# EUROPEAN PATENT SPECIFICATION

(11) **EP 2 809 150 B1**
(45) Date of publication and mention of the grant of the patent: **18.09.2019**
(21) Application number: 13704329.5
(22) Date of filing: 01.02.2013
(51) Int. Cl.: A01K 67/027, C07K 16/46, C12N 9/64

(54) **HUMANIZED MICE THAT EXPRESS HEAVY CHAINS CONTAINING VL DOMAINS**
SCHWERE KETTEN MIT VL-DOMÄNEN EXPRIMIERENDE HUMANISIERTE MÄUSE
SOURIS HUMANISÉS QUI EXPRIMENT DES CHAÎNES LOURDES CONTENANT DES DOMAINES VL

(30) Priority: 01.02.2012 US 201261593463 P; 31.07.2012 US 201261677538 P
(43) Date of publication of application: 10.12.2014
(60) Divisional application: 19191081.9
(73) Proprietor: Regeneron Pharmaceuticals, Inc., Tarrytown, NY 10591 (US)
(72) Inventor: MACDONALD, Lynn, White Plains, NY 10605 (US); GURER, Cagan, Valhalla, NY 10595 (US); MEAGHER, Karolina, A., Tarrytown, NY 10591 (US); STEVENS, Sean, San Francisco, CA 94158 (US); MURPHY, Andrew, J., Corton-on-Hudson, NY 10520 (US)
(74) Representative: J A Kemp
(86) International application number: PCT/US2013/024295
(87) International publication number: WO 2013/116609

(56) References cited:
- WO-A1-2011/158009
- WO-A1-2012/018764
- WO-A1-2012/141798
- WO-A2-2009/143472
- US-A1- 2012 167 237
- CECIL HAN ET AL: "Comprehensive analysis of reproductive ADAMs: relationship of ADAM4 and ADAM6 with an ADAM complex required for fertilization in mice", BIOLOGY OF REPRODUCTION, NEW YORK, NY [U.A.] : ACADEM. PRESS, US, vol. 80, no. 5, 7 January 2009 (2009-01-07), pages 1001-1008, XP002677427, ISSN: 0006-3363, DOI: 10.1095/BIOLREPROD.108.073700 [retrieved on 2009-01-07]
- KAREN FEATHERSTONE ET AL: "The Mouse Immunoglobulin Heavy Chain V-D Intergenic Sequence Contains Insulators That May Regulate Ordered V(D)J Recombination", JOURNAL OF BIOLOGICAL CHEMISTRY, AMERICAN SOCIETY FOR BIOCHEMISTRY AND MOLECULAR BIOLOGY, US, vol. 285, no. 13, 26 March 2010 (2010-03-26), pages 9327-9338, XP002677428, ISSN: 0021-9258, DOI: 10.1074/JBC.M109.098251 [retrieved on 2010-01-25]

### SEQUENCE LISTING

<110> Regeneron Pharmaceuticals, Inc.
<120> Humanized Rodents That Express Heavy Chains Containing VL Domains
<130> 7350A-WO
<140> To be assigned
   <141> Filed herewith
<150> 61/593,463
   <151> 2012-02-01
<150> 61/677,538
   <151> 2012-07-31
<160> 5
<170> FastSEQ for Windows Version 4.0
<210> 1
   <211> 754
   <212> PRT
   <213> Mus musculus
<400> 1
   Met Leu Ser Leu Thr Trp Gly Met Arg Leu Val Glu Arg Pro Val Val
    1               5                  10                  15
   Pro Arg Val Leu Leu Leu Leu Phe Ala Leu Trp Leu Leu Leu Leu Val
               20                  25                  30
   Pro Val Trp Cys Ser Gln Gly His Pro Thr Trp Arg Tyr Ile Ser Ser
           35                  40                  45
   Glu Val Val Ile Pro Arg Lys Glu Ile Tyr His Thr Lys Gly Leu Gln
       50                  55                  60
   Ala Gln Arg Leu Leu Ser Tyr Ser Leu Arg Phe Arg Gly Gln Arg His
   65                  70                  75                  80
   Ile Ile His Leu Arg Arg Lys Thr Leu Ile Trp Pro Arg His Leu Leu
                   85                  90                  95
   Leu Thr Thr Gln Asp Asp Gln Gly Ala Leu Gln Met Glu Tyr Pro Phe
               100                 105                 110
   Phe Pro Val Asp Cys Tyr Tyr Ile Gly Tyr Leu Glu Gly Ile Leu Gln
           115                 120                 125
   Ser Met Val Thr Val Asp Thr Cys Tyr Gly Gly Leu Ser Gly Val Ile
       130                 135                 140
   Lys Leu Asp Asn Leu Thr Tyr Glu Ile Lys Pro Leu Asn Asp Ser Gln
   145                 150                 155                 160
   Ser Phe Glu His Leu Val Ser Gln Ile Val Ser Glu Ser Asp Asp Thr
                   165                 170                 175
   Gly Pro Met Asn Ala Trp Lys His Trp Ser His Asn Thr Gly Ser Pro
               180                 185                 190
   Ser Ser Arg Leu Glu Tyr Ala Asp Gly Ala Pro Arg Leu Ser Ser Lys
           195                 200                 205
   Asn Tyr Ala Thr His Pro Ala Ala Ile Lys Gly His Phe Gln Ala Thr
       210                 215                 220
   His Ser Val Tyr Ser Ala Ser Gly Gly Asp Lys Leu Ser Ser Thr Val
   225                 230                 235                 240
   Glu Tyr Leu Phe Lys Val Ile Ser Leu Met Asp Thr Tyr Leu Thr Asn
                   245                 250                 255
   Leu His Met Arg Tyr Tyr Val Phe Leu Met Thr Val Tyr Thr Glu Ala
               260                 265                 270
   Asp Pro Phe Ser Gln Asp Phe Arg Val Pro Gly Gly Gln Ala His Thr
   275 280 285
   Phe Tyr Glu Arg Val Phe Tyr Ala His Phe Arg Pro Asp Ala Gly Ala
   290 295 300
   Ile Ile Asn Lys Asn Ser Pro Gly Asp Asp Ala Val Asn Pro Ala Glu
   305 310 315 320
   Arg Ser Ile Cys Ser Pro Ser Ala Leu Ile Cys Leu Gly Gln His Gly
   325 330 335
   Arg Asn Pro Leu Phe Leu Ser Ile Ile Ile Thr Asn Arg Val Gly Arg
   340 345 350
   Ser Leu Gly Leu Lys His Asp Glu Gly Tyr Cys Ile Cys Gln Arg Arg
   355 360 365
   Asn Thr Cys Ile Met Phe Lys Asn Pro Gln Leu Thr Asp Ala Phe Ser
   370 375 380
   Asn Cys Ser Leu Ala Glu Ile Ser Asn Ile Leu Asn Thr Pro Asp Leu
   385 390 395 400
   Met Pro Cys Leu Phe Tyr Asp Arg His Val Tyr Tyr Asn Thr Ser Leu
   405 410 415
   Thr Tyr Lys Phe Cys Gly Asn Phe Lys Val Asp Asn Asn Glu Gln Cys
   420 425 430
   Asp Cys Gly Ser Gln Lys Ala Cys Tyr Ser Asp Pro Cys Cys Gly Asn
   435 440 445
   Asp Cys Arg Leu Thr Pro Gly Ser Ile Cys Asp Lys Glu Leu Cys Cys
   450 455 460
   Ala Asn Cys Thr Tyr Ser Pro Ser Gly Thr Leu Cys Arg Pro Ile Gln
   465 470 475 480
   Asn Ile Cys Asp Leu Pro Glu Tyr Cys Ser Gly Ser Lys Phe Ile Cys
   485 490 495
   Pro Asp Asp Thr Tyr Leu Gln Asp Gly Thr Pro Cys Ser Glu Glu Gly
   500 505 510
   Tyr Cys Tyr Lys Gly Asn Cys Thr Asp Arg Asn Ile Gln Cys Met Glu
   515 520 525
   Ile Phe Gly Val Ser Ala Lys Asn Ala Asn Ile Lys Cys Tyr Asp Ile
   530 535 540
   Asn Lys Gln Arg Phe Arg Phe Gly His Cys Thr Arg Ala Glu Glu Ser
   545 550 555 560
   Leu Thr Phe Asn Ala Cys Ala Asp Gln Asp Lys Leu Cys Gly Arg Leu
   565 570 575
   Gln Cys Thr Asn Val Thr Asn Leu Pro Phe Leu Gln Glu His Val Ser
   580 585 590
   Phe His Gln Ser Val Ile Ser Gly Val Thr Cys Phe Gly Leu Asp Glu
   595 600 605
   His Arg Gly Thr Glu Thr Ala Asp Ala Gly Leu Val Arg His Gly Thr
   610 615 620
   Pro Cys Ser Arg Gly Lys Phe Cys Asp Arg Gly Ala Cys Asn Gly Ser
   625 630 635 640
   Leu Ser Arg Leu Gly Tyr Asp Cys Thr Pro Glu Lys Cys Asn Phe Arg
   645 650 655
   Gly Val Cys Asn Asn Arg Arg Asn Cys His Cys His Phe Gly Trp Ser
   660 665 670
   Pro Pro Lys Cys Lys Glu Glu Gly His Ser Gly Ser Ile Asp Ser Gly
   675 680 685
   Ser Pro Pro Val Gln Arg Arg Ile Ile Lys Gln Asn Leu Glu Pro Val
   690 695 700
   Val Tyr Leu Arg Ile Leu Phe Gly Arg Ile Tyr Phe Leu Phe Val Ala
   705 710 715 720
   Leu Leu Phe Gly Ile Ala Thr Arg Val Gly Val Thr Lys Ile Phe Arg
   725 730 735
   Phe Glu Asp Leu Gln Ala Ala Leu Arg Ser Trp Gln Glu Gln Ala Lys
   740 745 750
   Asp Lys
<210> 2
   <211> 756
   <212> PRT
   <213> Mus musculus
<400> 2
   Met Leu Ser Leu Thr Trp Gly Met Arg Leu Val Glu Arg Pro Val Val
    1               5                  10                  15
   Pro Arg Val Leu Leu Leu Leu Phe Ala Leu Trp Leu Leu Leu Leu Val
               20                  25                  30
   Pro Val Trp Cys Ser Gln Gly His Pro Thr Trp Arg Tyr Ile Ser Ser
           35                  40                  45
   Glu Val Val Ile Pro Arg Lys Glu Ile Tyr His Thr Lys Gly Leu Gln
       50                  55                  60
   Ala Gln Arg Leu Leu Ser Tyr Ser Leu His Phe Arg Gly Gln Arg His
   65                  70                  75                  80
   Ile Ile His Leu Arg Arg Lys Thr Leu Ile Trp Pro Arg His Leu Leu
                   85                  90                  95
   Leu Thr Thr Gln Asp Asp Gln Gly Ala Leu Gln Met Asp Tyr Pro Phe
               100                 105                 110
   Phe Pro Val Asp Cys Tyr Tyr Ile Gly Tyr Leu Glu Gly Ile Pro Gln
           115                 120                 125
   Ser Met Val Thr Val Asp Thr Cys Tyr Gly Gly Leu Ser Gly Val Met
       130                 135                 140
   Lys Leu Asp Asp Leu Thr Tyr Glu Ile Lys Pro Leu Asn Asp Ser Gln
   145                 150                 155                 160
   Ser Phe Glu His Leu Val Ser Gln Ile Val Ser Glu Ser Asp Asp Thr
                   165                 170                 175
   Gly Pro Met Asn Ala Trp Lys His Trp Ser His Asn Thr Gly Ser Pro
               180                 185                 190
   Ser Ser Arg Leu Glu Tyr Ala Asp Gly Ala Pro Arg Ile Ser Ser Lys
           195                 200                 205
   Asn Tyr Ala Thr His Pro Ala Ala Ile Lys Gly His Phe Gln Ala Thr
       210                 215                 220
   Asn Ser Val Tyr Asn Ser Ala Ala Gly Asp Lys Leu Ser Ser Thr Val
   225                 230                 235                 240
   Gly Tyr Leu Phe Gln Val Ile Ser Leu Met Asp Thr Tyr Leu Thr Asn
                   245                 250                 255
   Leu His Met Arg Tyr Tyr Val Phe Leu Met Thr Val Tyr Thr Asn Ser
               260                 265                 270
   Asp Pro Phe Arg Leu Glu Phe Ala Val Pro Gly Gly Ser Ala Tyr Asn
           275                 280                 285
   Tyr Tyr Val Ser Val Phe Tyr Asn Lys Phe Lys Pro Asp Ala Gly Val
       290                 295                 300
   Leu Leu Asn Lys Tyr Gly Pro Gln Asp Asn Gln Val Asn Pro Ala Glu
   305                 310                 315                 320
   Arg Ser Ile Cys Ser Ser Leu Ala Leu Ile Cys Ile Gly Lys Tyr Asp
                   325                 330                 335
   Arg Asn Pro Leu Phe Leu Ser Pro Ile Ile Thr Asn Arg Val Gly Arg
               340                 345                 350
   Ser Leu Gly Leu Lys Tyr Asp Glu Gly Tyr Cys Val Cys Gln Arg Arg
           355                 360                 365
   Asn Thr Cys Ile Met Phe Arg His Pro Gln Leu Thr Asp Ala Phe Ser
       370                 375                 380
   Asn Cys Ser Leu Ala Glu Ile Ser Asn Ile Leu Asn Thr Pro Gly Leu
   385                 390                 395                 400
   Met Pro Cys Leu Phe Tyr Asp Arg His Val Tyr Tyr Asn Thr Ser Leu
                   405                 410                 415
   Thr Tyr Lys Phe Cys Gly Asn Phe Lys Val Asp Asn Asp Glu Gln Cys
               420                 425                 430
   Asp Cys Gly Ser Gln Lys Ala Cys Tyr Ser Asp Pro Cys Cys Gly Asn
           435                 440                 445
   Asp Cys Arg Leu Thr Pro Gly Ser Ile Cys Asp Lys Glu Leu Cys Cys
       450                 455                 460
   Ala Asn Cys Thr Tyr Ser Pro Ser Gly Thr Leu Cys Arg Pro Ile Gln
   465                 470                 475                 480
   Asn Ile Cys Asp Leu Pro Glu Tyr Cys Asn Gly Thr Lys Tyr Ile Cys
                   485                 490                 495
   Pro Asp Asp Thr Tyr Leu Gln Asp Gly Thr Pro Cys Ser Glu Asp Gly
               500                 505                 510
   Tyr Cys Tyr Lys Gly Asn Cys Thr Asp Arg Asn Ile Gln Cys Met Glu
           515                 520                 525
   Ile Phe Gly Val Ser Ala Lys Asn Ala Asn Ile Lys Cys Tyr Asp Ile
       530                 535                 540
   Asn Lys Gln Arg Phe Arg Phe Gly His Cys Thr Arg Ala Glu Glu Ser
   545                 550                 555                 560
   Leu Thr Phe Asn Ala Cys Ala Asp Gln Asp Lys Leu Cys Gly Arg Leu
                   565                 570                 575
   Gln Cys Thr Asn Val Thr Asn Leu Pro Tyr Leu Gln Glu His Val Ser
               580                 585                 590
   Phe His Gln Ser Ile Ile Ser Gly Phe Thr Cys Phe Gly Leu Asp Glu
           595                 600                 605
   His Arg Gly Thr Glu Thr Thr Asp Ala Gly Met Val Arg His Gly Thr
       610                 615                 620
   Pro Cys Ser Lys Ser Lys Phe Cys Asp Gln Gly Ala Cys Ser Gly Ser
   625                 630                 635                 640
   Leu Ser His Leu Gly Tyr Asp Cys Thr Pro Glu Lys Cys Ser Phe Arg
                   645                 650                 655
   Gly Val Cys Asn Asn His Arg Asn Cys His Cys His Phe Gly Trp Lys
               660                 665                 670
   Pro Pro Glu Cys Lys Glu Glu Gly Leu Ser Gly Ser Ile Asp Ser Gly
           675                 680                 685
   Ser Pro Pro Val Gln Arg His Thr Ile Lys Gln Lys Gln Glu Pro Val
       690                 695                 700
   Val Tyr Leu Arg Ile Leu Phe Gly Arg Ile Tyr Phe Leu Phe Val Ala
   705                 710                 715                 720
   Leu Leu Phe Gly Ile Ala Thr Arg Val Gly Val Thr Lys Ile Phe Arg
                   725                 730                 735
   Phe Glu Asp Leu Gln Ala Thr Leu Arg Ser Gly Gln Gly Pro Ala Arg
               740                 745                 750
   Asp Lys Pro Lys
           755
<210> 3
   <211> 20468
   <212> DNA
   <213> Artificial Sequence
<220>
   <223> synthetic
<400> 3
   taactataac ggtcctaagg tagcgaggga tgacagattc tctgttcagt gcactcaggg 60
   tctgcctcca cgagaatcac catgtccttt ctcaagactg tgttctgtgc agtgccctgt 120
   cagtggaaat ctggagagca tgcttccatg agcttgtgag tagtatatct agtaagccat 180
   ggctttgtgt taatggtgat gttctacata tcagttctct ggcttaataa tgaggtgatg 240
   attctatgtt cctgtaacgc ttcctcaact gggtcctaag tctttcttca ctccatctat 300
   tcctctaagg aatgatcctg aaaatcccat cacaaactat aggagatggg aaccatcaaa 360
   aaacacagtg acaaagaggt gggaacgcat cagggttcag gaaccatatt ttaaaaagat 420
   atcgtaaata acttcttaaa agagatatag acaaatctcc attaatacgg agaccagagg 480
   cctaaggcta agaaccaatg gtggctcaag gtctcctgct acccgaggag caaacgtaga 540
   gcagtttcta atgatttatt taaaatatag aatcaaaagt accagtttgc aattttgaaa 600
   gatttatttc agcaatgcaa caacatcagg tggtgccgag tccaacacgt cttatgtccc 660
   atgatataaa caaaggccat ccagaactgt ggactggagt tctaccttgt cccctaatga 720
   cattcagatt ttttttccat tctctttatc ttagaggaga cagggggcta actcatttta 780
   cttgtccttt gcttgttctt gccaagaacg taaagcagct tgcaagtctt caaacctaaa 840
   tatcttagta actcctacac gagtggcaat gccaaagagc agtgcaacaa agaggaagta 900
   aatacgacca aagagtattc ttaaatacac tactggctct aggttctgtt ttattatgcg 960
   cctttgaacc ggaggggacc cactgtctat gctcccactg tgtccctctt ctttgcactt 1020
   tggagggctc caaccaaaat ggcaatggca attccgacga ttgttacaca ctcctctgaa 1080
   attgcatttt tctggggtgc agtcataacc caaacgagat aaacttccat tgcaagctcc 1140
   tcgatcacag aacttacccc ttgaacacgg ggtaccatgt ctcaccaatc cagcatctgc 1200
   tgtttctgtc ccacgatgtt catcaagccc aaagcaggta accccagaga taaccgattg 1260
   atggaatgaa acatgttctt gcaaaaatgg aagattggtg acattggtac actgcaacct 1320
   tccacacagc ttgtcctgat cagcacaagc attgaatgtg aggctttctt ctgctctagt 1380
   acaatgccca aatcgaaacc gttgtttgtt gatgtcatag cacttaatat tagcattctt 1440
   agcacttaca ccaaagattt ccatgcattg tatgttgcga tcagtgcagt tacctttata 1500
   gcagtaaccc tcttctgagc atggtgtccc atcttgcaga taagtgtcat ctgggcaaat 1560
   gaacttagag ccactacagt actctggaag atcacatatg ttctggatag gtctgcagag 1620
   tgtcccagaa ggactgtaag tgcaatttgc acagcataat tctttatcac aaatgctacc 1680
   aggtgttaac ctgcaatcat ttccacagca gggatctgaa taacatgcct tttgggagcc 1740
   acagtcacac tgctcattgt tatctacttt gaagtttcca caaaacttat aagtcaatga 1800
   tgtattataa taaacatgac ggtcatagaa aagacatggc atcagatcag gagtattaag 1860
   tatgttgctt atctctgcaa gggaacaatt gctgaaagca tctgttaatt gaggattttt 1920
   gaacatgatg caggtgttcc ttctctggca gatacagtac ccctcatcat gttttaggcc 1980
   taaactcctt ccaacacgat tggttattat aatagataaa aataaaggat ttcgaccatg 2040
   ttgaccaaga caaattaggg ctgagggaga acatatactc ctctcagctg gattaacagc 2100
   atcatctcct ggcgaattct tgttaattat agctcctgca tcaggcctaa aatgagcata 2160
   aaatactctc tcatagaaag tatgagcctg ccctcctgga actcgaaaat cttgtgaaaa 2220
   tggatcagcc tcggtataca cagtcatgag aaagacatag taccgcatat gaagattggt 2280
   cagataggtg tccattaaac taatgacttt aaacaaatac tcaacagtag atgaaagttt 2340
   gtcacctcca gaagcactat atacagaatg ggttgcttga aagtggcctt ttatagcagc 2400
   tggatgtgta gcgtaattct tactagatag tctgggagct ccatctgcat attccaatct 2460
   ggaggaggga gaacctgtat tatggctcca gtgcttccat gcattcatag gccctgtgtc 2520
   atcagactca gatactatct gagaaacaag gtgttcaaag ctctgtgaat cattgagggg 2580
   tttgatttca taggtaaggt tatccaactt tatgacccct gacaggcccc cataacaagt 2640
   atccacagtg accatggatt gcaggatccc ctccaggtag ccaatatagt aacaatctac 2700
   aggaaaaaag gggtactcca tctgtaaggc tccttggtca tcttgagttg tcagcaacaa 2760
   gtgtctgggc caaatgagtg tctttctccg caggtggatg atatgtctct ggccccgaaa 2820
   acgcaagcta tacgagagca gtctttgtgc ttgaagtcct ttggtatggt agatctcctt 2880
   ccgaggaata accacctccg atgagatgta acgccaagtg ggatggcctt gagaacacca 2940
   gactggaacc aggaggagca gccagagtgc aaatagcaag aggaggaccc tggggaccac 3000
   aggtctttcc actagcctca tgccccaggt cagagataac atcctgggtg gagctaactc 3060
   cctctgctgt ggccactgcc tggtctagaa aatactgaca gaggactaaa aacctcctca 3120
   ggctcccaac ctaagtggtt acccagacaa ctggagttag gtaacagtca ctgggtgtgg 3180
   caggaattga gtctgaatgt gttagctgag gttgaggtta aatattgtca aaagggatgt 3240
   ctataaatgt gcctggacaa gaaaagtcag aagcagcaag gagtgtctct gacaggctca 3300
   atcctttctt ttcttttttt gaagttcaaa atatcatttc cacgtgaatg tatttggttc 3360
   ccagtgtgac tctgggtctc tttctaggag tcaatatttc tttatatctt ggctcatgtt 3420
   tttcacagtt gttctaactt cttgttttgt tttgtttgtt tgtttgtttg aaagttagaa 3480
   gtaaatactg tctatattag ccttttagct ataaatgatt gtttttattt cttctaatca 3540
   tgttttgttt gagttttggt taaactattt acaaatgagt tttttttttc cttttgggtg 3600
   ttgctcgaaa gtttggagct ttctgttaat attgtgttgt tgtttctcca atattattag 3660
   acctgagaat tctacctggg tacctgtgaa ctccagaatt tttaaaaatt ccatctcttg 3720
   ggaacattat ctctgacccc gtctgaggcc gaagtggctg tccccctcca acctttagta 3780
   tctttctttc ctgactattg ggatttcttc aagcaatcag gctgatgggt tctcagcagt 3840
   gagaccagta gactgtcggt atgaacgtcg aagagtctgc cacacactcc gggttcatca 3900
   acagtgcttt cgcgtctctt acttttgtag aaggaaatgc agcctctgag ttttctccaa 3960
   gaaatcattg atgaaagggt gaaaagatgg gtatcacccg gagttcatga caagccctgg 4020
   ctcagacacg tgagcaaggt ctacagcccc aaagataggc tgccctgcaa catgtattta 4080
   taagatagga gaaaaaaatg ggtagttgga gggttgatca acttacttcc tctcaaacat 4140
   atatatctca tctaagtgtg caggggaaaa ctctgtagaa ctactgggat acctgctcac 4200
   ccccaggagc ctcatgaata agtctctgct tctgccttgt agccatgagc attactgcac 4260
   ctgatacccc tgcagcttcc tagggaagag ggaggaagtg acttggcccc tgtctggtta 4320
   aggtaagagg agataaatcc cttctcattg attagggtga gaggggtcat gtgctctatc 4380
   attggtgacc cagttgggac atgggtttat accaaagtca tcactctgag gttctgtgta 4440
   ccaccaggct gaactcccat atcctacatg gacataggac aacaccaagc agaaggaggt 4500
   tttaggacta aactgaagga cagagatgcg gtttctaaac aactagggag tgccagggcc 4560
   agcctctcta accactatag gacactgtgg agtctggtta caaagagaga ttactcaagg 4620
   tccttagcac tgattacaga gcatatctca gatgccttct gctgaccaga tgtatctttg 4680
   cataatctgc ctatccagat tcagaaaatt gatgccacat agccaagtgg actttcagga 4740
   acagacgatt taaaaacagg cagagagatg tgagagaaag gagaaggaga gagagaaggg 4800
   agagggagag aagagagagg gagacggaga aggaaagagg gagaaggaga aggagagaag 4860
   gggcatggac agagggaggg acagaaggag agaggagata gagaggggga taaggaagaa 4920
   gggagggagg gagagagaga gaaggctaag tctttccata cctgggtccc aatacctctt 4980
   ataacccaag cacatggttt cacatatcac aatgcggttg ggatatagat aactgtaaat 5040
   acttgtgaaa ataatggggc tgagatctgg ggttttcatg atagtttcaa agtcaccgta 5100
   ctgactaaaa ccttccactg gcccatctcc agcttcctaa tctgagggta tcaaatttcc 5160
   cactaagtgt gtttagaaag atctccacct ttttgccctt gtcttccagt gccccaccta 5220
   cgttctggtc tcccacatct gatgtcttct cagtgattct ggccctgcct gctccacagc 5280
   tacaaacccc ttcctataat gagctctgtg ctgagccatc atcctgaatc aatccacctt 5340
   aagcagatgt tttgcttatt tttcctgtgt ccatactaca gaggaaaggt aggcatgtag 5400
   aagctgaagc atctcacctc attccaagca ccctcagtct ctaaatgtgc ccccttgttt 5460
   ccagaagtgc aacctcaagc atcttttatt cattcatctt agagggccac atgtgctgta 5520
   gtgttataag atgaaattta aagcattaat tattcctaac aagccaatta aacaagccaa 5580
   aaacattcat cagtcattcc catggaacct ctgaagcatc ttcctgctct aaccttgggt 5640
   tttccagggc tgctctggga tcacaggagc tgtcctgtct accagccata taaaggcaga 5700
   cctatcagaa ttacaccaga cttctcacca tagactataa aagccagaat atcctggaca 5760
   gatgttatac agaaactaag agaacacaaa tgccagccca ggctactata cccagcaaaa 5820
   ctctcaatta ccatcgatga agaaaccaag atattccatt acaagtccaa atttacacaa 5880
   tatctttcca taaatccagc cctacaaagg atagcagatg gaaaactcca acacaggtag 5940
   gaaaactaca ccctagaaag agcactaaag taatcatctt tcaacacact caaaagaaga 6000
   taaccacaca aacataattc cacctctaac aacaaaaata aagtaggcaa caatcactat 6060
   tccttaatat ctcttttaac atcaatggac tcaattctcc aataaaaaga catagactaa 6120
   cagactgaat acataaacag gacacagcat tttgctgcat aaagcaaaca cagcgttact 6180
   tttttttttc taaatgacat tttttattag atattgtctt tattgacatt tcaaatgtta 6240
   tcccctttcc tggtttaccc tctgaaatcc cctatctcct ccccctcccc ctgctcacca 6300
   atccacccac tcccacttcc aggccctggc aatcccctat atttgggcat agagccttca 6360
   caggaccaag gtactctcct tgcattgatg accaactagt ccattctctg ctacaaatgc 6420
   agctagatct atgagtccca ccatgttttc ttttgttggt ggtttcatgc cagggagctc 6480
   ttggagtact gattggttca tattgttgtt ctccctatgg ggttacaaaa cccttcaact 6540
   tcttgggtcc tttctctggc tgcctcattg gggaccttgt gcgaagtcca atggatgact 6600
   gtgagcatcc acttctgtat ttgccaggca ctggcagagc ctctcagaag acagctatat 6660
   caagatcctg gcagcaagct cttgttggta tccacaaaag tgtctggtgg ttgtctatgg 6720
   gatggatccc caaaggggca gtctctggat ggtcattcct tcagtctctg ttccacactt 6780
   tgtctcttta actccttcca tgactatttt attcctccct ctaagaagga ccgaagtatt 6840
   catactttgg tcttccttct tgaaattcat gtgttttgtg aattgtatct ttgatattcc 6900
   gaacttctgg gctaatatcc acttatcagt gagtgaatat catgtgtgtt cttatgtgat 6960
   tgagttacct cactcaggat gatatcctcc agaaccatcc atttgtctaa gaatttaatg 7020
   aattcattgt ttttaatagc tgaggagtac tccattgtgt aaatgtacca cattttctgt 7080
   acccattgtt ctcttgaggg acatctgggt tctttaaagc ttctggacat taaatataag 7140
   gctgctatgg aaatagtgga gaatgtgtcc ttattacatg ttggagcatc ttctgggtat 7200
   atgcccagga gtgctattgc tggatcctct gatagtacta tgtccaattt tctgaggaac 7260
   tgccaaactg atttacagag tggttgtacc agcttgcaat tccaccagca atggagaaat 7320
   gttccccttc ctccacatcc tcaccaacat ctgctgtcac ctcaatttgt tcttagtgat 7380
   tcagacaggt gtgaggtgga atatcagggt tgtttggcat ttccctgatg actagtgata 7440
   ttgaaaaaaa ttttaagtgt ttctcagcca ttcagtattc ttcagttgag aattcactgt 7500
   ttagctctgt actcaggttt ttttaatagg gttatttggt tttctggagt ctaacgtctt 7560
   gaattctttc tatatattgg atattagccc tctgtcatat ttaggattgg taaagatctt 7620
   tcccaatatg ttggctgcct ttttgtgtcc tttgccttac agaacctttt taattttatg 7680
   aggtcccatt tgctaattct tcattttaca gcacaagcca ttggtgttct gttcaaaaat 7740
   ctttccccct gaaccctatc ttcgaggatc ttccccactt tctcctctat aagtttcagt 7800
   gtctctatta ttgtgctgag ggcgatcgca ccggtgaaac cctaggaagt tcctatactt 7860
   tctagagaat aggaacttcg gaataggaac ttctctaggg gctgcaggtc gaggtctgat 7920
   ggaattagaa cttggcaaaa caatactgag aatgaagtgt atgtggaaca gaggctgctg 7980
   atctcgttct tcaggctatg aaactgacac atttggaaac cacagtactt agaaccacaa 8040
   agtgggaatc aagagaaaaa caatgatccc acgagagatc tatagatcta tagatcatga 8100
   gtgggaggaa tgagctggcc cttaatttgg ttttgcttgt ttaaattatg atatccaact 8160
   atgaaacatt atcataaagc aatagtaaag agccttcagt aaagagcagg catttatcta 8220
   atcccacccc acccccaccc ccgtagctcc aatccttcca ttcaaaatgt aggtactctg 8280
   ttctcaccct tcttaacaaa gtatgacagg aaaaacttcc attttagtgg acatctttat 8340
   tgtttaatag atcatcaatt tctgcagact tacagcggat cttcagaaga actcgtcaag 8400
   aaggcgatag aaggcgatgc gctgcgaatc gggagcggcg ataccgtaaa gcacgaggaa 8460
   gcggtcagcc cattcgccgc caagctcttc agcaatatca cgggtagcca acgctatgtc 8520
   ctgatagcgg tccgccacac ccagccggcc acagtcgatg aatccagaaa agcggccatt 8580
   ttccaccatg atattcggca agcaggcatc gccatgggtc acgacgagat cctcgccgtc 8640
   gggcatgcgc gccttgagcc tggcgaacag ttcggctggc gcgagcccct gatgctcttc 8700
   gtccagatca tcctgatcga caagaccggc ttccatccga gtacgtgctc gctcgatgcg 8760
   atgtttcgct tggtggtcga atgggcaggt agccggatca agcgtatgca gccgccgcat 8820
   tgcatcagcc atgatggata ctttctcggc aggagcaagg tgagatgaca ggagatcctg 8880
   ccccggcact tcgcccaata gcagccagtc ccttcccgct tcagtgacaa cgtcgagcac 8940
   agctgcgcaa ggaacgcccg tcgtggccag ccacgatagc cgcgctgcct cgtcctgcag 9000
   ttcattcagg gcaccggaca ggtcggtctt gacaaaaaga accgggcgcc cctgcgctga 9060
   cagccggaac acggcggcat cagagcagcc gattgtctgt tgtgcccagt catagccgaa 9120
   tagcctctcc acccaagcgg ccggagaacc tgcgtgcaat ccatcttgtt caatcatggt 9180
   ggatggttta gttcctcacc ttgtcgtatt atactatgcc gatatactat gccgatgatt 9240
   aattgtcaac aattcgagag acccgggttc ccaatctaga gctcgacgtc taacaaaaaa 9300
   gccaaaaacg gccagaattt agcggacaat ttactagtct aacactgaaa attacatatt 9360
   gacccaaatg attacatttc aaaaggtgcc taaaaaactt cacaaaacac actcgccaac 9420
   cccgagcgca tagttcaaaa ccggagcttc agctacttaa gaagataggt acataaaacc 9480
   gaccaaagaa actgacgcct cacttatccc tcccctcacc agaggtccgg cgcctgtcga 9540
   ttcaggagag cctaccctag gcccgaaccc tgcgtcctgc gacggagaaa agcctaccgc 9600
   acacctaccg gcaggtggcc ccaccctgca ttataagcca acagaacggg tgacgtcacg 9660
   acacgacgag ggcgcgcgct cccaaaggta cgggtgcact gcccaacggc accgccataa 9720
   ctgccgcccc cgcaacagac gacaaaccga gttctccagt cagtgacaaa cttcacgtca 9780
   gggtccccag atggtgcccc agcccatctc acccgaataa gagctttccc gcattagcga 9840
   aggcctcaag accttgggtt cttgccgccc accatgcccc ccaccttgtt tcaacgacct 9900
   cacagcccgc ctcacaagcg tcttccattc aagactcggg aacagccgcc attttgctgc 9960
   gctcccccca acccccagtt cagggcaacc ttgctcgcgg acccagacta cagcccttgg 10020
   cggtctctcc acacgcttcc gtcccaccga gcggcccggc ggccacgaaa gccccggcca 10080
   gcccagcagc ccgctactca ccaagtgacg atcacagcga tccacaaaca agaaccgcga 10140
   cccaaatccc ggctgcgacg gaactagctg tgccacaccc ggcgcgtcct tatataatca 10200
   tcggcgttca ccgccccacg gagatccctc cgcagaatcg ccgagaaggg actacttttc 10260
   ctcgcctgtt ccgctctctg gaaagaaaac cagtgcccta gagtcaccca agtcccgtcc 10320
   taaaatgtcc ttctgctgat actggggttc taaggccgag tcttatgagc agcgggccgc 10380
   tgtcctgagc gtccgggcgg aaggatcagg acgctcgctg cgcccttcgt ctgacgtggc 10440
   agcgctcgcc gtgaggaggg gggcgcccgc gggaggcgcc aaaacccggc gcggaggcct 10500
   cggaggccag acgcgcgaga agttcctata ctttctagag aataggaact tcggaatagg 10560
   aacttcggta ccgcgatcgc gatgtgggaa cgcttcagtg ttcaggaacc atatgattta 10620
   tttaaaatat agaatcaaaa gtaccaattt gcagttttga aagatttatt ccagtgtaag 10680
   cattagcaat gcaccaacat caggtgattt ctgaatccaa cacgtcttat gtcctcatga 10740
   tattaaaaaa aaaaaaaggc catccagaac tgtgaacttg agttctacct tgttccctac 10800
   tgacattcag attttctttt ttgcattctc tttatcttac aggagacagg aggggagggc 10860
   taactcattt tactttggct tgtcccttgc tggtccttgc ccagaacgta aagtagcttg 10920
   caagtcttca aatctaaaaa tcttagtaac tcctacacga gtggcaatgc caaagagcag 10980
   tgcaacaaag aggaagtaaa tacgaccaaa gagtattctt aaatacacca ctggctcttg 11040
   tttttgtttt attgtgtgcc tttgaactgg aggggaccca ctgtctatgc tcccacttag 11100
   tccctcttct ttgcactctg gaggcttcca accaaaatga caatggcaat tccgatgatt 11160
   gttacacact cctctaaaac tgcatttttc tggggtgcag tcataaccca aatgagataa 11220
   acttccactg caagctcctt gatcacagaa cttacttttg gagcaggggg taccatgtct 11280
   caccattcca gcatctgttg tttctgtccc acgatgttca tcaagcccaa agcaggtaaa 11340
   cccagagata atcgattgat ggaatgaaac atgttcttgc aaatatggaa gattggtgac 11400
   attggtacac tgcaaccttc cacacagctt gtcctgatca gcacaagcat tgaatgtgag 11460
   gctttcttct gctctagtac aatgcccaaa tcgaaaccgt tgtttgttga tgtcatagca 11520
   cttaatatta gcattcttag cacttacacc aaagatttcc atgcattgta tgttgcgatc 11580
   agtgcagtta cctttatagc agtaaccatc ttctgagcat ggtgtcccat cttgcagata 11640
   agtgtcatct gggcaaatgt atttagtccc attacagtac tctggaagat cacatatgtt 11700
   ctggataggt ctgcagagtg tcccagaagg actgtaagtg caatttgcac agcataattc 11760
   tttatcacaa atgctaccag gtgttaacct gcaatcattt ccacagcagg gatctgaata 11820
   acatgccttt tgggagccac agtcacactg ctcatcgtta tctactttga agtttccaca 11880
   aaacttataa gtcaatgatg tattataata aacatgacgg tcatagaaaa gacatggcat 11940
   cagaccagga gtattaagta tgttgcttat ctctgcaagg gaacaattgc tgaaagcatc 12000
   tgttaattga ggatgtctga acataatgca ggtgttcctt ctctggcaga cacagtaccc 12060
   ctcatcatat tttaagccta aactccttcc aacacgattg gttattatag gagataaaaa 12120
   taaaggattt cgatcatatt taccaataca aattagggct aaggaagaac atatactcct 12180
   ctcagctgga ttaacctggt tatcttgtgg cccatactta ttaagtaaaa ctcctgcatc 12240
   aggcttaaat ttattataaa agactgacac atagtaatta taagccgacc ctcctggaac 12300
   tgcaaactca agtcgaaatg gatcagaatt ggtgtacaca gtcatgagaa agacatagta 12360
   ccgcatatga agattggtca gataggtgtc cattaaacta atgacttgaa acaaataccc 12420
   aacagtagat gaaagtttgt cacctgcagc agaattatat acagaattgg ttgcttgaaa 12480
   gtggcctttt atagcagctg gatgtgtagc gtagttctta ctagatattc tgggagctcc 12540
   atctgcatat tccaatctgg aggagggaga acctgtatta tggctccagt gcttccatgc 12600
   attcataggc cctgtgtcat cagactcaga tactatctga gaaacaaggt gttcaaagct 12660
   ctgtgaatca ttgaggggtt tgatttcata ggtaaggtca tctaacttca tgacccctga 12720
   caggccccca taacaagtat ccacagtgac catggattgt gggatcccct ccaggtagcc 12780
   aatatagtaa caatctacag gaaaaaaggg gtaatccatc tgtaaggctc cttggtcatc 12840
   ttgagttgtc agcaacaagt gtctgggcca aatgagtgtc tttctccgca ggtggatgat 12900
   atgtctctgg ccccgaaaat gcaagctata tgagagcagt ctttgtgctt gaagtccttt 12960
   ggtatggtag atctccttcc gaggaataac cacctccgat gagatgtaac gccaagtagg 13020
   atggccttga gaacaccaga ctggaaccag gaggagcagc cagagtgcaa atagcaagag 13080
   gaggaccctg gggaccacag gtctttccac tagcctcatg ccccaggtca gagataacat 13140
   cctgggtgga gctaaatccc tctgctgtgg ccactgcctg gtctagaaaa tactgacaga 13200
   ggactaaaaa cctcctcagg ctcccaacct aagtggttac ccagacaact ggagttaggt 13260
   aacagtcact gggtgtggca ggaattgagt ctgaatgtgt tagctgaggt tgaggttaaa 13320
   tattgtcaaa agggatgtct ataaatgtgc ctggacaaga aaagtcagaa gcagcaagga 13380
   gtgtctctga caggctcaat cctttctttt ctttttttga agttcaaaat atcatttcca 13440
   cgtgaatgta tttggttccc agtgtgactc tgggtctctt tctaggagtc aatatttctt 13500
   tatatcttgg ctcatgtttc tcacagttgt tctaatttct tgttttgttt tgtttgtttg 13560
   tttgaacgtt agtagtaaat actgtctata ttagcctttt agctataaat gattgttttt 13620
   atttcttcta atcatatttt gtttgagttt tggttaaact atttacaaat gagttttttt 13680
   tttttccttt tgggtgttgc tcgaaagttt ggagctttct gttaatattg tgttgttatt 13740
   tttccaatat tattagacct gagaattcta tctgggtacc tgtgaactct agaattttta 13800
   aaaattccat ctcttgggaa cattacctct gaccccgtct gaggccgaag tggctgtccc 13860
   cctccaacct ttagtatctt tctttcctga ctattgggat ttcttcaagc aatcaggctg 13920
   atgggttctc agcagtgaga ccagtagact gccggtatga acgtcgaaga gactgccaca 13980
   cactccaggt tcatcaacag tgctttcgcg tctcttactt ttgtagaagg aaaagcagcc 14040
   tctgagttat ctccaagaaa tcattaatga aagagttaaa agatgggtat cacccggagt 14100
   tcatgacaag ccctggctca gacacgtgag caaggtctac agccccaaag ataggctgcc 14160
   ctgcaacatg tatttataag atagaagaaa aaaatgggtg gttggagggt tgatcaactt 14220
   acttcctctc aaacatatat atctcatcta agtgtgcagg ggaaaactct gtaggactac 14280
   tgggattgtt attatcatta ttattattat tattattatt attattatta ttattattat 14340
   taacttaagg cattttatta gatattttct tcatttagtt ttcaaatgtt atccccggaa 14400
   cctcctatac tctctccctg ccctgctccc caacccaccc actcctacat cctggccctg 14460
   gcattcccct atactgtggc agatgatctt cgtaagacca agagcctttc ctcccattga 14520
   tggcctacta ggctatcctc ttttacatat gcaactagag tcacagctct ggggaggtat 14580
   tgcttagttc atattgtttt tcctcctata gggttgcaga tccctttagc tccttgggta 14640
   ctttctctag ctcctccatt gggggccctg tgttccatcc aatagatgac tgtgagcatc 14700
   cacttctgta tttgccaggt attggcatgg atcttactgc accttctgaa ctctctaagc 14760
   agctttcctg gtcacctcca ggagcctcat gaataagtct ctgcttcccc cttgtggcta 14820
   tgagcattac tgcacctgat acaccctgca gcttcctagg gaagagggag gaagtggctt 14880
   ggcccctgtc tggttaaggt aagaggagat aaatcccttc tcatgaatta gggtgagaag 14940
   ggtcatgtgc tctatcattg gtgaccaact tggggacatg ggcttataca gtcatcactc 15000
   tgaggctctg tgtaccacca gactgaactc ccatatccta catgcacata ggacaacacc 15060
   aagtagaagg aggttttagg actaaactga aggacagaga tggggtttct aaacaactag 15120
   ggagtgccag ggccagcctc tctaaccact ataggacact atggagtctg gttacaaaga 15180
   gagattactc aaggtcctta gcactgatta cagagcatat ctcagatgcc ttctgctgac 15240
   cagatgtatc tttgcataat ctgcctatcc agattcagaa aattgatgcc acatagccaa 15300
   gtggactttc aggaacagac gatttaaaaa caggcagaga gatgtgagag aaaggagaag 15360
   gagagagaga agggagaggg agagaagaga gagggagacg gagaaggaaa gagggagaag 15420
   gagaaggaga gaaggggcat ggacagaggg agggacagaa ggagagagga gatagagagg 15480
   gggataagga agaaaggagg gagggagaga gagagaaggc taagtctttc catacctggg 15540
   tcccaatacc tcttataacc caagcacatg gtttcagata tcacaatgcg gttgggatat 15600
   agataactgt aaatacttgt gaaaataatg gggctgagat ctggggtttt catgatagtt 15660
   tcaaagtcac tgtactgact aaaaccttcc actggcccat ctccagcttg ttaatctgag 15720
   ggtatcaaat ttcccactaa gtgtgtttag aaagatctcc acctttttgc cctagtcttc 15780
   cagtgcccca cctacgttct ggtctcccac atctgatgtc ttctcagtga ttctggccct 15840
   gcctgctcca cagctacaaa ccccttccta taatgagctc tgtgctgagc catcatcctg 15900
   aatcaatcca ccttaagcag atgttttgct tatttttcct gtgtccatac tacagaggaa 15960
   gggtaggcat gtagaagctg aggcatctca tctcactcta agcaccctca gtctctaaat 16020
   gtgccccttt gtttccagca gttcagcctc aagcatcttt tattcactcg tcttagaggg 16080
   acacatgtgc tgtagtgtta taagatgaaa tttaaagcat tagttattcc caacaagcca 16140
   attaaacaag ccaaaaacat tcatcagtca ttcccatgga acctctgaag catcttcctg 16200
   ctctaacctt gagtttccta gggctgctgt gggatcacag gagctgtcct gtttaccagc 16260
   ctatcctgtc ccacgggatt cagttattag tgggtgcgag ggggaccgca aacctggaag 16320
   aaaatgggat tggaagagaa aagagaaacg aagaccaagt agatcttttc ctatcaaggt 16380
   cttcgtttat taggctgagg tgcctggtgt aaagcatgca tcgcggggaa taggaagggg 16440
   tcgaggggga attttacaaa gaacaaagaa gcgggcatct gctgacatga gggccgaagt 16500
   caggctccag gcagcggaca ctctgcatct tatctctgga acatagatcc tccttgacag 16560
   ccttggggtg tcaggccagg ctcaggtgta actcatgtcc ttggatggca tgggagttca 16620
   ggaagagata gggaagaggg gactataatt cagcttttac agcctcaggt gccaggaagg 16680
   caacagggag gagggagtga ctacaggctc ctagcacgag gccatttggc ctgtcagggt 16740
   gggagattgt gaagggctca ctttctcatg gtatggtctc tgacaccagc cagaaaaaaa 16800
   aaaaatctcc agtcaatcta cagaaaggca gaaatatgga gaaccttcta gaagaacagc 16860
   aacccttgac tgactctgca gggcagtcta agcacacagg ttcctctgag cccagtccct 16920
   ggctgtattc actcacacag ggcccagcag tctggagatg cctgcagcag gaaggttctc 16980
   actgtacctc agtctcacag ctcagtcact ttgtagtctc cccacagcaa atcacaacca 17040
   atgcagttgt cacccttgag gccattcctc agtgacgcct gggaaacact gtccatttgc 17100
   cctgctcata tgctgtctca gctcacataa attccctcca agctccttta cagaaacaac 17160
   caattgacat acaaattaaa gtaatatgaa gctctgtcct gtaccaattc ctagaacacc 17220
   tcagctgcta aaatctaact gtgcaataca gagaactacc tagataatac ctaactccaa 17280
   tcttgaggta tctgttgaag aggctttcag ctatgaatta ccaagaggga tgcctgtgtc 17340
   tcctacatct taaggcatct ttctgaagct cacactaccc ttcacagcgc cacagcagca 17400
   gggagatctg tatctcttcc tctccacaag agggcagaat tcgggtgtca actgtgacaa 17460
   ccctccagca ttaatttcta ttagtagata ttttctttac ttacatttca aatgttatcc 17520
   ccttccccag tttttcctcc ctgaaacccc ctatcccatc acccatctcc ctgctcacca 17580
   acccacccat tccataagaa ccaatcagta ccccaccccc ccagagctcc cagggactaa 17640
   accaggaacc aaagagtaca catggaagga cccatagctc cagctgcata ggtagcacta 17700
   ctttgaaaag ggggaagatg atgaaatcat tactgtgggg gaaatgcaag gaagattcca 17760
   acacatctag gcatctatga agattttaag tcttcaaaat ccaaaaccac cacaaaattt 17820
   aaaaaaaaaa aaagtagatt ctaaatgcag tcacctgcac caggtgcctg gggaatcact 17880
   cagcagccct agactgagaa agcttggaga aagtagaaat agagaaagtg tacagccagt 17940
   atcctctagc tactcacatc caaacagggc ctcctgactg ctctgagcct gtcctaagaa 18000
   cagcaatgat gccacagaaa tttttagagt gagccctgaa ggaacttgag gctgatatga 18060
   gcaagccagt cccagaggaa aggaaaaccc atagagagaa aacaggtgag ttagtgcatt 18120
   aaaggggctg agcagggagt tctcatcgct ccccagcacc agaaataaga gcctctccgg 18180
   agctgctggg acatggaatg cagatgattc ggaccatcag ccccacagag acctttccca 18240
   ctctggctca gaaagaggca ctggaccaca gttggagagg agaatcgaaa gctgatatct 18300
   ctgtattcac ttagcctgtt acccacccat gcacccaagt ccaaggtggg agaaacactg 18360
   agggtctaaa cacagcccca gagcaactgc cagtattaaa taccagattt cagaagatgg 18420
   gaaatcacct ctctggtcat ttttgggaca tgtaaactgt aacaggaaac ataggaccaa 18480
   tttaagatgg agcagtccta tatccctaac ccagttgtaa ataaaacatt caagagtgcc 18540
   atcagacacc acagtggtac aggagagatg agtgtaccta gtgcatcaag agttccctca 18600
   ctagataaac caagatgtag ccccaggacc acccaggcac ctaccaggac tcccctccag 18660
   aggtctgagc cagttagctc tagttcattc tatgtttcag accaaaacat cagaaacaac 18720
   agcatctcca ctgcagatga acccctaagc catacagtgt acccaaaggc agcaccacag 18780
   atggggaatg tgggggcgaa gaagcttgta tcacattaag agtgttgccc aatgactatt 18840
   tgctatcttc acagtaagat gggcactgat cccacccacc aaaacatagt aggacaagga 18900
   cccttaaata acatctgtca aggggagctg tcaaatagcc actgagctga gatggctcat 18960
   acggggtgat atagaaaaac aggccaaaga acctcctgtg ttgcaagcac aaatgggaag 19020
   ctgtgaatct ccactacctt acaagaaaac tgtaccaatc tgcaagaaca ggggatacac 19080
   agctttccac actgtgtagg aaggcattgg actctcagat catccaagga ctagggctaa 19140
   agtggccatg tgtgggaaat acatccactt tataaaccta ccttgtatcc acatcacaca 19200
   tggagcatct gctcctggtc cctacctcat gtgggtgctg cggtgctgta cattgagtct 19260
   gggggcatga gtgtgcccgg taaattcctt atcactcaga tgaatttcca gtccacactc 19320
   atcaccttgg agtaggaatt ttaaaagtta gtgtagatat aagtaaagag agtcaggagg 19380
   gggggctgaa aggcagacct gcagaaattc tccagagggt agagctcaga agcagcaagc 19440
   aaaacttggg ctaacagaag agcaaacaca gagctcaggc agaactacct ggcaatgcga 19500
   ctgggcacac tgaaagcact gggcatcagc actgagcccc aaatatgcac tcaggatcct 19560
   ctgcataata atgtaacata acaggaaggt tagaacaggc caaaagagga aacagaacaa 19620
   atgcccctaa ccaaagaagt ataaacaaat tgggaagagt aaagaaggat tgtaaggatt 19680
   gagtaccaca cagaacatgc tcttaatggc ctcaatgctg aagctaggaa gaactaagtt 19740
   aaaagaaaca tgttcaacgg gattccctgt cactggactt cacaacaagc aaaattcaat 19800
   ctttctgtta aggagatgag aagagaatat ctgaaccttg tgttgacagt gccccacccc 19860
   gactgtcagg ctgtgggaaa tgccagagca atcactagga acacacaagg atgagggaga 19920
   cgagggttag gacacaacca tcatgatatc ccacaagtat ggaagagcaa gaacttgtag 19980
   agagcagaga atggcagaca aagcagcata tacataagta gatggccaga ctatacagga 20040
   aagatctaca tagcctgtga ggctttctga cagaaaaggg caggcatgtc tcaaagcaca 20100
   atgcctggct tgggacactg tctgccctgg atcctctcac tccacatgta aggagctcac 20160
   agcaaaacca cacagccttc cacaagagga gaagaaaagg tagcttgtca gtgaggaagt 20220
   cccccagaga cagaccattc cagtagttct tatcattcct cccaaagcag ccaccatcca 20280
   ggcactgaga gaccaaaggc tgttgggagg tcagcctaga ggcaggctca gacctttctt 20340
   gctccctagg accttcctga aaagatagaa gcacagaagt gaataatctg gacctcaact 20400
   caggatgaca actgaaactc aaccgtgctg cctgggcccc caatgctctc tacacctgca 20460
   ggcgcgcc                                                          20468
<210> 4
   <211> 67667
   <212> DNA
   <213> Artificial Sequence
<220>
   <223> synthetic
<400> 4
   taactataac ggtcctaagg tagcgaggga tgacagattc tctgttcagt gcactcaggg 60
   tctgcctcca cgagaatcac catgtccttt ctcaagactg tgttctgtgc agtgccctgt 120
   cagtggaaat ctggagagca tgcttccatg agcttgtgag tagtatatct agtaagccat 180
   ggctttgtgt taatggtgat gttctacata tcagttctct ggcttaataa tgaggtgatg 240
   attctatgtt cctgtaacgc ttcctcaact gggtcctaag tctttcttca ctccatctat 300
   tcctctaagg aatgatcctg aaaatcccat cacaaactat aggagatggg aaccatcaaa 360
   aaacacagtg acaaagaggt gggaacgcat cagggttcag gaaccatatt ttaaaaagat 420
   atcgtaaata acttcttaaa agagatatag acaaatctcc attaatacgg agaccagagg 480
   cctaaggcta agaaccaatg gtggctcaag gtctcctgct acccgaggag caaacgtaga 540
   gcagtttcta atgatttatt taaaatatag aatcaaaagt accagtttgc aattttgaaa 600
   gatttatttc agcaatgcaa caacatcagg tggtgccgag tccaacacgt cttatgtccc 660
   atgatataaa caaaggccat ccagaactgt ggactggagt tctaccttgt cccctaatga 720
   cattcagatt ttttttccat tctctttatc ttagaggaga cagggggcta actcatttta 780
   cttgtccttt gcttgttctt gccaagaacg taaagcagct tgcaagtctt caaacctaaa 840
   tatcttagta actcctacac gagtggcaat gccaaagagc agtgcaacaa agaggaagta 900
   aatacgacca aagagtattc ttaaatacac tactggctct aggttctgtt ttattatgcg 960
   cctttgaacc ggaggggacc cactgtctat gctcccactg tgtccctctt ctttgcactt 1020
   tggagggctc caaccaaaat ggcaatggca attccgacga ttgttacaca ctcctctgaa 1080
   attgcatttt tctggggtgc agtcataacc caaacgagat aaacttccat tgcaagctcc 1140
   tcgatcacag aacttacccc ttgaacacgg ggtaccatgt ctcaccaatc cagcatctgc 1200
   tgtttctgtc ccacgatgtt catcaagccc aaagcaggta accccagaga taaccgattg 1260
   atggaatgaa acatgttctt gcaaaaatgg aagattggtg acattggtac actgcaacct 1320
   tccacacagc ttgtcctgat cagcacaagc attgaatgtg aggctttctt ctgctctagt 1380
   acaatgccca aatcgaaacc gttgtttgtt gatgtcatag cacttaatat tagcattctt 1440
   agcacttaca ccaaagattt ccatgcattg tatgttgcga tcagtgcagt tacctttata 1500
   gcagtaaccc tcttctgagc atggtgtccc atcttgcaga taagtgtcat ctgggcaaat 1560
   gaacttagag ccactacagt actctggaag atcacatatg ttctggatag gtctgcagag 1620
   tgtcccagaa ggactgtaag tgcaatttgc acagcataat tctttatcac aaatgctacc 1680
   aggtgttaac ctgcaatcat ttccacagca gggatctgaa taacatgcct tttgggagcc 1740
   acagtcacac tgctcattgt tatctacttt gaagtttcca caaaacttat aagtcaatga 1800
   tgtattataa taaacatgac ggtcatagaa aagacatggc atcagatcag gagtattaag 1860
   tatgttgctt atctctgcaa gggaacaatt gctgaaagca tctgttaatt gaggattttt 1920
   gaacatgatg caggtgttcc ttctctggca gatacagtac ccctcatcat gttttaggcc 1980
   taaactcctt ccaacacgat tggttattat aatagataaa aataaaggat ttcgaccatg 2040
   ttgaccaaga caaattaggg ctgagggaga acatatactc ctctcagctg gattaacagc 2100
   atcatctcct ggcgaattct tgttaattat agctcctgca tcaggcctaa aatgagcata 2160
   aaatactctc tcatagaaag tatgagcctg ccctcctgga actcgaaaat cttgtgaaaa 2220
   tggatcagcc tcggtataca cagtcatgag aaagacatag taccgcatat gaagattggt 2280
   cagataggtg tccattaaac taatgacttt aaacaaatac tcaacagtag atgaaagttt 2340
   gtcacctcca gaagcactat atacagaatg ggttgcttga aagtggcctt ttatagcagc 2400
   tggatgtgta gcgtaattct tactagatag tctgggagct ccatctgcat attccaatct 2460
   ggaggaggga gaacctgtat tatggctcca gtgcttccat gcattcatag gccctgtgtc 2520
   atcagactca gatactatct gagaaacaag gtgttcaaag ctctgtgaat cattgagggg 2580
   tttgatttca taggtaaggt tatccaactt tatgacccct gacaggcccc cataacaagt 2640
   atccacagtg accatggatt gcaggatccc ctccaggtag ccaatatagt aacaatctac 2700
   aggaaaaaag gggtactcca tctgtaaggc tccttggtca tcttgagttg tcagcaacaa 2760
   gtgtctgggc caaatgagtg tctttctccg caggtggatg atatgtctct ggccccgaaa 2820
   acgcaagcta tacgagagca gtctttgtgc ttgaagtcct ttggtatggt agatctcctt 2880
   ccgaggaata accacctccg atgagatgta acgccaagtg ggatggcctt gagaacacca 2940
   gactggaacc aggaggagca gccagagtgc aaatagcaag aggaggaccc tggggaccac 3000
   aggtctttcc actagcctca tgccccaggt cagagataac atcctgggtg gagctaactc 3060
   cctctgctgt ggccactgcc tggtctagaa aatactgaca gaggactaaa aacctcctca 3120
   ggctcccaac ctaagtggtt acccagacaa ctggagttag gtaacagtca ctgggtgtgg 3180
   caggaattga gtctgaatgt gttagctgag gttgaggtta aatattgtca aaagggatgt 3240
   ctataaatgt gcctggacaa gaaaagtcag aagcagcaag gagtgtctct gacaggctca 3300
   atcctttctt ttcttttttt gaagttcaaa atatcatttc cacgtgaatg tatttggttc 3360
   ccagtgtgac tctgggtctc tttctaggag tcaatatttc tttatatctt ggctcatgtt 3420
   tttcacagtt gttctaactt cttgttttgt tttgtttgtt tgtttgtttg aaagttagaa 3480
   gtaaatactg tctatattag ccttttagct ataaatgatt gtttttattt cttctaatca 3540
   tgttttgttt gagttttggt taaactattt acaaatgagt tttttttttc cttttgggtg 3600
   ttgctcgaaa gtttggagct ttctgttaat attgtgttgt tgtttctcca atattattag 3660
   acctgagaat tctacctggg tacctgtgaa ctccagaatt tttaaaaatt ccatctcttg 3720
   ggaacattat ctctgacccc gtctgaggcc gaagtggctg tccccctcca acctttagta 3780
   tctttctttc ctgactattg ggatttcttc aagcaatcag gctgatgggt tctcagcagt 3840
   gagaccagta gactgtcggt atgaacgtcg aagagtctgc cacacactcc gggttcatca 3900
   acagtgcttt cgcgtctctt acttttgtag aaggaaatgc agcctctgag ttttctccaa 3960
   gaaatcattg atgaaagggt gaaaagatgg gtatcacccg gagttcatga caagccctgg 4020
   ctcagacacg tgagcaaggt ctacagcccc aaagataggc tgccctgcaa catgtattta 4080
   taagatagga gaaaaaaatg ggtagttgga gggttgatca acttacttcc tctcaaacat 4140
   atatatctca tctaagtgtg caggggaaaa ctctgtagaa ctactgggat acctgctcac 4200
   ccccaggagc ctcatgaata agtctctgct tctgccttgt agccatgagc attactgcac 4260
   ctgatacccc tgcagcttcc tagggaagag ggaggaagtg acttggcccc tgtctggtta 4320
   aggtaagagg agataaatcc cttctcattg attagggtga gaggggtcat gtgctctatc 4380
   attggtgacc cagttgggac atgggtttat accaaagtca tcactctgag gttctgtgta 4440
   ccaccaggct gaactcccat atcctacatg gacataggac aacaccaagc agaaggaggt 4500
   tttaggacta aactgaagga cagagatgcg gtttctaaac aactagggag tgccagggcc 4560
   agcctctcta accactatag gacactgtgg agtctggtta caaagagaga ttactcaagg 4620
   tccttagcac tgattacaga gcatatctca gatgccttct gctgaccaga tgtatctttg 4680
   cataatctgc ctatccagat tcagaaaatt gatgccacat agccaagtgg actttcagga 4740
   acagacgatt taaaaacagg cagagagatg tgagagaaag gagaaggaga gagagaaggg 4800
   agagggagag aagagagagg gagacggaga aggaaagagg gagaaggaga aggagagaag 4860
   gggcatggac agagggaggg acagaaggag agaggagata gagaggggga taaggaagaa 4920
   gggagggagg gagagagaga gaaggctaag tctttccata cctgggtccc aatacctctt 4980
   ataacccaag cacatggttt cacatatcac aatgcggttg ggatatagat aactgtaaat 5040
   acttgtgaaa ataatggggc tgagatctgg ggttttcatg atagtttcaa agtcaccgta 5100
   ctgactaaaa ccttccactg gcccatctcc agcttcctaa tctgagggta tcaaatttcc 5160
   cactaagtgt gtttagaaag atctccacct ttttgccctt gtcttccagt gccccaccta 5220
   cgttctggtc tcccacatct gatgtcttct cagtgattct ggccctgcct gctccacagc 5280
   tacaaacccc ttcctataat gagctctgtg ctgagccatc atcctgaatc aatccacctt 5340
   aagcagatgt tttgcttatt tttcctgtgt ccatactaca gaggaaaggt aggcatgtag 5400
   aagctgaagc atctcacctc attccaagca ccctcagtct ctaaatgtgc ccccttgttt 5460
   ccagaagtgc aacctcaagc atcttttatt cattcatctt agagggccac atgtgctgta 5520
   gtgttataag atgaaattta aagcattaat tattcctaac aagccaatta aacaagccaa 5580
   aaacattcat cagtcattcc catggaacct ctgaagcatc ttcctgctct aaccttgggt 5640
   tttccagggc tgctctggga tcacaggagc tgtcctgtct accagccata taaaggcaga 5700
   cctatcagaa ttacaccaga cttctcacca tagactataa aagccagaat atcctggaca 5760
   gatgttatac agaaactaag agaacacaaa tgccagccca ggctactata cccagcaaaa 5820
   ctctcaatta ccatcgatga agaaaccaag atattccatt acaagtccaa atttacacaa 5880
   tatctttcca taaatccagc cctacaaagg atagcagatg gaaaactcca acacaggtag 5940
   gaaaactaca ccctagaaag agcactaaag taatcatctt tcaacacact caaaagaaga 6000
   taaccacaca aacataattc cacctctaac aacaaaaata aagtaggcaa caatcactat 6060
   tccttaatat ctcttttaac atcaatggac tcaattctcc aataaaaaga catagactaa 6120
   cagactgaat acataaacag gacacagcat tttgctgcat aaagcaaaca cagcgttact 6180
   tttttttttc taaatgacat tttttattag atattgtctt tattgacatt tcaaatgtta 6240
   tcccctttcc tggtttaccc tctgaaatcc cctatctcct ccccctcccc ctgctcacca 6300
   atccacccac tcccacttcc aggccctggc aatcccctat atttgggcat agagccttca 6360
   caggaccaag gtactctcct tgcattgatg accaactagt ccattctctg ctacaaatgc 6420
   agctagatct atgagtccca ccatgttttc ttttgttggt ggtttcatgc cagggagctc 6480
   ttggagtact gattggttca tattgttgtt ctccctatgg ggttacaaaa cccttcaact 6540
   tcttgggtcc tttctctggc tgcctcattg gggaccttgt gcgaagtcca atggatgact 6600
   gtgagcatcc acttctgtat ttgccaggca ctggcagagc ctctcagaag acagctatat 6660
   caagatcctg gcagcaagct cttgttggta tccacaaaag tgtctggtgg ttgtctatgg 6720
   gatggatccc caaaggggca gtctctggat ggtcattcct tcagtctctg ttccacactt 6780
   tgtctcttta actccttcca tgactatttt attcctccct ctaagaagga ccgaagtatt 6840
   catactttgg tcttccttct tgaaattcat gtgttttgtg aattgtatct ttgatattcc 6900
   gaacttctgg gctaatatcc acttatcagt gagtgaatat catgtgtgtt cttatgtgat 6960
   tgagttacct cactcaggat gatatcctcc agaaccatcc atttgtctaa gaatttaatg 7020
   aattcattgt ttttaatagc tgaggagtac tccattgtgt aaatgtacca cattttctgt 7080
   acccattgtt ctcttgaggg acatctgggt tctttaaagc ttctggacat taaatataag 7140
   gctgctatgg aaatagtgga gaatgtgtcc ttattacatg ttggagcatc ttctgggtat 7200
   atgcccagga gtgctattgc tggatcctct gatagtacta tgtccaattt tctgaggaac 7260
   tgccaaactg atttacagag tggttgtacc agcttgcaat tccaccagca atggagaaat 7320
   gttccccttc ctccacatcc tcaccaacat ctgctgtcac ctcaatttgt tcttagtgat 7380
   tcagacaggt gtgaggtgga atatcagggt tgtttggcat ttccctgatg actagtgata 7440
   ttgaaaaaaa ttttaagtgt ttctcagcca ttcagtattc ttcagttgag aattcactgt 7500
   ttagctctgt actcaggttt ttttaatagg gttatttggt tttctggagt ctaacgtctt 7560
   gaattctttc tatatattgg atattagccc tctgtcatat ttaggattgg taaagatctt 7620
   tcccaatatg ttggctgcct ttttgtgtcc tttgccttac agaacctttt taattttatg 7680
   aggtcccatt tgctaattct tcattttaca gcacaagcca ttggtgttct gttcaaaaat 7740
   ctttccccct gaaccctatc ttcgaggatc ttccccactt tctcctctat aagtttcagt 7800
   gtctctatta ttgtgctgag gtcgcgatcg caccggtgaa accctaggaa gttcctatac 7860
   tttctagaga ataggaactt cggaatagga acttctctag gggctgcagg tcgaggtctg 7920
   atggaattag aacttggcaa aacaatactg agaatgaagt gtatgtggaa cagaggctgc 7980
   tgatctcgtt cttcaggcta tgaaactgac acatttggaa accacagtac ttagaaccac 8040
   aaagtgggaa tcaagagaaa aacaatgatc ccacgagaga tctatagatc tatagatcat 8100
   gagtgggagg aatgagctgg cccttaattt ggttttgctt gtttaaatta tgatatccaa 8160
   ctatgaaaca ttatcataaa gcaatagtaa agagccttca gtaaagagca ggcatttatc 8220
   taatcccacc ccacccccac ccccgtagct ccaatccttc cattcaaaat gtaggtactc 8280
   tgttctcacc cttcttaaca aagtatgaca ggaaaaactt ccattttagt ggacatcttt 8340
   attgtttaat agatcatcaa tttctgcaga cttacagcgg atcttcagaa gaactcgtca 8400
   agaaggcgat agaaggcgat gcgctgcgaa tcgggagcgg cgataccgta aagcacgagg 8460
   aagcggtcag cccattcgcc gccaagctct tcagcaatat cacgggtagc caacgctatg 8520
   tcctgatagc ggtccgccac acccagccgg ccacagtcga tgaatccaga aaagcggcca 8580
   ttttccacca tgatattcgg caagcaggca tcgccatggg tcacgacgag atcctcgccg 8640
   tcgggcatgc gcgccttgag cctggcgaac agttcggctg gcgcgagccc ctgatgctct 8700
   tcgtccagat catcctgatc gacaagaccg gcttccatcc gagtacgtgc tcgctcgatg 8760
   cgatgtttcg cttggtggtc gaatgggcag gtagccggat caagcgtatg cagccgccgc 8820
   attgcatcag ccatgatgga tactttctcg gcaggagcaa ggtgagatga caggagatcc 8880
   tgccccggca cttcgcccaa tagcagccag tcccttcccg cttcagtgac aacgtcgagc 8940
   acagctgcgc aaggaacgcc cgtcgtggcc agccacgata gccgcgctgc ctcgtcctgc 9000
   agttcattca gggcaccgga caggtcggtc ttgacaaaaa gaaccgggcg cccctgcgct 9060
   gacagccgga acacggcggc atcagagcag ccgattgtct gttgtgccca gtcatagccg 9120
   aatagcctct ccacccaagc ggccggagaa cctgcgtgca atccatcttg ttcaatcatg 9180
   gtggatggtt tagttcctca ccttgtcgta ttatactatg ccgatatact atgccgatga 9240
   ttaattgtca acaattcgag agacccgggt tcccaatcta gagctcgacg tctaacaaaa 9300
   aagccaaaaa cggccagaat ttagcggaca atttactagt ctaacactga aaattacata 9360
   ttgacccaaa tgattacatt tcaaaaggtg cctaaaaaac ttcacaaaac acactcgcca 9420
   accccgagcg catagttcaa aaccggagct tcagctactt aagaagatag gtacataaaa 9480
   ccgaccaaag aaactgacgc ctcacttatc cctcccctca ccagaggtcc ggcgcctgtc 9540
   gattcaggag agcctaccct aggcccgaac cctgcgtcct gcgacggaga aaagcctacc 9600
   gcacacctac cggcaggtgg ccccaccctg cattataagc caacagaacg ggtgacgtca 9660
   cgacacgacg agggcgcgcg ctcccaaagg tacgggtgca ctgcccaacg gcaccgccat 9720
   aactgccgcc cccgcaacag acgacaaacc gagttctcca gtcagtgaca aacttcacgt 9780
   cagggtcccc agatggtgcc ccagcccatc tcacccgaat aagagctttc ccgcattagc 9840
   gaaggcctca agaccttggg ttcttgccgc ccaccatgcc ccccaccttg tttcaacgac 9900
   ctcacagccc gcctcacaag cgtcttccat tcaagactcg ggaacagccg ccattttgct 9960
   gcgctccccc caacccccag ttcagggcaa ccttgctcgc ggacccagac tacagccctt 10020
   ggcggtctct ccacacgctt ccgtcccacc gagcggcccg gcggccacga aagccccggc 10080
   cagcccagca gcccgctact caccaagtga cgatcacagc gatccacaaa caagaaccgc 10140
   gacccaaatc ccggctgcga cggaactagc tgtgccacac ccggcgcgtc cttatataat 10200
   catcggcgtt caccgcccca cggagatccc tccgcagaat cgccgagaag ggactacttt 10260
   tcctcgcctg ttccgctctc tggaaagaaa accagtgccc tagagtcacc caagtcccgt 10320
   cctaaaatgt ccttctgctg atactggggt tctaaggccg agtcttatga gcagcgggcc 10380
   gctgtcctga gcgtccgggc ggaaggatca ggacgctcgc tgcgcccttc gtctgacgtg 10440
   gcagcgctcg ccgtgaggag gggggcgccc gcgggaggcg ccaaaacccg gcgcggaggc 10500
   ctcggaggcc agacgcgcga gaagttccta tactttctag agaataggaa cttcggaata 10560
   ggaacttcgg taccgcgatc gcccttgatc cacttgaact tgagcattgt tcaaggagat 10620
   aagaatggat caattcgaat tcttctacat gataacagcc agttgagcca gcaccatttg 10680
   ttgaaaattc tcttttttgc actggatggt tttagcactt ttgtcaaaga tcaagtgact 10740
   atggctcttc aactatggct cattccattg atcaacttgt ctgtcactgt acaagcacca 10800
   tgcaattttt attgcaattg cttagtatta caccttgagg tcaaggatgg tcattccacc 10860
   agaggttctt ttatggttga gaagagtttt tgctatccta ggtttttgtt attccagatg 10920
   aatttgcaaa tggccctttc taactcagtg aagaattgag gtggaatttt gatgggaatt 10980
   ttattgaatc tgtagattgc attcaacaag atagccattt ataatacatt aatcctgcca 11040
   gtccatgagc atgggagatc tttccatctt ccgagatctt cttcgatttc tttcttcaga 11100
   gacttgaagt ttttatcata cagatctttc acttccttag ttagagtcac accaaggtat 11160
   tttatattat ttgtgactac tgtgaaggtt gttgtttccc taatttcttt ctcagcctgt 11220
   tcatcctttg tgtagagaaa ggccactgat ttatttgagt taatattgta tccagctaat 11280
   tcactgaagt tgtttatcag gtttaggagt tctcttgtgg aatttttgga atcacatgtg 11340
   tatactatta tatcatctgc aattagtgat attttgactt cttctttccc aaattgtatc 11400
   cctttgatct ccttttgttg tctaattgcc cacactagga ctcgggcagc cttagtgcct 11460
   agtccctgat tttagtgtga tttgttcaag tttctctcca cttagtcgga tgttggctac 11520
   tgatttgctg tatattgctt ttattatgtt taggtatggg ccttgaattc ctgatctttc 11580
   caatactttt atcatgaatg ggtgttgaat tttgtcaaat gctttctcaa catctacaaa 11640
   gatgatcatg tagattttgt ctttcagttt gattatatag tgtattatgt tgatggattt 11700
   ccatatatta aaccatccct gcatccctgg gatgaagcct acttggtcat gatagacgat 11760
   tgttttgatg tgttcttgga ttcagttagt gagaaatata ttgagtattt ttacatcgat 11820
   attcataagg gaaattggtc tgaagttctc tttctttgtt gggtctttat gtggtttagt 11880
   tatcagagtc attgtagctt catagaacaa attgagtaga gtaccttctg tctctatttt 11940
   gtggtatagt ttgaggagat ttggaaatat gtcttcttgg aacgtctgag agaattctgc 12000
   actaaaccca tctgatcctg ggcttctttg gggggggggg actattaatg actgcttcta 12060
   tttctttagg ggaaatggga ctgtttagat tgttaatatg atcctgaata gaaatctgat 12120
   ctgatctaga aaattgtcca ttttattcag gttttccagt tttgttgagt attgcctttt 12180
   gtggtaggat ctgatgatgt tttggatttc cttaggttct gttgttatgt cttctttttc 12240
   atttctcatt ttgttaatta ggatactgtc cctgtgtcct ctagttactc tggctaagcg 12300
   tttatctatc ttattgattt tctcaaagaa ccagctcctg gtttggttga ttctttgtat 12360
   agttcttttt gtttccactt gattgatttc tgccctaagt ttgattgttt cctgctgtct 12420
   actcctcttg ggtgaatttg cttccttttg ttctagagct tttaggtgtg ctgtcaagct 12480
   gatagggtat gctctctcta gtttcttttt ggcggcactc atagctagga gtttcctctt 12540
   tagcagtgct ttcattacgt cctgtaagtt tgggtatgtt gtggcttcat ttgcattaaa 12600
   ttctaataag tctttaatct ctttccttct ttcttccttg accgagttat cattgactag 12660
   agtgttcatc agcttccaca tcaatgttgg ctttttatta tttatgtttt tattgaagat 12720
   cagcctttgt cggtggtgat cttctaggat gcatgggaaa ttttcaatat ttttgtatct 12780
   attgaggcct gttttgtgac caattatacg gtcaattttg gagaaagtac cgtgaggtac 12840
   tgagaagatg gtatatcttt ttgttttagg ataaaatgtt ctgtagatat ctgttaaatc 12900
   catttgtttc ataacttctg ttagtttcac tgtgtctctg tttagtttct gattccagaa 12960
   tctgtccaat gataagagta gggtattaaa ttctcccact actattgtgt gaggtacaat 13020
   gtgtggtttg agctttaaaa gagtttcctt aatgaatgtg gatggccttg catttggagc 13080
   atagttattc agaattgaga gttcctcttg gaagatttta cctttgatga gtataaaatg 13140
   cccctccttg tcttttttga tacctttggg ttagaagtgg attttattcg atattagaat 13200
   ggctaatcca tcttgtttct ttgagatgtt tgcttggaaa attattttcc tgccctttac 13260
   tcggtggtag tgtctgtctt agtccctgag gtgggtttcc tgtatacagc aaaatgttgg 13320
   gtcctggtta tgtagccagt ctgttagtct gtctttttat caggtaattg agtccattga 13380
   tattaagagc tattaaggaa aagtaattgg tgcttcctgt tatttttgtt gttagacttg 13440
   ggattctgtt cttgtggcta tcttctttta ggtttgttga aggattactt tcttgctttt 13500
   tttagggtgt aatttccctc tttgtgttgg agttttctct ttattatcct ttgaagggct 13560
   ggattcatgg aaagatgttg ggtgaatttg gttttgtcat ggaattcttt ggtttctcca 13620
   tctataattg agagttttgc tgggtatagt agcctaagct ggcatttgtg ctctcttagt 13680
   gtctataaca tctgtccagg atcttctggc tttcataatc tctggtgaga agtctggtgt 13740
   aattctgata ggcctgcctt tatatgttac ttgacctttt tcccttactg ctttaaatat 13800
   tctatcttca tttagtgcat tttttttctg attttttatg tgtcaggagg aatttctttt 13860
   ctgctccagt ctatttggat tctgtaggct acttctatgt tcatgggcat ctccttcttt 13920
   aggttacgga cgttttcttc tataattttg ttgaagatat ttactggccc tttaagttga 13980
   aaatctccat tctcatctat acctattatc tttaggtttg gtcttctcat tgtgtcctgg 14040
   atttcctgga tgttttgagt taggatcttt tttgcatttt gcattttttt ttattgttgt 14100
   gcccatgttt tctacggaat cttatgcacc tgagactctc tcttctacct cttgtattct 14160
   attggctgat gcttccacct atgtttctcg atttctttcc taggatttct atccccagag 14220
   ttgtctccct ttgtgatttc tttattgttt ctacttccat ttttagattt tgaatggttc 14280
   tgttcgattc catcgcctgt tgggttgtgt ttttctgtat ttctttgagg gatttttgtg 14340
   cttcatcttt aaggtcttct acctgtttag gagtgttttc ctataattct ttgagggatt 14400
   tttgtgtttt ctctttaagg gcttctagca atttagcagt gttctcctgt atttctttaa 14460
   gtgagttatt aatgcccttc ttaaaatcct ctaccaacat cattagatat gattttaaat 14520
   ccgaatcttg cttttcaggt gtgttggggt atccaggact cactgtgggg ggagtactgg 14580
   gttctgatga tgaaaactgg tcttggtttt tattagtaag attcctactt ttgccttcca 14640
   ccatctgata atatctgttg ttagatattc tagctgtctc tggctggagc ttgttcctcc 14700
   tgtgattctg tcagcctctg tcagcactcc tgggagtaca actcttttct gagtcccaat 14760
   gttcagagca ttctctgcag gcaagctctc ctctggcagg taaggtgccc agagctcttg 14820
   agctcagctc cacctcctga ctgcagatga agacccaaag ggaccctgtc caataagctc 14880
   tgttgcttct gccacccaca tgctctcctg tgcgaactgg tctctgagag acccgggata 14940
   caagatggta ctctcacctg aatcccaggg tcaaagccct ccctggaggc tgactctcct 15000
   cttgtgggaa ggtgcacaga ggtctggagc tcagctctgc ctcctggctg aagatgaagg 15060
   cccgaaggga ccctgtccaa gaagctttgt tgcttctggg acccacatgc tctcctacat 15120
   ggactggtct ctgagagacc agggattcaa gatggtgctc tcacctgagt cccagggtca 15180
   gagccctctc tggaggccaa ctctcctcag tgatcctaag atcctgggta tgctagggtg 15240
   cctatggcat ggagagtctt ctgaggaatg tgggactgtc tgctgagttt ccacccaagg 15300
   tggtgctggg ctggctccag tcagaatgaa cccagactct ggttgggcag gtttcctgtc 15360
   ctgctggccc aagcccctct gggttgtttt agaacagatg ttgctttcca ctcaccagtg 15420
   atcccaagat cctgggtgtg ctagggtgcc tgctatgtgg agagtccact ggggacctta 15480
   ggagcataca tcaagttcac acccatggtg gcaaggagct ggtgcctacc agaacaaacc 15540
   ccgggcactt ttactgaccc tttaagttga aaatcttcat tctcatctat acctattatc 15600
   cttaggtttg gtcttctcat tgtgtcctgg atttcctgga tgttttgact taagatcttt 15660
   ttgcattttg catttttttg attgttgtgt ccatgttctc tctggaatct tctacacctg 15720
   agattctctc ctctgtatct tgtattctgt tggtgatgct tgcatctatt gctcctgatc 15780
   tctttcctag ggtttctatc tccagagttt tctccctttg tgatttcttt attatttcta 15840
   cttccatttt tagatcctag atgattttgt taaattcctt cacctgtttg gttgtgtgtt 15900
   cctgtaattc tttaagggat ttttgtgttt cctctttaag ggcttctacc tgtttagctg 15960
   tgttctactg tatttcttta aaggacttat gaatgtcctt cttaaaaacc tctaccagca 16020
   tcatgagatg tgatcttaaa tgtcaatctt ccctttctgg tgtgttgggg tatccaggac 16080
   ttgctgtggt tggagttctg ggttctggta aacctgcctt agagggtcac cacagagtaa 16140
   tgatagcact acttttaaac aggggaagat gatgaaataa ttgctgtggg aaaatgcaag 16200
   gaagactcca acacatgtag gcatctatga aggtctcaaa tcttcaaaat ccaaaaccac 16260
   caagaaagaa agaaagaaag aaagaaagaa agaaagaaag aaagaaagaa agaaagaaag 16320
   aaagaaagaa agaaaggaag gaaggaagga aggaaggaag gaaggaagga aggaaggaag 16380
   gaaggaagga aggaaggaag gaaggaagat tctaaaagta gtcacctgca ccaggtgcct 16440
   ggggagtcac tcagcagccc tagactgaga aagcttgaag aaagtagaaa tagagaaagt 16500
   gtacagccag tatcctctag ctactcacat ccaaacaggg cctcctgact gctctgagcc 16560
   tgtcctaaga acagcaatga tgccacagaa atttttagag tgaaccctga aggaacttga 16620
   ggccgatatg agcaagccag tcccagagga aaggaaaacc catagagaga aaacaggtga 16680
   gttagtgcat taaaggggct gagcagggag ttctcatcgc tccccagcac cagaaataag 16740
   agtctctcca gagctgctgg gacatggaat gcagatgatt tggaccatca gccccacaga 16800
   gacctttccc actctggatc agaaagaggc aagcatgtaa ccatctggac cacagttgga 16860
   gaggagaatt gaaagctgat atctctgcat tcacttagcc tgttacccac ccatgcaccc 16920
   aagtccaagg tgggagaaat actgagggtc taaacacagc cccagatcaa ctgccagtat 16980
   taaataccag atttcagaag atgggaaatc gactctctgg tcagttttgg gacatgtaaa 17040
   ctgaaagaga aaacatagat ggaccaaatt aacatggagc ctaattaaaa tggagcagtc 17100
   ctatatccct aacccagttg taaataaaac attcaagagt gccatcagac accagagtgg 17160
   tacaggagag atgagtgtac ctagtacatc aagagttccc tcactagata aaccaagagt 17220
   agccccagga ccacccaggc acctaccagg agtcccctcc agaggtctga gccagttagc 17280
   tctggttcat tcagttccag accaaaatat cagaaacaat agcatctcca ctgcaggtga 17340
   atccctgagc aatacagtgt acccaaagaa aacaccatgg atggaggaca agggaggcag 17400
   aggagtctgt atcatactaa gagtattaca caatgagttt tttctctttt cacagtgacg 17460
   aagcactgat tccccctgtc ttagttaggg ttctactgct gtgaacagac attataacca 17520
   agacaggact tataaaggac aaaatttaat tggggctggc ttacaggttc agagtgtcag 17580
   tccattatca tcaatgcaag agcatggcaa catccaggca ggtatggtac agtaaaagct 17640
   tagaggtcta catcttcatc tgaaggctgc tagcagaata ctgacttcca ggcatctagg 17700
   atgaggggtt tttcaacaac atccacagtg acacacctac ttcaacaggg ccacatcttt 17760
   taactgtgcc aatacctagg cagagcatat acaaaccatg acatcacact atttggctcc 17820
   tgtaggctgg ttcaaacaca tgagtcaatg gtggccacat ctggtcatag tgtaattaaa 17880
   aaaaagtaca gttagtccaa cttccaacgt ccccatagtg tatagcaatc tcaacaatgt 17940
   taaaagtcaa aagttcaaag tctcttctaa aatccattca attacttaac tgtaacccca 18000
   aatcaagaca ggaaaccatc tgagcaaact ccacactctg catctccaag tctgacgtca 18060
   aagcaatttt cagatctcca atctatcttt tcatattgtg actacaacaa acttcttttc 18120
   ctgggatggt cctactccct gttagcagca ttcctcagaa gatactctgt ggctttgata 18180
   tttccaaaac cttgggtctt caaggcagat tcaatgttac agcttgtttc agtatctggg 18240
   atccacacat gattttctag gctcctccaa agggctggca ccacttcttt agctctgccc 18300
   tctgtggcat tctaagctca ggttgatcca ctccactgct gctgctgctg ctgttcttgg 18360
   tgaccatcca ccccatggta ttggcatctc cactatgatg aggacttctg ctgcaactag 18420
   gcttcaccaa tagcctctct taggctttct tcataatgac aagggtcaaa tcctttacat 18480
   gaccccttca gttctgggcc accaactgca actggggttg caccttcccc agtggtggtc 18540
   catagcctct cactccaagc ctcagatgtt cttcatgacc ccttcatgcc ttcaaaacca 18600
   gtaccacctg ggtgactctt atacattacc aagtatagtt gcagcacaag gtacaacctt 18660
   ggctatcact ggaacacaac ttctttgtgc tttcagaaaa caatttccag aagatttcac 18720
   ctttcttaat cactactaat ttcttagctc cagctaacca gtattaattg tcccagtatc 18780
   aattgtctcc ttctcctctt gactctaaag ccagaatcac atggacaaag gtgctgagtt 18840
   ctgctgcttg caggatctag atcatggctg ccttgttcta ttacattatc attagcttcc 18900
   tctttttcaa ctcctttact gcctaagctt ggctgtcctg gatcttgctc tatagattga 18960
   cctttagctc agagatctgc atgaatatct cctgggacta aaggtgtgtg ccaccatgcc 19020
   tggatttaag cttttcttca cctagaactt gctctgtctc aggctagcct tgaactcaaa 19080
   gatctgcttg gctttatctc ctgggattaa aagtgtgtag caccatgcct gtatctaaat 19140
   ttagctgggt aggatcttac cccaacatcc caccccctta attcaattta atatccttga 19200
   acataggatt cagctccatt tcacttcatg ttgcccattt aatactcgaa ccatacattt 19260
   tgtagttccc ttttctcagc gtgctgtgct tgtttgaaat gttcttcatg agaattaacc 19320
   agacaaaaag tctatgatgg atatttctga gacttccttt gtcagtgcaa ttaatctgag 19380
   tctctttacc ttagcctcag gcagattcct cagaaaagag caaaaaaaaa aaaaaagtag 19440
   ccactttctt caccaatata ccacaaaaac agtctctagg ccacatattg aaattctcct 19500
   ctgaaatctt gtgggccggg tccatacagt tcaaattgct ctcagtaaca aagacttcca 19560
   tagtcctact aggataaccc atcaagcacc atttaaatta ccccactgct ttccaaatcc 19620
   aaagtcccca aatccacatt ctcccaaaca aaaatgcatg gtcagaccta tcacagcaat 19680
   accccactga gtttactgtt gtgaacagac accatgactg aggcaagtct tatgaaggac 19740
   aacatttaac tgaggcaggc tcaaaggttc acagtctcag tccattatca tcaaggcagg 19800
   aacatggcag catccaagca ggcacggtgc aggaggagct gagagttctc catcttcatc 19860
   tgaaggctgt tagcagaatg ctgacgtcca gacagatagg atgagggtct tttcgacaac 19920
   atccacagtg acacacctac tccaacaggg caacatcttc taatcatgcc agtctttagg 19980
   caagcatata caaaccatta cacacacaca cacacacaca ccctcaaaaa aaagaaaaaa 20040
   gaaaacataa cagaacaagg acccttacat aacatctgac gaggggagct gtcaaaatag 20100
   ccactgagct taggtgtctc atacaggtta atatagaatg acaggccaat gaaactcctg 20160
   tgtggcaacc acaaagggga agctgtggat ctccatcacc ttacaagcaa actgtgccaa 20220
   tcagcaagga ctagggaagc agagacacac caagactttc acactgtgtg taaatggtgt 20280
   gggactctgc aggtcatcca aggactgtgg gcccagatga acatgtgtgg gaaatacata 20340
   cactttacat gcctaccttg tatttataac atatatgtaa caccttcttc tattttttat 20400
   cttctatgac tgctaaagtg ctgcacattg agcactgagg catgtccatg ccaggtaaat 20460
   ttcttaccac tctgatgcat ttccattccc aactcatcat cttgggaaag gaattttcaa 20520
   agttaatgta gatataagta aagagagtca ggagggtggg ctgaaaggca gacctgcaga 20580
   aattctccag agggtagagc tcagaagcag caagcagaac ttgggctaac agaagagcaa 20640
   acacagagct cagacagaac tacctggcaa tgcgactggg cacactggaa gcactgtgca 20700
   tcagcactga gccccaaata tgcccccaga atcctctgca taacaatgta acataacaga 20760
   aaggctggaa taggccaaaa gaggaaacaa aaaaagacac ccccccaaga aaaaataaac 20820
   aaattgggaa gagtaaaaaa gaattgtaag tgtaccacac agaacctgct cataatggct 20880
   tgcatgcata gaataggaag gaccacgtta aaagaaaaat gttcagcagg attccttctc 20940
   cctcaaactc ccaacaagca aaattcactc tcattaggag atgagaagag aaagtctgag 21000
   ccctgtgttg acagtgccag gaaccactgc cagcctgtgg gaaatgtctg gctctcctga 21060
   cagagcctgc tgcttctgcc ctgaatcctc tcactccacc tataaggggc tcatggcatc 21120
   accacacagc ctccccaaag aggggggaaa agggcagctt gtcattgagt aagtcaccca 21180
   gagacatgcc tttctagtag ttcttataat cgccctactc cagcagcctc catccaggca 21240
   ttgagagacc aaaggatatt tggaagtcag ccttatggta gccccagacc ctgcttgttg 21300
   cctatgatct tcctgaagag atagaagccc aggagaaaaa agttgtcccc caactcaaga 21360
   tggcccctga gactctgcac tgatgtctgt gcccccacta ggccaaaggt agtcacccta 21420
   tgcaaaccaa ccaatgcatg aaagggttac atgctacacc aattagaata agccatctag 21480
   ccctgttgcc taaatctgcc ccttatgagg tataaaaagt gtgttctttc tttgttcagg 21540
   gtctctcctc ttgcctgcat gctgaggggg attccccaac atattggatt aataataata 21600
   ataataataa taataataat aaatcttgtg gtttgcatca atttcagtct ctgttgtctt 21660
   tgtgagtgag ggtcttttgg tgaactccaa cacctactcc tgccaaacca gagctcttac 21720
   tttgcagggt ttttggagaa aggctctact actgtgtata taactaaagt ggtagctcac 21780
   acaatggtgt atctagtagc aaaagtttgt tgtgcccagc agattcttca gctcaaaaca 21840
   gtaattctag agtagctgac atctaagggt ctgtatctta gtcttgacca ctgaggctta 21900
   attaactgat attaccacac acacacagca attctgactg accacagaga catcaaagca 21960
   ccctaaagtc agatcccaat agccccttac ccagagtttg taaggccagc agagagcctc 22020
   tcagctctcc acctggacca atggctcctt ttagtaggaa gagacctaga gaactttccc 22080
   tcagaaaaac atgggaaaca ctccaagctg agctcagaca gccttccaca gccacttgga 22140
   aaatactgat ttggctatct ttacctggga ccaaaaagac attactacct tagtcacagc 22200
   ctaaacatgt tctttcagtc acttggtggt ggtccacaag gagcttatgg taggttctac 22260
   caagatcttt tgaagactac caggagcata ttgctaaatc agagtctaac ttcagctacc 22320
   taggagctcc ctgtatcatg gtggagcacc caaagaatgg aagcatctcc caatctactg 22380
   cggacctgag aaaggttttt ggttttgtga agttttttgg attttatgac ccatttattg 22440
   gaacatgatg gatctgttaa gggacagaat gccagtggta accatcctgc tgtcacaaat 22500
   ccaaagacct tcccatagcc tactctagaa gaccagatac aggcctacac aacttccctt 22560
   ctgattaatg ttggggacaa attttaacat ttggcttcta tatgtggatc tctgtcttct 22620
   ctgtcctctc tttgtcttac atactgcaga gactagggga agataactgt ggacatgaaa 22680
   catctgtgtc atgtagtgtt ggctgtgtca ctaggaagct acagtgtgtt tctcactatg 22740
   gaacagatac cagaaggtcc tgtgtctttg atgaactgac ccttagggcc catcccataa 22800
   ttcctgcaag tctggaccag gcatggccac atgacatcag actgtctcat atttcatctc 22860
   tgctagagcc atggtacaga tattgacata gatcaggtac agaaatagca gagtgctcct 22920
   gaagagggtt cttctccatc tgcaatcagc atggcaggaa gaccacagga gcagaattca 22980
   gtatctgcct ctctcactgt ggagtaccta ccacagtgat acagagcatc catggaagaa 23040
   cgacccctca tctacagtgg ctagccagtg aggataagtg ggacatgaag tttgacctgg 23100
   tgtcacagct ttgagtctag agaggcccag tggagttagg attgagagtc ccaagttttg 23160
   gtctagccat actgacacaa tctctataac agtatctgtg gtggcttcag gaagctggct 23220
   tctgaagtgc ccccacccaa cattccatct gcactggaac caaaaagttt cagatctcag 23280
   cagataaata cttggattaa aagaacacac acacacatac acacacacac acacacacac 23340
   acacacacac acacactaca aggtcaaata tgctccaggg gacacaaacc accatggctt 23400
   gtgaaaagac cacaagccaa ggagtttctg ttacaggttg ggttactcag taccatggta 23460
   acactcaatg atacagaaac acatccagga caattctaga agatgatcca ggctgttcag 23520
   atgaggagat aggtcctgga gagatcaatt cctcactctc cattctaaga tgactgaaac 23580
   aacccaacta gggcatgagc cagaattaca cacacttaca catgaactca cacacacata 23640
   ctcatatgca cgaacatgtg tgcatacaca tacacttgca ttcacacata catgcacaag 23700
   tgggcgtaca cacactcata tacattcata cacatatcca tgcacataag tgcacacaca 23760
   cattcatgta cataggcact ggtacacaca catatacaca tgcatgcaca catgcccttg 23820
   tatacacaca tacacacaca tgctcacata cacacacaca tacacaatgt cataagcaga 23880
   aagtacccaa cagtctttcc cactcaaacc agcccatcaa gagtgaaggg atgaagactg 23940
   atagtatcaa tgttacaggg ggccctgtta taatggggcc tggaagtaga cacacaatgg 24000
   agaggaatct gtgccagtct caggcactgt gcctacctat actagtggat ggggccacat 24060
   agtcatacgt acactatagc tgggcatggt ggcacatgca tttaatcaga gggctcagaa 24120
   tgcaaaatca agtagatctc taagttggac tccagcacag tcagggctac atgaagaaaa 24180
   ccaatatttt aaaaagaaac aaagaaataa agaaaagaaa aggaaaaaag aaaaaggaaa 24240
   agaaaaggaa aaaaggaaaa ggaaaaagaa aaggaaaaga aaaagaaaaa gaaaaagaaa 24300
   aagaaaaaga aaaagaagaa aaagaaaaag aaaaagaaag agaaaaagaa aaggcttgcc 24360
   ctgaaaccac tcagtgctca cacagctctg cccacagggg aaacctaccc atgcctgtcc 24420
   ccactcagca ggaaggctgt gaagtctctg acaggatttt gaacaagtta ctgtcacagt 24480
   gggcacagct cgggctacca ctgtaagaaa agctcaaacc aaaactgccc tccaaaactc 24540
   atccccagac ctattccctt tctggctttg tgtgtgtgtg tgtgtgtgtg tgtgtgtgtg 24600
   tgtgtgtgtg tgtgtgtgtg tgtgtgtgtg tgtgtgtgta catgtgcatg tatatgtgta 24660
   tacataccca accagcaagg tgctcctgaa tatcaagtcc tttataactt ggagtaggcc 24720
   atccttaccc tttcatcttg gctcaggact ctccaacaaa gctgaccctt gagggagaag 24780
   ctacaggcag ataggatcag tcttctctta ccaccatgaa tacaggcagt taactgcagg 24840
   tatgaacctg tgacatccag taatttctgc aaaagtaaca gacaccatac cacccctgga 24900
   gcaccatgac accaaggccc taaactgtca tcttctccat gtttccagca acgaatgatc 24960
   ttcctgttct gtcctccgag aagggttgga aaggagcttt gcatgaatag gcatgagaag 25020
   caacactgtg gtgtcaatgt caaccacaaa cccctcagaa aggctacaca agcagctgtt 25080
   cagacaatgg cactcagagt gaatggacac acagacatca gattggtgcc tggcacagag 25140
   gcccaggcca aagcactgga ttaaaaatac ctgtgcccct ttggacaatg gcagccagcc 25200
   atggtcatcc ccatggcaag gtcctccatt atgttcagga gttgaaactc agaatgacac 25260
   tcactgacct ccagtgatgg gtcccttgac cagcactggt cccagacaga cagacagaca 25320
   gtaacagaaa gacagagaga gaaagacaga gggagggaga ggagaggagg gagggagaga 25380
   gaaagggagg gagggaggga gggagggagg gagggaggga gggagggagg gagcaggaaa 25440
   gtcaaaacta gaagtagtta attttaacaa caggaaaggg gtttagaatc actcaccaaa 25500
   gtgttaagag cctgttttgg ataccaataa gatggctatt ctggctgact cttctctctt 25560
   tgctttgcta aggcaggcag tgttcaaaca gtgtcatcat aatcttgtgg ctgctccact 25620
   aggccaccct gaacgtgatc ctcccatagt cttttctgtg gtggttctcc actcctgtga 25680
   tgcaacacac ttccttcctc cctccctccc tccctccctc ccttccttcc ttccttcctt 25740
   ccttccttcc ttccttcctt ccttccttcc ttccttcctc ccttcctccc ttccttcctt 25800
   ccctccaggc aagacccgga gatccctaat ctgtaccaac agctagttat gccaaagtta 25860
   ctttccgaac ttcagattcc tatgctgtgc ccagccatgc ttctacatgg gtccctttca 25920
   cactttcatt cagagctccc taggctgtac ccagacctgc atgtgcctag gtacctttca 25980
   aaccttcact cagaggtccc tatgctgtac ctagccatgt ctataccaag gaaacattca 26040
   gactttcact cagagctctc tatgctgtat ccagcactgc ttgtgcctag gtccccgaaa 26100
   gaccttcact cagagctcac tatgaagtac ccagccctgc ttgtgccaag gtaattttca 26160
   taccttccct cagaaatccc tatgctgtac ccacacctga ttgtggctag atacctttca 26220
   gaccttcgct caaaactccc tatgctgtac ccagccctgc ttgtgccaag gtacatttca 26280
   gaccttcaat cagagctcct tatggtgtac caagcctggc ttgtgccaag gtaacttttt 26340
   tttccttttt ttttttataa tgtagcttac aagcacaatg cagtgacttt atataatttt 26400
   gttgtctatg gtataaatga ccatgatgtg cacttggtta tcagctgcac caccattaaa 26460
   gagcccatag aatattcaaa tccacacaca catacacaca catacacaca aacacacatt 26520
   tttatgagtt tcagacctag ttgtgaattg ttctgtatac agagtataag catcttcaaa 26580
   ttgacaacat aatttgtgtt ggttttccac cgtttctact tctgttaaaa acagcatatt 26640
   ttaactgcgg ccccgtctgc ctggttattt ctttgcaaac attgtgagtc tgatacagtc 26700
   cgatatgctc ggtgagtgga tggactccct ttctcaaccc agaacttcta caagaatgtc 26760
   tttctttttg ttttgttttt tttttctttt tttaatatct cttttctttt caaattttta 26820
   ttagatattt tcttcattta catttcaaat gctatcccta aagtccccta taccctcccc 26880
   ccggcctgct cccataccca cccactccca cttcatggtc ctgacattcc cctgtactgg 26940
   ggcatataaa gtgtgcaaga ccaagagacc tctcttccca atgatggccg actaggccat 27000
   cttctgctac atatgcatct agagacacgg gctctggggg tactggttag ttcatattgt 27060
   tgttccactt acagggctgc agaccccttc agctccttgg gtactttttc tagctcctcc 27120
   attgggagcc ctaggttcta tccaagagat gactgtgagc atccacttct gtagttgcca 27180
   ggcagtggca tagcctcaga agagaaagct atatcagggt cctttcagca aaatcttagt 27240
   ggcatatgca atagtgtctg tgtttggtgg ctgattatgg gatggatccc tggttggagc 27300
   agtctctgga tgttccatcc tttcatttca gctccaaaat tttctctgta atgccttcca 27360
   cgggtgtttt gttccctgtt ctaagaagga atgaactatc cacaaattgg tcgtccttct 27420
   tgattttctt ggtttttgtt ttgttttgtt ttgttttgtt ttttgcaaat tgtatcttgg 27480
   gtattctaag tttctgggct aatattcact tatcagtgag tgcatatcaa ctgagttctt 27540
   ttgtgattgg gttatcccct agatacatcc atttgcctaa gaatttcata aatttattgt 27600
   ttttaatagc tgagtagtac tccattgtgt aaatgtacca cattttctgt atacattcct 27660
   atgttgaggg acatctgggt tctttccagt ttgtggctat tataaataag gctgctatga 27720
   acatagtgaa gcacgtgtcc ttattaccag ttggaacatc ttctgggtta tgcccaggag 27780
   aggtatttcg ggatcctcca gtagtactat gtccactttt ctgaggaacc accagactga 27840
   tttccagagt agttgtacaa gcttgcaatc ccaccagcaa tggaggagtg ttcctttttc 27900
   tccacatcct cgccagcatc tgctgttacc tgagtttttg atcttagcca ttctgactgg 27960
   tatgaggtgg aatctcaggg ttgttttgat ttgcatttct ctgaagatta aggatgttga 28020
   atattttttc aggtgcttct cagtcattca gtattcctca gttgagaatt ctttagctct 28080
   gtaccccatt tttaatacga ttatttcaat ttctggagtc cagcttctta agctatttct 28140
   atatattgga tattagtccc ctatcatatt tagcattggt aaaaaatcct ttcccactct 28200
   gttggtggcc tttttgtctt atttacagta tcgtttgcct tacagaagct ttgcaatttt 28260
   atgaagcctc atttgtcaat ttttgatctt aaggtacaag ccattggtgt tctgttcaaa 28320
   aatctttccc tctgtaccct aacttcgagg atcttcccca ctttctcctc tataaatttc 28380
   agtgtctctg gttttatgtg gagttctatg atccacttag acttgagctt tatacaagga 28440
   gataagaatg gatcaattct cattcatcta catgataact gccagttgtg ccagcaccat 28500
   ttgttgaaaa tgctgtcatt tttccactcg acggttttag ctcccttgtc aaagatcaag 28560
   taccccttga taggctgtag gataggtatc tgggttcatt tctggatctt cagttctatt 28620
   ccattgatct acctgtctgt cactgtacca gtaccatgca gtttttatca caattgctct 28680
   gtagtatagc ttgaggtcag gcatggtgat tccaccagag gttcttttat tgttgagaac 28740
   agtttttact atcctagggt ttttgttatt ccagatgaat ttgcaaattg gcctttctaa 28800
   ctcagtgaag aattgaattg gaattttgat gaggattgca ttgaatctgt agatagcttt 28860
   cagtaggata gccattttga ctatattaat cctgccaatc catgagcatg ggagatcttt 28920
   ccaacttctg agatcttcaa tttctttctt cagagacttg aagttcttat catacagatc 28980
   tttcacttcc ttagttaggg tcacaccaag atattttata ttatttgtga ctattgtgaa 29040
   gggtgttgtt tccctaattt ctttctcagc ctgtttatcc tttgtgtaaa gaagggccac 29100
   tgatttgttt gagttaattt tatatccaac tactgcactg aagctgttta tcagctttag 29160
   gagttctcta gtggaatttt tagggtcact tatatatact atcatatcat ctgcaaatag 29220
   tgatgttttg acttcttcct ttccaatttg tatccccttg atctcctttt tttgtcgaat 29280
   tgctctggct aggatttcaa gtactaaatt gaatagatag ggagaaagtg ggtagccttg 29340
   cctagtccct gattttagta ggattgcttc aagtttttct ccacttagtt tgatgttggc 29400
   tactgatttt ctgtatatta cttttattat gtttagttat gggccttcaa ttcctgatat 29460
   ttccattact tttatcatga aggggtgttg gatattgtca aatgctttct cggcatctaa 29520
   cgagatgatc atgtgatttt tgtctttgag tttgtttata tagtggatta tgtcgataga 29580
   tttctgtata ttaaaccatc cctgcttccc tgggatggag ccaacttggt catcatggat 29640
   gatcatttcg atatgttctt ggattcggtt tgcgaggatt ttattgagta tttttgcagt 29700
   gatattcata agggaaactg gtctgaagtt ctctttcttt gttggatctt tgtgtagttt 29760
   aagtatcaga gtaattgtag cttcatagat tgaattgggt agagtacctt ctgtttctat 29820
   tttgtggaat agtttgagga gtgttggaat taggtcttct ttgaaggtct gatagaactc 29880
   tacactaaac ccatctggtc ctgggctttt ttttggttgg gagactatta atgactgctt 29940
   ctatttcttt aggggaagtg ggactgttta gattgtgtta atctgatcct gatttaactt 30000
   tggtacctgg tatctttcta gaaaattgtt catttcatcc aggttttcca gttttgttga 30060
   gtattgcctt ttgtggtagg atctgatgat gttttggatt tcctcaggtt ctgttgttat 30120
   gtctcctttt tcatttctca ttttgttaat taggatactg tccctgtgtc ctctagttag 30180
   tctggctaag ggtttatcta tcatagattt tctcaaagaa ccagctcctg gtttggttga 30240
   ttctttgaac agttcttttt gttccaactt ggttgatttc agccctaagt ttgattattt 30300
   cctgctgtct actcctcttt ggtgaatttg cttcctttcg ttctagagct tctaggtgtg 30360
   ctgtcaggct gctagtgtat gctctatcta gcttcttttt ggaggcactc ggggctatga 30420
   attttcctct taggactgcc ttcattgtgt cccataagtt tgggtatttt gtggctacat 30480
   tttcatttaa attctaaaaa gtcttgaatt tctttctttg tttctgcctt gaccaaggta 30540
   tgattgagaa gagtgttgtt cagtttccat gtgaatgttg gctttctatt atttatgctg 30600
   ttaatgaaca tcagccttag tctgtggtga tctgatagga tgcatgggat aatttcaata 30660
   tttttgtatc tgtttaggcc agttttttga ctgattatat ggtcagtttt ggaggatgta 30720
   ccatgaggtg ctgagaagaa gttatagcct tttgttttag gataaaatgt tctagagata 30780
   tctgttaaat ccatttcttt cataacttct gttagtttca acgtgtctct gtttagtttc 30840
   tgtttccagg atctgtccat tggtgagagt ggagtgttga agtcccccac tatttttgtg 30900
   tgaggtgcaa tgtgtgcttt gagctttact aaagtgtcat taatgaatgt ggctgccctt 30960
   gcatttggag catagatatt cagaattgag agttcctctt ggagtatttt acctttgatg 31020
   agtatgaagt gtccctcttt gtcttctttg ataactttgg gttggaagtc aattttattc 31080
   gatattagaa tggctactcc agcttgtttc ttcagaccat ttgtttggaa aattgttttc 31140
   cagcctttca ctctgaggta gtgtctgtct ttttccctga gatgggtttc ctgtaagcag 31200
   cagaatgttg ggtcctgttt gtactgtacc cagccctgct tgtgctaagg tacctttcag 31260
   gccttcagtc agagctccct atgctgtacc cagacttact tgtgcctacg tccacttcag 31320
   acgtttgctc agaaccctct atgctgtatc cagccctgct tgtttgtgcc tagatcccct 31380
   tgggtgcttt gctcacacta ctcaccatta ccaacaaatg aatggtttcc agatgttcca 31440
   gaaatttccc tgattcccaa aagaaaatga tacagaacag tcaactatat gcatgttttt 31500
   ttttaactac caaacttcaa aattagcaaa gtagggtcct aaatgagcaa gagcctccat 31560
   ctcagagctc tttgaaccat gcaccttttt gtcaacttga tatcagctgg aatcatctga 31620
   gaagcaggaa ccacagttta gaaaatgcct ccatagaaga ttggtccata gacaatctct 31680
   gggggtatcg tcttggttag tgtttgatgt gggagctagg cacacatcgt tgtgagtggt 31740
   accacccatg ggtaggtagt cctcaatgcc atttaaaaaa aatcaggtgg agaaagtcct 31800
   gaagaagaac ccttgttttt ccatgtctta tgttcaacca caacctctaa tttcctgccg 31860
   acttccctca gtgacagtgt gtgacctaga gttgtaagat gaaataaacc attttctctc 31920
   cattttgctt ttggtcatgg tgtttgtcac agcaatagaa acctaaatta gacatctagt 31980
   gtgtcaacac ctattcaacc ttattggaaa gaagacttga tctaataatc aataacccta 32040
   gattcagata tatttttcac atgtacagtc ttgaggattt ttttttgctt gaacccaacc 32100
   tcactgaata agatttacat gaatacacaa ttctatttat aaacttacat gcatcttttt 32160
   tcatgtaaaa taatatttag tgttgtgtat gatatagaat attgacaaac aaagttacct 32220
   acatgaatgt gaatatgttt tatttatgtg aatatgttta ctggataaaa atacaaaagt 32280
   gagcaaggaa gacaaaacca cctgaagtaa atgtcagcac aatgaggcag agatcaggaa 32340
   cccccacagt ggtaacagcg ggtttgtgtg tgtgagtgaa ctcactgggc aactctgtcc 32400
   ccagaaatac taggcaggat cagagggttg agatgttcag ccttccacct ccagtgaaga 32460
   aagggtctag aagctgaatt ggtcattaat ggccaacaga ttccatcctg cttatatgca 32520
   aggctgccat caaagccaaa aatatggtgt taggagaact cccacataac tggacacaga 32580
   ggagactgtg gggaggtaac ccagaggcac agaagccctt cacaggctgt cagagttcat 32640
   tcctcaccct gtgtgcctct tcatctctct gcttcctcct atcctttgta atccagcaat 32700
   catggtgggt gtccaactgc tccagtaaat ttgtctaaag agtatgtggc aggatcctta 32760
   accaaaagct ggtgagtcag cacaggctaa aacctgggat acacaaccag ctggcaatgt 32820
   agagagactt gtagtgctga agcgtcaaac cctgaggtct gtgctgtccc atgatggtta 32880
   gcatcagaaa cccctttccc agaaggttct gctgtcacta gtcagctaag tgaaggttct 32940
   tttgctctac ctaggtagac ctcatgggtc agcaggaagc tttgacctct ggtagccttc 33000
   ttctcacctg ccagggggca gccctggtgt ggggttcttc tttttgtcag ttcttcttga 33060
   gccagtggaa ggggaagaat gtcagtggag ggtaagactt ggtcttacga aattcccagg 33120
   taagactttg tcttacatga tatttagggt tgctgtataa taaaaagttt acttatctaa 33180
   gtctaagtta ctatgctatt atagctgacc tgccttctgt gttcctctga ggccagaaac 33240
   tgcagtctca ggcactttag cacctcatcc taaaacagcc ggagattaag taaacaatta 33300
   attgctagag ccttttccct agtgttcaga tgcctcttgc ttgtcaggct agggggatgt 33360
   ttgtagcaat aaacttctac tgaacccaaa gagaataaca cttgcagaag gtcaaacttt 33420
   tacataatcc aatatgcata atctcacata tgtacataaa atgattcatg tatgctcatt 33480
   tatacatttt cattcaaacc cagttgggcc cacatgcatg cattgtcaca attacatact 33540
   tacacacaca aatgcataca ttctcactgt gatggtttgt atattcttgg accagggaat 33600
   gctaccatct ggagatgtgg ctttgttgga ataggtgtga cctggttgga ataggtgtgt 33660
   cactgtgggt gtggacttaa gactctcacc ccagttgcct ggaagtcagt cttccactag 33720
   cagcctttgg atgaagacat agaactctca gctctgcctg caccatgcct tcctgaatac 33780
   tgccatgctc ccaccttgat gataatggac tgaacctgta agcctgcccc aattaaatgt 33840
   tgtttttata agacttccct tggtcatggt gtctgttcac agcagtaaaa ctctaactaa 33900
   taccgaagtt agtaccaggg actggggtat tgttgtgata ggcctgacca tgcttttatt 33960
   tgaaagaatg tgaattttgg gactttggat ttagaaagca gtggaattgg ggcttaatgg 34020
   gtcatcctag taggaatatg gaagactttg ttgctgggag taatttgaac tgtgttgaac 34080
   ctggcccaag agatttcaaa ggaaaagaat ttcagaatgt ggcataaaga ctgttttgtg 34140
   gtattttggt gaagaatgtg gctacttttt gcccctgtct gaaaagtctg cctgagactt 34200
   aggtgaagag actcaaatta attgcattgg caaatgaagt tttaaaaatg cccagcagag 34260
   actttgttct ctggttaagt cttatgaaga gaagtttgaa caagcattgc aaacttagaa 34320
   aggaaaaata taacatatat gatttgagta ttaaaggggt tgtgacatgg ttgaattcaa 34380
   agatgaagaa tgtgtaaaga aagcattaga aactatgaac aaatatgacc ttagtggaag 34440
   gcctttgaat attaaagagg atcccgatag agaaaatgct cgcaggacac tgcagcaaac 34500
   aggaacatca tttcaaggat catgtgcctc cgatgtggga tctgggttgc taaatttacc 34560
   accttccatt ctcaatagtc taaacattcc tcctgaggtt atcagcaatt ttcaggctgc 34620
   tagacttggt tccacaattt ttgttgctaa tcttgacttc aaagttggtt ggaagaaatt 34680
   aaaggaagtg ttcagcatag ctggaactgt gaagcgtcta ttagtgccag ccagctgaac 34740
   ataactggta taataagaaa tttgggtcca agtgttggtg agtttgcata cgtggtactc 34800
   tttaaggaag tagaaggaaa atcaagggat catgcagagc agctgaggat cggcactgtg 34860
   agaggccatg gaaggccatt ggtgaaggtg cagcctcagt tgcaattgat ggcccaggac 34920
   agaaggggtc atgcaaagga gatgaggctt ggcaccatga agaaagccta tgagaggctg 34980
   ttggtgaagc ctagttacag tggaagacag cagcattttg gagatgccag taccatgaga 35040
   tgaccaccaa gaacagcagc agtagttgag tacaggcagc tgaagcctag aagacaacat 35100
   gtgtgctaca aaggacaggg ctagagaagt gacccaagcc cttggaggag cccagaagat 35160
   cgtgagttgg ataccagaca ttggacagtt ggaatttaat ttttgttttt gattgtgaca 35220
   gtgccctgat atttttccct cttgaaggaa gaaaatattt tagtgaatcc cacagttaag 35280
   agacttttaa ttgtaaaaag actttggatt tcaaaagata ttgaatattt ttaaaggatt 35340
   gaacttttaa tatgtaaaga gtgtaggact tttaaagtta tttaagatcg tggggatgaa 35400
   tacgaactaa gggttgaggc ttactagtgt tgtgtttgtg tgtcaagttg acaatgggtc 35460
   aattgtactg gctagttttg tgtcaacctg acacaggctg gagttatcac agagaaagga 35520
   gcttcagttg gggaaatgcc tccatgagat ccagctgcaa ggcattttct caattagtga 35580
   tcaagggggg aagtcctctt gtgggtggtg ccatctctgg gctggtagtc ttgggttcta 35640
   taagagagcg gactgagcaa tccaggggag gcaagccagt aaagaacatc cttccatgtc 35700
   ctctgcatca gctcctgctt tctgacctgc ttgagttcca gtcttgactg ccttggtgat 35760
   gaacagcagt atagaagtgt aagctgaata aaccctttcc tccccaattt gttccgtggt 35820
   catgatgttt gtgcaggagt agaaacactg actaagacac tcacaattac atacttatac 35880
   acacacccat acttacatat gcatatggag caaaagacca agcactggtg cagtaagaac 35940
   ttactgattc tagatgaaaa ataagctcta atctttattg catagagaaa caaagggctt 36000
   ccaccctttt actgttaaat gaattaaacc catgtttgta agaaaaaaaa agctttgttc 36060
   cttttaataa gactaagcct gccttgttat taagaaaaga cctgttctgt cccacggtaa 36120
   ggagaaggct gcctgctcct tctgctctgc agtgtcttct tttttcccct ttccctcttc 36180
   attctatacc ttgctctctt aatattttat cttacatata gtttctacgt tattccactc 36240
   tgcattctcc ttctaattgt tttctctcct cctgaagtga tgtcacaata atggtcttac 36300
   tctcaatctc ttttctttaa aagctatacc tatgcttgaa actttacttg tctagggatg 36360
   ggcctcccct tttctttata aagcatgttt gctgacatta aaattgaacc ttgatcagaa 36420
   tgactgtctt ggtttcatct ttctttcatc tgcctagctc cctcttctct tccagattcc 36480
   aagatacctt tccagactag aacccagaca agtgagccgc tggctggcca caacaggtgt 36540
   acttttgcta agcctaccct gagactttaa ctcttatcca gtaaaatact gtaagaaagc 36600
   atgcaagaca ctccacaata ttgcagggac aaatctgggg cattctagct atgggaatgc 36660
   caaggtccag gaggctaagt ttccttgaaa ctctttgcct tgtgactgct tccaggcttt 36720
   taggcctacc aagtgaatca ctactggagt ggatgtagca tttggttgct tccagtcacc 36780
   ccagtcatct ggcatgagga agaggcagtt gctagtcttt ccacacattg atcaatcatt 36840
   cataaaaact catacccaga ttctcaaaga gaattttgtt accccatttc agcctggctt 36900
   atatgtgcaa tggtgtcaac agtttcccat tggtgcaaga ccttggagtt atcaatatgg 36960
   taatgcctat atcaacaata cacagtcact aaggacttca cactctgtcc ttctccattc 37020
   cctatacatt tgtcttctga ctgcagacat aatgtgacta gctacttcaa gctcctgctg 37080
   ccttgacttc cccaccatta aagataatac tttgaactgc tagcagaaat aagcctatgc 37140
   tttcttaggc tgcttttgtt aaagcatttt gtcagagcaa caaaagagta atggagacag 37200
   acattggtac caagaattgg gcgtcattgc agagatgaac ctgactgaca ttcctagggc 37260
   actgaaaatg tttctgtggg aggaatttgg agcttccagt ttgaaaagac ttagaattct 37320
   gtaagccaaa tgtaatcatt cattctgctg gaagctttgg aagacaaaaa taattaaaga 37380
   aatgaggaca atggtccccc agctcatgaa gttacataga tgaataagaa gtccaacagg 37440
   acctggggtt gggaccattt gtgtgatctt taagccaaga gttgggcttc attctgctat 37500
   gtcctgagaa cttgtgtgat actaaattta aatctcatag ttgaatttat gtagcagaag 37560
   aaatttgaag ccaggagagt attcagactg gtgccaggaa aacaactaca cctgttaaag 37620
   agataatgtc attgaaaaga agcctcctta agaagggtgt gctgagcttc ttaagaagat 37680
   gacaggtgac agaagaagaa cttcttattg tcatccacat ggtagtggtt ttggaattat 37740
   ggaagatgca agactgggga gagtcactgt ggcttgctcc atgtgttcaa agagctgctc 37800
   agggcaaaca atgtgtggca cagttagagt ccattcaaag agatcctgaa aggccactgt 37860
   ttgagtatgt gaagaggaag tctttccact gctgttctct tatctttccc tcttcctcca 37920
   gagttgctga tgctcattcc tttgctgaca gtagatctag catttgcaag ctttggtcat 37980
   ggactaaaga ccagctgcta tccaggaact tcaagggctc cagctttaga tttctgaagc 38040
   attctttcct taaaaatctg gaactcttgt agcaaccagg atctgcagaa tcaagaagtt 38100
   acagaggaga agaacgtatt agtaagaata tattaatcat atattcttga gatattttgg 38160
   caaagaatat ggtttccctt tgccattgtc caaaaaaatc tgcctgaggc tacattaaag 38220
   agttttggat tgcccagtcc ctccaagaga gagcaagtct cccaaggagt acaaagatca 38280
   caggtgagat gaccactcct gcccaaatac aaggtccaca tgggacctgc ccagagccca 38340
   caagacccag gaacaacaga gcagcctggt acaagatact tctggtttcc atctgaatcc 38400
   agggctgacc ctgtgccaca gctatctaga ccccagtccc acccccaccc aagtcctgcc 38460
   caaagaaagg aggtctcaca ggagtgctca caacactctt ctaacaacaa aaataacagg 38520
   aagcaatgat cactggtctt taatatctct taacatcaat ggactcagtc ccccaattaa 38580
   aagattaaca gactggatat gtaaagagga cccagcattt tgctgcatac aggaaacata 38640
   cctcagtgac aaaaacaaac actatctcaa agtaaaaggc tggaaaacaa ttttccaagc 38700
   aaatggtccc aagaaacaat ctggagtcgc cattttaata tcaaataaaa tcgactttca 38760
   aacaaaagtt atcaaaaaaa gatgaggaag acatttcata cttaaaaaaa gaaaaattct 38820
   gtcaaaatga actctcaatt ctgaacattt ttgctgcaaa tgttagggta cccacattca 38880
   taaaagaaaa actgataaag ctcaaagcat acattgcacc ttactcaata ataatgggag 38940
   actttgccac cccactctca tcaatggaca gatcatggaa acacaaagta aacagagaca 39000
   cagtgaaacg aacataagtt atgaatcaaa gagactgaac agatatatct agaacatttc 39060
   atcctaaaat gaaagaatat atcttctcag cacctcatgg taccttctcc aaaactgaca 39120
   atagaattgg tcacaaaaaa aaaagcctcc acagatacaa gaaatctgat atatcccata 39180
   tatcctataa gatcaccatg gactaaagct ggtcttcaat aacaaaaaca caaaagaaag 39240
   tccacatact aatggaagac gaacagttct ctactcaatg aaaattgggt cagggaagaa 39300
   acaaagaatg aaattaaaga ctttttagaa tttaatgaaa atgaaggcac aatattccca 39360
   aacttatggg acacaatgaa agcactgcta agagaaaact cataacactg agtgccttca 39420
   aaagaaacta aagagagcat acacttgcac attgaaagca cacctgaaag ctctagagca 39480
   aaagaagcaa atacacccaa gaggagtaga aagcaggaaa taatcaaact cgttactgaa 39540
   atcaaccaag tagaaaccaa aagaactata caaggaatca acaaaaccag gagatggttc 39600
   tttgagaaaa tcaacaagat agataaaccc ttagccagac taaccagata ctgaacagag 39660
   acagtatcct aattaacaaa accagaaatg aaaaggaaga catagcaaca aaaaccaaag 39720
   aaattaaata tatatatgat atatatatat atgatatata tatatatcat atcctactat 39780
   aaaccctata ctcaacaaaa ctggaaaatc tggatgaaat ggacaatttt ctagaaaggt 39840
   accaggtacc aaagttaaat cagggacaga taaataatct aaacggtccc ataaccccta 39900
   cagaaataga agccttcatt aaaagaaaga acactaccta attcattcta tgaagccaca 39960
   gttatgctta tacctaaacc aaacaaaaac ccaacaaaga cagagaacct caaaccaatt 40020
   tcacttatga atattgatgc aaaaatactg actaaaatgc tcacaaactg aatccaaaaa 40080
   gacattgaaa tgatcattca ccatgatcaa gtaggcttca tcctaaacat gaagggatgg 40140
   ttccatatat ggaaatccat caatgtaata cactatataa acaaactcaa agaaaaaaaa 40200
   gcaaatgatc atctccttag atgctaaaaa aagaatttga caaaattcaa ctccccttaa 40260
   tgttaaaagt cgtggaaaga tcaggaattc aaagcctata cctaaacata gtaaaagcgg 40320
   tatatggcaa accaggagcc atcatcaaac taaatgcaga gaaacaaagt aatcccacta 40380
   aaatccattc ttatcttcat gtacaaatct caagtcaaaa tgaaacaagc acctccataa 40440
   gaaaccagat acactgaatc taatagaaga gatagtgggt aaaattctca aacacataag 40500
   acaggaaaaa gatactgaaa agaacaccaa tggcttatgc tctacgacca aggactgaca 40560
   aatgggacct cataaaattg aaaagcttat ataaggcaaa ggacactgtc agtaagacaa 40620
   aacgggaacc aacagattgg gaaaagatat ttaccattcc ttcatcttat agagggttaa 40680
   tatccaatat gaacaaagaa ctcaagaagt tagaccccat agaaccaaaa aggtctttca 40740
   aaaaatgggg aacagagcta aacagagaat tctaaaccaa ggaagttcaa atggctgaga 40800
   agcacctaaa aaatgttcaa catccttagt ctttagggaa acacaaatca aaaccactct 40860
   gagattccac ctcataccag tcagaatggc taagatgaaa aactcaggtg acagcagatg 40920
   ctggtgaggc tgtagagaaa gagaaacact cctccattgc tggtgggatt gcaagttggt 40980
   acaaccactc tggaaatcag tgtggtgttt tctcagaaaa ctgaacatag tactacctaa 41040
   ggacccagct atatcactcc tgggcatata cccagaagat gttccaactt gtaataagga 41100
   cacatgctcc actatgttca taaaagcctt atttataata gctagaagtt tgaaagaacc 41160
   cagatgtccc tcaacatagg aatggataca gaaaatgtgg tacatttaca caatggaata 41220
   attagtagta actaaactat taaaagcaat gacttcatga atttcccagg caaatggatg 41280
   gaacttgaga atatcatcct gagtgaggtc actcagtcac ctaggaaaac aaacagtatg 41340
   tactcacaga taagtggata ttagcccaag agttcagaat acccaagatt caattcaaag 41400
   accatgtaaa acgtaagaag aaggaagatg aaaatgtgga tgtttcagta ctttttagaa 41460
   gggttcctat ttgttctgtt tgtcccatat ctagtccatt aaagaagccc actgtattaa 41520
   gcccttgcaa gttatggttt ccaattgtaa ttaaaaagaa gttcctgttc ttagatatga 41580
   tataaactgc aaaacatatt ttcaacctac actaaacttg aaacctcatt aatgtgatgt 41640
   atattcttct atttgttctt tctctttcat tgtatttttc ttttttagca tttttatgat 41700
   attttcttca tttacatttc aaatgctatc ctgaaggtcc cctataccct ccccctgccc 41760
   tgctccccta cccaaccact cccacttctt ggccctggca ttcccctgta ctggggcata 41820
   taaagtttgc aagaccaagg ggcctctttt cccaatgatg gctgactagg ccatcttctg 41880
   gtacatatgc agctagagac acttgctctg gagttgatgg ttagttcata ttgttgttcc 41940
   acctataggg ttgcagaccc cttcagctct tgggtacttt ctctacatcc tccactgggg 42000
   gcctgtgctc catccaatac atgactgtga gcatctactt ctgtatattc caggcactgg 42060
   catagcctta cactagacag ctatatcaga gtcctttcag caaaatcttg ctggcatgtg 42120
   caatattatc tgcgtttggt ggctgattat gggatggatc cccagttgtg gcagtctctg 42180
   gatggtctca actcaaaact ttgtctctgt aactccttcc atggaaattt tgttccctat 42240
   tataaagagg aatgaagtat ccacacattg gtcttccttc ttctggattt tcttgtgttt 42300
   tgcaaatttc agaatggcta agatcacaaa ttcaggtgac agcagatact agcgaggatg 42360
   tggagaaaga gggacactcc tccattgatg gtgggactgc aagcttgtaa agccattctg 42420
   gaaatcagtc tggcagttcc tcagaaaatt gaacatagta ttaccagaag atccagcaat 42480
   acctctcctg ggcatttacc cagaagatgt tccaactggt gataagaaca catgctccgc 42540
   tatgttcata gcagccttat ttataatagc cagaaactgg aaagaaccca gaggtccctc 42600
   aacagaggaa tgaatacaga aaatatgata catttacaca atggagtact actcagctat 42660
   taaaaacaat taatttataa aattcttagg caaatggatg aatctggagg atatcatcct 42720
   gagggaggta acccgctcac aaaagaactc acttgatatg cactaactga taagtggaca 42780
   ttagcccaga aatttagaat actcgtattt ttctaacaat agctaacagc cagcaatctc 42840
   acccttgtaa gcacttcctc cctcccataa aagggacttt aagaagcctt ctcaggctgt 42900
   tctctaaaac cctgacttag agggatacag ccaaatggca cccaaaacta aagcctgctt 42960
   cattcagaca gttgtggact tggtcttctc ctcacaattc taatgtttaa catacagtga 43020
   catccctagt aagatgtacc tggactgcca gctgagcaag tcagaggtaa caaggtggta 43080
   agtggcattc ttctattatc tctgcttcaa attctgcctt catttcctac cttgactctc 43140
   ctcaaacaca gatgatgacc tgtaagccag ataagccctt tcttcctcca gttgctttaa 43200
   aggcaggctg tttatcacag taacagaaaa tcaaactaca tgttgggccc agtcttctcc 43260
   atgctctcat ttcctctaca ctatggatca aaatttacct acctatttat accttcctga 43320
   gtgccagtct gctgtaagga acatctgagc cacactggta ctgtctttat aagagcagct 43380
   atgcctactt gtgaaggacc tttccctcct aacagaactg tatggagaag attattagaa 43440
   cctcacctga gcttccagcc actactccaa gccatttatg tgtagttttt ccataattac 43500
   aattagcatg aggagtctaa ggaagtgcta taatagcagc ttggagaggg taaatgacag 43560
   ggacaacacc tatgtggcca tgcttagtac agacacacca gcatgtctgc tgtaggatcg 43620
   ctggtattct tacccataca ttccattcat gttctgtaag gaagcataat aaactcactg 43680
   attctcccat ttttttttgg tgggttcaaa cttggactgg tcattgggtt ttgggagaca 43740
   tgccaatcca aggcttttcc tggaactcta atgtatgaag gaggaacata cttgacaaag 43800
   ctagtaatag tttagtgtct ggaccttggg ggaacttttt cctggggttc tgaccccagg 43860
   tatagacaag aaatgtattc cttctctaat accaggaata tgcttagcaa ggccagtaat 43920
   ggcctggggc tagggcttta aagaagttgt gatatttgga tcttaaaaac ccacctcttt 43980
   cccttttgat tggagccagc accgggtcac cttggacgca gagtcggcag atacccccaa 44040
   gttccctaga ggactctcca tgtgatctta ggacctctgg tgagtggaac acaacttctg 44100
   ttctaatcca attgtgcagg acctgagaac attaattagt gaagcagaaa acccagcctc 44160
   accagggtca caagtccctt ctggtcggtg ccacaccagg tcacattggg catggagtta 44220
   gcagacaccc ccaaggtgtc caaaagactc ttcaagtgat cttaggacct ctgctgagtg 44280
   gaacacaact tctgcaggaa gcaggttcga acccaagata tctgggcatc ttccctgcaa 44340
   gagaagagct tgcctgcaga gagtgctctg accactgaaa ctcaggagag aactagtctc 44400
   ccaggtctgc tgatagaggc taacagaatc acgagaggaa caagctctaa ccagagacaa 44460
   ctataacaac taactgaaga gaataccaga tggagaaagg caaacgtaag aatattacta 44520
   acagaaacca agactattca ccatcatcag aacccagcac tcccacctca cccagtcctg 44580
   ggcaccccaa aacacccgaa aagctagacc tggatttaaa agcatatctc aggatgatgg 44640
   tagagaacat aaagaaggac attaataact cacttaaaga aatacaggag aacactgcta 44700
   aagagttaca agtcctaaaa gaaaaacagg aaaacacaac caaaccggta gaagttcttg 44760
   aagaaaaaca ggaaaacaca accagacatg tgatggaaat gaacaaaaac atactaagaa 44820
   gggaagtaga cacaatagag aaaacccaaa gtgaggcaat gctgaagaca gaaaccctag 44880
   gaaagaaatc tggaaccata gaggcgagca tcagcaacag aatacaagag atggaagaga 44940
   gaatctcagg tgcagaagat ttcatagaga acataagcac aacaatcaaa gaaaatgcaa 45000
   aatgcaaaag gatcctaact caaaacatcc aggaaatcca ggacacaatg agaagaccaa 45060
   acctacagat aataggagta gatgggaatg aagattttca acttaaaggg ccagcaaata 45120
   tcttcaacaa aattttagaa gaaaacttcc caaacctaaa gaaagagatg cccatgaaca 45180
   tacaagaggc ccacagaact ccaaatagac tggaccagaa aagaaattcc tcccgacaca 45240
   taataatcaa aacaacaaat gcactaaata aagatagaat attaaaagca gcaagggaaa 45300
   aaggtcaggt aagagatata ggcaggccta tcagaattac accagatttt tcaccagaga 45360
   ctatgaaggc cagaagatcc tgaacagatg ttatacagac aaaaagaaca caaatgccag 45420
   ccaagcctac tatacccagc caaactctca attacaaaac caaattcaca cattaacttt 45480
   caaggaatcc agcccttcaa aggataataa caggaaaaaa aacaaacaaa aacaaaaaca 45540
   aaaaaaaaca aggacggaaa tcacacccta gaagaagcaa gaaagtaatc cctcaacaaa 45600
   ccaaaaagaa gacagccaca agaacagaat gccaactcta acaacaaaaa taataggaag 45660
   caacaattac ttttccttaa tatctcttaa tatcaatgga ctcaattccc caataagaag 45720
   acatagacta acagactgac tacacaaaca agacccaaca ttctgctgct tacaggaaac 45780
   ccatctcagg gaaaaagaca gacactacct cagagtgaaa ggctggaaaa caattttcca 45840
   agcaaatggc ctgaagaaac aagctggaat agccattcta atatcgaata aaatcgactt 45900
   ccaacccaaa gttatcaaaa aagacaagga gggacacttc atactcatca aaggtaaaat 45960
   cttccaggag taactctcaa ttctgtatat gtatgctcca aatgcaaggg cagccacata 46020
   cattaaagaa actttagtaa agctcaaagc acacattgca cctcccacaa taatagtggg 46080
   agacttcaac acaccacttt catcaatgga cagatcgtgg aagcagaaac taaacaggaa 46140
   cacagtgaaa ctaacagaag ttatgaaaca aatggacctg acagatatct acagaacatt 46200
   ttatcctaaa acaaaaggat ataccttctt ctgaacacct catggtacct tctccaaaat 46260
   tgagcatata attcatcaca aaacaggcct caacagatac aaaaatattg aaattgtccc 46320
   atgcatccta tcagactacc atggactaag gctgatcttc aataaaaaca taaataatag 46380
   aaatccaaca ttcacgtgga aactgaacaa cacacttctc aatgatacct tggtcaagga 46440
   aagaataagg aaagaaatta aagacttttt agagttcaat gaaaatgaaa ccacaacata 46500
   cccaaactta tgggacaaaa tgaaagcatt tctaagaggg aaacacatag ctctgagtgc 46560
   ctccaaaaag aaactagaga gagcacacac tagcggcttg acaacacaac taaaagctct 46620
   agaaataaaa aaggaagcaa attcactcaa gaggagtata cggcaggaaa taatcaaact 46680
   cagtggtgaa atcaaccaag tggaaataag aagaactatt caaagaatca accaaaggag 46740
   gagctggttc tttgataaaa tcaacaagat agataaaccc ttagccagac tcactagagg 46800
   gcacagggac agcatcctac ttaacaaaat cagaaatgaa aagggagaca taacaacaga 46860
   tcctgaagaa atccaaaaca ccatcagatc cttctacaaa aggctatact caacaaaact 46920
   ggaaaacctg gaagaaatgg acaaatttct agacagatac caggtacaat agttaaatca 46980
   ggatcaggtt aaggatctaa acagtcctat atcccctaaa gaaatagaag cagtcattaa 47040
   tagtctccca accaaaaaca gtccaggacc acatgggttt agggcagagc tctatcagac 47100
   cttcaaagaa gatctaattc cagttctgca caaactattc cacaaaatag aagcacaagg 47160
   tacgctaccc aattcattct atgaagccac aattactctg atacctaaat cacagacaga 47220
   tccaacaaag atagagaact ttcagaccaa tttcccttat gaatattgat gcaaaaatcc 47280
   tcaataaaat tctcgctaac cgaatgcaag aacccattaa aacaatcatc catcctgacc 47340
   aagtaggttt tattccaggg atgcagtaat ggtttaatat gcgaaaatcc atcaatgtaa 47400
   tccattatat aaacaaactc aaagacaaaa ccacatgatc atctcatttg agaaaatcca 47460
   acacccattc atgataaaag tcttggaaag atcaaaaatt caaggcccat acctaaacat 47520
   gacaaaagca atctatggca aaccagtagc caacatcaaa gtaagtggtg agaagctgga 47580
   agcaatccca ctaaaatcag ggactagaca aggctgccca ctttctccct acctattcaa 47640
   cattgtactt gaagtcctag tcagagcaat tcgacaaaaa aaaggagatc aaggggatac 47700
   aaattggaaa agaagcagtc aaaatatcac tttttgcaga tgatatgaca gtatatataa 47760
   gtgagcctaa aaattccacc agagaactcc taaacctgat aaatagcttc aatgaagtag 47820
   ctggatataa agtttactca aacaagtcaa tggcctttct ctacaccaag aataaacagg 47880
   ctgagaaaga aattagggaa acaacaccct tctcaatagt cacaaataat ataaaatatc 47940
   ttggcgtgac tctaactaag gaagtgaaag atctgtatga taaaaacttc aagtctctga 48000
   agaaagaaat taaagaagat ctcagaagat ggaaagatct cccatgctca tggattggca 48060
   gtatcaacac tgtaaaaatg gctatcttgc caaaagcaat ctacagattc agtgcaatcc 48120
   ccatcaaatt ccaactcaat tcttcaagga attataaaga gcaatctgca aattcacctg 48180
   gaataacaaa aaacctagga tagcaaaaac tcttctcaag gataaaagaa cctctggtgg 48240
   aatcaacatg cctgacctaa agctgtacta cagagcaatt gtgataaaaa ctgcatggta 48300
   ctggtatagc aacagacaag tagaccaatg gactagaatt gaagacccag aaatgaaacc 48360
   acatacctat ggtcacttga tctttgacat gggagctaaa accatccagt gaaaaaatga 48420
   cagcattttc aaaaaatggt gctcacacaa ctggctgtta tcatgtaaaa gaatgcaaat 48480
   tgatccattc ctatctcctt gtactaaggt caaaactaag tggatcaagg aacttcacat 48540
   aaaaccagag acactgaaac ttatagagga gaaagtgggg aaaaccttga agatatgggc 48600
   aaagggggaa aattcctgaa tagaacagca atggcttgtc ctgtaagatc aagaattgac 48660
   aaatggaacc tcatgaaact gcaaagcttt tgtaaggcaa aaggcaccgt caataagaca 48720
   aaaaggccac caacagactg ggaaaggatc tttacctatc ctaaatcaga taggagacta 48780
   atatccaata tgtagagagc acaagaacat ggactccaga aaatcaaata accccattaa 48840
   aaaatggtgc tcagggctaa aaaaagaatt ctcacctaag gaatactgaa tggctgagaa 48900
   gcacctgaaa acatgttcag catccttaat catcagggaa atgaaaatca gaacaacact 48960
   gagattcttc ttcacaccag tcagaatggc taagaaaaaa aattcaggtg acagcagatg 49020
   ctggcaagga tgtggagaaa gagaacactc ctccattgtt ggtgagattg aagcttatac 49080
   aaccactctg gaaatcagtc tggcagttcc tcagaaaact ggacatagta ctaccagagg 49140
   atcccacaat acctctcctg ggcatatatc cagaagatgt tccaactggt aagaaagaca 49200
   catgctccac tatgtccata gcagccttat ttataatagc cagaagctgg aaagaaccca 49260
   gatgcccctc agcagaggaa tgggtacaga aaatgtggta catttacata atggaatact 49320
   agtcagctat taaaaagaat gaatttatga aatttctagg caaatggatg gacctggagg 49380
   gcatcatcct gagtgaggta acacaaacac aaaagaactc acatgatatg tacacactga 49440
   taagtggata ttagcccaga aacttagaat acccaagata taagatacaa tttgtgaaac 49500
   acatgaaact caagaagaat gaagatcaaa gtgtgggcac tttgcccctt cttagaattg 49560
   ggagcaaaac acccatggaa ggagttacaa agacaaagtt tggagctgag acaaaaggat 49620
   ggaccatcta gagactgtca tatctgggga tccatcccat aatcagactc caaacactga 49680
   caccattgca tacactagca agattttgct aaaaggaccc agatatagct gtctcttttg 49740
   agactatgcc ggcgcctagc aagcacataa gtggatgctc acaatcagct attggatgga 49800
   tcacagggcc cccaatggag tagctagaga aagtacccaa ggagctaaag ggatatgcaa 49860
   ccctttaagt gaaacatcaa tataaactaa ccagtacccc actcccaccc cagagctcgt 49920
   atctatagct gcatatttag cagaagatgg cctagtcggc catcactgga aagagaggcc 49980
   cattggtcgt gcaaacttta tatgcctcag tacaggggaa cgccagggcc aagaattggg 50040
   agtgggtagg taggggagtg tgggggaggg tatgggggac ttttgggata acattagaaa 50100
   tgtaaatgag gaaaatacct aataaaaatg ttaaaaaaca aacaaacaaa caaacaactc 50160
   tcctgccact gaagggtttt agttatcagt cccaaggtca ctatgtgctt agtctttgag 50220
   tgcttgagat atcctctgtt ccctatgtgc aacatttctt gattataact ttgtctcatg 50280
   tatgatggat ttctgctgat tgacacattt ttccttgcaa actttatata atacacttta 50340
   attttttaat attttttatt acgtattttc ctcaattaca tttccaatgc tctcccaaaa 50400
   atcccccata ccctcccccc cacttcccta accacccatt cccatttttg gccctggcgt 50460
   tcccctgtac tggggcatat aaagtttgtg tgtccaatgg gcctctcttt ccagtgatgg 50520
   ccaactaggc catcttttga tacatatgca actatagtca ggagctgttt gtttatataa 50580
   tggattacat tgatggattt ccatagatta aaccatccct gcatccctgg aataaaacct 50640
   acttggtcag gattgatgat tgttttaaag tgttcttgga ttcggttagt gagaatttta 50700
   ttgagtattt ttttaattag gtattttcct cctttatatt ttcaaacgct gacaccattg 50760
   catacactag caagattttg ctgaaaggtc tcagatatag ctgtctcttg tgagactagg 50820
   ccgggcctag caaacacaga agtggatgct cacagtcagc tattggatgg atcacagggc 50880
   tccctatgga ggagctagag aaagtacaca aagagctaaa gggatctgca accctgtagg 50940
   tgcaacatta tgaactaacc agtaccaccg gagctcatgt ctctagctgc atatgtatca 51000
   gaagatggcc tagtcggcca tcactggaaa gagaggccca ttggacacac aaactttata 51060
   tgccccaata caggggaacg ccagggccaa aaaaatggga atgggtgggt cggagattgg 51120
   gggggtgggt ataagggacc tttgcgatag cattgaaaat gtaaactttg tctctgtaac 51180
   tcattccatg ggtgtttgtt cccaattcta aagaggggca tagagtccac acttcagtct 51240
   tcattcttga gtttcatgtg ttttgcaaat tgtatcttat atcttgggca tcctaagttt 51300
   ctgggctaat atccacttat cagtgagtac atattgtgtg agttcctttg tgattgggtt 51360
   acctcactca ggatgatgct ctccaggtcc aaccatttgc ctaggaattt cataaactca 51420
   ttctttttaa tagctgacta gtacttcatt gtgtaaatgt accacatttt ctgtatccat 51480
   tcctctgtta aggggcatct gggttctttc cagcttctgg ctattataaa taaggcttct 51540
   atgaacatag tggaacatgt gtccttctta acggttggga catcttctgg atatatgccc 51600
   aggagaggta ttgctggatc ttccggtagt attatgtcca attttctgag gaaccaccag 51660
   actgatttcc agagtggttg tacaagcttg caatcccatc tacagtagag gagtgttcct 51720
   ctttctccac atcctggcca gcatctgctt tcacctgaat tttggtcttt agccattctg 51780
   actggtgtga ggtggaatct cagggttgtt ttgatttgca tttccctgat gattaaggat 51840
   gctgaacatt ttttcaaatg cttctcttcc attcggtatt cctcaggtga gaattctttg 51900
   ttcaattctg agccccattt tttaatgggg ttatttgaaa ttctggagtc caccttcttg 51960
   ggttctttat atatattgga tattagtccc ctatctgatt taagataggt aaagatcctt 52020
   tcccaatctg ttggtggtct ttttgtctta ttgacggtgt cttttgcctt gcagaagctt 52080
   tgcagtttca tgaggtccca tttgtcaatt ctcaatctta ctgcacaagc cattgctgtt 52140
   ctattcagta attttcccct ttgcccatgt cttcaaggct ttcccccact ttctcctcta 52200
   taagtttcag tgtctggttt tatgtgaagt tccttgatcc acttagattt gaccttagta 52260
   caaggagata ggaatggatc aattcgcatt cttctacatg ataacaacca gttgtgccaa 52320
   caccatttgt tgaatatgct gtctttcttc cactggatgg ttttagctcc cttgtcgaat 52380
   atcaagtgac cataggtgtg tgggttcatt tctgggtctt caattctagt ccattggtct 52440
   acttgtctgt ctctatacca ctaccatgca gtttttatca caatctagat gcatgctcga 52500
   ggccatttca ttacctcttt ctccgcaccc gacatagata aagcttatcg ataccgtcga 52560
   cctcgagggg gggcccttgc tctgtagtaa agctttagat caggcatggt gattccacca 52620
   gaggttcttt tatccttgag aagagttttt gctatcctag gttttttgtt attccacatg 52680
   aatttgcaga ttgctctttc taattccttg aagaattgag ttggaatttg atggggattg 52740
   cattaaatct gtagattcct tttggcaaga cagccatttt tacaatgtta atcctgccaa 52800
   tccatgagca tgggagatct ttccatcttc tgagatcttc tttaatttct ttcttcagag 52860
   acttgaagtt tttatcatac agatctttca cttccttagt tagagtcacg ccaagatatt 52920
   ttatattatt tgtgactatt gagaagggtg ttgtttccct aatttctttc tcagcctgtt 52980
   tattcttggt gtagagaaag gccattgact tgtttgagtt agttttatat ccagctactt 53040
   cactgaagct gtttatcagg tttaggagtt ctctggtgga attttagggt cacttacata 53100
   tactatcaca tcctctgcaa aaagtgatat tttgacttca tcttttccaa tttgtatccc 53160
   cttgatctcg ttttgttgtc aaattgctct ggctaggact tcaagtataa tgttgaatag 53220
   gtatggagag agtggacagc cttgtctagt tcctgatttt agtgggattg cttcaagctt 53280
   ctcaacattt actttgatgt tggctactgg tttgctgtag attgctttta taatgtttag 53340
   gtatgggcct tgaattcctg atctttccaa gacttttatc atgaatgggt gttggatttg 53400
   tcgaatgctt tttccgcatc taacgagatg atcatgtggt ttttgtcttt gagtttgttt 53460
   atataatgga ttacgttgat ggatttccat atattaaacc atccctgcat ccctgaaatg 53520
   aaacctactt ggtcgggatg gatgattgct ttaatgtgtt cttggatttg gttagcgaga 53580
   attttattga ggatttttgc atctatattc ataagggaaa ttggtctgaa attctctatc 53640
   tttgttggat ctttctgtgg tttaggtatc agagtaatag tggcttcata gaatgagtat 53700
   ggtagagttc cttctacttc tattttgtgg aataatttgt gcagaactgg aattagatcg 53760
   tctttgaagg tctggtagaa ctctgcacta aaccctctga tccagggctt tttttggctg 53820
   ggagactatt aatgactgct tctatttctt taggggatat gggactgttt agatcattaa 53880
   cttgatcctg acttaacttt ggtaccttgt ttctgtctag aaatttgtcc atttcttcca 53940
   ggttttccag ttttgttgag tatagccttt tgtagaagga tctgatggtg ttttggattt 54000
   cttcaggatc tgttgttatg tctccctttt catttctgat tttgttaatt aggatgctgt 54060
   ccctttgccc tctagtgagt ctaggtaagg gtttatctat cttgttgatt ttatcaaaga 54120
   accaactcct cctttggtta attctttgaa tagttcttct tgtttccact tggttgattt 54180
   cacccctgag tttgattatt tcctgccgtc tactcctctt gggtgaattt tcttcctttt 54240
   tttctagggc ttttagatgt gttgtcaggc tgctagtgtg tgctctctcc agttactttt 54300
   tggaggcact cagagctatg agtttccctc ttagaaatgc tttcattctg tcccataggt 54360
   ttgggtatgt tgtggattca ttttcattaa gctctaaaga gtctttaatt tttttcttta 54420
   ttccttcatt gaccaagtta tcattgagaa gagtgatgtt cagtttccac gtgaatgttg 54480
   gctttccttt atttatgatg ttattgaaga tcagccttag gccatgatgg tctgatagga 54540
   tgcatgggac aatttcaata tttttgtatc tgttgaggcc tgttttgtga ccaattatat 54600
   ggtcaatttt ggagaaggtc ccgtgaggtg ctgtgaagaa ggtatatcct tttgttttag 54660
   aataaaatga tctgtagata tctgttaagt ccatttgttt catcacttct gttagtttca 54720
   ctgtgtcctt gtttagtttc tgtttccacg atctgtccat tgatgaaagt ggtgtgttga 54780
   agtctcccac tattattgtg tgaggtgcaa tgtgtgcttt gagctttact aaagtgtctt 54840
   taatgaatgt ggctgccctt gtatttggag catacatatt cagaattgag agttcctctt 54900
   ggaggatttt acctttgatg agtatgaagt gtccctcctt gtcttttttg ataactttgg 54960
   gttggaagtc gattttatcc gatattagaa tggctattcc agcttgttta ttcaggctat 55020
   ttgcttggaa aattgttttc cagcctttca ctctgaggta gtgtctgtct ttttccctga 55080
   gatgggtttc ctgtaagcag cagaatgttg ggtcttgttt gtgtagccag tctgttagtc 55140
   taagtctttt tattggggaa ttgagtctat tgatattaag agatattaag gaaaagtaat 55200
   tgttgcttcc ttttcttttt gttgttagat ttggcattct gttcttgtgg ctgtcttctt 55260
   tttggtttgt tgagggatta ctttctggct ttatctaggg tgtaatttct gtccttgtat 55320
   tggttttttt tctgttatta tcctttgaaa ggctggattc gtggaaagat aatgtgtgaa 55380
   tttggttttg tcgtggaata ctttggtttc tccatctatg gtgattgaga gtttggctgg 55440
   gtatcatagc ctggactggc atttgtgttc tcttagtgtc tgtataacat ctgtccagaa 55500
   tcttctggct ttcatagtct ctggtgaaaa gtctggtgta attctgatag gtctgccttt 55560
   atatgttact tgacctttct cccttactgc ttttaatatt ctctctttat ttagtgcatt 55620
   tgttgttctg attattatgt gtcgggagga atttcttttc tggtccagtc tatttggagt 55680
   tctgtaggtt tcttatatgt tcatgggcat gtcattcttt aggtttggga agttttcttc 55740
   tataattttg ttgaagatat ttgcaggccc tttaagttga aaatcttcat tctcatcaac 55800
   tcctattatc cgtaggtttg gtcttctcat tgtgtcctgg atttcctgga tgttttgagt 55860
   taggatcctt ttgcattttg tattttcttt gattgttgtg ccgatgttct ctatggaatc 55920
   ttctgcacct gagattctct cttccatctc ttgtattctg ttgctgatgc taacatctat 55980
   ggttccagat ttctttccta gggtttctat ctccagcatt gcctcacttt gggttttctt 56040
   tattgtgtct acttcccttt ttaggtctag tatggttttg ttcatttcca tcacctgttt 56100
   ggatgtgttt tcctgttttt ctataaggac ttctacctgt ttggttgagt tttcctgctt 56160
   ttctttaagg acttgtaact ctttagcagt gttctcctgt atttctttaa gtgagttatt 56220
   aaagttcttc ttgatgtcct ctaccatcat catgagatat gcttttaaat ctgggtctag 56280
   cttttcagtt gtgttggggt gcccaggacc aggtggggtg ggagtgctgc gttctgatgt 56340
   tggtgagtgg tcttgatttc tgttagtagg attcttgtgt ttgcctttcg ccatctggta 56400
   atctctgggg cctgtgcaga atacactcgg ttgggtcccg gaaccaagat ttctgctgct 56460
   gatgctcagg caaagcgctc cctctctgct tggaccccct tcctttggcc gggaaggtgg 56520
   ccagatgtct ggctacacta actttcttaa taaacttcct atttataagg aatcagctta 56580
   tgaaagatgt cacatcaaaa tgttgatact gccagactgt caggtgctgt ttaaacagag 56640
   aagctgaccc tgcacctgga atttgttcca ttccacttta aacatagtgt ttggttcaaa 56700
   aggtcctcac ctgggtctta atacctaaag agatgattgt tctactccag aacacacttt 56760
   tcccattgga caattaccct ccctttattt aaaaaatagc atgtctctct gcctttatta 56820
   cacacacaca cacacacaca cacacacaca cacacacaca cactttcctg tcttcattat 56880
   tttctcattt tagtccttct acttaggacc ctgtcaataa aaaaaaaaaa atggcctgat 56940
   ggtccagatc aattggaact ttataaaaaa ggagaggagg gaataaatgt tgttcatgac 57000
   tccctcaggg aaaaattcag tcgttgcaca tctgtcacag cttttgtagc tcttgttagc 57060
   tacagtatgc tagatgaagt cagccttgca tacataccct gcacacctac acatgcaagg 57120
   aataaaagag gctaaggtga accttagatc tggtttaaac accagttagt gggcttcagg 57180
   gcctccagag ggatgaactg cagactctca agatgttctg ggagctccac attcacacaa 57240
   aacaagtcaa tccctcttct ctgcttgctt ggtgacccag tggtagcttt tgtgttgcca 57300
   gaactttctt tgcatcggtt aagaattatt aggaagtgca ggatcaaggg caacagtaac 57360
   tatgatgtgt cccaaattct gccagtagag ggatgtgaat tatctcttgt caagctagag 57420
   aaaacctgag gatgaacagc tgagaaagga gtaaggcaga ctgcagtcag cagctacaga 57480
   cactgtacat catttttcag ttgactaata tacatcgatg cagctaagaa ggcagtgatc 57540
   ccaaggaagg ttattggcgt tccgaaaaat atatgagcac attaacagaa cagcccttac 57600
   cccaaactcc ttcagaataa actagtttca aacacagttg tctagcatgt aatcccatca 57660
   gtctcgtgga ggaagactct gagtgcatgg aacagagaat ctgtcagcag tgtaccctga 57720
   atctgaatat gaccttctgc tataggagaa aggaaccatc aaaaaagcac agtgataatg 57780
   atgtgggaac gcttcagtgt tcaggaacca tatgatttat ttaaaatata gaatcaaaag 57840
   taccaatttg cagttttgaa agatttattc cagtgtaagc attagcaatg caccaacatc 57900
   aggtgatttc tgaatccaac acgtcttatg tcctcatgat attaaaaaaa aaaaaaggcc 57960
   atccagaact gtgaacttga gttctacctt gttccctact gacattcaga ttttcttttt 58020
   tgcattctct ttatcttaca ggagacagga ggggagggct aactcatttt actttggctt 58080
   gtcccttgct ggtccttgcc cagaacgtaa agtagcttgc aagtcttcaa atctaaaaat 58140
   cttagtaact cctacacgag tggcaatgcc aaagagcagt gcaacaaaga ggaagtaaat 58200
   acgaccaaag agtattctta aatacaccac tggctcttgt ttttgtttta ttgtgtgcct 58260
   ttgaactgga ggggacccac tgtctatgct cccacttagt ccctcttctt tgcactctgg 58320
   aggcttccaa ccaaaatgac aatggcaatt ccgatgattg ttacacactc ctctaaaact 58380
   gcatttttct ggggtgcagt cataacccaa atgagataaa cttccactgc aagctccttg 58440
   atcacagaac ttacttttgg agcagggggt accatgtctc accattccag catctgttgt 58500
   ttctgtccca cgatgttcat caagcccaaa gcaggtaaac ccagagataa tcgattgatg 58560
   gaatgaaaca tgttcttgca aatatggaag attggtgaca ttggtacact gcaaccttcc 58620
   acacagcttg tcctgatcag cacaagcatt gaatgtgagg ctttcttctg ctctagtaca 58680
   atgcccaaat cgaaaccgtt gtttgttgat gtcatagcac ttaatattag cattcttagc 58740
   acttacacca aagatttcca tgcattgtat gttgcgatca gtgcagttac ctttatagca 58800
   gtaaccatct tctgagcatg gtgtcccatc ttgcagataa gtgtcatctg ggcaaatgta 58860
   tttagtccca ttacagtact ctggaagatc acatatgttc tggataggtc tgcagagtgt 58920
   cccagaagga ctgtaagtgc aatttgcaca gcataattct ttatcacaaa tgctaccagg 58980
   tgttaacctg caatcatttc cacagcaggg atctgaataa catgcctttt gggagccaca 59040
   gtcacactgc tcatcgttat ctactttgaa gtttccacaa aacttataag tcaatgatgt 59100
   attataataa acatgacggt catagaaaag acatggcatc agaccaggag tattaagtat 59160
   gttgcttatc tctgcaaggg aacaattgct gaaagcatct gttaattgag gatgtctgaa 59220
   cataatgcag gtgttccttc tctggcagac acagtacccc tcatcatatt ttaagcctaa 59280
   actccttcca acacgattgg ttattatagg agataaaaat aaaggatttc gatcatattt 59340
   accaatacaa attagggcta aggaagaaca tatactcctc tcagctggat taacctggtt 59400
   atcttgtggc ccatacttat taagtaaaac tcctgcatca ggcttaaatt tattataaaa 59460
   gactgacaca tagtaattat aagccgaccc tcctggaact gcaaactcaa gtcgaaatgg 59520
   atcagaattg gtgtacacag tcatgagaaa gacatagtac cgcatatgaa gattggtcag 59580
   ataggtgtcc attaaactaa tgacttgaaa caaataccca acagtagatg aaagtttgtc 59640
   acctgcagca gaattatata cagaattggt tgcttgaaag tggcctttta tagcagctgg 59700
   atgtgtagcg tagttcttac tagatattct gggagctcca tctgcatatt ccaatctgga 59760
   ggagggagaa cctgtattat ggctccagtg cttccatgca ttcataggcc ctgtgtcatc 59820
   agactcagat actatctgag aaacaaggtg ttcaaagctc tgtgaatcat tgaggggttt 59880
   gatttcatag gtaaggtcat ctaacttcat gacccctgac aggcccccat aacaagtatc 59940
   cacagtgacc atggattgtg ggatcccctc caggtagcca atatagtaac aatctacagg 60000
   aaaaaagggg taatccatct gtaaggctcc ttggtcatct tgagttgtca gcaacaagtg 60060
   tctgggccaa atgagtgtct ttctccgcag gtggatgata tgtctctggc cccgaaaatg 60120
   caagctatat gagagcagtc tttgtgcttg aagtcctttg gtatggtaga tctccttccg 60180
   aggaataacc acctccgatg agatgtaacg ccaagtagga tggccttgag aacaccagac 60240
   tggaaccagg aggagcagcc agagtgcaaa tagcaagagg aggaccctgg ggaccacagg 60300
   tctttccact agcctcatgc cccaggtcag agataacatc ctgggtggag ctaaatccct 60360
   ctgctgtggc cactgcctgg tctagaaaat actgacagag gactaaaaac ctcctcaggc 60420
   tcccaaccta agtggttacc cagacaactg gagttaggta acagtcactg ggtgtggcag 60480
   gaattgagtc tgaatgtgtt agctgaggtt gaggttaaat attgtcaaaa gggatgtcta 60540
   taaatgtgcc tggacaagaa aagtcagaag cagcaaggag tgtctctgac aggctcaatc 60600
   ctttcttttc tttttttgaa gttcaaaata tcatttccac gtgaatgtat ttggttccca 60660
   gtgtgactct gggtctcttt ctaggagtca atatttcttt atatcttggc tcatgtttct 60720
   cacagttgtt ctaatttctt gttttgtttt gtttgtttgt ttgaacgtta gtagtaaata 60780
   ctgtctatat tagcctttta gctataaatg attgttttta tttcttctaa tcatattttg 60840
   tttgagtttt ggttaaacta tttacaaatg agtttttttt ttttcctttt gggtgttgct 60900
   cgaaagtttg gagctttctg ttaatattgt gttgttattt ttccaatatt attagacctg 60960
   agaattctat ctgggtacct gtgaactcta gaatttttaa aaattccatc tcttgggaac 61020
   attacctctg accccgtctg aggccgaagt ggctgtcccc ctccaacctt tagtatcttt 61080
   ctttcctgac tattgggatt tcttcaagca atcaggctga tgggttctca gcagtgagac 61140
   cagtagactg ccggtatgaa cgtcgaagag actgccacac actccaggtt catcaacagt 61200
   gctttcgcgt ctcttacttt tgtagaagga aaagcagcct ctgagttatc tccaagaaat 61260
   cattaatgaa agagttaaaa gatgggtatc acccggagtt catgacaagc cctggctcag 61320
   acacgtgagc aaggtctaca gccccaaaga taggctgccc tgcaacatgt atttataaga 61380
   tagaagaaaa aaatgggtgg ttggagggtt gatcaactta cttcctctca aacatatata 61440
   tctcatctaa gtgtgcaggg gaaaactctg taggactact gggattgtta ttatcattat 61500
   tattattatt attattatta ttattattat tattattatt aacttaaggc attttattag 61560
   atattttctt catttagttt tcaaatgtta tccccggaac ctcctatact ctctccctgc 61620
   cctgctcccc aacccaccca ctcctacatc ctggccctgg cattccccta tactgtggca 61680
   gatgatcttc gtaagaccaa gagcctttcc tcccattgat ggcctactag gctatcctct 61740
   tttacatatg caactagagt cacagctctg gggaggtatt gcttagttca tattgttttt 61800
   cctcctatag ggttgcagat ccctttagct ccttgggtac tttctctagc tcctccattg 61860
   ggggccctgt gttccatcca atagatgact gtgagcatcc acttctgtat ttgccaggta 61920
   ttggcatgga tcttactgca ccttctgaac tctctaagca gctttcctgg tcacctccag 61980
   gagcctcatg aataagtctc tgcttccccc ttgtggctat gagcattact gcacctgata 62040
   caccctgcag cttcctaggg aagagggagg aagtggcttg gcccctgtct ggttaaggta 62100
   agaggagata aatcccttct catgaattag ggtgagaagg gtcatgtgct ctatcattgg 62160
   tgaccaactt ggggacatgg gcttatacag tcatcactct gaggctctgt gtaccaccag 62220
   actgaactcc catatcctac atgcacatag gacaacacca agtagaagga ggttttagga 62280
   ctaaactgaa ggacagagat ggggtttcta aacaactagg gagtgccagg gccagcctct 62340
   ctaaccacta taggacacta tggagtctgg ttacaaagag agattactca aggtccttag 62400
   cactgattac agagcatatc tcagatgcct tctgctgacc agatgtatct ttgcataatc 62460
   tgcctatcca gattcagaaa attgatgcca catagccaag tggactttca ggaacagacg 62520
   atttaaaaac aggcagagag atgtgagaga aaggagaagg agagagagaa gggagaggga 62580
   gagaagagag agggagacgg agaaggaaag agggagaagg agaaggagag aaggggcatg 62640
   gacagaggga gggacagaag gagagaggag atagagaggg ggataaggaa gaaaggaggg 62700
   agggagagag agagaaggct aagtctttcc atacctgggt cccaatacct cttataaccc 62760
   aagcacatgg tttcagatat cacaatgcgg ttgggatata gataactgta aatacttgtg 62820
   aaaataatgg ggctgagatc tggggttttc atgatagttt caaagtcact gtactgacta 62880
   aaaccttcca ctggcccatc tccagcttgt taatctgagg gtatcaaatt tcccactaag 62940
   tgtgtttaga aagatctcca cctttttgcc ctagtcttcc agtgccccac ctacgttctg 63000
   gtctcccaca tctgatgtct tctcagtgat tctggccctg cctgctccac agctacaaac 63060
   cccttcctat aatgagctct gtgctgagcc atcatcctga atcaatccac cttaagcaga 63120
   tgttttgctt atttttcctg tgtccatact acagaggaag ggtaggcatg tagaagctga 63180
   ggcatctcat ctcactctaa gcaccctcag tctctaaatg tgcccctttg tttccagcag 63240
   ttcagcctca agcatctttt attcactcgt cttagaggga cacatgtgct gtagtgttat 63300
   aagatgaaat ttaaagcatt agttattccc aacaagccaa ttaaacaagc caaaaacatt 63360
   catcagtcat tcccatggaa cctctgaagc atcttcctgc tctaaccttg agtttcctag 63420
   ggctgctgtg ggatcacagg agctgtcctg tttaccagcc tatcctgtcc cacgggattc 63480
   agttattagt gggtgcgagg gggaccgcaa acctggaaga aaatgggatt ggaagagaaa 63540
   agagaaacga agaccaagta gatcttttcc tatcaaggtc ttcgtttatt aggctgaggt 63600
   gcctggtgta aagcatgcat cgcggggaat aggaaggggt cgagggggaa ttttacaaag 63660
   aacaaagaag cgggcatctg ctgacatgag ggccgaagtc aggctccagg cagcggacac 63720
   tctgcatctt atctctggaa catagatcct ccttgacagc cttggggtgt caggccaggc 63780
   tcaggtgtaa ctcatgtcct tggatggcat gggagttcag gaagagatag ggaagagggg 63840
   actataattc agcttttaca gcctcaggtg ccaggaaggc aacagggagg agggagtgac 63900
   tacaggctcc tagcacgagg ccatttggcc tgtcagggtg ggagattgtg aagggctcac 63960
   tttctcatgg tatggtctct gacaccagcc agaaaaaaaa aaaatctcca gtcaatctac 64020
   agaaaggcag aaatatggag aaccttctag aagaacagca acccttgact gactctgcag 64080
   ggcagtctaa gcacacaggt tcctctgagc ccagtccctg gctgtattca ctcacacagg 64140
   gcccagcagt ctggagatgc ctgcagcagg aaggttctca ctgtacctca gtctcacagc 64200
   tcagtcactt tgtagtctcc ccacagcaaa tcacaaccaa tgcagttgtc acccttgagg 64260
   ccattcctca gtgacgcctg ggaaacactg tccatttgcc ctgctcatat gctgtctcag 64320
   ctcacataaa ttccctccaa gctcctttac agaaacaacc aattgacata caaattaaag 64380
   taatatgaag ctctgtcctg taccaattcc tagaacacct cagctgctaa aatctaactg 64440
   tgcaatacag agaactacct agataatacc taactccaat cttgaggtat ctgttgaaga 64500
   ggctttcagc tatgaattac caagagggat gcctgtgtct cctacatctt aaggcatctt 64560
   tctgaagctc acactaccct tcacagcgcc acagcagcag ggagatctgt atctcttcct 64620
   ctccacaaga gggcagaatt cgggtgtcaa ctgtgacaac cctccagcat taatttctat 64680
   tagtagatat tttctttact tacatttcaa atgttatccc cttccccagt ttttcctccc 64740
   tgaaaccccc tatcccatca cccatctccc tgctcaccaa cccacccatt ccataagaac 64800
   caatcagtac cccacccccc cagagctccc agggactaaa ccaggaacca aagagtacac 64860
   atggaaggac ccatagctcc agctgcatag gtagcactac tttgaaaagg gggaagatga 64920
   tgaaatcatt actgtggggg aaatgcaagg aagattccaa cacatctagg catctatgaa 64980
   gattttaagt cttcaaaatc caaaaccacc acaaaattta aaaaaaaaaa aagtagattc 65040
   taaatgcagt cacctgcacc aggtgcctgg ggaatcactc agcagcccta gactgagaaa 65100
   gcttggagaa agtagaaata gagaaagtgt acagccagta tcctctagct actcacatcc 65160
   aaacagggcc tcctgactgc tctgagcctg tcctaagaac agcaatgatg ccacagaaat 65220
   ttttagagtg agccctgaag gaacttgagg ctgatatgag caagccagtc ccagaggaaa 65280
   ggaaaaccca tagagagaaa acaggtgagt tagtgcatta aaggggctga gcagggagtt 65340
   ctcatcgctc cccagcacca gaaataagag cctctccgga gctgctggga catggaatgc 65400
   agatgattcg gaccatcagc cccacagaga cctttcccac tctggctcag aaagaggcac 65460
   tggaccacag ttggagagga gaatcgaaag ctgatatctc tgtattcact tagcctgtta 65520
   cccacccatg cacccaagtc caaggtggga gaaacactga gggtctaaac acagccccag 65580
   agcaactgcc agtattaaat accagatttc agaagatggg aaatcacctc tctggtcatt 65640
   tttgggacat gtaaactgta acaggaaaca taggaccaat ttaagatgga gcagtcctat 65700
   atccctaacc cagttgtaaa taaaacattc aagagtgcca tcagacacca cagtggtaca 65760
   ggagagatga gtgtacctag tgcatcaaga gttccctcac tagataaacc aagatgtagc 65820
   cccaggacca cccaggcacc taccaggact cccctccaga ggtctgagcc agttagctct 65880
   agttcattct atgtttcaga ccaaaacatc agaaacaaca gcatctccac tgcagatgaa 65940
   cccctaagcc atacagtgta cccaaaggca gcaccacaga tggggaatgt gggggcgaag 66000
   aagcttgtat cacattaaga gtgttgccca atgactattt gctatcttca cagtaagatg 66060
   ggcactgatc ccacccacca aaacatagta ggacaaggac ccttaaataa catctgtcaa 66120
   ggggagctgt caaatagcca ctgagctgag atggctcata cggggtgata tagaaaaaca 66180
   ggccaaagaa cctcctgtgt tgcaagcaca aatgggaagc tgtgaatctc cactacctta 66240
   caagaaaact gtaccaatct gcaagaacag gggatacaca gctttccaca ctgtgtagga 66300
   aggcattgga ctctcagatc atccaaggac tagggctaaa gtggccatgt gtgggaaata 66360
   catccacttt ataaacctac cttgtatcca catcacacat ggagcatctg ctcctggtcc 66420
   ctacctcatg tgggtgctgc ggtgctgtac attgagtctg ggggcatgag tgtgcccggt 66480
   aaattcctta tcactcagat gaatttccag tccacactca tcaccttgga gtaggaattt 66540
   taaaagttag tgtagatata agtaaagaga gtcaggaggg ggggctgaaa ggcagacctg 66600
   cagaaattct ccagagggta gagctcagaa gcagcaagca aaacttgggc taacagaaga 66660
   gcaaacacag agctcaggca gaactacctg gcaatgcgac tgggcacact gaaagcactg 66720
   ggcatcagca ctgagcccca aatatgcact caggatcctc tgcataataa tgtaacataa 66780
   caggaaggtt agaacaggcc aaaagaggaa acagaacaaa tgcccctaac caaagaagta 66840
   taaacaaatt gggaagagta aagaaggatt gtaaggattg agtaccacac agaacatgct 66900
   cttaatggcc tcaatgctga agctaggaag aactaagtta aaagaaacat gttcaacggg 66960
   attccctgtc actggacttc acaacaagca aaattcaatc tttctgttaa ggagatgaga 67020
   agagaatatc tgaaccttgt gttgacagtg ccccaccccg actgtcaggc tgtgggaaat 67080
   gccagagcaa tcactaggaa cacacaagga tgagggagac gagggttagg acacaaccat 67140
   catgatatcc cacaagtatg gaagagcaag aacttgtaga gagcagagaa tggcagacaa 67200
   agcagcatat acataagtag atggccagac tatacaggaa agatctacat agcctgtgag 67260
   gctttctgac agaaaagggc aggcatgtct caaagcacaa tgcctggctt gggacactgt 67320
   ctgccctgga tcctctcact ccacatgtaa ggagctcaca gcaaaaccac acagccttcc 67380
   acaagaggag aagaaaaggt agcttgtcag tgaggaagtc ccccagagac agaccattcc 67440
   agtagttctt atcattcctc ccaaagcagc caccatccag gcactgagag accaaaggct 67500
   gttgggaggt cagcctagag gcaggctcag acctttcttg ctccctagga ccttcctgaa 67560
   aagatagaag cacagaagtg aataatctgg acctcaactc aggatgacaa ctgaaactca 67620
   accgtgctgc ctgggccccc aatgctctct acacctgcag gcgcgcc               67667
<210> 5
   <211> 14781
   <212> DNA
   <213> Artificial Sequence
<220>
   <223> synthetic
<400> 5
   ggcgcgccga ccaggaaagc tgcttagaga gttcagaagg tgcagtaaga tccatgccaa 60
   tacctggcaa atacagaagt ggatgctcac agtcatctat tggatggaac acagggcccc 120
   caatggagga gctagagaaa gtacccaagg agctaaaggg atctgcaacc ctataggagg 180
   aaaaacaata tgaactaagc aatacctccc cagagctgtg actctagttg catatgtaaa 240
   agaggatagc ctagtaggcc atcaatggga ggaaaggctc ttggtcttac gaagatcatc 300
   tgccacagta taggggaatg ccagggccag gatgtaggag tgggtgggtt ggggagcagg 360
   gcagggagag agtataggag gttccgggga taacatttga aaactaaatg aagaaaatat 420
   ctaataaaat gccttaagtt aataataata ataataataa taataataat aataataata 480
   ataatgataa taacaatccc agtagtccta cagagttttc ccctgcacac ttagatgaga 540
   tatatatgtt tgagaggaag taagttgatc aaccctccaa ccacccattt ttttcttcta 600
   tcttataaat acatgttgca gggcagccta tctttggggc tgtagacctt gctcacgtgt 660
   ctgagccagg gcttgtcatg aactccgggt gatacccatc ttttaactct ttcattaatg 720
   atttcttgga gataactcag aggctgcttt tccttctaca aaagtaagag acgcgaaagc 780
   actgttgatg aacctggagt gtgtggcagt ctcttcgacg ttcataccgg cagtctactg 840
   gtctcactgc tgagaaccca tcagcctgat tgcttgaaga aatcccaata gtcaggaaag 900
   aaagatacta aaggttggag ggggacagcc acttcggcct cagacggggt cagaggtaat 960
   gttcccaaga gatggaattt ttaaaaattc tagagttcac aggtacccag atagaattct 1020
   caggtctaat aatattggaa aaataacaac acaatattaa cagaaagctc caaactttcg 1080
   agcaacaccc aaaaggaaaa aaaaaaaact catttgtaaa tagtttaacc aaaactcaaa 1140
   caaaatatga ttagaagaaa taaaaacaat catttatagc taaaaggcta atatagacag 1200
   tatttactac taacgttcaa acaaacaaac aaaacaaaac aagaaattag aacaactgtg 1260
   agaaacatga gccaagatat aaagaaatat tgactcctag aaagagaccc agagtcacac 1320
   tgggaaccaa atacattcac gtggaaatga tattttgaac ttcaaaaaaa gaaaagaaag 1380
   gattgagcct gtcagagaca ctccttgctg cttctgactt ttcttgtcca ggcacattta 1440
   tagacatccc ttttgacaat atttaacctc aacctcagct aacacattca gactcaattc 1500
   ctgccacacc cagtgactgt tacctaactc cagttgtctg ggtaaccact taggttggga 1560
   gcctgaggag gtttttagtc ctctgtcagt attttctaga ccaggcagtg gccacagcag 1620
   agggatttag ctccacccag gatgttatct ctgacctggg gcatgaggct agtggaaaga 1680
   cctgtggtcc ccagggtcct cctcttgcta tttgcactct ggctgctcct cctggttcca 1740
   gtctggtgtt ctcaaggcca tcctacttgg cgttacatct catcggaggt ggttattcct 1800
   cggaaggaga tctaccatac caaaggactt caagcacaaa gactgctctc atatagcttg 1860
   cattttcggg gccagagaca tatcatccac ctgcggagaa agacactcat ttggcccaga 1920
   cacttgttgc tgacaactca agatgaccaa ggagccttac agatggatta cccctttttt 1980
   cctgtagatt gttactatat tggctacctg gaggggatcc cacaatccat ggtcactgtg 2040
   gatacttgtt atgggggcct gtcaggggtc atgaagttag atgaccttac ctatgaaatc 2100
   aaacccctca atgattcaca gagctttgaa caccttgttt ctcagatagt atctgagtct 2160
   gatgacacag ggcctatgaa tgcatggaag cactggagcc ataatacagg ttctccctcc 2220
   tccagattgg aatatgcaga tggagctccc agaatatcta gtaagaacta cgctacacat 2280
   ccagctgcta taaaaggcca ctttcaagca accaattctg tatataattc tgctgcaggt 2340
   gacaaacttt catctactgt tgggtatttg tttcaagtca ttagtttaat ggacacctat 2400
   ctgaccaatc ttcatatgcg gtactatgtc tttctcatga ctgtgtacac caattctgat 2460
   ccatttcgac ttgagtttgc agttccagga gggtcggctt ataattacta tgtgtcagtc 2520
   ttttataata aatttaagcc tgatgcagga gttttactta ataagtatgg gccacaagat 2580
   aaccaggtta atccagctga gaggagtata tgttcttcct tagccctaat ttgtattggt 2640
   aaatatgatc gaaatccttt atttttatct cctataataa ccaatcgtgt tggaaggagt 2700
   ttaggcttaa aatatgatga ggggtactgt gtctgccaga gaaggaacac ctgcattatg 2760
   ttcagacatc ctcaattaac agatgctttc agcaattgtt cccttgcaga gataagcaac 2820
   atacttaata ctcctggtct gatgccatgt cttttctatg accgtcatgt ttattataat 2880
   acatcattga cttataagtt ttgtggaaac ttcaaagtag ataacgatga gcagtgtgac 2940
   tgtggctccc aaaaggcatg ttattcagat ccctgctgtg gaaatgattg caggttaaca 3000
   cctggtagca tttgtgataa agaattatgc tgtgcaaatt gcacttacag tccttctggg 3060
   acactctgca gacctatcca gaacatatgt gatcttccag agtactgtaa tgggactaaa 3120
   tacatttgcc cagatgacac ttatctgcaa gatgggacac catgctcaga agatggttac 3180
   tgctataaag gtaactgcac tgatcgcaac atacaatgca tggaaatctt tggtgtaagt 3240
   gctaagaatg ctaatattaa gtgctatgac atcaacaaac aacggtttcg atttgggcat 3300
   tgtactagag cagaagaaag cctcacattc aatgcttgtg ctgatcagga caagctgtgt 3360
   ggaaggttgc agtgtaccaa tgtcaccaat cttccatatt tgcaagaaca tgtttcattc 3420
   catcaatcga ttatctctgg gtttacctgc tttgggcttg atgaacatcg tgggacagaa 3480
   acaacagatg ctggaatggt gagacatggt accccctgct ccaaaagtaa gttctgtgat 3540
   caaggagctt gcagtggaag tttatctcat ttgggttatg actgcacccc agaaaaatgc 3600
   agttttagag gagtgtgtaa caatcatcgg aattgccatt gtcattttgg ttggaagcct 3660
   ccagagtgca aagaagaggg actaagtggg agcatagaca gtgggtcccc tccagttcaa 3720
   aggcacacaa taaaacaaaa acaagagcca gtggtgtatt taagaatact ctttggtcgt 3780
   atttacttcc tctttgttgc actgctcttt ggcattgcca ctcgtgtagg agttactaag 3840
   atttttagat ttgaagactt gcaagctact ttacgttctg ggcaaggacc agcaagggac 3900
   aagccaaagt aaaatgagtt agccctcccc tcctgtctcc tgtaagataa agagaatgca 3960
   aaaaagaaaa tctgaatgtc agtagggaac aaggtagaac tcaagttcac agttctggat 4020
   ggcctttttt tttttttaat atcatgagga cataagacgt gttggattca gaaatcacct 4080
   gatgttggtg cattgctaat gcttacactg gaataaatct ttcaaaactg caaattggta 4140
   cttttgattc tatattttaa ataaatcata tggttcctga acactgaagc gttcccacat 4200
   cgcgatcgcg gtaccgaagt tcctattccg aagttcctat tctctagaaa gtataggaac 4260
   ttctcgcgcg tctggcctcc gaggcctccg cgccgggttt tggcgcctcc cgcgggcgcc 4320
   cccctcctca cggcgagcgc tgccacgtca gacgaagggc gcagcgagcg tcctgatcct 4380
   tccgcccgga cgctcaggac agcggcccgc tgctcataag actcggcctt agaaccccag 4440
   tatcagcaga aggacatttt aggacgggac ttgggtgact ctagggcact ggttttcttt 4500
   ccagagagcg gaacaggcga ggaaaagtag tcccttctcg gcgattctgc ggagggatct 4560
   ccgtggggcg gtgaacgccg atgattatat aaggacgcgc cgggtgtggc acagctagtt 4620
   ccgtcgcagc cgggatttgg gtcgcggttc ttgtttgtgg atcgctgtga tcgtcacttg 4680
   gtgagtagcg ggctgctggg ctggccgggg ctttcgtggc cgccgggccg ctcggtggga 4740
   cggaagcgtg tggagagacc gccaagggct gtagtctggg tccgcgagca aggttgccct 4800
   gaactggggg ttggggggag cgcagcaaaa tggcggctgt tcccgagtct tgaatggaag 4860
   acgcttgtga ggcgggctgt gaggtcgttg aaacaaggtg gggggcatgg tgggcggcaa 4920
   gaacccaagg tcttgaggcc ttcgctaatg cgggaaagct cttattcggg tgagatgggc 4980
   tggggcacca tctggggacc ctgacgtgaa gtttgtcact gactggagaa ctcggtttgt 5040
   cgtctgttgc gggggcggca gttatggcgg tgccgttggg cagtgcaccc gtacctttgg 5100
   gagcgcgcgc cctcgtcgtg tcgtgacgtc acccgttctg ttggcttata atgcagggtg 5160
   gggccacctg ccggtaggtg tgcggtaggc ttttctccgt cgcaggacgc agggttcggg 5220
   cctagggtag gctctcctga atcgacaggc gccggacctc tggtgagggg agggataagt 5280
   gaggcgtcag tttctttggt cggttttatg tacctatctt cttaagtagc tgaagctccg 5340
   gttttgaact atgcgctcgg ggttggcgag tgtgttttgt gaagtttttt aggcaccttt 5400
   tgaaatgtaa tcatttgggt caatatgtaa ttttcagtgt tagactagta aattgtccgc 5460
   taaattctgg ccgtttttgg cttttttgtt agacgtcgag ctctagattg ggaacccggg 5520
   tctctcgaat tgttgacaat taatcatcgg catagtatat cggcatagta taatacgaca 5580
   aggtgaggaa ctaaaccatc caccatgatt gaacaagatg gattgcacgc aggttctccg 5640
   gccgcttggg tggagaggct attcggctat gactgggcac aacagacaat cggctgctct 5700
   gatgccgccg tgttccggct gtcagcgcag gggcgcccgg ttctttttgt caagaccgac 5760
   ctgtccggtg ccctgaatga actgcaggac gaggcagcgc ggctatcgtg gctggccacg 5820
   acgggcgttc cttgcgcagc tgtgctcgac gttgtcactg aagcgggaag ggactggctg 5880
   ctattgggcg aagtgccggg gcaggatctc ctgtcatctc accttgctcc tgccgagaaa 5940
   gtatccatca tggctgatgc aatgcggcgg ctgcatacgc ttgatccggc tacctgccca 6000
   ttcgaccacc aagcgaaaca tcgcatcgag cgagcacgta ctcggatgga agccggtctt 6060
   gtcgatcagg atgatctgga cgaagagcat caggggctcg cgccagccga actgttcgcc 6120
   aggctcaagg cgcgcatgcc cgacggcgag gatctcgtcg tgacccatgg cgatgcctgc 6180
   ttgccgaata tcatggtgga aaatggccgc ttttctggat tcatcgactg tggccggctg 6240
   ggtgtggcgg accgctatca ggacatagcg ttggctaccc gtgatattgc tgaagagctt 6300
   ggcggcgaat gggctgaccg cttcctcgtg ctttacggta tcgccgctcc cgattcgcag 6360
   cgcatcgcct tctatcgcct tcttgacgag ttcttctgaa gatccgctgt aagtctgcag 6420
   aaattgatga tctattaaac aataaagatg tccactaaaa tggaagtttt tcctgtcata 6480
   ctttgttaag aagggtgaga acagagtacc tacattttga atggaaggat tggagctacg 6540
   ggggtggggg tggggtggga ttagataaat gcctgctctt tactgaaggc tctttactat 6600
   tgctttatga taatgtttca tagttggata tcataattta aacaagcaaa accaaattaa 6660
   gggccagctc attcctccca ctcatgatct atagatctat agatctctcg tgggatcatt 6720
   gtttttctct tgattcccac tttgtggttc taagtactgt ggtttccaaa tgtgtcagtt 6780
   tcatagcctg aagaacgaga tcagcagcct ctgttccaca tacacttcat tctcagtatt 6840
   gttttgccaa gttctaattc catcagacct cgacctgcag cccctagaga agttcctatt 6900
   ccgaagttcc tattctctag aaagtatagg aacttcctag ggtttcaccg gtgcgatcgc 6960
   cctcagcaca ataatagaga cactgaaact tatagaggag aaagtgggga agatcctcga 7020
   agatagggtt cagggggaaa gatttttgaa cagaacacca atggcttgtg ctgtaaaatg 7080
   aagaattagc aaatgggacc tcataaaatt aaaaaggttc tgtaaggcaa aggacacaaa 7140
   aaggcagcca acatattggg aaagatcttt accaatccta aatatgacag agggctaata 7200
   tccaatatat agaaagaatt caagacgtta gactccagaa aaccaaataa ccctattaaa 7260
   aaaacctgag tacagagcta aacagtgaat tctcaactga agaatactga atggctgaga 7320
   aacacttaaa atttttttca atatcactag tcatcaggga aatgccaaac aaccctgata 7380
   ttccacctca cacctgtctg aatcactaag aacaaattga ggtgacagca gatgttggtg 7440
   aggatgtgga ggaaggggaa catttctcca ttgctggtgg aattgcaagc tggtacaacc 7500
   actctgtaaa tcagtttggc agttcctcag aaaattggac atagtactat cagaggatcc 7560
   agcaatagca ctcctgggca tatacccaga agatgctcca acatgtaata aggacacatt 7620
   ctccactatt tccatagcag ccttatattt aatgtccaga agctttaaag aacccagatg 7680
   tccctcaaga gaacaatggg tacagaaaat gtggtacatt tacacaatgg agtactcctc 7740
   agctattaaa aacaatgaat tcattaaatt cttagacaaa tggatggttc tggaggatat 7800
   catcctgagt gaggtaactc aatcacataa gaacacacat gatattcact cactgataag 7860
   tggatattag cccagaagtt cggaatatca aagatacaat tcacaaaaca catgaatttc 7920
   aagaaggaag accaaagtat gaatacttcg gtccttctta gagggaggaa taaaatagtc 7980
   atggaaggag ttaaagagac aaagtgtgga acagagactg aaggaatgac catccagaga 8040
   ctgccccttt ggggatccat cccatagaca accaccagac acttttgtgg ataccaacaa 8100
   gagcttgctg ccaggatctt gatatagctg tcttctgaga ggctctgcca gtgcctggca 8160
   aatacagaag tggatgctca cagtcatcca ttggacttcg cacaaggtcc ccaatgaggc 8220
   agccagagaa aggacccaag aagttgaagg gttttgtaac cccataggga gaacaacaat 8280
   atgaaccaat cagtactcca agagctccct ggcatgaaac caccaacaaa agaaaacatg 8340
   gtgggactca tagatctagc tgcatttgta gcagagaatg gactagttgg tcatcaatgc 8400
   aaggagagta ccttggtcct gtgaaggctc tatgcccaaa tataggggat tgccagggcc 8460
   tggaagtggg agtgggtgga ttggtgagca gggggagggg gaggagatag gggatttcag 8520
   agggtaaacc aggaaagggg ataacatttg aaatgtcaat aaagacaata tctaataaaa 8580
   aatgtcattt agaaaaaaaa aagtaacgct gtgtttgctt tatgcagcaa aatgctgtgt 8640
   cctgtttatg tattcagtct gttagtctat gtctttttat tggagaattg agtccattga 8700
   tgttaaaaga gatattaagg aatagtgatt gttgcctact ttatttttgt tgttagaggt 8760
   ggaattatgt ttgtgtggtt atcttctttt gagtgtgttg aaagatgatt actttagtgc 8820
   tctttctagg gtgtagtttt cctacctgtg ttggagtttt ccatctgcta tcctttgtag 8880
   ggctggattt atggaaagat attgtgtaaa tttggacttg taatggaata tcttggtttc 8940
   ttcatcgatg gtaattgaga gttttgctgg gtatagtagc ctgggctggc atttgtgttc 9000
   tcttagtttc tgtataacat ctgtccagga tattctggct tttatagtct atggtgagaa 9060
   gtctggtgta attctgatag gtctgccttt atatggctgg tagacaggac agctcctgtg 9120
   atcccagagc agccctggaa aacccaaggt tagagcagga agatgcttca gaggttccat 9180
   gggaatgact gatgaatgtt tttggcttgt ttaattggct tgttaggaat aattaatgct 9240
   ttaaatttca tcttataaca ctacagcaca tgtggccctc taagatgaat gaataaaaga 9300
   tgcttgaggt tgcacttctg gaaacaaggg ggcacattta gagactgagg gtgcttggaa 9360
   tgaggtgaga tgcttcagct tctacatgcc tacctttcct ctgtagtatg gacacaggaa 9420
   aaataagcaa aacatctgct taaggtggat tgattcagga tgatggctca gcacagagct 9480
   cattatagga aggggtttgt agctgtggag caggcagggc cagaatcact gagaagacat 9540
   cagatgtggg agaccagaac gtaggtgggg cactggaaga caagggcaaa aaggtggaga 9600
   tctttctaaa cacacttagt gggaaatttg ataccctcag attaggaagc tggagatggg 9660
   ccagtggaag gttttagtca gtacggtgac tttgaaacta tcatgaaaac cccagatctc 9720
   agccccatta ttttcacaag tatttacagt tatctatatc ccaaccgcat tgtgatatgt 9780
   gaaaccatgt gcttgggtta taagaggtat tgggacccag gtatggaaag acttagcctt 9840
   ctctctctct ccctccctcc cttcttcctt atccccctct ctatctcctc tctccttctg 9900
   tccctccctc tgtccatgcc ccttctctcc ttctccttct ccctctttcc ttctccgtct 9960
   ccctctctct tctctccctc tcccttctct ctctccttct cctttctctc acatctctct 10020
   gcctgttttt aaatcgtctg ttcctgaaag tccacttggc tatgtggcat caattttctg 10080
   aatctggata ggcagattat gcaaagatac atctggtcag cagaaggcat ctgagatatg 10140
   ctctgtaatc agtgctaagg accttgagta atctctcttt gtaaccagac tccacagtgt 10200
   cctatagtgg ttagagaggc tggccctggc actccctagt tgtttagaaa ccgcatctct 10260
   gtccttcagt ttagtcctaa aacctccttc tgcttggtgt tgtcctatgt ccatgtagga 10320
   tatgggagtt cagcctggtg gtacacagaa cctcagagtg atgactttgg tataaaccca 10380
   tgtcccaact gggtcaccaa tgatagagca catgacccct ctcaccctaa tcaatgagaa 10440
   gggatttatc tcctcttacc ttaaccagac aggggccaag tcacttcctc cctcttccct 10500
   aggaagctgc aggggtatca ggtgcagtaa tgctcatggc tacaaggcag aagcagagac 10560
   ttattcatga ggctcctggg ggtgagcagg tatcccagta gttctacaga gttttcccct 10620
   gcacacttag atgagatata tatgtttgag aggaagtaag ttgatcaacc ctccaactac 10680
   ccattttttt ctcctatctt ataaatacat gttgcagggc agcctatctt tggggctgta 10740
   gaccttgctc acgtgtctga gccagggctt gtcatgaact ccgggtgata cccatctttt 10800
   caccctttca tcaatgattt cttggagaaa actcagaggc tgcatttcct tctacaaaag 10860
   taagagacgc gaaagcactg ttgatgaacc cggagtgtgt ggcagactct tcgacgttca 10920
   taccgacagt ctactggtct cactgctgag aacccatcag cctgattgct tgaagaaatc 10980
   ccaatagtca ggaaagaaag atactaaagg ttggaggggg acagccactt cggcctcaga 11040
   cggggtcaga gataatgttc ccaagagatg gaatttttaa aaattctgga gttcacaggt 11100
   acccaggtag aattctcagg tctaataata ttggagaaac aacaacacaa tattaacaga 11160
   aagctccaaa ctttcgagca acacccaaaa ggaaaaaaaa aactcatttg taaatagttt 11220
   aaccaaaact caaacaaaac atgattagaa gaaataaaaa caatcattta tagctaaaag 11280
   gctaatatag acagtattta cttctaactt tcaaacaaac aaacaaacaa aacaaaacaa 11340
   gaagttagaa caactgtgaa aaacatgagc caagatataa agaaatattg actcctagaa 11400
   agagacccag agtcacactg ggaaccaaat acattcacgt ggaaatgata ttttgaactt 11460
   caaaaaaaga aaagaaagga ttgagcctgt cagagacact ccttgctgct tctgactttt 11520
   cttgtccagg cacatttata gacatccctt ttgacaatat ttaacctcaa cctcagctaa 11580
   cacattcaga ctcaattcct gccacaccca gtgactgtta cctaactcca gttgtctggg 11640
   taaccactta ggttgggagc ctgaggaggt ttttagtcct ctgtcagtat tttctagacc 11700
   aggcagtggc cacagcagag ggagttagct ccacccagga tgttatctct gacctggggc 11760
   atgaggctag tggaaagacc tgtggtcccc agggtcctcc tcttgctatt tgcactctgg 11820
   ctgctcctcc tggttccagt ctggtgttct caaggccatc ccacttggcg ttacatctca 11880
   tcggaggtgg ttattcctcg gaaggagatc taccatacca aaggacttca agcacaaaga 11940
   ctgctctcgt atagcttgcg ttttcggggc cagagacata tcatccacct gcggagaaag 12000
   acactcattt ggcccagaca cttgttgctg acaactcaag atgaccaagg agccttacag 12060
   atggagtacc ccttttttcc tgtagattgt tactatattg gctacctgga ggggatcctg 12120
   caatccatgg tcactgtgga tacttgttat gggggcctgt caggggtcat aaagttggat 12180
   aaccttacct atgaaatcaa acccctcaat gattcacaga gctttgaaca ccttgtttct 12240
   cagatagtat ctgagtctga tgacacaggg cctatgaatg catggaagca ctggagccat 12300
   aatacaggtt ctccctcctc cagattggaa tatgcagatg gagctcccag actatctagt 12360
   aagaattacg ctacacatcc agctgctata aaaggccact ttcaagcaac ccattctgta 12420
   tatagtgctt ctggaggtga caaactttca tctactgttg agtatttgtt taaagtcatt 12480
   agtttaatgg acacctatct gaccaatctt catatgcggt actatgtctt tctcatgact 12540
   gtgtataccg aggctgatcc attttcacaa gattttcgag ttccaggagg gcaggctcat 12600
   actttctatg agagagtatt ttatgctcat tttaggcctg atgcaggagc tataattaac 12660
   aagaattcgc caggagatga tgctgttaat ccagctgaga ggagtatatg ttctccctca 12720
   gccctaattt gtcttggtca acatggtcga aatcctttat ttttatctat tataataacc 12780
   aatcgtgttg gaaggagttt aggcctaaaa catgatgagg ggtactgtat ctgccagaga 12840
   aggaacacct gcatcatgtt caaaaatcct caattaacag atgctttcag caattgttcc 12900
   cttgcagaga taagcaacat acttaatact cctgatctga tgccatgtct tttctatgac 12960
   cgtcatgttt attataatac atcattgact tataagtttt gtggaaactt caaagtagat 13020
   aacaatgagc agtgtgactg tggctcccaa aaggcatgtt attcagatcc ctgctgtgga 13080
   aatgattgca ggttaacacc tggtagcatt tgtgataaag aattatgctg tgcaaattgc 13140
   acttacagtc cttctgggac actctgcaga cctatccaga acatatgtga tcttccagag 13200
   tactgtagtg gctctaagtt catttgccca gatgacactt atctgcaaga tgggacacca 13260
   tgctcagaag agggttactg ctataaaggt aactgcactg atcgcaacat acaatgcatg 13320
   gaaatctttg gtgtaagtgc taagaatgct aatattaagt gctatgacat caacaaacaa 13380
   cggtttcgat ttgggcattg tactagagca gaagaaagcc tcacattcaa tgcttgtgct 13440
   gatcaggaca agctgtgtgg aaggttgcag tgtaccaatg tcaccaatct tccatttttg 13500
   caagaacatg tttcattcca tcaatcggtt atctctgggg ttacctgctt tgggcttgat 13560
   gaacatcgtg ggacagaaac agcagatgct ggattggtga gacatggtac cccgtgttca 13620
   aggggtaagt tctgtgatcg aggagcttgc aatggaagtt tatctcgttt gggttatgac 13680
   tgcaccccag aaaaatgcaa tttcagagga gtgtgtaaca atcgtcggaa ttgccattgc 13740
   cattttggtt ggagccctcc aaagtgcaaa gaagagggac acagtgggag catagacagt 13800
   gggtcccctc cggttcaaag gcgcataata aaacagaacc tagagccagt agtgtattta 13860
   agaatactct ttggtcgtat ttacttcctc tttgttgcac tgctctttgg cattgccact 13920
   cgtgtaggag ttactaagat atttaggttt gaagacttgc aagctgcttt acgttcttgg 13980
   caagaacaag caaaggacaa gtaaaatgag ttagccccct gtctcctcta agataaagag 14040
   aatggaaaaa aaatctgaat gtcattaggg gacaaggtag aactccagtc cacagttctg 14100
   gatggccttt gtttatatca tgggacataa gacgtgttgg actcggcacc acctgatgtt 14160
   gttgcattgc tgaaataaat ctttcaaaat tgcaaactgg tacttttgat tctatatttt 14220
   aaataaatca ttagaaactg ctctacgttt gctcctcggg tagcaggaga ccttgagcca 14280
   ccattggttc ttagccttag gcctctggtc tccgtattaa tggagatttg tctatatctc 14340
   ttttaagaag ttatttacga tatcttttta aaatatggtt cctgaaccct gatgcgttcc 14400
   cacctctttg tcactgtgtt ttttgatggt tcccatctcc tatagtttgt gatgggattt 14460
   tcaggatcat tccttagagg aatagatgga gtgaagaaag acttaggacc cagttgagga 14520
   agcgttacag gaacatagaa tcatcacctc attattaagc cagagaactg atatgtagaa 14580
   catcaccatt aacacaaagc catggcttac tagatatact actcacaagc tcatggaagc 14640
   atgctctcca gatttccact gacagggcac tgcacagaac acagtcttga gaaaggacat 14700
   ggtgattctc gtggaggcag accctgagtg cactgaacag agaatctgtc atccctcgct 14760
   accttaggac cgttatagtt a                                           14781

## Description

### FIELD OF INVENTION

Genetically modified non-human fertile animals that express human immunoglobulin-like binding proteins comprising an immunoglobulin heavy chain constant region fused with an immunoglobulin light chain variable domain are described, as well as binding proteins having an immunoglobulin light chain variable domain fused to a light chain constant domain and an immunoglobulin light chain variable domain fused to a heavy chain constant domain. Genetically modified mice, cells, embryos, and tissues that comprise a nucleic acid sequence that encodes a functional ADAM6 protein are described, wherein the mice, cells, embryos, and tissues comprise human immunoglobulin light chain gene segments operably linked to one or more non-human immunoglobulin heavy chain constant genes. Modifications include human and/or humanized immunoglobulin loci. Mice that comprise ADAM6 function are described, including mice that comprise an ectopic nucleic acid sequence that encodes an ADAM6 protein. Genetically modified male mice that comprise a genetic modification of an endogenous mouse immunoglobulin V_{H} region locus, and that further comprise ADAM6 activity are described, including mice that comprise an ectopic nucleic acid sequence that restores or maintains fertility to the male mouse. Exemplary fertility is fertility that is comparable to wild-type mice.

Genetically modified non-human fertile animals that comprise a deletion or a modification of an endogenous ADAM6 gene or homolog or ortholog thereof, and that comprise a genetic modification that restores ADAM6 (or homolog or ortholog thereof) function in whole or in part are described, wherein the non-human animals express a human immunoglobulin light chain variable sequence in the context of a heavy chain constant sequence. Cells expressing such binding proteins, rodents (*e.g*., mice) that make them, and related methods and compositions are also described.

Genetically engineered animals that express antibodies comprising light chain variable regions fused with heavy chain constant regions, wherein the non-human animals lack a functional endogenous ADAM6 gene but retain ADAM6 function, are described, including rodents (*e.g*., mice) that comprise a modification of an endogenous immunoglobulin heavy chain variable (V_{H}) region locus that renders the mouse incapable of making a functional ADAM6 protein and results in a loss in fertility. The genetically modified mice comprise an immunoglobulin V_{H} locus characterized by a plurality of human V_{L}, J_{L} and optionally D_{H} gene segments or a combination thereof, and that further comprise ADAM6 function are described, including mice that comprise an ectopic nucleic acid sequence that restores fertility to a male mouse. The genetically modified mice express antibodies that lack heavy chain variable domains and instead comprise variable domains comprising rearranged light chain gene segments.

Genetically modified rodents (*e.g*., mice), cells, embryos, and tissues that comprise a nucleic acid sequence encoding a functional ADAM6 locus are described, wherein the mice, cells, embryos, and tissues express an immunoglobulin heavy chain comprising a human light chain variable domain. Further, the mice, cells, embryos, and tissues lack a functional endogenous ADAM6 gene but retain ADAM6 function characterized by the presence of an ectopic nucleic acid sequence that encodes an ADAM6 protein. Methods for making antibody sequences in fertile non-human animals that are useful for binding antigens are described.

### BACKGROUND

Over the last two decades, pharmaceutical applications for antibodies fueled a great deal of research into making antibodies that are suitable for use as human therapeutics. Early antibody therapeutics, based on mouse antibodies, were not ideal as human therapeutics because repeatedly administering mouse antibodies to humans results in immunogenicity problems that can confound long-term treatment regimens. Solutions based on humanizing mouse antibodies to make them appear more human and less mouse-like were developed. Methods for expressing human immunoglobulin sequences for use in antibodies followed, mostly based on *in vitro* expression of human immunoglobulin libraries in phage, bacteria, or yeast. Finally, attempts were made to make useful human antibodies from human lymphocytes *in vitro,* in mice engrafted with human hematopoietic cells, and in transchromosomal or transgenic mice with disabled endogenous immunoglobulin loci.

For the creation of these mice, it was necessary to disable the endogenous mouse immunoglobulin genes so that the randomly integrated fully human transgenes would function as the expressed repertoire of immunoglobulins in the mouse. Such mice can make human antibodies suitable for use as human therapeutics, but these mice display substantial problems with their immune systems. These problems lead to several experimental hurdles, for example, the mice are impractical for generating sufficiently diverse antibody repertoires, require the use of extensive re-engineering fixes, provide a suboptimal clonal selection process likely due to incompatibility between human and mouse elements, and an unreliable source of large and diverse populations of human variable sequences needed to be truly useful for making human therapeutics.

Transgenic mice that contain fully human antibody transgenes contain randomly inserted transgenes that contain unrearranged human immunoglobulin heavy chain variable sequences (V, D, and J sequences) linked to human heavy chain constant sequences, and unrearranged human immunoglobulin light chain variable sequences (V and J) linked to human light chain constant sequences. The mice therefore generate rearranged antibody genes from loci other than endogenous loci, where the rearranged antibody genes are fully human. In general, the mice contain human heavy chain sequences and human κ light chain sequences, although mice with at least some human λ sequences have also been reported. The transgenic mice generally have damaged and nonfunctional endogenous immunoglobulin loci, or knockouts of endogenous immunoglobulin loci, so that the mice are incapable of rearranging human antibody sequences at an endogenous immunoglobulin locus. The vagaries of such transgenic mice render them less than optimal for generating a sufficiently diverse human antibody repertoire in mice, likely due at least in part to a suboptimal clonal selection process that interfaces fully human antibody molecules within an endogenous selection system and deleterious effects from changes to the endogenous genetic makeup of such mice.

There remains a need in the art for making improved genetically modified non-human animals that are useful in generating immunoglobulin sequences, including human antibody sequences, and that are useful in generating a diverse repertoire of immunoglobulin-like molecules that exhibit diversity apart from traditional antibody molecules, while at the same time reducing or eliminating deleterious changes that might result from the genetic modifications. There also remains a need for non-human animals that are capable of rearranging immunoglobulin gene segments to form useful rearranged immunoglobulin genes, including human immunoglobulin light chain variable domains in the context of heavy chain constant domains that are cognate with human immunoglobulin light chain variable domains in the context of light chain constant domains, or that are capable of making proteins from altered immunoglobulin loci, including loci that contain a sufficiently diverse collection of human light chain variable gene segments. There remains a need for non-human animals that can generate immunoglobulin-like binding proteins, wherein the binding proteins comprise human immunoglobulin light chain variable domains linked to heavy chain constant domains.

### SUMMARY OF INVENTION

The invention provides a mouse whose genome comprises:
(a) an insertion of one or more human VL gene segments and one or more human JL gene segments upstream of a mouse immunoglobulin light chain constant region gene, wherein the one or more human VL gene segments and one or more human JL gene segments are operably linked to the mouse immunoglobulin light chain constant region gene;
(b) an insertion of one or more human VL gene segments and one or more human JL gene segments upstream of a mouse immunoglobulin heavy chain constant region gene, wherein the one or more human VL gene segments and one or more human JL gene segments are operably linked to the mouse immunoglobulin heavy chain constant region gene; and
(c) an ectopic nucleic acid sequence that encodes a mouse ADAM6a protein or a functional fragment thereof and an ectopic nucleic acid sequence that encodes a mouse ADAM6b protein or a functional fragment thereof, wherein the mouse ADAM6a and ADAM6b proteins or functional fragments thereof are expressed from the ectopic nucleic acid sequences, wherein the mouse expresses an antibody comprising (i) a polypeptide that comprises a human light chain variable domain linked to a mouse heavy chain constant domain; and (ii) a polypeptide that comprises a human light chain variable domain linked to a mouse light chain constant domain.

The invention further provides a cell derived from the mouse of the invention. The cell may be a B cell. The invention further provides a hybridoma made from the B cell of the invention.

The invention also provides a method for making an antibody that binds to an antigen of interest, wherein the method comprises
(a) exposing a mouse of the invention to an antigen of interest;
(b) isolating one or more B cells of the mouse, wherein the one or more B cells express an antibody that binds the antigen of interest;
(c) identifying a nucleic acid sequence that encodes one of the light variable chain domains of the antibody that binds that antigen of interest, wherein the antibody comprises a human immunoglobulin κ light chain variable domain linked to a mouse light chain constant domain and a human immunoglobulin κ light chain variable domain linked to a mouse heavy chain constant domain; and
(d) employing the nucleic acid sequence of (c) with a human immunoglobulin constant region nucleic acid sequence to make a human antibody that binds the antigen of interest.

Genetically modified non-human animals having immunoglobulin loci are described, wherein the immunoglobulin loci comprise a plurality of human light chain variable (V_{L}) gene segments operably linked to one or more non-human constant regions, *e.g*., human Vκ and Jκ or human Vλ and Jλ, and in various aspects the loci lack a sequence that encodes an endogenous functional ADAM6 protein. The non-human animals include rodents, *e.g*., mice and rats.

Loci are provided that are capable of rearranging and forming a gene encoding a light chain variable domain that is derived from a rearrangement involving a human light chain Vκ or Vλ gene segment and a human Jκ or Jλ gene segment and in various aspects, additionally a D_{H} gene segment, wherein in various aspects the loci lack an endogenous functional ADAM6 gene or functional fragment thereof.

Modified immunoglobulin loci include loci that lack an endogenous functional ADAM6 gene and comprise human immunoglobulin sequences are described, *e.g*., a human V_{L} segment operably linked to a human or (or human/non-human chimeric) non-human immunoglobulin constant sequence (and in operable linkage with, *e.g*., a V and/or a J segment). Modified loci that comprise multiple V_{L} gene segments and an ectopic nucleotide sequence that encodes an ADAM6 protein or fragment thereof that is functional in the non-human animal, are described. Modified loci that comprise multiple V_{L} gene segments, operably linked with one or more D_{H} segments and/or one or more J_{L} or J_{H} segments, operably linked to a non-human immunoglobulin constant sequence, *e.g.,* a rodent (*e.g*., mouse or rat) or human sequence, are described. Non-human animals comprising such humanized loci are also described, wherein the non-human animals exhibit wild-type fertility.

Non-human animals that comprise an immunoglobulin heavy chain variable locus (*e.g*., on a transgene or as an insertion or replacement at an endogenous non-human animal heavy chain variable locus) that comprises a plurality of human V_{L} gene segments operably linked to a human D and/or human J gene segment are described. In various aspects, the plurality of human V_{L} gene segments are operably linked to one or more human D and/or one or more human J gene segments at the endogenous immunoglobulin heavy chain variable gene locus of the non-human animal. In various aspects, the non-human animals further comprise an ectopic nucleotide sequence that encodes an ADAM6 protein or homolog or ortholog thereof that is functional in the male non-human animal that comprises the modified heavy chain locus. In various aspects, the ectopic nucleotide sequence is contiguous with at least one human V_{L} segment, a D_{H} gene segment, or a J_{L} gene segment. In various aspects, the ectopic nucleotide sequence is contiguous with a non-immunoglobulin sequence in the genome of the non-human animal. In one aspect, the ectopic nucleotide sequence is on the same chromosome as the modified heavy chain locus. In one aspect, the ectopic nucleotide sequence is on a different chromosome as the modified heavy chain locus.

Non-human animals are described that are modified at their immunoglobulin heavy chain variable region loci to delete all or substantially all (*e.g*., all functional segments, or nearly all functional segments) endogenous immunoglobulin V_{H} segments and that comprise a plurality of human V_{L} gene segments operably linked to a D_{H} and J segment or a J_{L} gene segment at the endogenous immunoglobulin heavy chain variable region locus of the non-human animal. Non-human animals comprising such loci and that lack an endogenous ADAM6 gene are also described.

Methods are described for making human immunoglobulin sequences in non-human animals. In various aspects, the human immunoglobulin sequences are derived from a repertoire of immunoglobulin heavy chain sequences that comprise human V_{L} gene segments rearranged and in operable linkage with immunoglobulin heavy chain constant regions, *e.g*., V_{L}, and one or more D_{H} and J segments or one or more J_{L} segments. Methods for making human immunoglobulin sequences in non-human animals, tissues, and cells are described, wherein the human immunoglobulin sequences bind an antigen of interest.

In one aspect, nucleic acid constructs, cells, embryos, rodents (*e.g*., mice), and methods are described for making rodents (*e.g*., mice) that comprise a modification that results in a nonfunctional endogenous rodent (*e.g*., mouse) ADAM6 protein or ADAM6 gene (*e.g*., a knockout of or a deletion in an endogenous ADAM6 gene), wherein the rodents (*e.g*., mice) comprise a nucleic acid sequence that encodes an ADAM6 protein or ortholog or homolog or fragment thereof that is functional in a male rodent of the same kind (*e.g*., mouse). In one aspect, the mice comprise an ectopic nucleotide sequence encoding a rodent ADAM6 protein or ortholog or homolog or functional fragment thereof; in a specific aspect, the rodent ADAM6 protein is a mouse ADAM6 protein. In one aspect, the mice comprise an ectopic nucleotide sequence that encodes one or more rodent ADAM6 proteins, wherein the one or more proteins comprise SEQ ID NO: 1 or SEQ ID NO: 2 or a fragment thereof that is functional in the mice.

In various aspects, the sequence that encodes ADAM6 activity is contiguous with a human immunoglobulin sequence. In various aspects, the sequence that encodes ADAM6 activity is contiguous with a non-human immunoglobulin sequence. In various aspects, the sequence is present on the same chromosome as the endogenous non-human immunoglobulin heavy chain locus of the non-human animal. In various aspects, the sequence is present on a different chromosome than the immunoglobulin heavy chain locus of the non-human animal.

Genetically modified non-human animals are described that comprise a modification that maintains activity of an ADAM6 gene or homolog or ortholog thereof, wherein the modification includes insertion of one or more human immunoglobulin light chain gene segments upstream of a non-human immunoglobulin heavy chain constant region, and the non-human animals further comprise modifications that enable them to express human immunoglobulin light chain variable regions cognate with human immunoglobulin light chain variable regions. In various aspects, the human immunoglobulin light chain variable regions are expressed in the context of light and heavy chain constant regions.

In various aspects, the insertion of one or more human immunoglobulin light chain gene segments is performed 3' or downstream of the ADAM6 gene of the non-human animal. In various aspects, the insertion of one or more human immunoglobulin light chain gene segments is performed in a manner such that the ADAM6 gene(s) of the non-human animal is not disrupted, deleted and/or functionally silenced such that the ADAM6 activity of the non-human animal is at the same or comparable level as in a non-human animal that does not contain such an insertion. Exemplary disruptions, deletions and/or functionally silencing modifications include any modification that results in a reduction, elimination and/or loss of activity of the ADAM6 protein(s) encoded by the ADAM6 gene(s) of the non-human animal.

In one aspect, nucleic acid constructs, cells, embryos, mice, and methods are described for making mice that comprise a modification of an endogenous immunoglobulin locus, wherein the mice comprise an ADAM6 protein or ortholog or homolog or fragment thereof that is functional in a male mouse. In one aspect, the endogenous immunoglobulin locus is an immunoglobulin heavy chain locus, and the modification reduces or eliminates ADAM6 activity of a cell or tissue of a male mouse.

In one aspect, mice are described that comprise an ectopic nucleotide sequence encoding a mouse ADAM6 or ortholog or homolog or functional fragment thereof; mice are also described that comprise an endogenous nucleotide sequence encoding a mouse ADAM6 or ortholog or homolog or fragment thereof, and at least one genetic modification of a heavy chain immunoglobulin locus. In one aspect, the endogenous nucleotide sequence encoding a mouse ADAM6 or ortholog or homolog or functional fragment thereof is located at an ectopic position as compared to an endogenous ADAM6 gene of a wild type mouse.

In one aspect, methods are described for making mice that comprise a modification of an endogenous immunoglobulin locus, wherein the mice comprise an ADAM6 protein or ortholog or homolog or fragment thereof that is functional in a male mouse. In various aspects, the modification comprises an insertion of one or more human V_{L} gene segments at the endogenous immunoglobulin locus.

In one aspect, methods are described for making mice that comprise a genetic modification of a heavy chain immunoglobulin locus, wherein application of the methods result in male mice that comprise a modified heavy chain immunoglobulin locus (or a deletion thereof), and the male mice are capable of generating offspring by mating. In one aspect, the male mice are capable of producing sperm that can transit from a mouse uterus through a mouse oviduct to fertilize a mouse egg.

In one aspect, methods are described for making mice that comprise a genetic modification of an immunoglobulin heavy chain locus and an immunoglobulin light chain locus, wherein application of the methods to modify the heavy chain locus result in male mice that exhibit a reduction in fertility, and the mice comprise a genetic modification that restores in whole or in part the reduction in fertility. In various aspects, the reduction in fertility is characterized by an inability of the sperm of the male mice to migrate from a mouse uterus through a mouse oviduct to fertilize a mouse egg. In various aspects, the reduction in fertility is characterized by sperm that exhibit an *in vivo* migration defect. In various aspects, the genetic modification that restores in whole or in part the reduction in fertility is a nucleic acid sequence encoding a mouse ADAM6 gene or ortholog or homolog or fragment thereof that is functional in a male mouse.

In one aspect, the genetic modification comprises replacing endogenous immunoglobulin heavy chain variable loci with immunoglobulin light chain variable loci of another species (*e.g*., a non-mouse species). In one aspect, the genetic modification comprises insertion of immunoglobulin light chain variable loci of another species (*e.g*., a non-mouse species) into endogenous immunoglobulin heavy chain variable loci. In a specific aspect, the species is human. In one aspect, the genetic modification comprises deletion of an endogenous immunoglobulin heavy chain variable locus in whole or in part, wherein the deletion results in a loss of endogenous ADAM6 function. In a specific aspect, the loss of endogenous ADAM6 function is associated with a reduction in fertility in male mice.

In one aspect, the genetic modification comprises inactivation of an endogenous non-human immunoglobulin heavy chain variable locus in whole or in part, wherein the inactivation does not result in a loss of endogenous ADAM6 function. Inactivation may include replacement or deletion of one or more endogenous non-human gene segments resulting in an endogenous non-human immunoglobulin heavy chain locus that is substantially incapable of rearrangement to encode a heavy chain of an antibody that comprises endogenous non-human gene segments. Inactivation may include other modifications that render the endogenous immunoglobulin heavy chain locus incapable of rearranging to encode the heavy chain of an antibody, wherein the modification does not include replacement or deletion of endogenous gene segments. Exemplary modifications include chromosomal inversions and/or translocations mediated by molecular techniques, *e.g*., using precise placement of site-specific recombination sites (*e.g*., Cre-lox technology). Other exemplary modifications include disabling the operable linkage between the non-human immunoglobulin variable gene segments and the non-human immunoglobulin constant regions.

In one aspect, the genetic modification comprises inserting into the genome of the non-human animal a DNA fragment containing one or more human V_{L} gene segments and one or more human J_{L} gene segments, and optionally one or more human D_{H} gene segments, of another species (*e.g*., a non-mouse species) operably linked to one or more constant region sequences (*e.g*., an IgM and/or an IgG gene). In one aspect, the DNA fragment is capable of undergoing rearrangement in the genome of the non-human animal to form a sequence that encodes a light chain variable domain operably linked to a heavy chain constant region. In one aspect, the species is human. In one aspect, the genetic modification comprises insertion of one or more human immunoglobulin light chain gene segments downstream or 3' of an endogenous ADAM6 gene of the non-human animal such that ADAM6 activity (*e.g*., expression and/or function of an encoded protein) is the same or comparable to a non-human animal that does not comprise the insertion.

In one aspect, methods are described for making mice that comprise a genetic modification of an immunoglobulin heavy chain locus, wherein application of the methods result in male mice that comprise a modified immunoglobulin heavy chain locus (or a deletion thereof), and the male mice exhibit a reduction in fertility, and the mice comprise a genetic modification that restores in whole or in part the reduction in fertility. In various aspects, the reduction in fertility is characterized by an inability of the sperm of the male mice to migrate from a mouse uterus through a mouse oviduct to fertilize a mouse egg. In various aspects, the reduction in fertility is characterized by sperm that exhibit an in vivo migration defect. In various aspects, the genetic modification that restores in whole or in part the reduction in fertility is a nucleic acid sequence encoding a mouse ADAM6 gene or ortholog or homolog or fragment thereof that is functional in a male mouse.

In one aspect, the genetic modification comprises replacing endogenous immunoglobulin heavy chain variable loci with immunoglobulin light chain variable loci, *e.g*., one or more light chain variable (V_{L}) gene segments, one or more heavy chain diversity (D_{H}) gene segments and one or more joining (J) gene segments, or one or more light chain joining (J_{L}) gene segments of another species (*e.g*., a non-mouse species). In one aspect, the genetic modification comprises insertion of a single orthologous immunoglobulin light chain variable loci V_{L} gene segment, at least one D_{H} gene segment and at least one J gene segment, or at least one J_{L} gene segment into endogenous immunoglobulin heavy chain variable loci. In a specific aspect, the species is human. In one aspect, the genetic modification comprises a deletion of an endogenous immunoglobulin heavy chain variable locus in whole or in part, wherein the deletion results in a loss of endogenous ADAM6 function. In a specific aspect, the loss of endogenous ADAM6 function is associated with a reduction in fertility in male mice. In one aspect, the genetic modification comprises inactivation of an endogenous immunoglobulin heavy chain variable locus in whole or in part, wherein the deletion does not result in a loss of endogenous ADAM6 function. Inactivation may include replacement or deletion of one or more endogenous gene segments resulting in an endogenous immunoglobulin heavy chain locus that is substantially incapable of rearrangement to encode a heavy chain of an antibody that comprises endogenous gene segments. Inactivation may include other modifications that render the endogenous immunoglobulin heavy chain locus incapable of rearranging to encode the heavy chain of an antibody, wherein the modification does not include replacement or deletion of endogenous gene segments. Exemplary modifications include chromosomal inversions and/or alterations that result in a heavy chain locus that is not in operable linkage with one or more endogenous constant regions.

In one aspect, the genetic modification comprises inserting into the genome of the mouse a DNA fragment containing one or more human V_{L} gene segments, one or more J gene segments, and optionally one or more D gene segments of another species (*e.g*., a non-mouse species) operably linked to one or more constant region sequences (*e.g*., an IgM and/or an IgG gene). In various aspects, the J gene segments include J_{H} or J_{L} gene segments. In one aspect, the DNA fragment is capable of undergoing rearrangement to form a sequence that encodes a heavy chain of an antibody, wherein the heavy chain comprises a rearranged human light chain variable gene segment fused to a heavy chain constant region. In one aspect, the genetic modification comprises insertion of at least six, at least 16, at least 30, or at least 40 or more human V_{L} gene segments, and at least one or at least 5 human J_{L} gene segments into the genome of the mouse. In a specific aspect, the species is human and the gene segments are human κ light chain gene segments. In one aspect, the genetic modification comprises deletion of an endogenous immunoglobulin heavy chain variable locus in whole or in part to render the endogenous immunoglobulin heavy chain locus nonfunctional, wherein the deletion further results in a loss of endogenous ADAM6 function. In a specific aspect, the loss of endogenous ADAM6 function is associated with a reduction in fertility in male mice.

In one aspect, mice are described that comprise a modification that reduces or eliminates mouse ADAM6 expression from an endogenous ADAM6 allele such that a male mouse having the modification exhibits a reduced fertility (*e.g*., a highly reduced ability to generate offspring by mating), or is essentially infertile, due to the reduction or elimination of endogenous ADAM6 function, wherein the mice further comprise an ectopic ADAM6 sequence or homolog or ortholog or functional fragment thereof. In one aspect, the modification that reduces or eliminates mouse ADAM6 expression is a modification (*e.g*., an insertion, a deletion, a replacement, *etc*.) in a mouse immunoglobulin locus. In one aspect, the immunoglobulin locus is an immunoglobulin heavy chain locus.

In one aspect, the reduction or loss of ADAM6 function comprises an inability or substantial inability of the mouse to produce sperm that can travel from a mouse uterus through a mouse oviduct to fertilize a mouse egg. In a specific aspect, at least about 95%, 96%, 97%, 98%, or 99% of the sperm cells produced in an ejaculate volume of the mouse are incapable of traversing through an oviduct in vivo following copulation and fertilizing a mouse ovum.

In one aspect, the reduction or loss of ADAM6 function comprises an inability to form or substantial inability to form a complex of ADAM2 and/or ADAM3 and/or ADAM6 on a surface of a sperm cell of the mouse. In one aspect, the loss of ADAM6 function comprises a substantial inability to fertilize a mouse egg by copulation with a female mouse.

In one aspect, a mouse is described that lacks a functional endogenous ADAM6 gene, and comprises a protein (or an ectopic nucleotide sequence that encodes a protein) that confers ADAM6 functionality on the mouse. In one aspect, the mouse is a male mouse and the functionality comprises enhanced fertility as compared with a mouse that lacks a functional endogenous ADAM6 gene.

In one aspect, the protein is encoded by a genomic sequence located within an immunoglobulin locus in the germline of the mouse. In a specific aspect, the immunoglobulin locus is a heavy chain locus. In another specific aspect, the heavy chain locus comprises at least one human V_{H}, at least one human D_{H} and at least one human J_{H} gene segment. In another specific aspect, the heavy chain locus comprises at least one human V_{L} and at least one human J_{L} gene segment. In another specific aspect, the heavy chain locus comprises at least one human V_{L}, at least one human D_{H}, and at least one human J_{L}. In another specific aspect, the heavy chain locus comprises at least one human V_{L}, at least one human D_{H}, and at least one human J_{H} gene segment. In another specific aspect, the heavy chain locus comprises at least one human V_{L} and at least one human J_{L} gene segment. In another specific aspect, the heavy chain locus comprises at least one human V_{L} and at least one human J_{H} gene segment. In another specific aspect, the heavy chain locus comprises six human Vκ and five human Jκ gene segments. In another specific aspect, the heavy chain locus comprises 16 human Vκ and five human Jκ gene segments. In another specific aspect, the heavy chain locus comprises 30 human Vκ and five human Jκ gene segments. In another specific aspect, the heavy chain locus comprises 40 human Vκ and five human Jκ gene segments.

In one aspect, the ectopic protein is encoded by a genomic sequence located within a non-immunoglobulin locus in the germline of the mouse. In one aspect, the non-immunoglobulin locus is a transcriptionally active locus. In a specific aspect, the transcriptionally active locus is the ROSA locus. In a specific aspect, the transcriptionally active locus is associated with tissue-specific expression. In one aspect, the tissue-specific expression is present in reproductive tissues. In one aspect, the protein is encoded by a genomic sequence randomly inserted into the germline of the mouse.

In one aspect, the mouse comprises a human or chimeric human/mouse or chimeric human/rat light chain (*e.g*., human variable, mouse or rat constant) and a chimeric human variable/mouse or rat constant heavy chain. In a specific aspect, the mouse comprises a transgene that comprises a chimeric human variable/rat or mouse constant light chain gene operably linked to a transcriptionally active promoter, *e.g*., a ROSA26 promoter. In a further specific aspect, the chimeric human/mouse or rat light chain transgene comprises a rearranged human light chain variable region sequence in the germline of the mouse.

In one aspect, the ectopic nucleotide sequence is located within an immunoglobulin locus in the germline of the mouse. In a specific aspect, the immunoglobulin locus is a heavy chain locus. In one aspect, the heavy chain locus comprises at least one human V_{L} and at least one human J_{L} gene segment. In a specific aspect, the heavy chain locus comprises at least six and up to 40 human Vκ gene segments, and five human Jκ gene segments. In one aspect, the ectopic nucleotide sequence is located within a non-immunoglobulin locus in the germline of the mouse. In one aspect, the non-immunoglobulin locus is a transcriptionally active locus. In a specific aspect, the transcriptionally active locus is the ROSA26 locus. In one aspect, the ectopic nucleotide sequence is positioned randomly inserted into the germline of the mouse.

In one aspect, a mouse is described that lacks a functional endogenous ADAM6 gene, wherein the mouse comprises an ectopic nucleotide sequence that complements the loss of mouse ADAM6 function. In one aspect, the ectopic nucleotide sequence confers upon the mouse an ability to produce offspring that is comparable to a corresponding wild-type mouse that contains a functional endogenous ADAM6 gene. In one aspect, the sequence confers upon the mouse an ability to form a complex of ADAM2 and/or ADAM3 and/or ADAM6 on the surface of sperm cell of the mouse. In one aspect, the sequence confers upon the mouse an ability to travel from a mouse uterus through a mouse oviduct to a mouse ovum to fertilize the ovum.

In one aspect, the mouse lacking the functional endogenous ADAM6 gene and comprising the ectopic nucleotide sequence produces at least about 50%, 60%, 70%, 80%, or 90% of the number of litters a wild-type mouse of the same age and strain produces in a six-month time period.

In one aspect, the mouse lacking the functional endogenous ADAM6 gene and comprising the ectopic nucleotide sequence produces at least about 1.5-fold, about 2-fold, about 2.5-fold, about 3-fold, about 4-fold, about 6-fold, about 7-fold, about 8-fold, or about 10-fold or more progeny when bred over a six-month time period than a mouse of the same age and the same or similar strain that lacks the functional endogenous ADAM6 gene and that lacks the ectopic nucleotide sequence that is bred over substantially the same time period and under substantially the same conditions.

In one aspect, the mouse lacking the functional endogenous ADAM6 gene and comprising the ectopic nucleotide sequence produces an average of at least about 2-fold, 3-fold, or 4-fold higher number of pups per litter in a four or six month breeding period than a mouse that lacks the functional endogenous ADAM6 gene and that lacks the ectopic nucleotide sequence, and that is bred for the same period of time.

In one aspect, the mouse lacking the functional endogenous ADAM6 gene and comprising the ectopic nucleotide sequence is a male mouse, and the male mouse produces sperm that when recovered from oviducts at about 5-6 hours post-copulation reflects an oviduct migration that is at least 10-fold, at least 20-fold, at least 30-fold, at least 40-fold, at least 50-fold, at least 60-fold, at least 70-fold, at least 80-fold, at least 90-fold, 100-fold, 110-fold, or 120-fold or higher than a mouse that lacks the functional endogenous ADAM6 gene and that lacks the ectopic nucleotide sequence.

In one aspect, the mouse lacking the functional endogenous ADAM6 gene and comprising the ectopic nucleotide sequence when copulated with a female mouse generates sperm that is capable of traversing the uterus and entering and traversing the oviduct within about six hours at an efficiency that is about equal to sperm from a wild-type mouse.

In one aspect, the mouse lacking the functional endogenous ADAM6 gene and comprising the ectopic nucleotide sequence produces about 1.5-fold, about 2-fold, about 3-fold, or about 4-fold or more litters in a comparable period of time than a mouse that lacks the functional ADAM6 gene and that lacks the ectopic nucleotide sequence.

In one aspect, a mouse comprising in its germline a non-mouse nucleic acid sequence that encodes an immunoglobulin protein is described, wherein the non-mouse immunoglobulin sequence comprises an insertion of a mouse ADAM6 gene or homolog or ortholog or functional fragment thereof. In one aspect, the non-mouse immunoglobulin sequence comprises a human immunoglobulin sequence. In one aspect, the sequence comprises a human immunoglobulin heavy chain sequence. In one aspect, the sequence comprises a human immunoglobulin light chain sequence. In one aspect, the sequence comprises a human heavy chain sequence contiguous with a human light chain sequence. In one aspect, the sequence comprises one or more V gene segments, one or more D gene segments, and one or more J gene segments; in one aspect, the sequence comprises one or more V gene segments and one or more J gene segments. In one aspect, the one or more V, D, and J gene segments, or one or more V and J gene segments, are unrearranged. In one aspect, the one or more V, D, and J gene segments, or one or more V and J gene segments, are rearranged. In one aspect, following rearrangement of the one or more V, D, and J gene segments, or one or more V and J gene segments, the mouse comprises in its genome at least one nucleic acid sequence encoding a mouse ADAM6 gene or homolog or ortholog or functional fragment thereof. In one aspect, following rearrangement the mouse comprises in its genome at least two nucleic acid sequences encoding a mouse ADAM6 gene or homolog or ortholog or functional fragment thereof. In one aspect, following rearrangement the mouse comprises in its genome at least one nucleic acid sequence encoding a mouse ADAM6 gene or homolog or ortholog or functional fragment thereof. In one aspect, the mouse comprises the ADAM6 gene or homolog or ortholog or functional fragment thereof in a B cell. In one aspect, the mouse comprises the ADAM6 gene or homolog or ortholog or functional fragment thereof in a non-B cell.

In one aspect, mice are described that express a human immunoglobulin heavy chain variable region or functional fragment thereof from an endogenous immunoglobulin heavy chain locus, wherein the mice comprise an ADAM6 activity that is functional in a male mouse. In one aspect, the heavy chain locus comprises one or more human V_{L} gene segments and one or more J_{L} gene segments, and optionally one or more D_{H} gene segments. In one aspect, the heavy chain locus comprises at least six human Vκ gene segments and five human Jκ gene segments. In one aspect, the heavy chain locus comprises at least 16 human Vκ gene segments and five human Jκ gene segments. In one aspect, the heavy chain locus comprises at least 30 human Vκ gene segments and five human Jκ gene segments. In one aspect, the heavy chain locus comprises at least 40 human Vκ gene segments and five human Jκ gene segments.

In one aspect, mice are described that express a human immunoglobulin light chain variable region or functional fragment thereof from an endogenous immunoglobulin heavy chain locus, wherein the mice comprise an ADAM6 activity that is functional in a male mouse.

In one aspect, the male mice comprise a single unmodified endogenous ADAM6 allele or ortholog of homolog or functional fragment thereof at an endogenous ADAM6 locus.

In one aspect, the male mice comprise an ectopic mouse ADAM6 sequence or homolog or ortholog or functional fragment thereof that encodes a protein that confers ADAM6 function.

In one aspect, the male mice comprise an ADAM6 sequence or homolog or ortholog or functional fragment thereof at a location in the mouse genome that approximates the location of the endogenous ADAM6 allele, *e.g*., 3' of a final V gene segment sequence and 5' of an initial J gene segment.

In one aspect, the male mice comprise an ADAM6 sequence or homolog or ortholog or functional fragment thereof at a location in the mouse genome that is different from that of the endogenous ADAM6 allele, *e.g*., 5' of the 5'-most V gene segment sequence of a V gene locus.

In one aspect, the male mice comprise an ADAM6 sequence or homolog or ortholog or functional fragment thereof flanked upstream, downstream, or upstream and downstream (with respect to the direction of transcription of the ADAM6 sequence) of a nucleic acid sequence encoding an immunoglobulin V gene segment and/or an immunoglobulin J gene segment. In a specific aspect, the immunoglobulin V and J gene segments are human gene segments. In one aspect, the immunoglobulin V and J gene segments are human gene segments, and the sequence encoding the mouse ADAM6 or ortholog or homolog or fragment thereof functional in a mouse is between the human V and J gene segments; in one aspect, the mouse comprises two or more human V gene segments, and the sequence is at a position 5' of the 5'-most human V gene segment; in one aspect, the mouse comprises two or more human V gene segments, and the sequence is at a position between the final V gene segment and the penultimate V gene segment; in one aspect, the mouse comprises a plurality of human V gene segments, and the sequence is at a position upstream of the 5'-most human V gene segment; in one aspect, the mouse further comprises a D gene segment, and the sequence is at a position following the 3'-most V gene segment and a 5'-most D gene segment; in one aspect, the sequence is at a position between a V gene segment and a J gene segment.

In one aspect, the human V gene segments are light chain V gene segments. In a specific aspect, the light chain V gene segments are Vκ gene segments. In another specific aspect, the light chain V gene segments are Vλ gene segments. In one aspect, the J gene segment is selected from a J_{H} and a J_{L} gene segment. In a specific aspect, the J_{L} gene segment is a Jκ gene segment. In another specific aspect, the J_{L} gene segment is a Jλ gene segment.

In one aspect, the male mice comprise an ADAM6 sequence or homolog or ortholog or functional fragment thereof that is located at a position in an endogenous immunoglobulin locus that is the same or substantially the same as in a wild type male mouse. In a specific aspect, the endogenous locus is incapable of encoding the heavy chain variable region of an antibody, wherein the variable region comprises or is derived from an endogenous non-human gene segment. In a specific aspect, the endogenous locus is positioned at a location in the genome of the male mouse that renders the heavy chain gene segments of the locus incapable of encoding a heavy chain variable region of an antibody. In various aspects, the male mice comprise an ADAM6 sequence located on the same chromosome as human immunoglobulin gene segments and the ADAM6 sequence encodes a functional ADAM6 protein.

In one aspect, a male mouse is described that comprises a nonfunctional endogenous ADAM6 gene, or a deletion of an endogenous ADAM6 gene, in its germline; wherein sperm cells of the mouse are capable of transiting an oviduct of a female mouse and fertilizing an egg. In one aspect, the mice comprise an extrachromosomal copy of a mouse ADAM6 gene or ortholog or homolog or functional fragment thereof that is functional in a male mouse. In one aspect, the mice comprise an ectopic mouse ADAM6 gene or ortholog or homolog or functional fragment thereof that is functional in a male mouse.

In one aspect, a male mouse is described that comprises a functional endogenous ADAM6 gene and a modification to an endogenous immunoglobulin heavy chain locus. In one aspect, the modification is made downstream, or 3' of an endogenous ADAM6 gene or locus. In one aspect, the modification is a replacement of one or more endogenous immunoglobulin heavy chain gene segments with one or more human immunoglobulin light chain gene segments. In one aspect, the modification is an insertion of one or more human immunoglobulin light chain gene segments upstream of an endogenous immunoglobulin heavy chain constant region gene.

In one aspect, mice are described that comprise a genetic modification that reduces endogenous ADAM6 function, wherein the mouse comprises at least some ADAM6 functionality provided either by an endogenous unmodified allele that is functional in whole or in part (*e.g*., a heterozygote), or by expression from an ectopic sequence that encodes an ADAM6 or an ortholog or homolog or functional fragment thereof that is functional in a male mouse. In various aspects, the ADAM6 or ortholog or homolog or functional fragment thereof comprises a nucleic acid sequence that encodes an ADAM6 protein set forth in SEQ ID NO: 1, SEQ ID NO: 2, or a combination thereof.

In one aspect, the mice comprise ADAM6 function sufficient to confer upon male mice the ability to generate offspring by mating, as compared with male mice that lack a functional ADAM6. In one aspect, the ADAM6 function is conferred by the presence of an ectopic nucleotide sequence that encodes a mouse ADAM6 or a homolog or ortholog or functional fragment thereof. In one aspect, the ADAM6 function is conferred by an endogenous ADAM6 gene present in an endogenous immunoglobulin locus, wherein the mouse is incapable of expressing an antibody comprising an endogenous immunoglobulin heavy chain gene segment. ADAM6 homologs or orthologs or fragments thereof that are functional in a male mouse include those that restore, in whole or in part, the loss of ability to generate offspring observed in a male mouse that lacks sufficient endogenous ADAM6 activity, *e.g*., the loss in ability observed in an ADAM6 knockout mouse. In this sense ADAM6 knockout mice include mice that comprise an endogenous locus or fragment thereof, but that is not functional, *i.e.,* that does not express ADAM6 (ADAM6a and/or ADAM6b) at all, or that expresses ADAM6 (ADAM6a and/or ADAM6b) at a level that is insufficient to support an essentially normal ability to generate offspring of a wild-type male mouse. The loss of function can be due, *e.g*., to a modification in a structural gene of the locus (*i.e*., in an ADAM6a or ADAM6b coding region) or in a regulatory region of the locus (*e.g*., in a sequence 5' to the ADAM6a gene, or 3' of the ADAM6a or ADAM6b coding region, wherein the sequence controls, in whole or in part, transcription of an ADAM6 gene, expression of an ADAM6 RNA, or expression of an ADAM6 protein). In various aspects, orthologs or homologs or fragments thereof that are functional in a male mouse are those that enable a sperm of a male mouse (or a majority of sperm cells in the ejaculate of a male mouse) to transit a mouse oviduct and fertilize a mouse ovum.

In one aspect, male mice that express the human immunoglobulin variable region or functional fragment thereof comprise sufficient ADAM6 activity to confer upon the male mice the ability to generate offspring by mating with female mice and, in one aspect, the male mice exhibit an ability to generate offspring when mating with female mice that is in one aspect at least 25%, in one aspect, at least 30%, in one aspect at least 40%, in one aspect at least 50%, in one aspect at least 60%, in one aspect at least 70%, in one aspect at least 80%, in one aspect at least 90%, and in one aspect about the same as, that of mice with one or two endogenous unmodified ADAM6 alleles.

In one aspect male mice express sufficient ADAM6 (or an ortholog or homolog or functional fragment thereof) to enable a sperm cell from the male mice to traverse a female mouse oviduct and fertilize a mouse egg.

In one aspect, the ADAM6 functionality is conferred by a nucleic acid sequence that is contiguous with a mouse chromosomal sequence (*e.g*., the nucleic acid is randomly integrated into a mouse chromosome; or placed at a specific location, *e.g*., by targeting the nucleic acid to a specific location, *e.g*., by site-specific recombinase-mediated (*e.g*., Cre-mediated) insertion or homologous recombination). In one aspect, the ADAM6 sequence is present on a nucleic acid that is distinct from a chromosome of the mouse (*e.g*., the ADAM6 sequence is present on an episome, *i.e*., extra-chromosomally, *e.g*., in an expression construct, a vector, a YAC, a trans-chromosome, *etc*.).

In one aspect, genetically modified mice and cells are described that comprise a modification of an endogenous heavy chain immunoglobulin locus, wherein the mice express at least a portion of a immunoglobulin light chain sequence, *e.g*., at least a portion of a human sequence, wherein the mice comprise an ADAM6 activity that is functional in a male mouse. In one aspect, the modification reduces or eradicates an ADAM6 activity of the mouse. In one aspect, the mouse is modified such that both alleles that encode ADAM6 activity are either absent or express an ADAM6 that does not substantially function to support normal mating in a male mouse. In one aspect, the mouse further comprises an ectopic nucleic acid sequence encoding a mouse ADAM6 or ortholog or homolog or functional fragment thereof. In one aspect, the modification maintains ADAM6 activity of the mouse and renders an endogenous immunoglobulin heavy chain locus incapable of encoding a heavy chain of an antibody. In a specific aspect, the modification includes chromosomal inversions and or translocations that render the endogenous immunoglobulin heavy chain locus incapable of rearranging to encode a heavy chain variable region of an antibody.

In one aspect, genetically modified mice and cells are described that comprise a modification of an endogenous immunoglobulin heavy chain locus, wherein the modification reduces or eliminates ADAM6 activity expressed from an ADAM6 sequence of the locus, and wherein the mice comprise an ADAM6 protein or ortholog or homolog or functional fragment thereof. In various aspects, the ADAM6 protein or fragment thereof is encoded by an ectopic ADAM6 sequence. In various aspects, the ADAM6 protein or fragment thereof is expressed from an endogenous ADAM6 allele. In various aspects, the mouse comprises a first heavy chain allele comprises a first modification that reduces or eliminates expression of a functional ADAM6 from the first heavy chain allele, and the mouse comprises a second heavy chain allele that comprises a second modification that does not substantially reduce or does not eliminate expression of a functional ADAM6 from the second heavy chain allele.

In various aspects, the modification is the insertion of one or more human immunoglobulin light chain gene segments upstream, or 5', of an endogenous immunoglobulin heavy chain constant region gene. In various aspects, the modification maintains the endogenous ADAM6 gene located at the endogenous immunoglobulin heavy chain locus.

In one aspect, the second modification is located 3' (with respect to the transcriptional directionality of the mouse V gene segment) of a final mouse V gene segment and located 5' (with respect to the transcriptional directionality of the constant sequence) of a mouse (or chimeric human/mouse) immunoglobulin heavy chain constant gene or fragment thereof (*e.g*., a nucleic acid sequence encoding a human and/or mouse: C_{H}1 and/or hinge and/or C_{H}2 and/or C_{H}3).

In one aspect, the modification is at a first immunoglobulin heavy chain allele at a first locus that encodes a first ADAM6 allele, and the ADAM6 function results from expression of an endogenous ADAM6 at a second immunoglobulin heavy chain allele at a second locus that encodes a functional ADAM6, wherein the second immunoglobulin heavy chain allele comprises at least one modification of a V, D, and/or J gene segment. In a specific aspect, the at least one modification of the V, D, and or J gene segment is a deletion, a replacement with a human V, D, and/or J gene segment, a replacement with a camelid V, D, and/or J gene segment, a replacement with a humanized or camelized V, D, and/or J gene segment, a replacement of a heavy chain sequence with a light chain sequence, and a combination thereof. In one aspect, the at least one modification is the deletion of one or more heavy chain V, D, and/or J gene segments and a replacement with one or more light chain V and/or J gene segments (*e.g*., a human light chain V and/or J gene segment) at the heavy chain locus.

In one aspect, the modification is at a first immunoglobulin heavy chain allele at a first locus and a second immunoglobulin heavy chain allele at a second locus, and the ADAM6 function results from expression of an ectopic ADAM6 at a non-immunoglobulin locus in the germline of the mouse. In a specific aspect, the non-immunoglobulin locus is the ROSA26 locus. In a specific aspect, the non-immunoglobulin locus is transcriptionally active in reproductive tissue.

In one aspect, the modification is at a first immunoglobulin heavy chain allele at a first locus and a second immunoglobulin heavy chain allele at a second locus, and the ADAM6 function results from an endogenous ADAM6 gene in the germline of the mouse. In a specific aspect, the endogenous ADAM6 gene is juxtaposed by mouse immunoglobulin heavy chain gene segments.

In one aspect, the modification is at a first immunoglobulin heavy chain allele at a first locus and a second immunoglobulin heavy chain allele at a second locus, and the ADAM6 function results from expression of an ectopic ADAM6 sequence at the first immunoglobulin heavy chain allele. In one aspect, the modification is at a first immunoglobulin heavy chain allele at a first locus and a second immunoglobulin heavy chain allele at a second locus, and the ADAM6 function or activity results from expression of an ectopic ADAM6 at the second immunoglobulin heavy chain allele.

In one aspect, a mouse comprising a heterozygous or a homozygous knockout of ADAM6 is described. In one aspect, the mouse further comprises a modified immunoglobulin sequence that is a human or a humanized immunoglobulin sequence, or a camelid or camelized human or mouse immunoglobulin sequence. In one aspect, the modified immunoglobulin sequence is present at the endogenous immunoglobulin heavy chain locus. In one aspect, the modified immunoglobulin sequence comprises a human light chain variable region sequence at an endogenous immunoglobulin heavy chain locus. In one aspect, the human light chain variable region sequence replaces an endogenous heavy chain variable sequence at the endogenous immunoglobulin heavy chain locus. In one aspect, the modified immunoglobulin sequence comprises a human κ light chain variable region sequence at an endogenous immunoglobulin heavy chain locus. In one aspect, the modified immunoglobulin sequence comprises a human λ light chain variable region sequence at an endogenous immunoglobulin heavy chain locus.

In one aspect, a mouse incapable of expressing a functional endogenous ADAM6 from an endogenous ADAM6 locus is described. In one aspect, the mouse comprises an ectopic nucleic acid sequence that encodes an ADAM6, or functional fragment thereof, that is functional in the mouse. In a specific aspect, the ectopic nucleic acid sequence encodes a protein that rescues a loss in the ability to generate offspring exhibited by a male mouse that is homozygous for an ADAM6 knockout. In a specific aspect, the ectopic nucleic acid sequence encodes a mouse ADAM6 protein.

In one aspect, a mouse is described that lacks a functional endogenous ADAM6 locus, and that comprises an ectopic nucleic acid sequence that confers upon the mouse ADAM6 function. In one aspect, the nucleic acid sequence comprises an endogenous ADAM6 sequence or functional fragment thereof. In one aspect, the endogenous ADAM6 sequence comprises ADAM6a- and ADAM6b-encoding sequence located in a wild-type mouse between the 3'-most mouse immunoglobulin heavy chain V gene segment (V_{H}) and the 5'-most mouse immunoglobulin heavy chain D gene segment (D_{H}).

In one aspect, the nucleic acid sequence comprises a sequence encoding mouse ADAM6a or functional fragment thereof and/or a sequence encoding mouse ADAM6b or functional fragment thereof, wherein the ADAM6a and/or ADAM6b or functional fragment(s) thereof is operably linked to a promoter. In one aspect, the promoter is a human promoter. In one aspect, the promoter is the mouse ADAM6 promoter. In a specific aspect, the ADAM6 promoter comprises sequence located between the first codon of the first ADAM6 gene closest to the mouse 5'-most D_{H} gene segment and the recombination signal sequence of the 5'-most D_{H} gene segment, wherein 5' is indicated with respect to direction of transcription of the mouse immunoglobulin genes. In one aspect, the promoter is a viral promoter. In a specific aspect, the viral promoter is a cytomegalovirus (CMV) promoter. In one aspect, the promoter is an ubiquitin promoter.

In one aspect, the promoter is an inducible promoter. In one aspect, the inducible promoter regulates expression in non-reproductive tissues. In one aspect, the inducible promoter regulates expression in reproductive tissues. In a specific aspect, the expression of the mouse ADAM6a and/or ADAM6b sequences or functional fragments(s) thereof is developmentally regulated by the inducible promoter in reproductive tissues.

In one aspect, the mouse ADAM6a and/or ADAM6b are selected from the ADAM6a of SEQ ID NO: 1 and/or ADAM6b of sequence SEQ ID NO: 2.

In one aspect, the mouse ADAM6 promoter is a promoter of SEQ ID NO: 3. In a specific aspect, the mouse ADAM6 promoter comprises the nucleic acid sequence of SEQ ID NO: 3 directly upstream (with respect to the direction of transcription of ADAM6a) of the first codon of ADAM6a and extending to the end of SEQ ID NO: 3 upstream of the ADAM6 coding region. In another specific aspect, the ADAM6 promoter is a fragment extending from within about 5 to about 20 nucleotides upstream of the start codon of ADAM6a to about 0.5kb, 1kb, 2kb, or 3kb or more upstream of the start codon of ADAM6a.

In one aspect, the nucleic acid sequence comprises SEQ ID NO: 3 or a fragment thereof that when placed into a mouse that is infertile or that has low fertility due to a lack of ADAM6 improves fertility or restores fertility to about a wild-type fertility. In one aspect, SEQ ID NO: 3 or a fragment thereof confers upon a male mouse the ability to produce a sperm cell that is capable of traversing a female mouse oviduct in order to fertilize a mouse egg.

In one aspect, the mouse ADAM6 promoter is a promoter of SEQ ID NO: 4. In a specific aspect, the mouse ADAM6 promoter comprises the nucleic acid sequence of SEQ ID NO: 4 directly upstream (with respect to the direction of transcription of ADAM6a) of the first codon of ADAM6a and extending to the end of SEQ ID NO: 4 upstream of the ADAM6 coding region. In another specific aspect, the ADAM6 promoter is a fragment extending from within about 5 to about 20 nucleotides upstream of the start codon of ADAM6a to about 0.5kb, 1kb, 2kb, or 3kb or more upstream of the start codon of ADAM6a.

In one aspect, the nucleic acid sequence comprises SEQ ID NO: 4 or a fragment thereof that when placed into a mouse that is infertile or that has low fertility due to a lack of ADAM6 improves fertility or restores fertility to about a wild-type fertility. In one aspect, SEQ ID NO: 4 or a fragment thereof confers upon a male mouse the ability to produce a sperm cell that is capable of traversing a female mouse oviduct in order to fertilize a mouse egg.

In one aspect, the mouse ADAM6 promoter is a promoter of SEQ ID NO: 5. In a specific aspect, the mouse ADAM6 promoter comprises the nucleic acid sequence of SEQ ID NO: 5 directly upstream (with respect to the direction of transcription of ADAM6a) of the first codon of ADAM6a and extending to the end of SEQ ID NO: 5 upstream of the ADAM6 coding region. In another specific aspect, the ADAM6 promoter is a fragment extending from within about 5 to about 20 nucleotides upstream of the start codon of ADAM6a to about 0.5kb, 1kb, 2kb, or 3kb or more upstream of the start codon of ADAM6a.

In one aspect, the nucleic acid sequence comprises SEQ ID NO: 5 or a fragment thereof that when placed into a mouse that is infertile or that has low fertility due to a lack of ADAM6 improves fertility or restores fertility to about a wild-type fertility. In one aspect, SEQ ID NO: 5 or a fragment thereof confers upon a male mouse the ability to produce a sperm cell that is capable of traversing a female mouse oviduct in order to fertilize a mouse egg.

In various aspects, the ectopic nucleic acid sequence that confers upon the mouse ADAM6 function encodes one or more ADAM6 proteins, wherein the one or more ADAM6 proteins comprise SEQ ID NO: 1, SEQ ID NO: 2 or a combination thereof.

In various aspects, the ectopic nucleic acid sequence comprises a sequence selected from SEQ ID NO: 3, SEQ ID NO: 4 and SEQ ID NO: 5, wherein the ectopic nucleic acid sequence confers upon the mouse ADAM6 function through one or more ADAM6 proteins encoded by the ectopic nucleic acid sequence.

In one aspect, the nucleic acid sequence is any sequence encoding an ADAM6 gene or homolog or ortholog or functional fragment thereof that when placed into or maintained in a mouse yields a level of fertility that is the same or comparable to a wild-type mouse. An exemplary level of fertility may be demonstrated by the ability of a male mouse to produce a sperm cell that is capable of traversing a female mouse oviduct in order to fertilize a mouse egg.

In one aspect, a mouse is described that comprises a deletion of an endogenous nucleotide sequence that encodes an ADAM6 protein, a replacement of an endogenous V_{H} gene segment with a human V_{H} gene segment, and an ectopic nucleotide sequence that encodes a mouse ADAM6 protein or ortholog or homolog or fragment thereof that is functional in a male mouse.

In one aspect, a mouse is described that comprises a deletion of an endogenous nucleotide sequence that encodes an ADAM6 protein, a replacement of an endogenous V_{H} gene segment with a human V_{L} gene segment, and an ectopic nucleotide sequence that encodes a mouse ADAM6 protein or ortholog or homolog or fragment thereof that is functional in a male mouse. In one aspect, the human V_{L} gene segment is a Vκ gene segment. In one aspect, the V_{L} gene segment is a Vλ gene segment.

In one aspect, the mouse further comprises a human J_{L} gene segment, and the ectopic nucleotide sequence that encodes a mouse ADAM6 protein or ortholog or homolog or fragment thereof that is functional in a male mouse is positioned between a human V_{L} gene segment and the human J_{L} gene segment. In one aspect, the mouse comprises a one or more human V_{L} gene segments and one or more human V_{L} gene segments and the ectopic nucleotide sequence that encodes a mouse ADAM6 protein or ortholog or homolog or fragment thereof that is functional in a male mouse is positioned upstream (or 5') of the one or more human V_{L} gene segments. In a specific aspect, the human V_{L} and J_{L} gene segments are Vκ and Jκ gene segments.

In one aspect, the mouse comprises an immunoglobulin heavy chain locus that comprises a deletion of an endogenous immunoglobulin locus nucleotide sequence that comprises an endogenous ADAM6 gene, comprises a nucleotide sequence encoding one or more human immunoglobulin gene segments, and wherein the ectopic nucleotide sequence encoding the mouse ADAM6 protein is within or directly adjacent to the nucleotide sequence encoding the one or more human immunoglobulin gene segments.

In one aspect, the mouse comprises a replacement of all or substantially all endogenous V_{H} gene segments with a nucleotide sequence encoding one or more human V_{L} gene segments, and the ectopic nucleotide sequence encoding the mouse ADAM6 protein is within, or directly adjacent to, the nucleotide sequence encoding the one or more human V_{L} gene segments. In one aspect, the mouse further comprises a replacement of one or more endogenous D_{L} gene segments with one or more human V_{L} and/or human J_{L} gene segments at the endogenous D_{H} gene locus. In one aspect, the mouse further comprises a replacement of one or more endogenous J_{H} gene segments with one or more human J_{L} gene segments at the endogenous J_{H} gene locus. In one aspect, the mouse comprises a replacement of all or substantially all endogenous V_{H}, D_{H}, and J_{H} gene segments and a replacement at the endogenous V_{H}, D_{H}, and J_{H} gene loci with human V_{L} and J_{L} gene segments, wherein the mouse comprises an ectopic sequence encoding a mouse ADAM6 protein. In one aspect, the mouse comprises an insertion of one or more human V_{L} and J_{L} gene segments at an endogenous immunoglobulin heavy chain locus, wherein the mouse comprises an ADAM6 gene that is functional in the mouse. In a specific aspect, the human V_{L} and J_{L} gene segments are V_{κ} and J_{κ} gene segments. In a specific aspect, the ectopic sequence encoding the mouse ADAM6 protein is placed between the penultimate 3'-most V_{L} gene segment of the human V_{L} gene segments present, and the ultimate 5'-most J_{L} gene segment of the human J_{L} gene segments present. In a specific aspect, the ectopic sequence encoding the mouse ADAM6 protein is placed upstream (or 5') of the 5'-most V_{L} gene segment of the human V_{L} gene segments present. In a specific aspect, the mouse comprises a deletion of all or substantially all mouse V_{H} gene segments, and a replacement with at least 40 human V_{L} gene segments, and the ectopic nucleotide sequence encoding the mouse ADAM6 protein is placed downstream of human Vκ4-1 gene segment and upstream of human Jκ1 gene segment. In a specific aspect, the mouse comprises a deletion of all or substantially all mouse V_{H} gene segments, and a replacement with at least 40 human V_{L} gene segments, and the ectopic nucleotide sequence encoding the mouse ADAM6 protein is placed upstream of a human Vκ2-40 gene segment.

In a specific aspect, the mouse comprises a replacement of all or substantially all endogenous V_{H} gene segments with a nucleotide sequence encoding one or more human V_{L} gene segments, and the ectopic nucleotide sequence encoding the mouse ADAM6 protein is within, or directly adjacent to, the nucleotide sequence encoding the one or more human V_{L} gene segments.

In one aspect, the V_{L} gene segments are Vκ gene segments. In one aspect, the V_{L} gene segments are Vλ gene segments.

In one aspect, the ectopic nucleotide sequence that encodes the mouse ADAM6 protein is present on a transgene in the genome of the mouse. In one aspect, the ectopic nucleotide sequence that encodes the mouse ADAM6 protein is present extra-chromosomally in the mouse.

In one aspect, a mouse is described that comprises a modification of an endogenous heavy chain immunoglobulin locus, wherein the mouse expresses a B cell that comprises a rearranged immunoglobulin sequence operably linked to a heavy chain constant region gene sequence, and the B cell comprises in its genome (*e.g*., on a B cell chromosome) a gene encoding an ADAM6 or ortholog or homolog or fragment thereof that is functional in a male mouse. In one aspect, the rearranged immunoglobulin sequence operably linked to the heavy chain constant region gene sequence comprises a human light chain V, J, and optionally a D gene sequence; a mouse heavy chain V, D, and/or J sequence; a human or mouse light chain V and/or J sequence. In one aspect, the heavy chain constant region gene sequence comprises a human or a mouse heavy chain sequence selected from the group consisting of a C_{H}1, a hinge, a C_{H}2, a C_{H}3, and a combination thereof.

In one aspect, the human light chain V and/or J sequence is selected from a human Vκ, Vλ, Jκ and Jλ sequence.

In one aspect, a mouse is described that comprises a functionally silenced endogenous immunoglobulin heavy chain locus, wherein ADAM6 function is maintained in the mouse, and further comprises an insertion of one or more human immunoglobulin gene segments, wherein the one or more human immunoglobulin gene segments include human V_{L} and JL gene segments, and optionally human D_{H} gene segments. In one aspect, the one or more human immunoglobulin gene segments includes a human Vκ, Vλ, Jκ and Jλ gene segments.

In one aspect, a genetically modified mouse is described, wherein the mouse comprises a functionally silenced immunoglobulin light chain gene, and further comprises a replacement of one or more endogenous immunoglobulin heavy chain variable region gene segments with one or more human immunoglobulin light chain variable region gene segments, wherein the mouse lacks a functional endogenous ADAM6 locus, and wherein the mouse comprises an ectopic nucleotide sequence that expresses a mouse ADAM6 protein or an ortholog or homolog or fragment thereof that is functional in a male mouse.

In one aspect, a genetically modified mouse is described, wherein the mouse comprises a functionally silenced immunoglobulin light chain gene locus, and further comprises a replacement of one or more endogenous immunoglobulin light chain variable gene segments with one or more human immunoglobulin light chain variable gene segments, wherein the mouse lacks a functional endogenous ADAM6 locus, and wherein the mouse comprises an ectopic nucleotide sequence that encodes a mouse ADAM6 protein or an ortholog or homolog or fragment thereof that is functional in a male mouse.

In one aspect, the one or more human immunoglobulin light chain variable gene segments are contiguous with the ectopic nucleotide sequence.

In one aspect, a mouse is described that lacks a functional endogenous ADAM6 locus or sequence and that comprises an ectopic nucleotide sequence encoding a mouse ADAM6 locus or functional fragment of a mouse ADAM6 locus or sequence, wherein the mouse is capable of mating with a mouse of the opposite sex to produce a progeny that comprises the ectopic ADAM6 locus or sequence. In one aspect, the mouse is male. In one aspect, the mouse is female.

In one aspect, a genetically modified mouse is described, wherein the mouse comprises a human immunoglobulin light chain variable region gene segment at an endogenous immunoglobulin heavy chain variable region gene locus, the mouse lacks an endogenous functional ADAM6 sequence at the endogenous immunoglobulin heavy chain variable region gene locus, and wherein the mouse comprises an ectopic nucleotide sequence that expresses a mouse ADAM6 protein or an ortholog or homolog or fragment thereof that is functional in a male mouse.

In one aspect, a genetically modified mouse is described, wherein the mouse comprises a human immunoglobulin light chain variable gene segment at an endogenous immunoglobulin heavy chain variable region gene locus, the mouse lacks an endogenous functional ADAM6 sequence at the endogenous immunoglobulin heavy chain variable gene locus, and wherein the mouse comprises an ectopic nucleotide sequence that expresses a mouse ADAM6 protein or an ortholog or homolog or fragment thereof that is functional in a male mouse.

In one aspect, the ectopic nucleotide sequence that expresses the mouse ADAM6 protein is extra-chromosomal. In one aspect, the ectopic nucleotide sequence that expresses the mouse ADAM6 protein is integrated at one or more loci in a genome of the mouse. In a specific aspect, the one or more loci include an immunoglobulin locus.

In one aspect, a mouse is described that expresses an immunoglobulin light chain sequence from a modified endogenous immunoglobulin heavy chain locus, wherein the heavy chain is derived from a human V_{L} gene segment, a J gene segment, and optionally a D_{H} gene segment, wherein the mouse comprises an ADAM6 activity that is functional in the mouse. In one aspect, the human V_{L} gene segment is selected from a human Vκ and a human Vλ gene segment. In various aspects, the J gene segment is a J_{H}, a Jκ or a Jλ gene segment or a combination thereof.

In one aspect the mouse comprises a plurality of human V_{L} gene segments and a plurality of J gene segments. In a specific aspect, the J gene segments are J_{L} gene segments.

In one aspect, a mouse is described that expresses an immunoglobulin light chain sequence from a modified endogenous immunoglobulin heavy chain locus, wherein the heavy chain is derived from a human V_{L} gene segment and a J_{L} gene segment, wherein the mouse comprises an ADAM6 activity that is functional in the mouse.

In one aspect, the mouse comprises a plurality of human V gene segments, a plurality of J gene segments, and optionally a plurality of D gene segments. In one aspect, the D gene segments are human D gene segments. In one aspect, the J gene segments are human J gene segments. In one aspect, the mouse further comprises a humanized heavy chain constant region sequence, wherein the humanization comprises replacement of a sequence selected from a C_{H}1, hinge, C_{H}2, C_{H}3, and a combination thereof. In a specific aspect, the heavy chain is derived from a human V gene segment, a human J gene segment, a human C_{H}1 sequence, a human or mouse hinge sequence, a mouse C_{H}2 sequence, and a mouse C_{H}3 sequence. In another specific aspect, the mouse further comprises a human light chain constant sequence. In one aspect, the mouse comprises an ADAM6 gene that is flanked 5' and 3' by endogenous immunoglobulin heavy chain gene segments. In a specific aspect, the endogenous immunoglobulin heavy chain variable gene segments are incapable of encoding a heavy chain variable region of an antibody. In a specific aspect, the ADAM6 gene of the mouse is at a position that is the same as in a wild-type mouse and the endogenous immunoglobulin heavy chain variable gene loci of the mouse are incapable of rearranging to encode a heavy chain of an antibody.

In one aspect, the plurality of human V gene segments are flanked 5' (with respect to transcriptional direction of the V gene segments) by a sequence encoding an ADAM6 activity that is functional in the mouse. In a specific aspect, the plurality of human V gene segments include human Vκ gene segments Vκ4-1, Vκ5-2, Vκ7-3, Vκ2-4, Vκ1-5, Vκ1-6, Vκ3-7, Vκ1-8, Vκ1-9, Vκ2-10, Vκ3-11, Vκ1-12, Vκ1-13, Vκ2-14, Vκ3-15, Vκ1-16, Vκ1-17, Vκ2-18, Vκ2-19, Vκ3-20, Vκ6-21, Vκ1-22, Vκ1-23, Vκ2-24, Vκ3-25, Vκ2-26, Vκ1-27, Vκ2-28, Vκ2-29, Vκ2-30, Vκ3-31, Vκ1-32, Vκ1-33, Vκ3-34, Vκ1-35, Vκ2-36, Vκ1-37, Vκ2-38, Vκ1-39, and Vκ2-40 and the human Vκ2-40 gene segment is flanked 5' (with respect to transcriptional direction of the human Vκ2-40 gene segment) by a sequence encoding an ADAM6 activity that is functional in the mouse. In a specific aspect, the sequence encoding an ADAM6 activity that is functional in the mouse is placed in the same transcription orientation with respect to the human Vκ gene segments. In a specific aspect, the sequence encoding an ADAM6 activity that is functional in the mouse is placed in the opposite transcription orientation with respect to the human Vκ gene segments.

In one aspect, the V gene segment is flanked 3' (with respect to transcriptional direction of the V gene segment) by a sequence encoding an ADAM6 activity that is functional in the mouse.

In one aspect, the D gene segment is flanked 5' (with respect to transcriptional direction of the D gene segment) by a sequence encoding an ADAM6 activity that is functional in the mouse.

In one aspect, the J gene segment is flanked 5' (with respect to transcriptional direction of the J gene segment) by a sequence encoding an ADAM6 activity that is functional in the mouse.

In one aspect, the ADAM6 activity that is functional in the mouse results from expression of a nucleotide sequence located 5' of the 5'-most D gene segment and 3' of the most 3'-most V gene segment (with respect to the direction of transcription of the V gene segment) of the modified endogenous heavy chain immunoglobulin locus.

In one aspect, the ADAM6 activity that is functional in the mouse results from expression of a nucleotide sequence located 5' of the 5'-most J gene segment and 3' of the 3'-most V gene segment (with respect to the direction of transcription of the V gene segment) of the modified endogenous immunoglobulin heavy chain locus.

In one aspect, the ADAM6 activity that is functional in the mouse results from expression of a nucleotide sequence located between two human V gene segments in the modified endogenous immunoglobulin heavy chain locus. In one aspect, the two human V gene segments are a human Vκ5-2 gene segment and a Vκ4-1 gene segment.

In one aspect, the ADAM6 activity that is functional in the mouse results from expression of a nucleotide sequence located between a human V gene segment and a human J gene segment in the modified endogenous immunoglobulin heavy chain locus. In one aspect, the human V gene segment is a human Vκ4-1 gene segment and the human J segment is a Jκ1 gene segment.

In one aspect, the nucleotide sequence comprises a sequence selected from a mouse ADAM6b sequence or functional fragment thereof, a mouse ADAM6a sequence or functional fragment thereof, and a combination thereof.

In various aspects, the sequence encoding an ADAM6 activity that is functional in the mouse encodes a ADAM6b protein set forth in SEQ ID NO: 2 and/or encodes a ADAM6a protein set forth in SEQ ID NO: 1.

In one aspect, the nucleotide sequence between the two human V gene segments is placed in opposite transcription orientation with respect to the human V gene segments. In a specific aspect, nucleotide sequence encodes, from 5' to 3' with respect to the direction of transcription of ADAM6 genes, an ADAM6a sequence followed by an ADAM6b sequence. In one aspect, t

In one aspect, the nucleotide sequence between the human V gene segment and the human J gene segment is placed in opposite transcription orientation with respect to the human V and J gene segments. In a specific aspect, the nucleotide sequence encodes, from 5' to 3' with respect to the direction of transcription of ADAM6 genes, an ADAM6a sequence followed by an ADAM6b sequence.

In one aspect, the mouse comprises a hybrid immunoglobulin sequence, wherein the hybrid immunoglobulin sequence comprises a human immunoglobulin κ light chain sequence contiguous with a non-human ADAM6 sequence.

In one aspect, the mouse comprises a human sequence contiguous with a mouse sequence at an endogenous immunoglobulin heavy chain locus, wherein the contiguous sequence comprises at least one human V_{L} gene segment, a mouse ADAM6 sequence or ortholog or homolog or functional fragment thereof, and a human J_{L} gene segment. In a specific aspect, the mouse ADAM6 sequence or ortholog or homolog or functional fragment thereof is positioned immediately adjacent to the at least one human V_{L} gene segment. In one aspect, the human V_{L} gene segment is a human Vκ gene segment. In one aspect, the mouse ADAM6 sequence or ortholog or homolog or functional fragment thereof is positioned immediately adjacent and 3' to the at least one human V_{L} gene segment and immediately adjacent and 5' to the human J_{L} gene segment. In a specific aspect, the human V_{L} gene segment is a human Vκ gene segment and the human J_{L} gene segment is a human Jκ gene segment.

In one aspect, the sequence encoding the ADAM6 activity that is functional in the mouse is a mouse ADAM6 sequence or functional fragment thereof.

In one aspect, the mouse comprises an endogenous mouse DFL16.1 gene segment (*e.g*., in a mouse heterozygous for the modified endogenous mouse immunoglobulin heavy chain locus), or a human D_{H}1-1 gene segment. In one aspect, the D gene segment of the immunoglobulin heavy chain expressed by the mouse is derived from an endogenous mouse DFL16.1 gene segment or a human D_{H}1-1 gene segment.

In one aspect, a mouse is described that comprises a nucleic acid sequence encoding a mouse ADAM6 (or homolog or ortholog or functional fragment thereof) in a DNA-bearing cell of non-rearranged B cell lineage, but does not comprise the nucleic acid sequence encoding the mouse ADAM6 (or homolog or ortholog or functional fragment thereof) in a B cell that comprise rearranged immunoglobulin loci, wherein the nucleic acid sequence encoding the mouse ADAM6 (or homolog or ortholog or functional fragment thereof) occurs in the genome at a position that is different from a position in which a mouse ADAM6 gene appears in a wild-type mouse. In one aspect, the nucleic acid sequence encoding the mouse ADAM6 (or homolog or ortholog or functional fragment thereof) is present in all or substantially all DNA-bearing cells that are not of rearranged B cell lineage; in one aspect, the nucleic acid sequence is present in germline cells of the mouse, but not in a chromosome of a rearranged B cell.

In one aspect, a mouse is described that comprises a nucleic acid sequence encoding a mouse ADAM6 (or homolog or ortholog or functional fragment thereof) in all or substantially all DNA-bearing cells, including B cells that comprise rearranged immunoglobulin loci, wherein the nucleic acid sequence encoding the mouse ADAM6 (or homolog or ortholog or functional fragment thereof) occurs in the genome at a position that is different from a position in which a mouse ADAM6 gene appears in a wild-type mouse. In one aspect, the nucleic acid sequence encoding the mouse ADAM6 (or homolog or ortholog or functional fragment thereof) is on a nucleic acid that is contiguous with the rearranged immunoglobulin locus. In one aspect, the nucleic acid that is contiguous with the rearranged immunoglobulin locus is a chromosome. In one aspect, the chromosome is a chromosome that is found in a wild-type mouse and the chromosome comprises a modification of a mouse immunoglobulin locus.

In one aspect, a genetically modified mouse is described, wherein the mouse comprises a B cell that comprises in its genome an ADAM6 sequence or ortholog or homolog thereof. In one aspect, the ADAM6 sequence or ortholog or homolog thereof is at an immunoglobulin heavy chain locus. In a specific aspect, the heavy chain locus comprises endogenous immunoglobulin heavy chain gene segments that are incapable of rearranging to encode the heavy chain of an antibody. In one aspect, the ADAM6 sequence or ortholog or homolog thereof is at a locus that is not an immunoglobulin locus. In one aspect, the ADAM6 sequence is on a transgene driven by a heterologous promoter. In a specific aspect, the heterologous promoter is a non-immunoglobulin promoter. In a specific aspect, B cell expresses an ADAM6 protein or ortholog or homolog thereof.

In one aspect, 90% or more of the B cells of the mouse comprise a gene encoding an ADAM6 protein or an ortholog thereof or a homolog thereof or a fragment thereof that is functional in the mouse. In a specific aspect, the mouse is a male mouse.

In one aspect, the B cell genome comprises a first allele and a second allele comprising the ADAM6 sequence or ortholog or homolog thereof. In one aspect, the B cell genome comprises a first allele but not a second allele comprising the ADAM6 sequence or ortholog or homolog thereof.

In one aspect, a mouse is described that comprises a modification at one or more endogenous immunoglobulin heavy chain alleles, wherein the modification maintains one or more endogenous ADAM6 alleles.

In one aspect, the modification renders the mouse incapable of expressing a functional heavy chain that comprises rearranged endogenous heavy chain gene segments from at least one heavy chain allele and maintains an endogenous ADAM6 allele located within the at least one endogenous immunoglobulin heavy chain allele.

In one aspect, the mice are incapable of expressing a functional heavy chain that comprises rearranged endogenous heavy chain gene segments from at least one of the endogenous immunoglobulin heavy chain alleles, and the mice express and ADAM6 protein from an endogenous ADAM6 allele. In a specific aspect, the mice are incapable of expressing a functional heavy chain that comprises rearranged endogenous heavy chain gene segments from two endogenous immunoglobulin heavy chain alleles, and the mice express an ADAM6 protein from one or more endogenous ADAM6 alleles.

In one aspect, the mice are incapable of expressing a functional heavy chain from each endogenous heavy chain allele, and the mice comprise a functional ADAM6 allele located within 1, 2, 3, 4, 5, 10, 20, 30, 40, 50, 60, 70, 80, 90, 100, 110, or 120 or more Mbp upstream (with respect to the direction of transcription of the mouse immunoglobulin heavy chain locus) of a mouse immunoglobulin heavy chain constant region sequence. In a specific aspect, the functional ADAM6 allele is at the endogenous heavy chain locus (e.g., in an intergenic V-D region, between two V gene segments, between a V and a D gene segment, between a D and a J gene segment, *etc*.). In a specific aspect, the functional ADAM6 allele is located within a 90 to 100 kb intergenic sequence between the final mouse V gene segment and the first mouse D gene segment. In another specific aspect, the endogenous 90 to 100 kb intergenic V-D sequence is removed, and the ectopic ADAM6 sequence is placed between the final V and the first D gene segment.

In one aspect, a mouse is described that comprises a modification at one or more endogenous ADAM6 alleles.

In one aspect, the modification renders the mouse incapable of expressing a functional ADAM6 protein from at least one of the one or more endogenous ADAM6 alleles. In a specific aspect, the mouse is incapable of expressing a functional ADAM6 protein from each of the endogenous ADAM6 alleles.

In one aspect, the mouse is incapable of expressing a functional ADAM6 protein from each endogenous ADAM6 allele, and the mice comprise an ectopic ADAM6 sequence.

In one aspect, the mouse is incapable of expressing a functional ADAM6 protein from each endogenous ADAM6 allele, and the mouse comprises an ectopic ADAM6 sequence located within 1, 2, 3, 4, 5, 10, 20, 30, 40, 50, 60, 70, 80, 90, 100, 110, or 120 or more kb upstream (with respect to the direction of transcription of the mouse heavy chain locus) of a mouse immunoglobulin heavy chain constant region sequence. In a specific aspect, the ectopic ADAM6 sequence is at the endogenous immunoglobulin heavy chain locus (*e.g*., in an intergenic V-D region, between two V gene segments, between a V and a J gene segment, between a D and a J gene segment, *etc*.). In a specific aspect, the ectopic ADAM6 sequence is located within a 90 to 100 kb intergenic sequence between the final mouse V gene segment and the first mouse D gene segment. In another specific aspect, the endogenous 90 to 100 kb intergenic V-D sequence is removed, and the ectopic ADAM6 sequence is placed between a human Vκ gene segment and a first human Jκ gene segment. In another specific aspect, the endogenous 90 to 100 kb intergenic V-D sequence is removed, and the ectopic ADAM6 sequence is placed 5' or upstream of a human Vκ gene segment, wherein the human Vκ gene segment is selected from a human Vκ4-1 or Vκ2-40 gene segment.

In one aspect, the mouse is capable of expressing a functional ADAM6 protein from one or more endogenous ADAM6 alleles and the modification includes an insertion of a human sequence that encodes an immunoglobulin variable domain. In one aspect, the human sequence comprises unrearranged immunoglobulin gene segments. In a specific aspect, the human sequence comprises a V gene segment and a J gene segment. In another specific aspect, the human sequence comprises a V gene segment, a J gene segment, and a D gene segment.

In one aspect, an infertile male mouse is described, wherein the mouse comprises a deletion of two or more endogenous ADAM6 alleles. In one aspect, a female mouse is described that is a carrier of a male infertility trait, wherein the female mouse comprises in its germline a nonfunctional ADAM6 allele or a knockout of an endogenous ADAM6 allele.

In one aspect, a mouse comprising an endogenous immunoglobulin heavy chain V, D, and or J gene segment that are incapable of rearranging to encode an heavy chain of an antibody is described, wherein the majority of the B cells of the mouse comprise an functional ADAM6 gene. In various aspects, the majority of the B cells further comprise one or more human V_{L} and J_{L} gene segments upstream of a mouse immunoglobulin heavy chain constant region. In one aspect, the human V_{L} and J_{L} gene segments are Vκ and Jκ gene segments.

In one aspect, the mouse comprises an intact endogenous immunoglobulin heavy chain V, D, and J gene segments that are incapable of rearranging to encode a functional heavy chain of an antibody. In one aspect, the mouse comprises at least one and up to 89 V gene segments, at least one and up to 13 D gene segments, at least one and up to four J gene segments, and a combination thereof; wherein the at least one and up to 89 V gene segments, at least one and up to 13 D gene segments, at least one and up to four J gene segments are incapable of rearranging to encode a heavy chain variable region of an antibody. In a specific aspect, the mouse comprises a functional ADAM6 gene located within the intact endogenous immunoglobulin heavy chain V, D, and J gene segments. In one aspect, the mouse comprises an endogenous heavy chain locus that includes an endogenous ADAM6 locus, wherein the endogenous heavy chain locus comprises 89 V gene segments, 13 D gene segments, and four J gene segments, wherein the endogenous heavy chain gene segments are incapable of rearranging to encode a heavy chain variable region of an antibody and the ADAM6 locus encodes an ADAM6 protein that is functional in the mouse.

In one aspect, a method is described for making an infertile male mouse, comprising rendering an endogenous ADAM6 allele of a donor ES cell nonfunctional (or knocking out said allele), introducing the donor ES cell into a host embryo, gestating the host embryo in a surrogate mother, and allowing the surrogate mother to give birth to progeny derived in whole or in part from the donor ES cell. In one aspect, the method further comprises breeding progeny to obtain an infertile male mouse.

In one aspect, a method is described for making a mouse with a genetic modification of interest, wherein the mouse is infertile, the method comprising the steps of (a) making a genetic modification of interest in a genome; (b) modifying the genome to knockout an endogenous ADAM6 allele, or render an endogenous ADAM6 allele nonfunctional; and, (c) employing the genome in making a mouse. In various aspects, the genome is from an ES cell or used in a nuclear transfer experiment.

In one aspect, a mouse that lacks an endogenous immunoglobulin heavy chain V, D, and J gene segment is described, wherein a majority of the B cells of the mouse comprise an ADAM6 sequence or ortholog or homolog thereof.

In one aspect, the mouse lacks endogenous immunoglobulin heavy chain gene segments selected from two or more V gene segments, two or more D gene segments, two or more J gene segments, and a combination thereof. In one aspect, the mouse lacks immunoglobulin heavy chain gene segments selected from at least one and up to 89 V gene segments, at least one and up to 13 D gene segments, at least one and up to four J gene segments, and a combination thereof. In one aspect, the mouse lacks a genomic DNA fragment from chromosome 12 comprising about three megabases of the endogenous immunoglobulin heavy chain locus. In a specific aspect, the mouse lacks all functional endogenous heavy chain V, D, and J gene segments. In a specific aspect, the mouse lacks 89 V_{H} gene segments, 13 D_{H} gene segments and four J_{H} gene segments.

In one aspect, a mouse is described, wherein the mouse has a genome in the germline comprising a modification of an immunoglobulin heavy chain locus, wherein the modification to the immunoglobulin heavy chain locus comprises the replacement of one or more mouse immunoglobulin variable region sequences with one or more non-mouse immunoglobulin variable region sequences, and wherein the mouse comprises a nucleic acid sequence encoding a mouse ADAM6 protein. In one aspect, the D_{H} and J_{H} sequences and at least 3, at least 10, at least 20, at least 40, at least 60, or at least 80 V_{H} sequences of the endogenous immunoglobulin heavy chain locus are replaced by non-mouse immunoglobulin light chain sequences. In one aspect, the D_{H}, J_{H}, and all V_{H} sequences of the endogenous immunoglobulin heavy chain locus are replaced by a plurality of non-mouse immunoglobulin V_{L} gene segments, one or more J_{L} gene segment, and optionally one or more D gene segment sequences. The non-mouse immunoglobulin sequences can be unrearranged. In one aspect, the non-mouse immunoglobulin sequences comprise complete unrearranged V_{L} and J_{L} regions of the non-mouse species. In one aspect, the non-mouse immunoglobulin sequences are capable of forming a complete variable region, *i.e*., a rearranged variable region containing V_{L} and J_{L} gene segments joined together to form an sequence that encodes a light chain variable region, of the non-mouse species, operably linked to one or more endogenous constant regions. The non-mouse species can be Homo sapiens and the non-mouse immunoglobulin sequences can be human sequences.

In one aspect, a genetically modified mouse comprising a nucleotide sequence encoding an ADAM6 protein or functional fragment thereof that is contiguous with a human immunoglobulin light chain variable gene segment is described.

In one aspect, the mouse lacks an endogenous unmodified ADAM6 gene sequence. In one aspect, the mouse lacks a functional endogenous ADAM6 gene sequence.

In one aspect, the human immunoglobulin light chain variable gene segment is an immunoglobulin κ light chain variable gene segment. In one aspect, the human immunoglobulin light chain variable gene segment is an immunoglobulin λ light chain variable gene segment. In one aspect, the human immunoglobulin light chain variable gene segment is operably linked to an immunoglobulin heavy chain constant gene sequence.

In one aspect, the immunoglobulin heavy chain constant gene sequence is a mouse or rat or human heavy chain gene sequence. In one aspect, the heavy chain constant gene sequence comprises a C_{H}1 and/or a hinge region.

In one aspect, the mouse comprises deletion of, or replacement of, one or more endogenous immunoglobulin heavy chain gene sequences.

In one aspect, the mouse further comprises an unrearranged human Vκ or unrearranged human Vλ gene segment operably linked to a human or mouse or rat light chain constant region sequence. In one aspect, the mouse comprises a plurality of unrearranged human Vκ gene segments (*e.g*., two or more human Vκ segments and one or more human Jκ segments) or a plurality of unrearranged human Vλ gene segments (*e.g*., two or more human Yλ segments and one or more human Jλ segments). In one aspect, the unrearranged human Vκ or unrearranged human Vλ gene segments are operably linked to a constant region sequence at an endogenous immunoglobulin light chain locus.

In one aspect, the mouse further comprises a modification that renders an endogenous κ light chain locus and/or an endogenous λ light chain locus nonfunctional. In one aspect, the mouse comprises a knockout or a deletion of an endogenous mouse κ and/or an endogenous mouse λ light chain locus.

In one aspect, a method for maintaining a mouse strain is described, wherein the mouse strain comprises a replacement of a mouse immunoglobulin heavy chain sequence with one or more human immunoglobulin light chain sequences. In one aspect, the one or more human immunoglobulin light chain sequences are human immunoglobulin Vκ and/or JK gene segments.

In one aspect, the mouse strain comprises a deletion of one or more mouse V_{H}, D_{H}, and/or J_{H} gene segments. In one aspect, the mouse further comprises one or more human V_{L} gene segments and one or more human J_{L} gene segments. In one aspect, the mouse comprises at least 6, at least 16, at least 30, or at least 40 human Vκ gene segments and at least five Jκ gene segments. In a specific aspect, the human light chain gene segments are operably linked to a constant region gene. In one aspect, the constant region gene is a mouse constant region gene. In one aspect, the constant region gene comprises a mouse constant region gene sequence selected from a C_{H}1, a hinge, a C_{H}2, a C_{H}3, and/or a C_{H}4 or a combination thereof.

In one aspect, the method comprises generating a male mouse heterozygous for the replacement of the mouse immunoglobulin heavy chain sequence, and breeding the heterozygous male mouse with a wild-type female mouse or a female mouse that is homozygous or heterozygous for the human heavy chain sequence. In one aspect, the method comprises maintaining the strain by repeatedly breeding heterozygous males with females that are wild type or homozygous or heterozygous for the human heavy chain sequence.

In one aspect, the method comprises obtaining cells from male or female mice homozygous or heterozygous for the human heavy chain sequence, and employing those cells as donor cells or nuclei therefrom as donor nuclei, and using the cells or nuclei to make genetically modified animals using host cells and/or gestating the cells and/or nuclei in surrogate mothers.

In one aspect, only male mice that are heterozygous for the replacement at the heavy chain locus are bred to female mice. In a specific aspect, the female mice are homozygous, heterozygous, or wild type with respect to a replaced heavy chain locus.

In one aspect, the mouse further comprises a replacement of λ and/or κ light chain variable sequences at an endogenous immunoglobulin light chain locus with heterologous immunoglobulin light chain sequences. In one aspect, the heterologous immunoglobulin light chain sequences are human immunoglobulin λ and/or κ light chain variable sequences.

In one aspect, the mouse further comprises a transgene at a locus other than an endogenous immunoglobulin locus, wherein the transgene comprises a sequence encoding a rearranged or unrearranged heterologous λ or κ light chain sequence (*e.g*., unrearranged V_{L} and unrearranged J_{L}, or rearranged VJ) operably linked (for unrearranged) or fused (for rearranged) to an immunoglobulin light chain constant region sequence. In one aspect, the heterologous λ or κ light chain sequence is human. In one aspect, the constant region sequence is selected from rodent, human, and non-human primate. In one aspect, the constant region sequence is selected from mouse, rat, and hamster. In one aspect, the transgene comprises a non-immunoglobulin promoter that drives expression of the light chain sequences. In a specific aspect, the promoter is a transcriptionally active promoter. In a specific aspect, the promoter is a ROSA26 promoter.

In one aspect, a fertile mouse is described that comprises a modification of an endogenous ADAM6 gene, wherein the mouse comprises an ectopic sequence that confers ADAM6 function to the mouse, and wherein the mouse comprises in its germline an unrearranged human immunoglobulin light chain gene segment operably linked to a nucleic acid sequence encoding an immunoglobulin heavy chain sequence.

In one aspect, a fertile mouse is described that comprises a modification of an endogenous ADAM6 locus, wherein the modification renders the ADAM6 locus nonfunctional, and wherein the mouse expresses an immunoglobulin binding protein comprising a human immunoglobulin light chain variable domain contiguous with a heavy chain constant sequence.

In one aspect, the immunoglobulin binding protein further comprises a cognate human immunoglobulin light chain variable domain fused with a light chain constant sequence.

In one aspect, the heavy chain constant sequence and the light chain constant sequence are non-human.

In one aspect, a mouse is described, comprising an immunoglobulin heavy chain locus comprising a replacement of one or more immunoglobulin heavy chain variable region (V_{H}) gene segments, heavy chain diversity (D_{H}) gene segments, and heavy chain joining (J_{H}) gene segments at an endogenous immunoglobulin heavy chain locus with one or more light chain variable region (V_{L}) gene segments and one or more light chain joining region (J_{L}) gene segments, wherein the mouse is capable of expressing an ADAM6 protein.

In one aspect, a mouse is described, comprising an immunoglobulin heavy chain locus that comprises a replacement of all or substantially all V_{H}, D_{H}, and J_{H} gene segments with one or more V_{L} gene segments and one or more J_{L} gene segments to form a V_{L} gene segment sequence at an endogenous heavy chain locus (VL_{H} locus), wherein the VL_{H} locus is capable of recombining with an endogenous C_{H} gene to form a rearranged gene that is derived from a V_{L} gene segment, a J_{L} gene segment, and an endogenous C_{H} gene.

In one aspect, the V_{L} segments are human V_{L}. In one aspect, the J_{L} segments are human J_{L}. In a specific aspect, the V_{L} and J_{L} segments are human V_{L} and human J_{L} segments.

In one aspect, all or substantially all V_{H}, D_{H}, and J_{H} gene segments are replaced with at least six human Vκ gene segments and at least one Jκ gene segment. In one aspect, all or substantially all V_{H}, D_{H}, and J_{H} gene segments are replaced with at least 16 human Vκ gene segments (human Vκ) and at least one Jκ gene segment. In one aspect, all or substantially all V_{H}, D_{H}, and J_{H} gene segments are replaced with at least 30 human Vκ gene segments and at least one Jκ gene segment. In one aspect, all or substantially all V_{H}, D_{H}, and J_{H} gene segments are replaced with at least 40 human Vκ gene segments and at least one Jκ gene segment. In one aspect, the at least one Jκ gene segment comprises two, three, four, or five human Jκ gene segments.

In one aspect, the V_{L} segments are human Vκ segments. In one aspect, the human Vκ segments comprise 4-1, 5-2, 7-3, 2-4, 1-5, and 1-6. In one aspect, the human Vκ segments comprise 3-7, 1-8, 1-9, 2-10, 3-11, 1-12, 1-13, 2-14, 3-15, and 1-16. In one aspect, the human Vκ segments comprise 1-17, 2-18, 2-19, 3-20, 6-21, 1-22, 1-23, 2-24, 3-25, 2-26, 1-27, 2-28, 2-29, and 2-30. In one aspect, the human Vκ segments comprise 3-31, 1-32, 1-33, 3-34, 1-35, 2-36, 1-37, 2-38, 1-39, and 2-40.

In one aspect, the V_{L} segments are human Vκ segments and comprise 4-1, 5-2, 7-3, 2-4, 1-5, 1-6, 3-7, 1-8, 1-9, 2-10, 3-11, 1-12, 1-13, 2-14, 3-15, and 1-16. In one aspect, the Vκ segments further comprise 1-17, 2-18, 2-19, 3-20, 6-21, 1-22, 1-23, 2-24, 3-25, 2-26, 1-27, 2-28, 2-29, and 2-30. In one aspect, the Vκ segments further comprise 3-31, 1-32, 1-33, 3-34, 1-35, 2-36, 1-37, 2-38, 1-39, and 2-40.

In one aspect, the V_{L} segments are human Vλ segments and comprise a fragment of cluster A of the human λ light chain locus. In a specific aspect, the fragment of cluster A of the human λ light chain locus extends from hVλ3-27 through hVλ3-1.

In one aspect, the V_{L} segments comprise a fragment of cluster B of the human λ light chain locus. In a specific aspect, the fragment of cluster B of the human λ light chain locus extends from hVλ5-52 through hVλ1-40.

In one aspect, the V_{L} segments comprise a human λ light chain variable region sequence that comprises a genomic fragment of cluster A and a genomic fragment of cluster B. In a one aspect, the human λ light chain variable region sequence comprises at least one gene segment of cluster A and at least one gene segment of cluster B.

In one aspect, the V_{L} segments comprise at least one gene segment of cluster B and at least one gene segment of cluster C.

In one aspect, the V_{L} segments comprise hVλ 3-1, 4-3, 2-8, 3-9, 3-10, 2-11, and 3-12. In a specific aspect, the V_{L} segments comprise a contiguous sequence of the human λ light chain locus that spans from Vλ3-12 to Vλ3-1. In one aspect, the contiguous sequence comprises at least 2, 3, 4, 5, 6, 7, 8, 9, 10, 11 or 12 hVλs. In a specific aspect, the hVλs include 3-1, 4-3, 2-8, 3-9, 3-10, 2-11, and 3-12. In a specific aspect, the hVλs comprises a contiguous sequence of the human λ locus that spans from Vλ3-12 to Vλ3-1.

In one aspect, the hVλs comprises 13 to 28 or more hVλs. In a specific aspect, the hVλs include 2-14, 3-16, 2-18, 3-19, 3-21, 3-22, 2-23, 3-25, and 3-27. In a specific aspect, the hVλs comprise a contiguous sequence of the human λ locus that spans from Yλ3-27 to Vλ3-1.

In one aspect, the V_{L} segments comprise 29 to 40 hVλs. In a specific aspect, the V_{L} segments comprise a contiguous sequence of the human λ locus that spans from Vλ3-29 to Vλ3-1, and a contiguous sequence of the human λ locus that spans from Vλ5-52 to Vλ1-40. In a specific aspect, all or substantially all sequence between hVλ1-40 and hVλ3-29 in the genetically modified mouse consists essentially of a human λ sequence of approximately 959 bp found in nature (*e.g*., in the human population) downstream of the hVλ1-40 gene segment (downstream of the 3' untranslated portion), a restriction enzyme site (*e.g*., Pl-Scel), followed by a human λ sequence of approximately 3,431 bp upstream of the hVλ3-29 gene segment found in nature.

In one aspect, the Jκ is human and is selected from the group consisting of Jκ1, Jκ2, Jκ3, Jκ4, Jκ5, and a combination thereof. In a specific aspect, the Jκ comprises Jκ1 through Jκ5.

In one aspect, the V_{L} segments are human Vλ segments, and the Jκ gene segment comprises an RSS having a 12-mer spacer, wherein the RSS is juxtaposed at the upstream end of the Jκ gene segment. In one aspect, the V_{L} gene segments are human Vλ and the VL_{H} locus comprises two or more Jκ gene segments, each comprising an RSS having a 12-mer spacer wherein the RSS is juxtaposed at the upstream end of each Jκ gene segment.

In a specific aspect, the V_{L} segments comprise contiguous human κ gene segments spanning the human κ locus from Vκ4-1 through Vκ2-40, and the J_{L} segments comprise contiguous gene segments spanning the human κ locus from Jκ1 through Jκ5.

In one aspect, where the V_{L} segments are Vλ segments and no D_{H} segment is present between the V_{L} segments and J segments, the V_{L} segments are flanked downstream (*i.e*., juxtaposed on the downstream side) with 23-mer RSS, and Jκ segments if present or Jλ segments if present are flanked upstream (*i.e*., juxtaposed on the upstream side) with 12-mer RSS.

In one aspect, where the V gene segments are Vκ gene segments and no D_{H} gene segment is present between the V gene segments and J gene segments, the Vκ gene segments are each juxtaposed on the downstream side with a 12-mer RSS, and Jκ segments if present or Jλ segments if present are each juxtaposed on the upstream side with a 23-mer RSS.

In one aspect, the mouse comprises a rearranged gene that is derived from a V_{L} gene segment, a J_{L} gene segment, and an endogenous C_{H} gene. In one aspect, the rearranged gene is somatically mutated. In one aspect, the rearranged gene comprises 1, 2, 3, 4, 5, 6, 7, 8, 9, 10 or more N additions. In one aspect, the N additions and/or the somatic mutations observed in the rearranged gene derived from the V_{L} segment and the J_{L} segment are 1.5-fold, 2-fold, 2.5-fold, 3-fold, 3.5-fold, 4-fold, 4.5-fold, or at least 5-fold more than the number of N additions and/or somatic mutations observed in a rearranged light chain variable domain (derived from the same V_{L} gene segment and the same J_{L} gene segment) that is rearranged at an endogenous light chain locus. In one aspect, the rearranged gene is in a B cell that specifically binds an antigen of interest, wherein the B cell binds the antigen of interest with a K_{D} in the low nanomolar range or lower (*e.g*., a K_{D} of 10 nanomolar or lower). In a specific aspect, the V_{L} segment, the J_{L} segment, or both, are human gene segments. In a specific aspect, the V_{L} and J_{L} segments are human κ gene segments. In one aspect, the mouse C_{H} gene is selected from IgM, IgD, IgG, IgA and IgE. In a specific aspect, the mouse IgG is selected from IgG1, IgG2A, IgG2B, IgG2C and IgG3. In another specific aspect, the mouse IgG is IgG1.

In one aspect, the mouse comprises a B cell, wherein the B cell expresses from a locus on a chromosome of the B cell a binding protein consisting essentially of four polypeptide chains, wherein the four polypeptide chains consist essentially of (a) two identical polypeptides that comprise an endogenous C_{H} region fused with a V_{L}; and, (b) two identical polypeptides that comprise an endogenous C_{L} region fused with a V_{L} region that is cognate with respect to the V_{L} region that is fused with the mouse C_{H} region, and, in one aspect, is a human *(e.g*., a human κ) V_{L} region. In one aspect, the V_{L} region fused to the endogenous C_{H} region is a human V_{L} region. In a specific aspect, the human V_{L} region fused with the mouse C_{H} region is a Vκ region. In a specific aspect, the human V_{L} region fused with the mouse C_{H} region is identical to a V region encoded by a rearranged human germline light chain nucleotide sequence. In a specific aspect, the human V_{L} region fused to the mouse C_{H} region comprises two, three, four, five, six, or more somatic hypermutations. In one aspect, the human V_{L} region fused to the mouse C_{H} region is encoded by a rearranged gene that comprises 1, 2, 3, 4, 5, 6, 7, 8, 9, or 10 or more N additions.

In one aspect, at least 50% of all IgG molecules expressed by the mouse comprise a polypeptide that comprises an IgG isotype C_{H} region and a V_{L} region, wherein the length of said polypeptide is no longer than 535, 530, 525, 520, or 515 amino acids. In one aspect, at least 75% of all IgG molecules comprise the polypeptide recited in this paragraph. In one aspect, at least 80%, 85%, 90%, or 95% of all IgG molecules comprise the polypeptide recited in this paragraph. In a specific aspect, all IgG molecules expressed by the mouse comprise a polypeptide that is no longer than the length of the polypeptide recited in this paragraph.

In one aspect, the mouse expresses a binding protein comprising a first polypeptide that comprises an endogenous C_{H} region fused with a variable domain encoded by a rearranged human V gene segment and a J gene segment but not a D_{H} gene segment, and a second polypeptide that comprises an endogenous C_{L} region fused with a V domain encoded by a rearranged human V gene segment and a J gene segment but not a D_{H} gene segment, and the binding protein specifically binds an antigen with an affinity in the micromolar, nanomolar, or picomolar range. In one aspect, the J segment is a human J segment (*e.g*., a human κ gene segment). In one aspect, the human V segment is a human Vκ segment. In one aspect, the variable domain that is fused with the endogenous C_{H} region comprises a greater number of somatic hypermutations than the variable region that is fused with the endogenous C_{L} region; in a specific aspect, the variable region fused with the endogenous C_{H} region comprises about 1.5, 2-, 3-, 4-, or 5-fold or more somatic hypermutations than the V region fused to the endogenous C_{L} region; in a specific aspect, the V region fused with the mouse C_{H} region comprises at least 6, 7, 8, 9, 10, 11, 12, 13, 14, or 15 or more somatic hypermutations than the V region fused with the mouse C_{L} region. In one aspect, the V region fused with the mouse C_{H} region is encoded by a rearranged gene that comprises 1, 2, 3, 4, 5, 6, 7, 8, 9, or 10 or more N additions.

In one aspect, the mouse expresses a binding protein comprising a first light chain variable domain (V_{L}1) fused with an immunoglobulin heavy chain constant region sequence and a second light chain variable domain (V_{L}2) fused with an immunoglobulin light chain constant region, wherein V_{L}1 comprises a number of somatic hypermutations that is about 1.5- to about 5-fold higher or more than the number of somatic hypermutations present in V_{L}2. In one aspect, the number of somatic hypermutations in V_{L}1 is about 2- to about 4-fold higher than in V_{L}2. In one aspect, the number of somatic hypermutations in V_{L}1 is about 2- to about 3-fold higher than in V_{L}2. In one aspect, V_{L}1 is encoded by a sequence that comprises 1, 2, 3, 4, 5, 6, 7, 8, 9, or 10 or more N additions.

In one aspect, a genetically modified mouse is described that expresses an immunoglobulin that consists essentially of the following polypeptides: a first two identical polypeptides that each consists essentially of a C_{H} region fused with a variable domain that is derived from gene segments that consist essentially of a V_{L} gene segment and a J_{L} gene segment, and a second two identical polypeptides that each consists essentially of a C_{L} region fused with a variable domain that is derived from gene segments that consist essentially of a V_{L} segment and a J_{L} segment.

In a specific aspect, the two identical polypeptides that have the C_{H} region have a mouse C_{H} region.

In a specific aspect, the two identical polypeptides that have the C_{L} region have a mouse C_{L} region.

In one aspect, the variable domain fused with the C_{L} region is a variable domain that is cognate with the variable domain fused to the C_{H} region.

In one aspect, the variable domain that is fused with the endogenous C_{H} region comprises a greater number of somatic hypermutations than the variable domain that is fused with the endogenous C_{L} region; in a specific aspect, the variable domain fused with the endogenous C_{H} region comprises about 1.5-fold, 2-fold, 2.5-fold, 3-fold, 3.5-fold, 4-fold, 4.5-fold, or 5-fold or more somatic hypermutations than the variable domain fused to the endogenous C_{L} region. In one aspect, the variable domain fused with the endogenous C_{L} region is encoded by a gene that comprises 1, 2, 3, 4, 5, 6, 7, 8, 9, 10 or more N additions.

In one aspect, one or more of the V segments and the J segments are human gene segments. In a specific aspect, both the V segments and the J segments are human κ gene segments. In another specific aspect, both of the V segments and the J segments are human λ gene segments. In one aspect, the V segments and the J segments are independently selected from human κ and human λ gene segments. In a specific aspect, the V segments are Vκ segments and the J segments are Jλ segments. In another specific aspect, the V segments are Vλ segments and the J segments are Jκ segments.

In one aspect, one or more of the variable domains fused with the C_{L} region and the variable domains fused with the C_{H} region are human variable domains. In a specific aspect, the human variable domains are human Vκ domains. In another specific aspect, the human variable domains are Vλ domains. In one aspect, the human domains are independently selected from human Vκ and human Vλ□domains. In a specific aspect, the human variable domain fused with the C_{L} region is a human Vλ domain and the human variable domain fused with the C_{H} region is a human Vκ domain. In another aspect, the human variable domain fused with the C_{L} region is a human Vκ domain and the human variable domain fused with the C_{H} is a human Vλ domain.

In one aspect, the V_{L} gene segment of the first two identical polypeptides is selected from a human Vλ segment and a human Vκ segment. In one aspect, the V_{L} segment of the second two identical polypeptides is selected from a human Vλ segment and a human Vκ segment. In a specific aspect, the V_{L} segment of the first two identical polypeptides is a human Vκ segment and the V_{L} segment of the second two identical polypeptides is selected from a human Vκ segment and a human Vλ segment. In a specific aspect, the V_{L} segment of the first two identical polypeptides is a human Vλ segment and the V_{L} segment of the second two identical polypeptides is selected from a human Vλ segment and a human Vκ segment. In a specific aspect, the human V_{L} segment of the first two identical polypeptides is a human Vκ segment, and the human V_{L} segment of the second two identical polypeptides is a human Vκ segment.

In one aspect, the IgG of the mouse comprises a binding protein made in response to an antigen, wherein the binding protein comprises a polypeptide that consists essentially of a variable domain and a C_{H} region, wherein the variable domain is encoded by a nucleotide sequence that consists essentially of a rearranged V_{L} segment and a rearranged J segment, and wherein the binding protein specifically binds an epitope of the antigen with a K_{D} in the micromolar, nanomolar, or picomolar range.

In one aspect, a mouse is described, wherein all or substantially all of the IgG made by the mouse in response to an antigen comprises a heavy chain that comprises a variable domain, wherein the variable domain is encoded by a rearranged gene derived from gene segments that consist essentially of a V gene segment and a J gene segment. In one aspect, the rearranged gene comprises 1, 2, 3, 4, 5, 6, 7, 8, 9, or 10 or more N additions.

In one aspect, the V segment is a V segment of a light chain. In one aspect, the light chain is selected from a κ light chain and a λ light chain. In a specific aspect, the light chain is a κ light chain. In a specific aspect, the V segment is a human V segment. In a specific aspect, the V segment is a human Vκ segment and the J segment is a human Jκ segment.

In one aspect, the J segment is a J segment of a light chain. In one aspect, the light chain is selected from a κ light chain and a λ light chain. In a specific aspect, the light chain is a κ light chain. In a specific aspect, the J segment is a human J segment. In another aspect, the J segment is a J segment of a heavy chain (*i.e.,* a J_{H}). In a specific aspect, the heavy chain is of mouse origin. In another specific aspect, the heavy chain is of human origin.

In one aspect, the variable domain of the heavy chain that is made from no more than a V segment and a J segment is a somatically mutated variable domain.

In one aspect, the variable domain of the heavy chain that is made from no more than a V segment and a J segment is fused to a mouse C_{H} region.

In a specific aspect, all or substantially all of the IgG made by the mouse in response to an antigen comprises a variable domain that is derived from no more than one human V segment and no more than one human J segment, and the variable domain is fused to a mouse IgG constant region, and the IgG further comprises a light chain that comprises a human V_{L} domain fused with a mouse C_{L} region. In a specific aspect, the V_{L} domain fused with the mouse C_{L} region is derived from a human Vκ segment and a human Jκ segment. In a specific aspect, the V_{L} domain fused with the mouse C_{L} region is derived from a human Vλ segment and a human Jλ segment.

In one aspect, a mouse is described that makes an IgG comprising a first CDR3 on a polypeptide comprising a C_{H} region and a second CDR3 on a polypeptide comprising a C_{L} region, wherein both the first CDR3 and the second CDR3 are each independently derived from no more than two gene segments, wherein the two gene segments consist essentially of a V_{L} gene segment and a J_{L} gene segment. In one aspect, the CDR3 on the polypeptide comprising the C_{H} region comprises a sequence that is derived from a CDR3 nucleotide sequence that comprises 1, 2, 3, 4, 5, 6, 7, 8, 9, or 10 or more N additions.

In one aspect, the V_{L} segment and the J_{L} segment are human gene segments. In one aspect, the V_{L} segment and the J_{L} segment are κ gene segments. In one aspect, the V_{L} segment and the J_{L} segment are λ gene segments.

In one aspect, a mouse is described that makes an IgG comprising a first CDR3 on a first polypeptide comprising a C_{H} region and a second CDR3 on a second polypeptide comprising a C_{L} region, wherein both the first CDR3 and the second CDR3 each comprise a sequence of amino acids wherein more than 75% of the amino acids are derived from a V gene segment. In one aspect, the CDR3 on the polypeptide comprising the C_{H} region comprises a sequence that is derived from a CDR3 nucleotide sequence that comprises 1, 2, 3, 4, 5, 6, 7, 8, 9, or 10 or more N additions.

In one aspect, more than 80%, more than 90%, or more than 95% of the amino acids of the first CDR3, and more than 80%, more than 90%, or more than 95% of the amino acids of the second CDR3, are derived from a light chain V segment.

In one aspect, no more than two amino acids of the first CDR3 are derived from a gene segment other than a light chain V segment. In one aspect, no more than two amino acids of the second CDR3 are derived from a gene segment other than a light chain V segment. In a specific aspect, no more than two amino acids of the first CDR3 and no more than two amino acids of the second CDR3 are derived from a gene segment other than a light chain V segment. In one aspect, no CDR3 of the IgG comprises an amino acid sequence derived from a D gene segment. In one aspect, the CDR3 of the first polypeptide does not comprise a sequence derived from a D segment.

In one aspect, the V segment is a human V gene segment. In a specific aspect, the V segment is a human Vκ gene segment.

In one aspect, the first and/or the second CDR3 have at least one, two, three, four, five, or six somatic hypermutations. In one aspect, the first CDR3 is encoded by a nucleic acid sequence that comprises 1, 2, 3, 4, 5, 6, 7, 8, 9, or 10 or more N additions.

In one aspect, the first CDR3 consists essentially of amino acids derived from a human light chain V gene segment and a human light chain J gene segment, and the second CDR3 consists essentially of amino acids derived from a human light chain V gene segment and a human light chain J gene segment. In one aspect, the first CDR3 is derived from a nucleic acid sequence that comprises 1, 2, 3, 4, 5, 6, 7, 8, 9, or 10 or more N additions. In one aspect, the first CDR3 is derived from no more than two gene segments, wherein the no more than two gene segments are a human Vκ gene segment and a human Jκ gene segment; and the second CDR3 is derived from no more than two gene segments, wherein the no more than two gene segments are a human Vκ gene segment and a J gene segment selected from a human Jκ segment, a human Jλ segment, and a human J_{H} segment. In one aspect, the first CDR3 is derived from no more than two gene segments, wherein the no more than two gene segments are a human Vλ segment and a J segment selected from a human Jκ segment, a human Jλ segment, and a human J_{H} segment.

In one aspect, a mouse is described that makes an IgG that does not contain an amino acid sequence derived from a D_{H} gene segment, wherein the IgG comprises a first polypeptide having a first V_{L} domain fused with a mouse C_{L} region and a second polypeptide having a second V_{L} domain fused with a mouse C_{H} region, wherein the first V_{L} domain and the second V_{L} domain are not identical. In one aspect, the first and second V_{L} domains are derived from different V segments. In another aspect, the first and second V_{L} domains are derived from different J segments. In one aspect, the first and second V_{L} domains are derived from identical V and J segments, wherein the second V_{L} domain comprises a higher number of somatic hypermutations as compared to the first V_{L} domain.

In one aspect, the first and the second V_{L} domains are independently selected from human and mouse V_{L} domains. In one aspect, the first and second V_{L} domains are independently selected from Vκ and Vλ domains. In a specific aspect, the first V_{L} domain is selected from a Vκ domain and a Vλ domain, and the second V_{L} domain is a Vκ domain. In another specific aspect, the Vκ domain is a human Vκ domain.

In one aspect, a mouse is described, wherein all or substantially all of the IgG made by the mouse consists essentially of a light chain having a first human V_{L} domain fused with a mouse C_{L} domain, and a heavy chain having a second human V_{L} domain fused with a mouse C_{H} domain.

In one aspect, the human V_{L} domain fused with the mouse C_{H} domain is a human VK domain.

In one aspect, the first and the second human V_{L} domains are not identical.

In one aspect, a mouse is described, wherein at least 10%, 20%, 30%, 40%, 50%, 60%, 70%, 80%, 90%, 95%, or about 100% of the immunoglobulin G made by the mouse consists essentially of a dimer of (a) a first polypeptide that consists essentially of an immunoglobulin V_{L} domain and an immunoglobulin C_{L} region; and, (b) a second polypeptide of no more than 535 amino acids in length, wherein the second polypeptide consists essentially of a C_{H} region and a V domain that lacks a sequence derived from a D_{H} gene segment.

In one aspect, the second polypeptide is about 435-535 amino acids in length. In a specific aspect, the second polypeptide is about 435-530 amino acids in length. In a specific aspect, the second polypeptide is about 435-525 amino acids in length. In a specific aspect, the second polypeptide is about 435-520 amino acids in length. In a specific aspect, the second polypeptide is about 435-515 amino acids in length.

In one aspect, in about 90% or more of the IgG made by the mouse the second polypeptide is no more than about 535 amino acids in length.

In one aspect, in about 50% or more of the IgG made by the mouse the second polypeptide is no more than about 535 amino acids in length. In one aspect, in about 50% or more of the immunoglobulin G made by the mouse the second polypeptide is no more than about 530, 525, 520, 515, 510, 505, 500, 495, 490, 485, 480, 475, 470, 465, 460, 455, or 450 amino acids in length. In one aspect, about 60%, 70%, 80%, 90%, or 95 % or more of the IgG made by the mouse is of the recited length. In a specific aspect, all or substantially all of the IgG made by the mouse is of the recited length.

In one aspect, the V domain of the second polypeptide is a V_{L} domain. In a specific aspect, the V domain of the second polypeptide is selected from a Vκ and a Vλ domain. In a specific aspect, the V domain of the second polypeptide is a human Vκ or Vλ domain.

In one aspect, a mouse is described that expresses from a nucleotide sequence in its germline a polypeptide that comprises a light chain variable sequence (e.g., a V and/or J sequence), a D_{H} sequence, and a heavy chain constant region.

In one aspect, the mouse expresses the polypeptide from an endogenous heavy chain locus that comprises a replacement of all or substantially all functional endogenous heavy chain variable locus gene segments with a plurality of human gene segments at the endogenous heavy chain locus.

In one aspect, the polypeptide comprises a V_{L} sequence derived from a Vλ or a Vκ gene segment, the polypeptide comprises a CDR3 derived from a D_{H} gene segment, and the polypeptide comprises a sequence derived from a J_{H} or Jλ or Jκ gene segment.

In one aspect, the mouse comprises an endogenous heavy chain immunoglobulin locus comprising a replacement of all functional V_{H} gene segments with one or more human light chain Vλ gene segments wherein the one or more human Vλ segments each have juxtaposed on the downstream side a 23-mer spaced recombination signal sequence (RSS), wherein the Vλ segments are operably linked to a human or mouse D_{H} segment that has juxtaposed upstream and downstream a 12-mer spaced RSS; the D_{H} gene segment is operably linked with a J segment juxtaposed upstream with a 23-mer spaced RSS that is suitable for recombining with the 12-mer spaced RSS juxtaposing the D_{H} gene segment; wherein the V, D_{H}, and J segments are operably linked to a nucleic acid sequence encoding a heavy chain constant region.

In one aspect, the mouse comprises an endogenous heavy chain immunoglobulin locus comprising a replacement of all functional V_{H} gene segments with one or more human Vκ gene segments each juxtaposed on the downstream side with a 12-mer spaced recombination signal sequence (RSS), wherein the V segments are operably linked to a human or mouse D_{H} segment that is juxtaposed both upstream and downstream with a 23-mer spaced RSS; the D_{H} segment is operably linked with a J segment juxtaposed on the upstream side with a 12-mer spaced RSS that is suitable for recombining with the 23-mer spaced RSS juxtaposing the D_{H} segment; wherein the V, D_{H}, and gene segments are operably linked to a nucleic acid sequence encoding a heavy chain constant region.

In one aspect, the heavy chain constant region is an endogenous heavy chain constant region. In one aspect, the nucleic acid sequence encodes a sequence selected from a C_{H}1, a hinge, a C_{H}2, a C_{H}3, and a combination thereof. In one aspect, one or more of the C_{H}1, hinge, C_{H}2, and C_{H}3 are human.

In one aspect, the mouse comprises an endogenous heavy chain immunoglobulin locus comprising a replacement of all functional V_{H} gene segments with a plurality of human Vλ or Vκ gene segments each juxtaposed downstream with 23-mer spaced RSS, a plurality of human D_{H} segments juxtaposed both upstream and downstream by a 12-mer spaced RSS, a plurality of human J segments (J_{H} or Jλ or Jκ) juxtaposed both upstream and downstream with a 23-mer spaced RSS, wherein the locus comprises an endogenous constant region sequence selected from C_{H}1, hinge, C_{H}2, C_{H}3, and a combination thereof. In a specific aspect, the mouse comprises all or substantially all functional human Vλ or Vκ segments, all or substantially all functional human D_{H} segments, and all or substantially all J_{H} or Jλ or Jκ segments.

In one aspect, the mouse expresses an antigen-binding protein comprising (a) a polypeptide that comprises a human light chain sequence linked to a heavy chain constant sequence comprising a mouse sequence; and (b) a polypeptide that comprises a human light chain variable region linked to a human or mouse light chain constant sequence. In a specific aspect, the light chain sequence is a human light chain sequence, and upon exposure to a protease that is capable of cleaving an antibody into an Fc and a Fab, a fully human Fab is formed that comprises at least four light chain CDRs, wherein the at least four light chain CDRs are selected from λ sequences, κ sequences, and a combination thereof. In one aspect, the Fab comprises at least five light chain CDRs. In one aspect, the Fab comprises six light chain CDRs. In one aspect, at least one CDR of the Fab comprises a sequence derived from a Vλ segment or a Vκ segment, and the at least one CDR further comprises a sequence derived from a D segment. In one aspect, the at least one CDR is a CDR3 and the CDR is derived from a human Vκ segment, a human D segment, and a human Jκ segment.

In one aspect, the polypeptide of comprises a variable region derived from a human Vλ or Vκ gene segment, a human D_{H} gene segment, and a human J_{H} or Jλ or Jκ gene segment. In a specific aspect, the heavy chain constant sequence is derived from a human C_{H}1 and a mouse C_{H}2 and a mouse C_{H}3 sequence.

In one aspect, a mouse is described that comprises in its germline an unrearranged human Vκ or Vλ gene segment operably linked to a human J gene segment and a heavy chain constant region sequence, wherein the mouse expresses a V_{L} binding protein that comprises a human Vκ domain fused with a heavy chain constant region and a human V_{L} domain fused with a light chain constant domain. In one aspect, the human V_{L} domain comprises a rearranged human V_{L} gene segment selected from a human Vκ and a human Vλ gene segment. In a specific aspect, the human V_{L} domain further comprises a rearranged human J_{L} gene segment selected from a human Jκ and a human Jλ gene segment.

In one aspect, a mouse is described that expresses an immunoglobulin protein from a modified endogenous heavy chain locus in its germline, wherein the modified endogenous heavy chain locus lacks a functional mouse heavy chain V gene segment and the locus comprises unrearranged light chain V gene segments and unrearranged J gene segments, wherein the unrearranged light chain V gene segments and unrearranged J gene segments are operably linked with a heavy chain constant region sequence; wherein the immunoglobulin protein consists essentially of a first polypeptide and a second polypeptide, wherein the first polypeptide comprises an immunoglobulin light chain sequence and an immunoglobulin heavy chain constant sequence, and the second polypeptide comprises an immunoglobulin light chain variable domain and a light chain constant region.

In one aspect, a mouse is described that expresses an immunoglobulin protein, wherein the immunoglobulin protein lacks a heavy chain immunoglobulin variable domain, and the immunoglobulin protein comprises a first variable domain derived from a light chain gene, and a second variable domain derived from a light chain gene, wherein the first variable domain and the second variable domain are cognate with respect to one another, wherein the first and the second light chain variable domains are not identical, and wherein the first and the second light chain variable domains associate and when associated specifically bind an antigen of interest.

In one aspect, a mouse is described that expresses from unrearranged gene segments in its germline an immunoglobulin protein comprising variable regions that are wholly derived from gene segments that consist essentially of unrearranged human gene segments, wherein the immunoglobulin protein comprises an immunoglobulin light chain constant sequence and an immunoglobulin heavy chain constant sequence selected from the group consisting of a C_{H}1, a hinge, a C_{H}2, a C_{H}3, and a combination thereof.

In one aspect, a mouse is described that expresses from unrearranged gene segments in its germline an immunoglobulin protein comprising variable regions, wherein all CDR3s of all variable regions are generated entirely from light chain V and J gene segments, and optionally one or more somatic hypermutations, e.g., one or more N additions.

In one aspect, a mouse is described that expresses a somatically mutated immunoglobulin protein derived from unrearranged human immunoglobulin light chain variable region gene segments in the germline of the mouse, wherein the immunoglobulin protein lacks a CDR that comprises a sequence derived from a D gene segment, wherein the immunoglobulin protein comprises a first CDR3 on a light chain variable domain fused with a light chain constant region, comprises a second CDR3 on a light chain variable domain fused with a heavy chain constant region, and wherein the second CDR3 is derived from a rearranged light chain variable region sequence that comprises 1, 2, 3, 4, 5, 6, 7, 8, 9, or 10 or more N additions.

In one aspect, a mouse as described herein is described, wherein the mouse comprises a functionally silenced light chain locus selected from a λ locus, a κ locus, and a combination thereof. In one aspect, the mouse comprises a deletion of a λ and/or a κ locus, in whole or in part, such that the λ and/or κ locus is nonfunctional.

In one aspect, a mouse embryo is described, comprising a cell that comprises a modified immunoglobulin locus as described herein. In one aspect, the mouse is a chimera and at least 10%, 20%, 30%, 40%, 50%, 60%, 70%, 80%, 90%, or 95% of the cells of the embryo comprise a modified immunoglobulin locus as described herein. In one aspect, at least 96%. 97%, 98%, 99%, or 99.8% of the cells of the embryo comprise a modified immunoglobulin locus as described herein. In one aspect, the embryo comprises a host cell and a cell derived from a donor ES cell, wherein the cell derived from the donor ES cell comprises a modified immunoglobulin locus as described herein. In one aspect, the embryo is a 2-, 4-, 8, 16-, 32, or 64-cell stage host embryo, or a blastocyst, and further comprises a donor ES cell comprising a modified immunoglobulin locus as described herein.

In one aspect, a mouse or a cell made using a nucleic acid construct as described herein is described.

In one aspect, a mouse made using a cell as described herein is described. In one aspect, the cell is a mouse ES cell.

In one aspect, mouse cells and mouse embryos are described, including but not limited to ES cells, pluripotent cells, and induced pluripotent cells, that comprise genetic modifications as described herein. Cells that are XX and cells that are XY are described. Cells that comprise a nucleus containing a modification as described herein are also described, e.g., a modification introduced into a cell by pronuclear injection. Cells, embryos, and mice that comprise a virally introduced ADAM6 gene are also described, e.g., cells, embryos, and mice comprising a transduction construct comprising an ADAM6 gene that is functional in the mouse.

In one aspect, a cell or tissue derived from a mouse as described herein is described. In one aspect, the cell or tissue is derived from spleen, lymph node or bone marrow of a mouse as described herein. In one aspect, the cell is a B cell. In one aspect the cell is an embryonic stem cell. In one aspect, the cell is a germ cell.

In one aspect, the tissue is selected from connective, muscle, nervous and epithelial tissue. In a specific aspect, the tissue is reproductive tissue.

In one aspect, the cell and/or tissue derived from a mouse as described herein is isolated for use in one or more *ex vivo* assays. In various aspects, the one or more *ex vivo* assays include measurements of physical, thermal, electrical, mechanical or optical properties, a surgical procedure, measurements of interactions of different tissue types, the development of imaging techniques, or a combination thereof.

In aspect, use of cell or tissue derived from a mouse as described herein to make an antibody is described. In one aspect, use of a cell or tissue derived from a mouse as described herein to make a hybridoma or quadroma is described.

In one aspect, a non-human cell comprising a chromosome or fragment thereof of a non-human animal as described herein. In one aspect, the non-human cell comprises a nucleus of a non-human animal as described herein. In one aspect, the non-human cell comprises the chromosome or fragment thereof as the result of a nuclear transfer.

In one aspect, a nucleus derived from a mouse as described herein is described. In one aspect, the nucleus is from a diploid cell that is not a B cell.

In one aspect, a genetically modified mouse cell is described, wherein the cell is incapable of expressing a heavy chain comprising rearranged endogenous immunoglobulin heavy chain gene segments, and the cell comprises a functional ADAM6 gene that encodes a mouse ADAM6 protein or functional fragment thereof. In one aspect, the cell further comprises an insertion of human immunoglobulin light chain gene segments. In a specific aspect, the human immunoglobulin light chain gene segments are Vκ and/or Jκ gene segments that are operably linked to mouse heavy chain constant regions such that upon rearrangement encode a functional light chain variable domain fused to a mouse heavy chain constant domain.

In one aspect, a genetically modified mouse cell is described; wherein the cell lacks a functional endogenous ADAM6 locus, and the cell comprises an ectopic nucleotide sequence that encodes a mouse ADAM6. In one aspect, the cell further comprises a modification of an endogenous immunoglobulin heavy chain variable region sequence. In a specific aspect, the modification of the endogenous immunoglobulin heavy chain variable region sequence comprises a deletion selected from a deletion of a mouse V_{H} gene segment, a deletion of a mouse D_{H} gene segment, a deletion of a mouse J_{H} gene segment, and a combination thereof. In a specific aspect, the mouse comprises a replacement of one or more mouse immunoglobulin V_{H}, D_{H}, and/or J_{H} sequences with a human immunoglobulin sequence. In a specific aspect, the human immunoglobulin sequence is selected from a human V_{H}, a human V_{L}, a human D_{H}, a human J_{H}, a human J_{L}, and a combination thereof.

In one aspect, the cell is a totipotent cell, a pluripotent cell, or an induced pluripotent cell. In a specific aspect, the cell is a mouse ES cell.

In one aspect, a mouse B cell is described, wherein the mouse B cell comprises a rearranged immunoglobulin gene, wherein the B cell comprises on a chromosome of the B cell a nucleic acid sequence encoding an ADAM6 protein or ortholog or homolog or fragment thereof that is functional in a male mouse. In one aspect, the mouse B cell comprises two alleles of the nucleic acid sequence. In one aspect, the rearranged immunoglobulin gene comprises a rearranged immunoglobulin light chain sequence contiguous with a heavy chain constant sequence. In one aspect, the light chain sequence is a κ sequence; in one aspect, the light chain sequence is a λ sequence.

In one aspect, the nucleic acid sequence is on a nucleic acid molecule (*e.g*., a B cell chromosome) that is contiguous with the rearranged mouse heavy chain immunoglobulin locus.

In one aspect, the nucleic acid sequence is on a nucleic acid molecule (e.g., a B cell chromosome) that is distinct from the nucleic acid molecule that comprises the rearranged mouse heavy chain immunoglobulin locus.

In one aspect, the mouse B cell comprises a rearranged non-mouse light chain immunoglobulin variable region sequence operably linked to a mouse or human heavy chain immunoglobulin constant region gene, wherein the B cell comprises a nucleic acid sequence that encodes an ADAM6 protein or ortholog or homolog or fragment thereof that is functional in a male mouse.

In one aspect, a somatic mouse cell is described, comprising a chromosome that comprises a modified immunoglobulin heavy chain locus, and a nucleic acid sequence encoding a mouse ADAM6 or ortholog or homolog or fragment thereof that is functional in a male mouse. In one aspect, the nucleic acid sequence is on the same chromosome as the modified immunoglobulin heavy chain locus. In one aspect, the nucleic acid is on a different chromosome than the modified immunoglobulin heavy chain locus. In one aspect, the somatic cell comprises a single copy of the nucleic acid sequence. In one aspect, the somatic cell comprises at least two copies of the nucleic acid sequence. In a specific aspect, the somatic cell is a B cell. In one aspect, the modified immunoglobulin heavy chain locus comprises an unrearranged immunoglobulin light chain gene segment operably linked to a heavy chain constant region sequence.

In one aspect, a mouse germ cell is described, comprising a nucleic acid sequence encoding a mouse ADAM6 (or homolog or ortholog or functional fragment thereof) on a chromosome of the germ cell, wherein the nucleic acid sequence encoding the mouse ADAM6 (or homolog or ortholog or functional fragment thereof) is at a position in the chromosome that is different from a position in a chromosome of a wild-type mouse germ cell, wherein the mouse further comprises a modification comprising an unrearranged light chain immunoglobulin gene segment (a Vκ and/or Vλ and/or Vκ and Jκ and/or Vλ and JA.) operably linked to a heavy chain constant region sequence. In one aspect, the nucleic acid sequence is at a mouse immunoglobulin locus. In one aspect, the nucleic acid sequence is on the same chromosome of the germ cell as a mouse immunoglobulin locus. In one aspect, the nucleic acid sequence is on a different chromosome of the germ cell than the mouse immunoglobulin locus. In one aspect, the mouse immunoglobulin locus comprises a replacement of at least one mouse immunoglobulin sequence with at least one non-mouse immunoglobulin sequence. In a specific aspect, the at least one non-mouse immunoglobulin sequence is a human immunoglobulin sequence.

In one aspect, a pluripotent, induced pluripotent, or totipotent cell derived from a mouse as described herein is described. In a specific aspect, the cell is a mouse embryonic stem (ES) cell.

In one aspect, a cell is described, comprising a modified immunoglobulin locus as described herein. In one aspect, the cell is selected from a totipotent cell, a pluripotent cell, an induced pluripotent stem cell (iPS), and an ES cell. In a specific aspect, the cell is a mouse cell, e.g., a mouse ES cell. In one aspect, the cell is homozygous for the modified immunoglobulin locus.

In one aspect, a cell is described, comprising a nucleic acid sequence encoding a first polypeptide that comprises a first somatically mutated human Vκ or Vλ sequence fused to a human heavy chain constant region sequence.

In one aspect, the cell further comprises a second polypeptide chain that comprises a second somatically mutated human Vκ or Vλ sequence fused to a human light chain constant region sequence.

In one aspect, the human Vκ or Vλ sequence of the first polypeptide is cognate with the human Vκ or Vλ sequence of the second polypeptide.

In one aspect, the Vκ or Vλ of the first polypeptide and the human Vκ or Vλ of the second polypeptide when associated specifically bind an antigen of interest. In a specific aspect, the first polypeptide comprises a variable domain consisting essentially of a human Vκ, and the second polypeptide comprises a variable domain consisting of a human Vκ that is cognate with the human Vκ of the first polypeptide, and the human constant region sequence is an IgG sequence.

In one aspect, the cell is selected from a CHO cell, a COS cell, a 293 cell, a HeLa cell, and a human retinal cell expressing a viral nucleic acid sequence (*e.g*., a PERC.6™ cell.

In one aspect, a somatic rodent (*e.g*., mouse) cell is described, comprising a chromosome that comprises a genetic modification as described herein.

In one aspect, a rodent (*e.g*., mouse) germ cell is described, comprising a nucleic acid sequence that comprises a genetic modification as described herein.

In one aspect, a pluripotent, induced pluripotent, or totipotent cell derived from a rodent (*e.g*., mouse) as described herein is described. In a specific aspect, the cell is a mouse embryonic stem (ES) cell.

In one aspect, use of a cell as described herein for the manufacture of a rodent (*e.g*., a mouse), a cell, or a therapeutic protein (*e.g*., an antibody or other antigen-binding protein) is described. In one aspect, use of a cell as described herein for the manufacture of a therapeutic protein is described, wherein the therapeutic protein comprises a human variable domain. In a specific aspect, the human variable domain comprises rearranged Vκ and Jκ gene segments.

In one aspect, a rodent (e.g., a mouse) made using a targeting vector, nucleotide construct, or cell as described herein is described.

In one aspect, use of a targeting vector as described herein for the manufacture of a rodent (e.g., a mouse) or a cell (e.g., a mouse ES cell, a mouse fibroblast, *etc*.) is described. In one aspect, the targeting vector comprises a human genomic fragment that contains unrearranged immunoglobulin gene segments. In a specific aspect, the unrearranged immunoglobulin gene segments include V and J gene segments. In a specific aspect, the unrearranged immunoglobulin gene segments include V, D and J gene segments.

In one aspect, a nucleic acid construct is described, comprising an upstream homology arm and a downstream homology arm, wherein the upstream homology arm comprises a sequence that is identical or substantially identical to a human immunoglobulin heavy chain variable region sequence, the downstream homology arm comprises a sequence that is identical or substantially identical to a human or mouse immunoglobulin variable region sequence, and disposed between the upstream and downstream homology arms is a sequence that comprises a nucleotide sequence encoding a mouse ADAM6 protein. In a specific aspect, the sequence encoding the mouse ADAM6 gene is operably linked with a mouse promoter with which the mouse ADAM6 is linked in a wild type mouse.

In one aspect, a targeting vector is described, comprising (a) a nucleotide sequence that is identical or substantially identical to a human variable region gene segment nucleotide sequence; and, (b) a nucleotide sequence encoding a mouse ADAM6 or ortholog or homolog or fragment thereof that is functional in a mouse.

In one aspect, the targeting vector further comprises a promoter operably linked to the sequence encoding the mouse ADAM6. In a specific aspect, the promoter is a mouse ADAM6 promoter.

In one aspect, a nucleotide construct for modifying a mouse immunoglobulin heavy chain variable locus is described, wherein the construct comprises at least one site-specific recombinase recognition site and a sequence encoding an ADAM6 protein or ortholog or homolog or fragment thereof that is functional in a mouse.

In one aspect, a nucleic acid construct is described that comprises a human D_{H} gene segment juxtaposed upstream and downstream with a 23-mer spaced RSS. In a specific aspect, the nucleic acid construct comprises a homology arm that is homologous to a human genomic sequence comprising human Vκ gene segments. In one aspect, the targeting construct comprises all or substantially all human D_{H} gene segments each juxtaposed upstream and downstream with a 23-mer spaced RSS.

In one aspect, a nucleic acid construct is described that comprises a human Jκ gene segment juxtaposed upstream with a 12-mer spaced RSS. In a specific aspect, the nucleic acid construct comprises a first homology arm that contains homology to a human genomic D_{H} gene sequence that is juxtaposed upstream and downstream with a 23-mer spaced RSS. In one aspect, the nucleic acid construct comprises a second homology arm that contains homology to a human genomic J gene sequence or that contains homology to a mouse heavy chain constant region sequence or that contains homology to a J-C intergenic sequence upstream of a mouse constant region heavy chain sequence.

In one aspect, a nucleic acid construct is described that comprises a human Vλ segment juxtaposed downstream with a 23-mer spaced RSS, a human D_{H} segment juxtaposed upstream and downstream with a 12-mer spaced RSS, and a human J segment selected from a Jκ segment juxtaposed upstream with a 23-mer spaced RSS, a human Jλ segment juxtaposed upstream with a 23-mer spaced RSS, and a human J_{H} segment juxtaposed upstream with a 23-mer spaced RSS. In one aspect, the construct comprises a homology arm that contains homology to a mouse constant region sequence, a J-C intergenic mouse sequence, and/or a human Vλ sequence.

In one aspect, the nucleic acid construct comprises a human λ light chain variable region sequence that comprises a fragment of cluster A of the human λ light chain locus. In a specific aspect, the fragment of cluster A of the human λ light chain locus extends from hVλ3-27 through hVλ3-1.

In one aspect, the nucleic acid construct comprises a human λ light chain variable region sequence that comprises a fragment of cluster B of the human λ light chain locus. In a specific aspect, the fragment of cluster B of the human λ light chain locus extends from hVλ5-52 through hVλ1-40.

In one aspect, nucleic acid construct comprises a human λ light chain variable region sequence that comprises a genomic fragment of cluster A and a genomic fragment of cluster B. In a one aspect, the human λ light chain variable region sequence comprises at least one gene segment of cluster A and at least one gene segment of cluster B.

In one aspect, the human λ light chain variable region sequence comprises at least one gene segment of cluster B and at least one gene segment of cluster C.

In one aspect, a nucleic acid construct is described, comprising a human D_{H} segment juxtaposed upstream and downstream with a 23-mer spaced RSS normally found in nature flanking either a Jκ, a J_{H}, a Vλ, or a V_{H} segment. In one aspect, the nucleic acid construct comprises a first homology arm homologous to a human V-J intergenic region or homologous to a human genomic sequence comprising a human V gene segment. In one aspect, the nucleic acid construct comprises a second homology arm homologous to a human or mouse heavy chain constant region sequence. In a specific aspect, the human or mouse heavy chain constant region sequence is selected from a C_{H}1, hinge, C_{H}2, C_{H}3, and a combination thereof. In one aspect, the nucleic acid construct comprises a human J gene segment flanked upstream with a 12-mer RSS. In one aspect, the nucleic acid construct comprises a second homology arm that contains homology to a J gene segment flanked upstream with a 12-mer RSS. In one aspect, the J gene segment is selected from a human Jκ, a human Jλ, and a human J_{H}.

In one aspect, a nucleic acid construct is described that comprises a human D_{H} segment juxtaposed upstream and downstream with a 23-mer spaced RSS, and a site-specific recombinase recognition sequence, e.g., a sequence recognized by a site-specific recombinase such as a Cre, a Flp, or a Dre protein.

In one aspect, a nucleic acid construct is described that comprises a human Vλ or a human Vκ segment, a D_{H} segment juxtaposed upstream and downstream with a 12-mer or a 23-mer spaced RSS, and a human J segment with a 12-mer or a 23-mer spaced RSS, wherein the 12-mer or 23-mer spaced RSS is positioned immediately 5' to the human J segment (*i.e.,* with respect to the direction of transcription). In one aspect, the construct comprises a human Vλ juxtaposed with a 3' 23-mer spaced RSS, a human D_{H} segment juxtaposed upstream and downstream with a 12-mer spaced RSS, and a human Jκ segment juxtaposed with a 5' 23-mer spaced RSS. In one aspect, the construct comprises a human Vκ juxtaposed with a 3' 12-mer spaced RSS, a human D_{H} segment juxtaposed upstream and downstream with a 23-mer spaced RSS, and a human Jλ segment juxtaposed with a 5' 12-mer spaced RSS.

In one aspect, a targeting vector is described, comprising (a) a first targeting arm and a second targeting arm, wherein the first and second targeting arms are independently selected from human and mouse targeting arms, wherein the targeting arms direct the vector to an endogenous or modified immunoglobulin V region gene locus; and, (b) a contiguous sequence of human V_{L} gene segments or a contiguous sequence of human V_{L} gene segments and at least one human Jκ gene segment, wherein the contiguous sequence is selected from the group consisting of (i) hVκ4-1 through hVκ1-6 and Jκ1, (ii) hVκ4-1 through hVκ1-6 and Jκ1 through Jκ2, (iii) hVκ4-1 through hVκ1-6 and Jκ1 through Jκ3, (iv) hVκ4-1 through hVκ1-6 and Jκ1 through Jκ4, (v) hVκ4-1 through hVκ1-6 and Jκ1 through Jκ5, (vi) hVκ3-7 through hVκ1-16, (vii) hVκ1-17 through hVκ2-30, (viii) hVκ3-31 through hVκ2-40, and (ix) a combination thereof.

In one aspect, the targeting arms that direct the vector to an endogenous or modified immunoglobulin locus are identical or substantially identical to a sequence at the endogenous or modified immunoglobulin locus.

In one aspect, a method for making a genetically modified mouse is described, comprising replacing one or more immunoglobulin heavy chain gene segments upstream (with respect to transcription of the immunoglobulin heavy chain gene segments) of an endogenous ADAM6 locus of the mouse with one or more human immunoglobulin light chain and/or heavy chain gene segments, and replacing one or more immunoglobulin gene segments downstream (with respect to transcription of the immunoglobulin heavy chain gene segments) of the ADAM6 locus of the mouse with one or more human immunoglobulin heavy chain or light chain gene segments. In one aspect, the one or more human immunoglobulin gene segments replacing one or more endogenous immunoglobulin gene segments upstream of an endogenous ADAM6 locus of the mouse include V gene segments. In one aspect, the human immunoglobulin gene segments replacing one or more endogenous immunoglobulin gene segments upstream of an endogenous ADAM6 locus of the mouse include V and D gene segments. In one aspect, the one or more human immunoglobulin gene segments replacing one or more endogenous immunoglobulin gene segments downstream of an endogenous ADAM6 locus of the mouse include J gene segments. In one aspect, the one or more human immunoglobulin gene segments replacing one or more endogenous immunoglobulin gene segments downstream of an endogenous ADAM6 locus of the mouse include D and J gene segments. In one aspect, the one or more human immunoglobulin gene segments replacing one or more endogenous immunoglobulin gene segments downstream of an endogenous ADAM6 locus of the mouse include V, D and J gene segments.

In one aspect, a method for making a genetically modified mouse is described, comprising replacing one or more immunoglobulin heavy chain gene segments upstream (with respect to transcription of the immunoglobulin heavy chain gene segments) of an endogenous ADAM6 locus of the mouse with one or more human immunoglobulin light chain gene segments, and replacing one or more immunoglobulin gene segments downstream (with respect to transcription of the immunoglobulin heavy chain gene segments) of the ADAM6 locus of the mouse with one or more human immunoglobulin light chain segments. In one aspect, the one or more human immunoglobulin gene segments replacing one or more endogenous immunoglobulin gene segments upstream of an endogenous ADAM6 locus of the mouse include V gene segments. In one aspect, the human immunoglobulin gene segments replacing one or more endogenous immunoglobulin gene segments upstream of an endogenous ADAM6 locus of the mouse include V and J gene segments. In one aspect, the one or more human immunoglobulin gene segments replacing one or more endogenous immunoglobulin gene segments downstream of an endogenous ADAM6 locus of the mouse include J gene segments. In one aspect, the one or more human immunoglobulin gene segments replacing one or more endogenous immunoglobulin gene segments downstream of an endogenous ADAM6 locus of the mouse include V and J gene segments. In one aspect, the one or more human immunoglobulin gene segments replacing one or more endogenous immunoglobulin gene segments downstream of an endogenous ADAM6 locus of the mouse include V gene segments.

In a specific aspect, the V gene segments are V_{L} gene segments. In another specific aspect, the J gene segments are J_{L} gene segments.

In one aspect, the one or more immunoglobulin heavy chain gene segments upstream and/or downstream of the ADAM6 gene are replaced in a pluripotent, induced pluripotent, or totipotent cell to form a genetically modified progenitor cell; the genetically modified progenitor cell is introduced into a host; and, the host comprising the genetically modified progenitor cell is gestated to form a mouse comprising a genome derived from the genetically modified progenitor cell. In one aspect, the host is an embryo. In a specific aspect, the host is selected from a mouse pre-morula (*e*.*g*., 8- or 4-cell stage), a tetraploid embryo, an aggregate of embryonic cells, or a blastocyst.

In one aspect, a method for making a genetically modified mouse is described, comprising replacing a mouse nucleotide sequence that comprises a mouse immunoglobulin gene segment and a mouse ADAM6 (or ortholog or homolog or fragment thereof functional in a male mouse) nucleotide sequence with a sequence comprising a human immunoglobulin gene segment to form a first chimeric locus, then inserting a sequence comprising a mouse ADAM6-encoding sequence (or a sequence encoding an ortholog or homolog or functional fragment thereof) into the sequence comprising the human immunoglobulin gene segment to form a second chimeric locus.

In one aspect, the second chimeric locus comprises a human immunoglobulin heavy chain variable gene segment (V_{H}). In one aspect, the second chimeric locus comprises a human immunoglobulin light chain variable gene segment (V_{L}). In a specific aspect, the second chimeric locus comprises a human V_{H} gene segment or a human V_{L} gene segment operably linked to a human D_{H} gene segment and a human J_{H} gene segment. In a specific aspect, the second chimeric locus comprises a human V_{L} gene segment operably linked to a human J_{H} or a human J_{L} gene segment. In a further specific aspect, the second chimeric locus is operably linked to a third chimeric locus that comprises a human C_{H}1 sequence, or a human C_{H}1 and human hinge sequence, fused with a mouse C_{H}2 + C_{H}3 sequence.

In one aspect, a method is described for modifying a heavy chain immunoglobulin locus of a mouse, comprising: (a) making a first modification of a mouse heavy chain immunoglobulin locus that results in a reduction or elimination of endogenous mouse ADAM6 activity in a male mouse; and, (b) making a second modification to add a nucleic acid sequence that confers upon the mouse ADAM6 activity that is functional in a male mouse.

In one aspect, the first modification comprises the addition of a human immunoglobulin sequence or the replacement of a mouse immunoglobulin sequence with a human immunoglobulin sequence.

In one aspect, the human immunoglobulin sequence is a heavy chain sequence. In one aspect, the human immunoglobulin sequence is a light chain sequence.

In one aspect, the first and the second modification are made in a single ES cell, and the single ES cell is introduced into a host embryo to make the mouse.

In one aspect, a progeny of a mating of a mouse as described herein with a second mouse that is a wild-type mouse or genetically modified is described.

In one aspect, use of a mouse that comprises an ectopic nucleotide sequence comprising a mouse ADAM6 locus or sequence to make a fertile male mouse is described, wherein the use comprises mating the mouse comprising the ectopic nucleotide sequence that comprises the mouse ADAM6 locus or sequence to a mouse that lacks a functional endogenous ADAM6 locus or sequence, and obtaining a progeny that is a female capable of producing progeny having the ectopic ADAM6 locus or sequence or that is a male that comprises the ectopic ADAM6 locus or sequence, and the male exhibits a fertility that is approximately the same as a fertility exhibited by a wild-type male mouse.

In one aspect, use of a mouse as described herein to introduce an ectopic ADAM6 sequence into a mouse that lacks a functional endogenous ADAM6 sequence is described, wherein the use comprises mating a mouse as described herein with the mouse that lacks the functional endogenous ADAM6 sequence.

In one aspect, use of genetic material from a mouse as described herein to make a mouse having an ectopic ADAM6 sequence is described. In one aspect, the *use* comprises nuclear transfer using a nucleus of a cell of a mouse as described herein. In one aspect, the use comprises cloning a cell of a mouse as described herein to produce an animal derived from the cell. In one aspect, the use comprises employing a sperm or an egg of a mouse as described herein in a process for making a mouse comprising the ectopic ADAM6 sequence.

In one aspect, a method for making a fertile male mouse comprising a modified immunoglobulin heavy chain locus is described, comprising fertilizing a first mouse germ cell that comprises a modification of an endogenous heavy chain immunoglobulin locus with a second mouse germ cell that comprises an ADAM6 gene or ortholog or homolog or fragment thereof that is functional in a male mouse; forming a fertilized cell; allowing the fertilized cell to develop into an embryo; and, gestating the embryo in a surrogate to obtain a mouse.

In one aspect, the fertilization is achieved by mating a male mouse and a female mouse. In one aspect, the female mouse comprises the ADAM6 gene or ortholog or homolog or fragment thereof. In one aspect, the male mouse comprises the ADAM6 gene or ortholog or homolog or fragment thereof.

In one aspect, use of a nucleic acid sequence encoding a mouse ADAM6 protein or an ortholog or homolog thereof or a functional fragment of the corresponding ADAM6 protein for restoring or enhancing the fertility of a mouse having a genome comprising a modification of an immunoglobulin heavy chain locus is described, wherein the modification reduces or eliminates endogenous ADAM6 function.

In one aspect, the nucleic acid sequence is integrated into the genome of the mouse at an ectopic position. In one aspect, the nucleic acid sequence is integrated into the genome of the mouse at an endogenous immunoglobulin locus. In a specific aspect, the endogenous immunoglobulin locus is a heavy chain locus. In one aspect, the nucleic acid sequence is integrated into the genome of the mouse at a position other than an endogenous immunoglobulin locus.

In one aspect, a method for making a genetically modified mouse is described, comprising replacing at an endogenous heavy chain locus one or more immunoglobulin heavy chain gene segments of a mouse with one or more human immunoglobulin light chain gene segments. In one aspect, the replacement is of all or substantially all functional mouse immunoglobulin heavy chain segments (*i*.*e*., V_{H}, D_{H}, and J_{H} segments) with one or more functional human light chain segments (*i*.*e*., V_{L} and J_{L} segments). In one aspect, the replacement is of all or substantially all functional mouse heavy chain V_{H}, D_{H}, and J_{H} segments with all or substantially all human Vλ or Vκ segments and at least one Jλ or Jκ segment. In a specific aspect, the replacement includes all or substantially all functional human Jλ or Jκ segments.

In one aspect, a method is described for making a mouse that expresses a polypeptide that comprises a sequence derived from a human immunoglobulin Vλ or Vκ and/or Jλ or Jκ segment fused with a mouse heavy chain constant region, comprising replacing endogenous heavy chain immunoglobulin variable segments (V_{H}, D_{H}, and J_{H}) with at least one human Vλ or Vκ segment and at least one human Jλ or Jκ segment, wherein the replacement is in a pluripotent, induced pluripotent, or totipotent mouse cell to form a genetically modified mouse progenitor cell; the genetically modified mouse progenitor cell is introduced into a mouse host; and, the mouse host comprising the genetically modified progenitor cell is gestated to form a mouse comprising a genome derived from the genetically modified mouse progenitor cell. In one aspect, the host is an embryo. In a specific aspect, the host is selected from a mouse pre-morula (*e*.*g*., 8- or 4-cell stage), a tetraploid embryo, an aggregate of embryonic cells, or a blastocyst.

In one aspect, a method is described for making a genetically modified mouse as described herein, comprising introducing by nuclear transfer a nucleic acid containing a modification as described herein into a cell, and maintaining the cell under suitable conditions (*e*.*g*., including culturing the cell and gestating an embryo comprising the cell in a surrogate mother) to develop into a mouse as described herein.

In one aspect, a method for making a modified mouse is described, comprising modifying as described herein a mouse ES cell or pluripotent or totipotent or induced pluripotent mouse cell to include one or more unrearranged immunoglobulin light chain variable gene segments operably linked to an immunoglobulin heavy chain constant sequence, culturing the ES cell, introducing the cultured ES cell into a host embryo to form a chimeric embryo, and introducing the chimeric embryo into a suitable host mouse to develop into a modified mouse. In one aspect, the one or more unrearranged immunoglobulin light chain variable region gene segments are human λ or human κ gene segments. In one aspect, the one or more unrearranged immunoglobulin light chain variable region gene segments comprise human Yλ or human Vκ segments and one or more Jλ, Jκ, or J_{H} segments. In one aspect, the heavy chain constant gene sequence is a human sequence selected from C_{H}1, hinge, C_{H}2, C_{H}3, and a combination thereof. In one aspect, the one or more unrearranged immunoglobulin light chain variable gene segments replace all or substantially all functional endogenous heavy chain variable region gene segments at the endogenous heavy chain locus, and the heavy chain constant sequence is a mouse sequence comprising a C_{H}1, a hinge, a C_{H}2, and a C_{H}3.

In one aspect, nucleic acids constructs, cells, embryos, mice, and methods are described for making proteins that comprise one or more κ and/or λ light chain variable region immunoglobulin sequences and an immunoglobulin heavy chain constant region sequence, including proteins that comprise a human λ or κ light chain variable domain and a human or mouse heavy chain constant region sequence.

In one aspect, a nucleotide sequence encoding an immunoglobulin variable region made in a mouse as described herein is described.

In one aspect, a heavy chain or light chain variable region amino acid sequence of an antibody made in a mouse as described herein is described.

In one aspect, a heavy chain or light chain variable region nucleotide sequence encoding a variable region of an antibody made in a mouse as described herein is described.

In one aspect, an antibody or antigen-binding fragment thereof (*e*.*g*., Fab, F(ab)₂, scFv) made in a mouse as described herein is described.

In one aspect, binding proteins are described that comprise immunoglobulin variable domains that are derived from light chain (*i*.*e*., kappa (κ) and/or lambda (λ)) immunoglobulin variable domains, but not from full-length heavy chain immunoglobulin variable domains. Methods and compositions for making binding proteins, including genetically modified mice, are also described.

In one aspect, a method is described for making an antigen-binding protein that does not comprise an immunoglobulin heavy chain variable domain, comprising a step of immunizing a mouse as described herein with an antigen of interest, and maintaining the mouse under conditions that allow it to make an antigen-binding protein that specifically binds the antigen of interest.

In one aspect, a method is described for making an antigen-binding protein that comprises a first immunoglobulin light chain variable domain contiguous with a heavy chain constant regions sequence, and a second immunoglobulin light chain variable domain that is contiguous with a light chain constant sequence, comprising a step of immunizing a mouse as described herein with an antigen of interest, and maintaining the mouse under conditions that allow it to make an antigen-binding protein that specifically binds the antigen of interest.

In one aspect, a method is described for making an antigen-binding protein that comprises an immunoglobulin light chain variable domain contiguous with a heavy chain constant regions sequence, wherein the antigen-binding protein lacks an immunoglobulin light chain comprising a step of immunizing a mouse as described herein that comprises a knockout of an endogenous κ and λ locus (or that comprises a nonfunctional endogenous κ and/or λ locus) with an antigen of interest, and maintaining the mouse under conditions that allow it to make an antigen-binding protein that specifically binds the antigen of interest.

In one aspect, use of a mouse as described herein to make an immunoglobulin variable region nucleotide sequence is described. In one aspect, the variable region nucleotide sequence comprises rearranged V_{L}, D_{H} and J_{L} gene segments. In one aspect, the variable region nucleotide sequence comprises rearranged V_{L}, D_{H} and J_{H} gene segments. In one aspect, the variable region nucleotide sequence comprises rearranged Vκ and Jκ gene segments. In one aspect, the variable region nucleotide sequence comprises rearranged Vλ and Jλ gene segments.

In one aspect, use of a mouse as described herein to make a fully human Fab or a fully human F(ab)₂ is described. In one aspect, the fully human Fab or a fully human F(ab)₂ comprises rearranged V_{L}, D_{H} and J_{L} gene segments. In one aspect, the fully human Fab or a fully human F(ab)₂ comprises rearranged V_{L}, D_{H} and J_{H} gene segments. In one aspect, the fully human Fab or a fully human F(ab)₂ comprises rearranged Vκ and Jκ gene segments. In one aspect, the fully human Fab or a fully human F(ab)₂ comprises rearranged Vλ and Jλ gene segments.

In one aspect, use of a mouse as described herein to make a fully human Fab (comprising a first human V_{L} fused with a human light chain constant region, and a second human V_{L} fused with a human heavy chain constant region sequence) or a fully human F(ab)₂ is described.

In one aspect, use of a mouse as described herein to make an immortalized cell line is described. In one aspect, the immortalized cell line comprises a nucleic acid sequence encoding a human Vλ or Vκ domain operably linked to a nucleic acid sequence that comprises a mouse constant region nucleic acid sequence. In one aspect, the immortalized cell line expresses an antibody that comprises a human variable domain. In a specific aspect, the human variable domain is a light chain variable domain.

In one aspect, use of a mouse as described herein to make a hybridoma or quadroma is described. In one aspect, the hybridoma or quadroma expresses a polypeptide comprising a human variable domain that binds an antigen of interest.

In one aspect, use of a mouse as described herein to make a phage library containing human light chain variable regions is described. In one aspect, the light chain variable regions are human Vκ regions.

In one aspect, use of a mouse as described herein to generate a variable region sequence for making a human antigen-binding protein is described, comprising (a) immunizing a mouse as described herein with an antigen of interest, (b) isolating a lymphocyte from the immunized mouse of (a), (c) exposing the lymphocyte to one or more labeled antibodies, (d) identifying a lymphocyte that is capable of binding to the antigen of interest, and (e) amplifying one or more human light chain variable region nucleic acid sequences from the lymphocyte thereby generating a variable region sequence.

In one aspect, the lymphocyte is derived from the spleen of the mouse. In one aspect, the lymphocyte is derived from a lymph node of the mouse. In one aspect, the lymphocyte is derived from the bone marrow of the mouse.

In one aspect, the labeled antibody is a fluorophore-conjugated antibody. In one aspect, the one or more fluorophore-conjugated antibodies are selected from an IgM, an IgG, and/or a combination thereof.

In one aspect, the lymphocyte is a B cell.

In one aspect, the one or more variable region nucleic acid sequences comprises a light chain variable region sequence. In a specific aspect, the light chain variable region sequence is an immunoglobulin κ light chain variable region sequence. In one aspect, the one or more variable region nucleic acid sequence is a λ light chain variable region sequence.

In one aspect, use of a mouse as described herein to generate one or more κ light chain variable region sequences for making a human antigen-binding protein is described, comprising (a) immunizing a mouse as described herein with an antigen of interest, (b) isolating the spleen from the immunized mouse of (a), (c) exposing B lymphocytes from the spleen to one or more labeled antibodies, (d) identifying a B lymphocyte of (c) that is capable of binding to the antigen of interest, and (e) amplifying a κ light chain variable region nucleic acid sequence from the B lymphocyte thereby generating the κ light chain variable region sequence.

In one aspect, use of a mouse as described herein to generate a κ light chain variable region sequence for making a human antigen-binding protein is described, comprising (a) immunizing a mouse as described herein with an antigen of interest, (b) isolating one or more lymph nodes from the immunized mouse of (a), (c) exposing B lymphocytes from the one or more lymph nodes to one or more labeled antibodies, (d) identifying a B lymphocyte of (c) that is capable of binding to the antigen of interest, and (e) amplifying a κ light chain variable region nucleic acid sequence from the B lymphocyte thereby generating κ light chain variable region sequence.

In one aspect, use of a mouse as described herein to generate a κ light chain variable region sequence for making a human antigen-binding protein is described, comprising (a) immunizing a mouse as described herein with an antigen of interest, (b) isolating bone marrow from the immunized mouse of (a), (c) exposing B lymphocytes from the bone marrow to one or more labeled antibodies, (d) identifying a B lymphocyte of (c) that is capable of binding to the antigen of interest, and (e) amplifying a κ light chain variable region nucleic acid sequence from the B lymphocyte thereby generating the κ light chain variable region sequence. In various aspects, the one or more labeled antibodies are selected from an IgM, an IgG, and/or a combination thereof.

In various aspects, use of a mouse as described herein to generate a κ light chain variable region sequence for making a human antigen-binding protein is described, further comprising fusing the amplified κ light chain variable region sequence to human heavy or light chain constant region sequences or optionally a human heavy chain variable region seuqence, expressing the fused sequences in a cell, and recovering the expressed sequences thereby generating a human antigen-binding protein.

In various aspects, the human heavy chain constant regions are selected from IgM, IgD, IgA, IgE and IgG. In various specific aspects, the IgG is selected from an IgG1, an IgG2, an IgG3 and an IgG4. In various aspects, the human heavy chain constant region comprises a C_{H}1, a hinge, a C_{H}2, a C_{H}3, a C_{H}4, or a combination thereof. In various aspects, the light chain constant region is an immunoglobulin κ constant region. In various aspects, the cell is selected from a HeLa cell, a DU145 cell, a Lncap cell, a MCF-7 cell, a MDA-MB-438 cell, a PC3 cell, a T47D cell, a THP-1 cell, a U87 cell, a SHSY5Y (human neuroblastoma) cell, a Saos-2 cell, a Vero cell, a CHO cell, a GH3 cell, a PC12 cell, a human retinal cell (*e*.*g*., a PER.C6™ cell), and a MC3T3 cell. In a specific aspect, the cell is a CHO cell.

In one aspect, a method for generating a human light chain variable region specific against an antigen of interest is described, comprising the steps of immunizing a mouse as described herein with the antigen, isolating at least one cell from the mouse producing a human light chain variable region specific against the antigen, generating at least one cell producing a human antigen-binding protein comprising the light chain variable region specific against the antigen, and culturing at least one cell producing the human antigen-binding protein, and obtaining said human antigen-binding protein. In one aspect, the human light chain variable region is a human Vκ region.

In various aspects, the at least one cell isolated from the mouse producing a human light chain variable region specific against the antigen is a splenocyte or a B cell.

In various aspects, the antigen-binding protein is an antibody.

In various aspects, immunization with the antigen of interest is carried out with protein, DNA, a combination of DNA and protein, or cells expressing the antigen.

In one aspect, use of a mouse as described herein to make a nucleic acid sequence encoding an immunoglobulin variable region or fragment thereof is described. In one aspect, the nucleic acid sequence is used to make a human antibody or antigen-binding fragment thereof. In one aspect, the mouse is used to make an antigen-binding protein selected from an antibody, a multispecific antibody (*e*.*g*., a bispecific antibody), an scFv, a bis-scFv, a diabody, a triabody, a tetrabody, a V-NAR, a V_{HH}, a V_{L}, a F(ab), a F(ab)₂, a DVD *(i.e.,* dual variable domain antigen-binding protein), a an SVD (*i*.*e*., single variable domain antigen-binding protein), or a bispecific T-cell engager (BiTE).

In one aspect, use of the mouse as described herein for the manufacture of a medicament (*e*.*g*., an antigen-binding protein), or for the manufacture of a sequence encoding a variable sequence of a medicament (*e*.*g*., an antigen-binding protein), for the treatment of a human disease or disorder is described.

In one aspect, use of a mouse as described herein to make a nucleic acid sequence encoding a first human light chain immunoglobulin variable sequence (V_{L}1) that is cognate with a second human light chain immunoglobulin variable sequence (V_{L}2), wherein the V_{L}1 fused with a human immunoglobulin light chain constant region (polypeptide 1) expresses with V_{L}2 fused with a human immunoglobulin heavy chain constant region (polypeptide 2), as a dimer of polypeptide1/polypeptide 2, to form a V_{L}1-V_{L}2 antibody.

In one aspect, use of a mouse as described herein to make a nucleic acid sequence encoding a human immunoglobulin light chain variable sequence that is fused with a human immunoglobulin heavy chain sequence, wherein the nucleic acid sequence encodes a human V_{L}-C_{H} polypeptide, wherein the human V_{L}-C_{H} polypeptide expresses as a dimer, and wherein the dimer expresses in the absence of an immunoglobulin light chain (*e*.*g*., in the absence of a human λ or human κ light chain). In one aspect, the V_{L}-C_{H} dimer specifically binds an antigen of interest in the absence of a λ light chain and in the absence of a κ light chain.

In one aspect, use of a mouse as described herein to make a nucleic acid sequence encoding all or a portion of an immunoglobulin variable domain. In one aspect, the immunoglobulin variable domain is a human Vλ or human Vκ domain.

In one aspect, use of a nucleic acid construct as described herein for the manufacture of a mouse, a cell, or a therapeutic protein (*e*.*g*., an antibody or other antigen-binding protein) is described.

In one aspect, use of a nucleic acid sequence from a mouse as described herein to make a cell line for the manufacture of a human therapeutic is described. In one aspect, the human therapeutic is a binding protein comprising a human light chain variable sequence (*e*.*g*., derived from a human Vλ or human Vκ segment) fused with a human heavy chain constant sequence. In one aspect, the human therapeutic comprises a first polypeptide that is a human λ or κ immunoglobulin light chain, and a second polypeptide that comprises a human Vλ or human Vκ variable sequence fused with a human heavy chain constant sequence.

In one aspect, an expression system is described, comprising a mammalian cell transfected with a DNA construct that encodes a polypeptide that comprises a somatically mutated human V_{L} domain fused with a human C_{H} domain.

In one aspect, the expression system further comprises a nucleotide sequence that encodes an immunoglobulin V_{L} domain fused with a human C_{L} domain, wherein the V_{L} domain fused with the human C_{L} domain is a cognate light chain with the V_{L} domain fused with the human C_{H} domain.

In one aspect, the mammalian cell is selected from a CHO cell, a COS cell, a Vero cell, a 293 cell, and a retinal cell that expresses a viral gene (*e*.*g*., a PER.C6™ cell).

In one aspect, a method for making a binding protein is described, comprising obtaining a nucleotide sequence encoding a V_{L} domain from a gene encoding a V_{L} region fused to a C_{H} region from a cell of a mouse as described herein, and cloning the nucleotide sequence encoding the V_{L} region sequence in frame with a gene encoding a human C_{H} region to form a human binding protein sequence, expressing the human binding protein sequence in a suitable cell.

In one aspect, the mouse has been immunized with an antigen of interest, and the V_{L} region fused to the C_{H} region specifically binds (*e*.*g*., with a K_{D} in the micromolar, nanomolar, or picomolar range) an epitope of the antigen of interest. In one aspect, nucleotide sequence encoding the V_{L} region fused to the C_{H} region is somatically mutated in the mouse.

In one aspect, the suitable cell is selected from a B cell, a hybridoma, a quadroma, a CHO cell, a COS cell, a 293 cell, a HeLa cell, and a human retinal cell expressing a viral nucleic acid sequence (*e*.*g*., a PERC.6™ cell).

In one aspect, the C_{H} region comprises a human IgG isotype. In a specific aspect, the human IgG is selected from an IgG1, IgG2, and IgG4. In another specific aspect, the human IgG is IgG1. In another specific aspect, the human IgG is IgG4. In another specific aspect, the human IgG4 is a modified IgG4. In one aspect, the modified IgG4 comprises a substitution in the hinge region. In a specific aspect, the modified IgG4 comprises a substitution at amino acid residue 228 relative to a wild-type human IgG4, numbered according to the EU numbering index of Kabat. In a specific aspect, the substitution at amino acid residue 228 is a S228P substitution, numbered according to the EU numbering index of Kabat.

In one aspect, the cell further comprises a nucleotide sequence encoding a V_{L} domain from a light chain that is cognate to the V_{L} domain fused to the C_{H} region, and the method further comprises expressing the nucleotide sequence encoding the cognate V_{L} domain fused to a human Cκ or Cλ domain.

In one aspect, a method for making a bispecific antigen-binding protein is described, comprising exposing a first mouse as described herein to a first antigen of interest and identifying a sequence of a first human V_{L} domain that specifically binds the first antigen of interest; exposing a second mouse as described herein to a second antigen of interest and identifying a sequence of a second human V_{L} domain that specifically binds the second antigen of interest; wherein the first human V_{L} domain does not bind the second antigen of interest, and the second human V_{L} domain does not bind the first antigen of interest; and fusing the sequence of the first human V_{L} domain to a first heavy chain constant sequence to form a first antigen-binding polypeptide, and fusing the sequence of the second human V_{L} domain to a second heavy chain constant sequence to form a second antigen-binding polypeptide; and, employing the first and the second antigen-binding polypeptides in a bispecific binding protein.

In one aspect, the antigen-binding protein further comprises a first immunoglobulin light chain that comprises a human κ or λ variable domain that is cognate with the first human V_{L} domain, and a second immunoglobulin light chain that comprises a human κ or λ variable domain that is cognate with the second human V_{L} domain.

In one aspect, the first heavy chain constant sequence is identical to the second heavy chain constant sequence. In one aspect, the first heavy chain constant sequence comprises a modification that reduces or eliminates binding of the first heavy chain constant region to protein A, and the second heavy chain constant sequence binds protein A.

In one aspect, the first and second human V_{L} domains comprise a sequence derived from a human Vκ and a human Jκ gene segment. In one aspect, the first and second human V_{L} domains comprise a sequence derived from a human Vλ and a human Jλ gene segment. In one aspect, the first and second human V_{L} domains comprise a sequence derived from a human D_{H} gene segment. In one aspect, the first and second human V_{L} domains comprise a sequence derived from a human J_{H} gene segment. In a specific aspect, the first and second human V_{L} domains comprise a sequence derived from a human D_{H} and a human J_{H} gene segment. In a specific aspect, the first and second human V_{L} domains comprise a sequence derived from a human D_{H} and a human Jκ gene segment.

In one aspect, the first human V_{L} domain comprises a sequence derived from a human Vκ and a human Jκ gene segment and the second human V_{L} domain comprises a sequence derived from a human Vλ and a human Jλ gene segment. In one aspect, the first human V_{L} domain comprises a sequence derived from a human Vλ and a human Jλ gene segment and the second human V_{L} domain comprises a sequence derived from a human Vκ and a human Jκ gene segment.

In one aspect, an immunoglobulin variable region (VR) (*e*.*g*., comprising a human V_{L} sequence fused with a human J_{L}, or J_{H}, or D_{H} and J_{H}, or D_{H} and J_{L}) made in a mouse as described herein is described. In a specific aspect, the immunoglobulin VR is derived from a germline human gene segment selected from a Vκ segment and a Vλ segment, wherein the VR is encoded by a rearranged sequence from the mouse wherein the rearranged sequence is somatically hypermutated. In one aspect, the rearranged sequence comprises 1 to 5 somatic hypermutations. In one aspect, the rearranged sequence comprises at least 6, 7, 8, 9, or 10 somatic hypermutations. In one aspect, the rearranged sequence comprises more than 10 somatic hypermutations. In one aspect, the rearranged sequence is fused with one or more human or mouse heavy chain constant region sequences (*e*.*g*., selected from a human or mouse C_{H}1, hinge, C_{H}2, C_{H}3, and a combination thereof).

In one aspect, an immunoglobulin variable domain amino acid sequence of a binding protein made in a mouse as described herein is described. In one aspect, the VR is fused with one or more human or mouse heavy chain constant region sequences (*e*.*g*., selected from a human or mouse C_{H}1, hinge, C_{H}2, C_{H}3, and a combination thereof).

In one aspect, a light chain variable domain encoded by a nucleic acid sequence derived from a mouse as described herein is described.

In one aspect, an antibody or antigen-binding fragment thereof (*e*.*g*., Fab, F(ab)₂, scFv) made in a mouse as described herein, or derived from a sequence made in a mouse as described herein, is described.

### BRIEF DESCRIPTION OF THE FIGURES

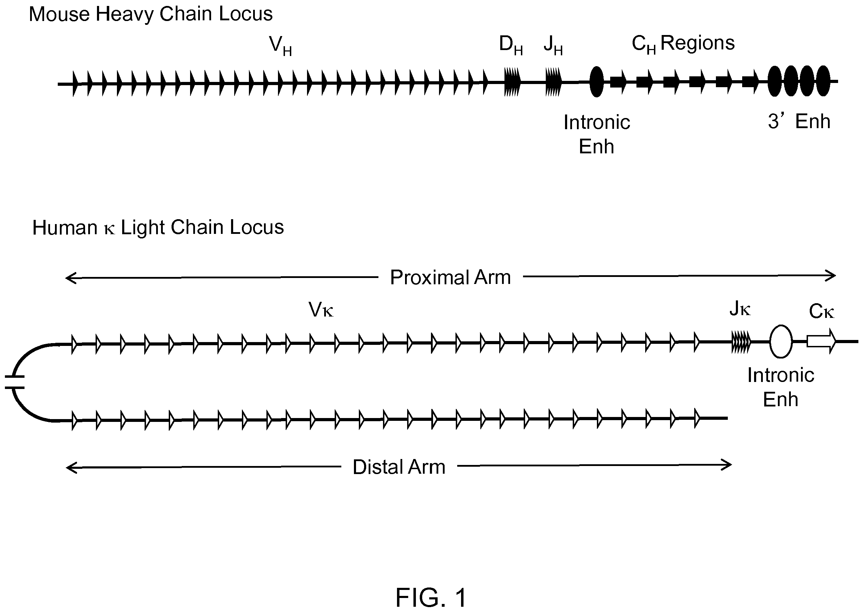

**FIG. 1** illustrates schematics (not to scale) of the mouse heavy chain locus (top) and the human κ light chain locus (bottom). The mouse heavy chain locus is about 3 Mb in length and contains approximately 200 heavy chain variable (V_{H}) gene segments, 13 heavy chain diversity (D_{H}) gene segments and 4 heavy chain joining (J_{H}) gene segments as well as enhancers (Enh) and heavy chain constant (C_{H}) regions. The human κ light chain locus is duplicated into distal and proximal contigs of opposite polarity spanning about 440 kb and 600 kb, respectively. Between the two contigs is about 800 kb of DNA that is believed to be free of Vκ gene segments. The human κ light chain locus contains about 76 Vκ gene segments, 5 Jκ gene segments, an intronic enhancer (Enh) and a single constant region (CK).
**FIG. 2** shows an exemplary targeting strategy for progressive insertion of 40 human Vκ and five human Jκ gene segments into the mouse heavy chain locus resulting in a modified mouse immunoglobulin heavy chain locus comprising human Vκ and Jκ gene segments operably linked to mouse immunoglobulin heavy chain constant regions. Hygromycin (hyg) and neomycin (neo) selection cassettes are shown with recombinase recognition sites (R1, R2, etc.).
**FIG.** 3 shows an exemplary targeting strategy for progressive insertion of a plurality of human Vλ and a single human Jλ gene segment into the mouse heavy chain locus. Hygromycin (hyg) and neomycin (neo) selection cassettes are shown with recombinase recognition sites (R1, R2, etc.).
**FIG. 4** shows an exemplary targeting strategy for progressive insertion of a plurality of human Vλ and four human Jλ gene segments into the mouse heavy chain locus. Hygromycin (hyg) and neomycin (neo) selection cassettes are shown with recombinase recognition sites (R1, R2, etc.).
**FIG. 5** shows an exemplary targeting strategy for progressive insertion of human Vλ, human D_{H} and human J_{H} gene segments into the mouse heavy chain locus. Hygromycin (hyg) and neomycin (neo) selection cassettes are shown with recombinase recognition sites (R1, R2, etc.).
**FIG. 6** shows an exemplary targeting strategy for progressive insertion of human Vλ, human D_{H} and human Jκ gene segments into the mouse heavy chain locus. Hygromycin (hyg) and neomycin (neo) selection cassettes are shown with recombinase recognition sites (R1, R2, etc.).
**FIG.** 7 shows the steps for cloning a genomic fragment encoding mouse ADAM6 genes from a mouse immunoglobulin heavy chain V-D intergenic region and engineering steps to modify the genomic fragment for insertion into a modified immunoglobulin heavy chain locus.
**FIG. 8** shows a targeting strategy for insertion of a genomic fragment encoding mouse ADAM6 genes into the Vκ-Jκ intergenic region of a modified mouse immunoglobulin heavy chain locus containing human Vκ and Jκ gene segments operably linked to mouse immunoglobulin heavy chain constant regions.
**FIG. 9** shows a targeting strategy for insertion of a genomic fragment encoding mouse ADAM6 genes upstream (5') of human Vκ gene segments (*i*.*e*., hVκ2-40) of a modified mouse immunoglobulin heavy chain locus containing human Vκ and Jκ gene segments operably linked to mouse immunoglobulin heavy chain constant regions.

### DETAILED DESCRIPTION

This invention is not limited to particular methods, and experimental conditions described, as such methods and conditions may vary. It is also to be understood that the terminology used herein is for the purpose of describing particular aspects only, and is not intended to be limiting, since the scope of the present invention is defined by the claims.

Unless defined otherwise, all terms and phrases used herein include the meanings that the terms and phrases have attained in the art, unless the contrary is clearly indicated or clearly apparent from the context in which the term or phrase is used. Although any methods and materials similar or equivalent to those described herein can be used in the practice or testing of the present invention, particular methods and materials are now described.

The phrase "substantial" or "substantially" when used to refer to an amount of gene segments (*e*.*g*., "substantially all" V gene segments) includes both functional and non functional gene segments and include, in various aspects, e.g., 80% or more, 85% or more, 90% or more, 95% or more 96% or more, 97% or more, 98% or more, or 99% or more of all gene segments; in various aspects, "substantially all" gene segments includes, e.g., at least 95%, 96%, 97%, 98%, or 99% of functional (*i*.*e*., non-pseudogene) gene segments.

The term "replacement" includes wherein a DNA sequence is placed into a genome of a cell in such a way as to replace a sequence within the genome with a heterologous sequence (*e*.*g*., a human sequence in a mouse), at the locus of the genomic sequence. The DNA sequence so placed may include one or more regulatory sequences that are part of source DNA used to obtain the sequence so placed (*e*.*g*., promoters, enhancers, 5'- or 3'-untranslated regions, appropriate recombination signal sequences, *etc*.). For example, in various aspects, the replacement is a substitution of an endogenous sequence for a heterologous sequence that results in the production of a gene product from the DNA sequence so placed (comprising the heterologous sequence), but not expression of the endogenous sequence; the replacement is of an endogenous genomic sequence with a DNA sequence that encodes a protein that has a similar function as a protein encoded by the endogenous genomic sequence (*e*.*g*., the endogenous genomic sequence encodes an immunoglobulin gene or domain, and the DNA fragment encodes one or more human immunoglobulin genes or domains). In various aspects, an endogenous gene or fragment thereof is replaced with a corresponding human gene or fragment thereof. A corresponding human gene or fragment thereof is a human gene or fragment that is an ortholog of, a homolog of, or is substantially identical or the same in structure and/or function, as the endogenous gene or fragment thereof that is replaced.

The term "contiguous" includes reference to occurrence on the same nucleic acid molecule, *e*.*g*., two nucleic acid sequences are "contiguous" if they occur on the same nucleic molecule but are interrupted by another nucleic acid sequence. For example, a rearranged V(D)J sequence is "contiguous" with a constant region gene sequence, although the final codon of the V(D)J sequence is not followed immediately by the first codon of the constant region sequence. In another example, two V gene segment sequences are "contiguous" if they occur on the same genomic fragment, although they may be separated by sequence that does not encode a codon of the V region, *e*.*g*., they may be separated by a regulatory sequence, *e*.*g*., a promoter or other noncoding sequence. In one aspect, a contiguous sequence includes a genomic fragment that contains genomic sequences arranged as found in a wild-type genome.

The phrase "derived from" when used concerning a variable region "derived from" a cited gene or gene segment includes the ability to trace the sequence back to a particular unrearranged gene segment or gene segments that were rearranged to form a gene that expresses the variable domain (accounting for, where applicable, splice differences and somatic mutations).

The phrase "functional" when used concerning a variable region gene segment or joining gene segment refers to usage in an expressed antibody repertoire; *e*.*g*., in humans Vλ gene segments 3-1, 4-3, 2-8, *etc.* are functional, whereas Vλ gene segments 3-2, 3-4, 2-5, *etc.* are nonfunctional.

A "heavy chain locus" includes a location on a chromosome, *e*.*g*., a mouse chromosome, wherein in a wild-type mouse heavy chain variable (V_{H}), heavy chain diversity (D_{H}), heavy chain joining (J_{H}), and heavy chain constant (C_{H}) region DNA sequences are found.

The phrase "bispecific binding protein" includes a binding protein capable of selectively binding two or more epitopes. Bispecific binding proteins comprise two different polypeptides that comprise a first light chain variable domain (V_{L}1) fused with a first C_{H} region and a second light chain variable domain (V_{L}2) fused with a second C_{H} region. In general, the first and the second C_{H} regions are identical, or they differ by one or more amino acid substitutions (*e*.*g*., as described herein). V_{L}1 and V_{L}2 specifically binding different epitopes-either on two different molecules (*e*.*g*., antigens) or on the same molecule (*e*.*g*., on the same antigen). If a bispecific binding protein selectively binds two different epitopes (a first epitope and a second epitope), the affinity of V_{L}1 for the first epitope will generally be at least one to two or three or four orders of magnitude lower than the affinity of V_{L}1 for the second epitope, and vice versa with respect to V_{L}2. The epitopes recognized by the bispecific binding protein can be on the same or a different target (*e*.*g*., on the same or a different antigen). Bispecific binding proteins can be made, for example, by combining a V_{L}1 and a V_{L}2 that recognize different epitopes of the same antigen. For example, nucleic acid sequences encoding V_{L} sequences that recognize different epitopes of the same antigen can be fused to nucleic acid sequences encoding different C_{H} regions, and such sequences can be expressed in a cell that expresses an immunoglobulin light chain, or can be expressed in a cell that does not express an immunoglobulin light chain. A typical bispecific binding protein has two heavy chains each having three light chain CDRs, followed by (N-terminal to C-terminal) a C_{H}1 domain, a hinge, a C_{H}2 domain, and a C_{H}3 domain, and an immunoglobulin light chain that either does not confer antigen-binding specificity but that can associate with each heavy chain, or that can associate with each heavy chain and that can bind one or more of the epitopes bound by V_{L}1 and/or V_{L}2, or that can associate with each heavy chain and enable binding or assist in binding of one or both of the heavy chains to one or both epitopes.

Therefore, two general types of bispecific binding proteins are (1) V_{L}1-C_{H} (dimer), and (2) V_{L}1-C_{H}:light chain + V_{L}2-C_{H}:light chain, wherein the light chain is the same or different. In either case, the C_{H} (*i*.*e*., the heavy chain constant region) can be differentially modified (*e*.*g*., to differentially bind protein A, to increase serum half-life, *etc*.) as described herein, or can be the same.

The term "cell," when used in connection with expressing a sequence includes any cell that is suitable for expressing a recombinant nucleic acid sequence. Cells include those of prokaryotes and eukaryotes (single-cell or multiple-cell), bacterial cells (*e*.*g*., strains of *E*. *coli, Bacillus spp., Streptomyces spp., etc.),* mycobacteria cells, fungal cells, yeast cells (*e.g., S. cerevisiae, S. pombe, P. pastoris, P. methanolica, etc*.), plant cells, insect cells (*e.g., SF-9, SF-21, baculovirus-infected insect cells, Trichoplusia ni, etc*.), non-human animal cells, human cells, B cells, or cell fusions such as, for example, hybridomas or quadromas. In some aspects, the cell is a human, monkey, ape, hamster, rat, or mouse cell. In some aspects, the cell is eukaryotic and is selected from the following cells: CHO (*e*.*g*., CHO K1, DXB-11 CHO, Veggie-CHO), COS (*e*.*g*., COS-7), retinal cell, Vero, CV1, kidney (*e*.*g*., HEK293, 293 EBNA, MSR 293, MDCK, HaK, BHK), HeLa, HepG2, WI38, MRC 5, Colo205, HB 8065, HL-60, (*e*.*g*., BHK21), Jurkat, Daudi, A431 (epidermal), CV-1, U937, 3T3, L cell, C127 cell, SP2/0, NS-0, MMT 060562, Sertoli cell, BRL 3A cell, HT1080 cell, myeloma cell, tumor cell, and a cell line derived from an aforementioned cell. In some aspects, the cell comprises one or more viral genes, *e.g.* a retinal cell that expresses a viral gene (*e*.*g*., a PER.C6™ cell).

The term "cognate," when used in the sense of "cognate with," *e*.*g*., a first V_{L} domain that is "cognate with" a second V_{L} domain, is intended to include reference to the relation between two V_{L} domains from a same binding protein made by a mouse in accordance with the invention. For example, a mouse that is genetically modified in accordance with an aspect of the invention, *e*.*g*., a mouse having a heavy chain locus in which V_{H}, D_{H}, and J_{H} regions are replaced with V_{L} and J_{L} regions, makes antibody-like binding proteins that have two identical polypeptide chains made of the same mouse C_{H} region (*e*.*g*., an IgG isotype) fused with a first human V_{L} domain, and two identical polypeptide chains made of the same mouse C_{L} region fused with a second human V_{L} domain. During clonal selection in the mouse, the first and the second human V_{L} domains were selected by the clonal selection process to appear together in the context of a single antibody-like binding protein. Thus, first and second V_{L} domains that appear together, as the result of the clonal selection process, in a single antibody-like molecule are referred to as being "cognate." In contrast, a V_{L} domain that appears in a first antibody-like molecule and a V_{L} domain that appears in a second antibody-like molecule are *not* cognate, *unless* the first and the second antibody-like molecules have identical heavy chains (*i*.*e*., unless the V_{L} domain fused to the first human heavy chain region and the V_{L} domain fused to the second human heavy chain region are identical).

The phrase "complementarity determining region," or the term "CDR," includes an amino acid sequence encoded by a nucleic acid sequence of an organism's immunoglobulin genes that normally *(i.e.,* in a wild-type animal) appears between two framework regions in a variable region of a light or a heavy chain of an immunoglobulin molecule (*e*.*g*., an antibody or a T cell receptor). A CDR can be encoded by, for example, a germline sequence or a rearranged or unrearranged sequence, and, for example, by a naive or a mature B cell or a T cell. In some circumstances (*e*.*g*., for a CDR3), CDRs can be encoded by two or more sequences (*e*.*g*., germline sequences) that are not contiguous (*e*.*g*., in an unrearranged nucleic acid sequence) but are contiguous in a B cell nucleic acid sequence, *e*.*g*., as the result of splicing or connecting the sequences (*e*.*g*., V-D-J recombination to form a heavy chain CDR3).

The phrase "gene segment," or "segment" includes reference to a V (light or heavy) or D or J (light or heavy) immunoglobulin gene segment, which includes unrearranged sequences at immunoglobulin loci (in *e*.*g*., humans and mice) that can participate in a rearrangement (mediated by, *e*.*g*., endogenous recombinases) to form a rearranged V/J or V/D/J sequence. Unless indicated otherwise, the V, D, and J segments comprise recombination signal sequences (RSS) that allow for V/J recombination or V/D/J recombination according to the 12/23 rule. Unless indicated otherwise, the segments further comprise sequences with which they are associated in nature or functional equivalents thereof (*e*.*g*., for V segments promoter(s) and leader(s)).

The phrase "heavy chain," or "immunoglobulin heavy chain" includes an immunoglobulin heavy chain constant region sequence from any organism, and unless otherwise specified includes a heavy chain variable domain (V_{H}). V_{H} domains include three heavy chain CDRs and four framework (FR) regions, unless otherwise specified. Fragments of heavy chains include CDRs, CDRs and FRs, and combinations thereof. A typical heavy chain consists essentially of, following the variable domain (from N-terminal to C-terminal), a C_{H}1 domain, a hinge, a C_{H}2 domain, a C_{H}3 domain, and optionally a C_{H}4 domain (*e*.*g*., in the case of IgM or IgE) and a transmembrane (M) domain (*e*.*g*., in the case of membrane-bound immunoglobulin on lymphocytes). A heavy chain constant region is a region of a heavy chain that extends (from N-terminal side to C-terminal side) from outside FR4 to the C-terminal of the heavy chain. Heavy chain constant regions with minor deviations, *e*.*g*., truncations of one, two, three or several amino acids from the C-terminal, would be encompassed by the phrase "heavy chain constant region," as well as heavy chain constant regions with sequence modifications, e.g., 1, 2, 3, 4, 5, 6, 7, 8, 9, or 10 amino acid substitutions. Amino acid substitutions can be made at one or more positions selected from, e.g. (with reference to EU numbering of an immunoglobulin constant region, *e*.*g*., a human IgG constant region), 228, 233, 234, 235, 236, 237, 238, 239, 241, 248, 249, 250, 252, 254, 255, 256, 258, 265, 267, 268, 269, 270, 272, 276, 278, 280, 283, 285, 286, 289, 290, 292, 293, 294, 295, 296, 297, 298, 301, 303, 305, 307, 308, 309, 311, 312, 315, 318, 320, 322, 324, 326, 327, 328, 329, 330, 331, 332, 333, 334, 335, 337, 338, 339, 340, 342, 344, 356, 358, 359, 360, 361, 362, 373, 375, 376, 378, 380, 382, 383, 384, 386, 388, 389, 398, 414, 416, 419, 428, 430, 433, 434, 435, 437, 438, and 439.

For example, and not by way of limitation, a heavy chain constant region can be modified to exhibit enhanced serum half-life (as compared with the same heavy chain constant region without the recited modification(s)) and have a modification at position 250 (*e*.*g*., E or Q); 250 and 428 (*e*.*g*., L or F); 252 (*e*.*g*., L/Y/F/W or T), 254 (*e*.*g*., S or T), and 256 (*e*.*g*., S/R/Q/E/D or T); or a modification at 428 and/or 433 (*e*.*g*., L/R/SI/P/Q or K) and/or 434 (*e*.*g*., H/F or Y); or a modification at 250 and/or 428; or a modification at 307 or 308 (*e*.*g*., 308F, V308F), and 434. In another example, the modification can comprise a 428L (*e*.*g*., M428L) and 434S (*e*.*g*., N434S) modification; a 428L, 259I (*e*.*g*., V259I), and a 308F (*e*.*g*., V308F) modification; a 433K (*e*.*g*., H433K) and a 434 (*e*.*g*., 434Y) modification; a 252, 254, and 256 (*e*.*g*., 252Y, 254T, and 256E) modification; a 250Q and 428L modification (*e*.*g*., T250Q and M428L); a 307 and/or 308 modification (*e*.*g*., 308F or 308P).

The phrase "light chain" includes an immunoglobulin light chain constant (C_{L}) region sequence from any organism, and unless otherwise specified includes human κ and λ light chains. Light chain variable (V_{L}) domains typically include three light chain CDRs and four framework (FR) regions, unless otherwise specified. Generally, a full-length light chain (V_{L} + C_{L}) includes, from amino terminus to carboxyl terminus, a V_{L} domain that includes FR1-CDR1-FR2-CDR2-FR3-CDR3-FR4, and a C_{L} region. Light chains (V_{L} + C_{L}) that can be used with this invention include those, *e*.*g*., that do not selectively bind either a first or second (in the case of bispecific binding proteins) epitope selectively bound by the binding protein (*e*.*g*., the epitope(s) selectively bound by the V_{L} domain fused with the C_{H} domain). V_{L} domains that do not selectively bind the epitope(s) bound by the V_{L} that is fused with the C_{H} domain include those that can be identified by screening for the most commonly employed light chains in existing antibody libraries (wet libraries or *in silico*), wherein the light chains do not substantially interfere with the affinity and/or selectivity of the epitope binding domains of the binding proteins. Suitable light chains include those that can bind (alone or in combination with its cognate V_{L} fused with the C_{H} region) an epitope that is specifically bound by the V_{L} fused to the C_{H} region.

The phrase "micromolar range" is intended to mean 1-999 micromolar; the phrase "nanomolar range" is intended to mean 1-999 nanomolar; the phrase "picomolar range" is intended to mean 1-999 picomolar.

The term "non-human animals" is intended to include any non-human animals such as cyclostomes, bony fish, cartilaginous fish such as sharks and rays, amphibians, reptiles, mammals, and birds. Suitable non-human animals include mammals. Suitable mammals include non-human primates, goats, sheep, pigs, dogs, cows, and rodents. Suitable non-human animals are selected from the rodent family including rat and mouse. In one aspect, the non-human animals are mice.

The mouse as a genetic model has been greatly enhanced by transgenic and knockout technologies, which have allowed for the study of the effects of the directed over-expression or deletion of specific genes. Despite all of its advantages, the mouse still presents genetic obstacles that render it an imperfect model for human diseases and an imperfect platform to test human therapeutics or make them. First, although about 99% of human genes have a mouse homolog (Waterston et al. (2002), Initial sequencing and comparative analysis of the mouse genome, Nature 420, 520-562), potential therapeutics often fail to cross-react, or cross-react inadequately, with mouse orthologs of the intended human targets. To obviate this problem, selected target genes can be "humanized," that is, the mouse gene can be eliminated and replaced by the corresponding human orthologous gene sequence (*e*.*g*., US 6,586,251, US 6,596,541 and US 7,105,348). Initially, efforts to humanize mouse genes by a "knockout-plus-transgenic humanization" strategy entailed crossing a mouse carrying a deletion (*i*.*e*., knockout) of the endogenous gene with a mouse carrying a randomly integrated human transgene (see, *e*.*g*., Bril et al. (2006), Tolerance to factor VIII in a transgenic mouse expressing human factor VIII cDNA carrying an Arg(593) to Cys substitution, Thromb Haemost 95:341-347; Homanics et al. (2006), Production and characterization of murine models of classic and intermediate maple syrup urine disease, BMC Med Genet 7:33; Jamsai et al. (2006), A humanized BAC transgenic/knockout mouse model for HbE/beta-thalassemia, Genomics 88(3):309-15; Pan et al. (2006), Different role for mouse and human CD3delta/epsilon heterodimer in preT cell receptor (preTCR) function: human CD3delta/epsilon heterodimer restores the defective preTCR function in CD3gamma- and CD3gammadelta-deficient mice, Mol Immunol 43:1741-1750). But those efforts were hampered by size limitations; conventional knockout technologies were not sufficient to directly replace large mouse genes with their large human genomic counterparts. A straightforward approach of direct homologous replacement, in which an endogenous mouse gene is directly replaced by the human counterpart gene at the same precise genetic location of the mouse gene *(i.e.,* at the endogenous mouse locus), is rarely attempted because of technical difficulties. Until now, efforts at direct replacement involved elaborate and burdensome procedures, thus limiting the length of genetic material that could be handled and the precision with which it could be manipulated.

Exogenously introduced human immunoglobulin transgenes rearrange in precursor B-cells in mice (Alt et al. (1985), Immunoglobulin genes in transgenic mice, Trends Genet 1:231-236). This finding was exploited by engineering mice using the knockout-plus-transgenic approach to express human antibodies (Green et al. (1994), Antigen-specific human monoclonal antibodies from mice engineered with human Ig heavy and light chain YACs, Nat Genet 7:13-21; Lonberg, N. (2005), Human antibodies from transgenic animals. Nat Biotechnol 23:1117-1125; Lonberg et al. (1994), Antigen-specific human antibodies from mice comprising four distinct genetic modifications, Nature 368:856-859; Jakobovits et al. (2007), From XenoMouse technology to panitumumab, the first fully human antibody product from transgenic mice, Nat Biotechnol 25:1134-1143). The endogenous mouse immunoglobulin heavy chain and κ light chain loci were inactivated in these mice by targeted deletion of small but critical portions of each endogenous locus, followed by introducing human immunoglobulin gene loci as randomly integrated large transgenes, as described above, or minichromosomes (Tomizuka et al. (2000), Double trans-chromosomic mice: maintenance of two individual human chromosome fragments containing Ig heavy and kappa loci and expression of fully human antibodies, PNAS USA 97:722-727). Such mice represented an important advance in genetic engineering; fully human monoclonal antibodies isolated from them yielded promising therapeutic potential for treating a variety of human diseases (Gibson et al. (2006), Randomized phase III trial results of panitumumab, a fully human anti-epidermal growth factor receptor monoclonal antibody, in metastatic colorectal cancer, Clin Colorectal Cancer 6:29-31; Jakobovits *et al.,* 2007; Kim et al. (2007), Clinical efficacy of zanolimumab (HuMax-CD4): two Phase II studies in refractory cutaneous T-cell lymphoma, Blood 109(11):4655-62; Lonberg, 2005; Maker et al. (2005), Tumor regression and autoimmunity in patients treated with cytotoxic T lymphocyte-associated antigen 4 blockade and interleukin 2: a phase I/II study, Ann Surg Oncol 12:1005-1016; McClung et al. (2006), Denosumab in postmenopausal women with low bone mineral density, N Engl J Med 354:821-831). But, as discussed above, these mice exhibit compromised B cell development and immune deficiencies when compared to wild type mice. Such problems potentially limit the ability of the mice to support a vigorous humoral response and, consequently, generate fully human antibodies against some antigens. The deficiencies may be due to: (1) inefficient functionality due to the random introduction of the human immunoglobulin transgenes and resulting incorrect expression due to a lack of upstream and downstream control elements (Garrett et al. (2005), Chromatin architecture near a potential 3' end of the Igh locus involves modular regulation of histone modifications during B-Cell development and in vivo occupancy at CTCF sites, Mol Cell Biol 25:1511-1525; Manis et al. (2003), Elucidation of a downstream boundary of the 3' IgH regulatory region, Mol Immunol 39:753-760; Pawlitzky et al. (2006), Identification of a candidate regulatory element within the 5' flanking region of the mouse Igh locus defined by pro-B cell-specific hypersensitivity associated with binding of PU.1, Pax5, and E2A, J Immunol 17616839-6851); (2) inefficient interspecies interactions between human constant domains and mouse components of the B-cell receptor signaling complex on the cell surface, which may impair signaling processes required for normal maturation, proliferation, and survival of B cells (Hombach et al. (1990), Molecular components of the B-cell antigen receptor complex of the IgM class, Nature 343:760-762); and (3) inefficient interspecies interactions between soluble human immunoglobulins and mouse Fc receptors that might reduce affinity selection (Rao et al. (2002). Differential expression of the inhibitory IgG Fc receptor FcgammaRIIB on germinal center cells: implications for selection of high-affinity B cells, J Immunol 169:1859-1868) and immunoglobulin serum concentrations (Brambell et al. (1964), A Theoretical Model of Gamma-Globulin Catabolism, Nature 203:1352-1354; Junghans and Anderson, (1996), The protection receptor for IgG catabolism is the beta2-microglobulin-containing neonatal intestinal transport receptor, PNAS USA93:5512-5516; Rao *et al.,* 2002; Hjelm et al. (2006), Antibody-mediated regulation of the immune response, Scand J Immunol 64:177-184; Nimmerjahn and Ravetch, (2007), Fc-receptors as regulators of immunity, Adv Immunol 96:179-204). These deficiencies can be corrected by *in situ* humanization of only the variable regions of the mouse immunoglobulin loci within their natural locations at the endogenous heavy and light chain loci. This would effectively result in mice that make "reverse chimeric" (i.e., human V: mouse C) antibodies which would be capable of normal interactions and selection with the mouse environment based on retaining mouse constant regions. Further such reverse chimeric antibodies may be readily reformatted into fully human antibodies for therapeutic purposes.

Genetically modified animals that comprise an insertion or a replacement at the endogenous immunoglobulin heavy chain locus with heterologous (*e*.*g*., from another species) immunoglobulin sequences can be made in conjunction with insertions or replacements at endogenous immunoglobulin light chain loci or in conjunction with immunoglobulin light chain transgenes (*e*.*g*., chimeric immunoglobulin light chain transgenes or fully human fully mouse, etc.). The species from which the heterologous immunoglobulin sequences are derived can vary widely. Exemplary heterologous immunoglobulin sequences include human sequences.

Immunoglobulin variable region nucleic acid sequences, *e*.*g*., V, D, and/or J segments, are in various aspects obtained from a human or a non-human animal. Non-human animals suitable for providing V, D, and/or J segments include, for example bony fish, cartilaginous fish such as sharks and rays, amphibians, reptiles, mammals, birds (*e*.*g*., chickens). Non-human animals include, for example, mammals. Mammals include, for example, non-human primates, goats, sheep, pigs, dogs, bovine (*e*.*g*., cow, bull, buffalo), deer, camels, ferrets and rodents and non-human primates (*e*.*g*., chimpanzees, orangutans, gorillas, marmosets, rhesus monkeys baboons). Suitable non-human animals are selected from the rodent family including rats, mice, and hamsters. In one aspect, the non-human animals are mice. As clear from the context, various non-human animals can be used as sources of variable domains or variable region gene segments (*e*.*g*., sharks, rays, mammals, *e*.*g*., camels, rodents such as mice and rats).

According to the context, non-human animals are also used as sources of constant region sequences to be used in connection with variable sequences or segments, for example, rodent constant sequences can be used in transgenes operably linked to human or non-human variable sequences (*e*.*g*., human or non-human primate variable sequences operably linked to, *e*.*g*., rodent, *e*.*g*., mouse or rat or hamster, constant sequences). Thus, in various aspects, human V, D, and/or J segments are operably linked to rodent (*e*.*g*., mouse or rat or hamster) constant region gene sequences. In some aspects, the human V, D, and/or J segments (or one or more rearranged VDJ or VJ genes) are operably linked or fused to a mouse, rat, or hamster constant region gene sequence in, *e*.*g*., a transgene integrated at a locus that is not an endogenous immunoglobulin locus.

In a specific aspect, a mouse is described that comprises a replacement of V_{H}, D_{H}, and J_{H} gene segments at an endogenous immunoglobulin heavy chain locus with one or more human V_{L} and one or more human J_{L} gene segments, wherein the one or more human V_{L} and one or more J_{L} gene segments are operably linked to an endogenous immunoglobulin heavy chain gene; wherein the mouse comprises a transgene at a locus other than an endogenous immunoglobulin locus, wherein the transgene comprises an unrearranged or rearranged human V_{L} and human J_{L} gene segment operably linked to a mouse or rat or human constant region. In various aspects, the one or more human V_{L} gene segments include human Vκ or human Vλ gene segments. In one aspect, the one or more human J_{L} gene segments include human Jκ or human Jλ gene segments.

A method for a large *in situ* genetic replacement of the mouse germline immunoglobulin heavy chain variable genes with human germline immunoglobulin light chain variable genes while maintaining the ability of the mice to generate offspring is described. Specifically, the precise replacement of the mouse heavy chain variable gene loci with human light chain variable gene loci while leaving the mouse constant regions intact is described. As a result, mice have been created that express immunoglobulin-like binding proteins in the context of endogenous constant regions. The human light chain variable regions are linked to mouse heavy chain constant regions to form chimeric human-mouse immunoglobulin loci that rearrange and express unique immunoglobulin-like molecules. The immunoglobulin-like molecules expressed are "reverse chimeras," *i*.*e*., they comprise human variable region sequences and mouse constant region sequences.

The engineering of human immunoglobulin sequences in the genome of a mouse, even at precise locations, *e*.*g*., at the endogenous mouse immunoglobulin loci, may present certain challenges due to divergent evolution of the immunoglobulin loci between mouse and man. For example, intergenic sequences interspersed within the immunoglobulin loci are not identical between mice and humans and, in some circumstances, may not be functionally equivalent. Differences between mice and humans in their immunoglobulin loci can still result in abnormalities in humanized mice, particularly when humanizing or manipulating certain portions of endogenous mouse immunoglobulin heavy chain loci. Some modifications at mouse immunoglobulin heavy chain loci are deleterious. Deleterious modifications can include, for example, loss of the ability of the modified mice to mate and produce offspring. In various aspects, engineering human immunoglobulin sequences in the genome of a mouse includes methods that maintain endogenous sequences that when absent in modified mouse strains are deleterious. Exemplary deleterious effects may include inability to propagate modified strains, loss of function of essential genes, inability to express polypeptides, *etc.* Such deleterious effects may be directly or indirectly related to the modification engineered into the genome of the mouse.

Notwithstanding the near wild-type humoral immune function observed in mice with replaced immunoglobulin loci, there are other challenges encountered when employing a direct replacement of the immunoglobulin that is not encountered in some approaches that employ randomly integrated transgenes. Differences in the genetic composition of the immunoglobulin loci between mice and humans has lead to the discovery of sequences beneficial for the propagation of mice with replaced immunoglobulin gene segments. Specifically, mouse ADAM genes located within the endogenous immunoglobulin heavy chain locus are optimally present in mice with replaced immunoglobulin loci, due to their role in fertility.

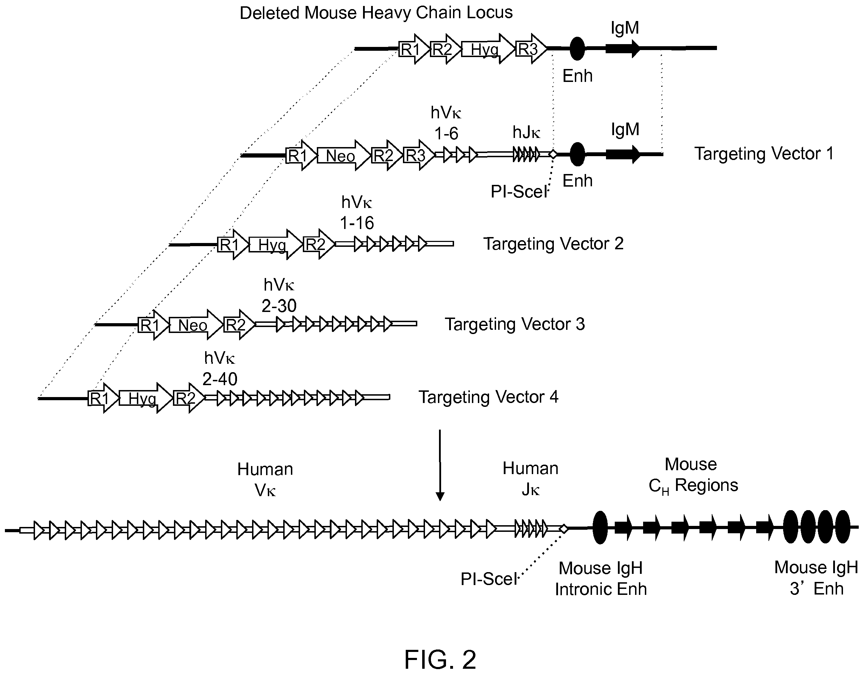

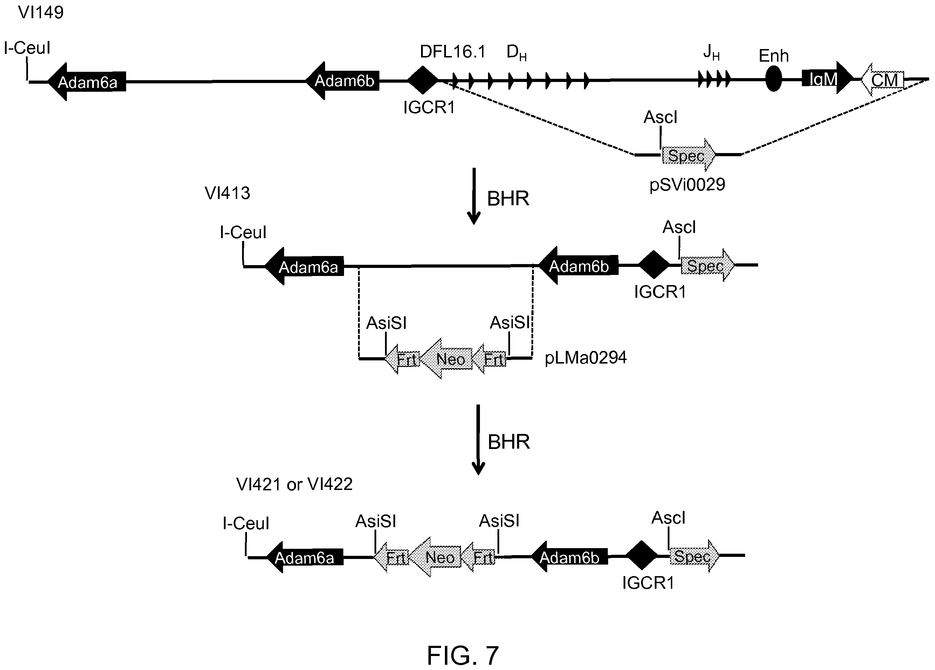

A precise, *in situ* replacement of six megabases of the variable regions of the mouse heavy chain immunoglobulin loci (V_{H}-D_{H}-J_{H}) with human immunoglobulin light chain variable gene loci (V_{L}-J_{L}) is performed, while leaving the flanking mouse sequences intact and functional within the hybrid loci, including all mouse constant chain genes and locus transcriptional control regions (FIG. 2 - FIG. 6). Engineering steps to maintain mouse sequences that confer on the mouse the ability to mate and produce offspring in a manner comparable to a wild-type mouse were performed (FIG. 7 - FIG. 9). Specifically, about half a megabase of the human immunoglobulin κ light chain locus containing the proximal arm (*i*.*e*., 40 functional human Vκ gene segments and five human Jκ gene segments) and mouse ADAM6 genes were introduced through chimeric BAC targeting vectors into mouse ES cells using VELOCIGENE® genetic engineering technology (see, *e*.*g*., US Pat. No. 6,586,251 and Valenzuela et al., 2003, High-throughput engineering of the mouse genome coupled with high-resolution expression analysis, Nat Biotechnol 21:652-659).

### Genomic Location and Function of Mouse ADAM6

Male mice that lack the ability to express any functional ADAM6 protein exhibit a severe defect in the ability of the mice to mate and to generate offspring. The mice lack the ability to express a functional ADAM6 protein by virtue of a replacement of all or substantially all mouse immunoglobulin heavy chain variable gene segments with human light chain variable gene segments. The loss of ADAM6 function results because the ADAM6 locus is located within a region of the endogenous immunoglobulin heavy chain variable gene locus, proximal to the 3' end of the V_{H} gene segment locus that is upstream of the D_{H} gene segments. In order to breed mice that are homozygous for a replacement of all or substantially all endogenous heavy chain variable gene segments with human light chain variable gene segments, it is generally a cumbersome approach to set up males and females that are each homozygous for the replacement and await a productive mating. Successful litters are relatively rare, and average litter size is very low. Instead, males heterozygous for the replacement have been employed to mate with females homozygous for the replacement to generate progeny that are heterozygous for the replacement, then breed a homozygous mouse therefrom. The inventors have determined that the likely cause of the loss in fertility in the male mice is the absence in homozygous male mice of a functional ADAM6 protein.

In various aspects, male mice that comprise a damaged *(i.e.,* nonfunctional or marginally functional) ADAM6 gene exhibit a reduction or elimination of fertility. Because in mice (and other rodents) the ADAM6 gene is located in the immunoglobulin heavy chain locus, the inventors have determined that in order to propagate mice, or create and maintain a strain of mice, that comprise modifications to an endogenous immunoglobulin heavy chain locus, various modified breeding or propagation schemes are employed. The low fertility, or infertility, of male mice homozygous for a replacement of the endogenous immunoglobulin heavy chain variable gene locus renders maintaining such a modification in a mouse strain difficult. In various aspects, maintaining the strain comprises avoiding infertility problems exhibited by male mice homozygous for a replacement.

In one aspect, a method for maintaining a strain of mouse as described herein is described. The strain of mouse need not comprise an ectopic ADAM6 sequence, and in various aspects the strain of mouse is homozygous or heterozygous for a knockout (*e*.*g*., a functional knockout) of ADAM6.

The mouse strain comprises a modification of an endogenous immunoglobulin heavy chain locus that results in a reduction or loss in fertility in a male mouse. In one aspect, the modification comprises a deletion of a regulatory region and/or a coding region of an ADAM6 gene. In a specific aspect, the modification comprises a modification of an endogenous ADAM6 gene (regulatory and/or coding region) that reduces or eliminates fertility of a male mouse that comprises the modification; in a specific aspect, the modification reduces or eliminates fertility of a male mouse that is homozygous for the modification.

In one aspect, the mouse strain is homozygous or heterozygous for a knockout (e.g., a functional knockout) or a deletion of an ADAM6 gene.

In one aspect, the mouse strain is maintained by isolating from a mouse that is homozygous or heterozygous for the modification a cell, and employing the donor cell in host embryo, and gestating the host embryo and donor cell in a surrogate mother, and obtaining from the surrogate mother a progeny that comprises the genetic modification. In one aspect, the donor cell is an ES cell. In one aspect, the donor cell is a pluripotent cell, e.g., an induced pluripotent cell.

In one aspect, the mouse strain is maintained by isolating from a mouse that is homozygous or heterozygous for the modification a nucleic acid sequence comprising the modification, and introducing the nucleic acid sequence into a host nucleus, and gestating a cell comprising the nucleic acid sequence and the host nucleus in a suitable animal. In one aspect, the nucleic acid sequence is introduced into a host oocyte embryo.

In one aspect, the mouse strain is maintained by isolating from a mouse that is homozygous or heterozygous for the modification a nucleus, and introducing the nucleus into a host cell, and gestating the nucleus and host cell in a suitable animal to obtain a progeny that is homozygous or heterozygous for the modification.

In one aspect, the mouse strain is maintained by employing *in vitro* fertilization (IVF) of a female mouse (wild-type, homozygous for the modification, or heterozygous for the modification) employing a sperm from a male mouse comprising the genetic modification. In one aspect, the male mouse is heterozygous for the genetic modification. In one aspect, the male mouse is homozygous for the genetic modification.

In one aspect, the mouse strain is maintained by breeding a male mouse that is heterozygous for the genetic modification with a female mouse to obtain progeny that comprises the genetic modification, identifying a male and a female progeny comprising the genetic modification, and employing a male that is heterozygous for the genetic modification in a breeding with a female that is wild-type, homozygous, or heterozygous for the genetic modification to obtain progeny comprising the genetic modification. In one aspect, the step of breeding a male heterozygous for the genetic modification with a wild-type female, a female heterozygous for the genetic modification, or a female homozygous for the genetic modification is repeated in order to maintain the genetic modification in the mouse strain.

In one aspect, a method is described for maintaining a mouse strain that comprises a replacement of an endogenous immunoglobulin heavy chain variable gene locus with one or more human immunoglobulin light chain sequences, and optionally one or more human D_{H} gene segments, comprising breeding the mouse strain so as to generate heterozygous male mice, wherein the heterozygous male mice are bred to maintain the genetic modification in the strain. In a specific aspect, the strain is not maintained by any breeding of a homozygous male with a wild-type female, or a female homozygous or heterozygous for the genetic modification.

The ADAM6 protein is a member of the A Disintegrin And Metalloprotease (ADAM) family of proteins, which is a large family with diverse functions including cell adhesion. Some members of the ADAM family are implicated in spermatogenesis and fertilization. For example, ADAM2 encodes a subunit of the protein fertilin, which is implicated in sperm-egg interactions. ADAM3, or cyritestin, appears necessary for sperm binding to the zona pellucida. The absence of either ADAM2 or ADAM3 results in infertility. It has been postulated that ADAM2, ADAM3, and ADAM6 form a complex on the surface of mouse sperm cells.

In humans, an ADAM6 gene, reportedly a pseudogene, is located between human V_{H} gene segments V_{H}1-2 and V_{H}6-1. In mice, there are two ADAM6 genes-ADAM6a and ADAM6b-that are located in an intergenic region between mouse V_{H} and D_{H} gene segments, and are oriented in opposite transcriptional orientation to that of the surrounding immunoglobulin gene segments. In mice, a functional ADAM6 locus is apparently required for normal fertilization. A functional ADAM6 locus or sequence, then, refers to an ADAM6 locus or sequence that can complement, or rescue, the drastically reduced fertilization exhibited in male mice with missing or damaged endogenous ADAM6 loci.

The position of the intergenic sequence in mice that encodes ADAM6a and ADAM6b renders the intergenic sequence susceptible to modification when modifying an endogenous heavy chain. When V_{H} gene segments are deleted or replaced, or when D_{H} gene segments are deleted or replaced, there is a high probability that a resulting mouse will exhibit a severe deficit in fertility. In order to compensate for the deficit, the mouse is modified to include a nucleotide sequence that encodes a protein that will complement the loss in ADAM6 activity due to a modification of the endogenous ADAM6 locus. In various aspects, the complementing nucleotide sequence is one that encodes a mouse ADAM6a, a mouse ADAM6b, or a homolog or ortholog or functional fragment thereof that rescues the fertility deficit. In various aspects, the complementing nucleotide sequence encodes a mouse ADAM6a protein as set forth in SEQ ID NO: 1, and/or encodes a mouse ADAM6b protein as set forth in SEQ ID NO: 2. Alternatively, suitable methods to preserve the endogenous ADAM6 locus can be employed, while rendering the endogenous immunoglobulin heavy chain sequences flanking the mouse ADAM6 locus incapable of rearranging to encode a functional endogenous heavy chain variable region. Exemplary alternative methods include manipulation of large portions of mouse chromosomes that position the endogenous immunoglobulin heavy chain variable region loci in such a way that they are incapable of rearranging to encode a functional heavy chain variable region that is operably linked to an endogenous heavy chain constant gene. In various aspects, the methods include inversions and/or translocations of mouse chromosomal fragments containing endogenous immunoglobulin heavy chain gene segments.

The nucleotide sequence that rescues fertility can be placed at any suitable position. It can be placed in an intergenic region (*e*.*g*., between V and J gene segments or upstream of V gene segments), or in any suitable position in the genome *(i.e.,* ectopically). In one aspect, the nucleotide sequence can be introduced into a transgene that randomly integrates into the mouse genome. In one aspect, the sequence can be maintained episomally, that is, on a separate nucleic acid rather than on a mouse chromosome. Suitable positions include positions that are transcriptionally permissive or active, *e*.*g*., a ROSA26 locus (Zambrowicz et al., 1997, PNAS USA 94:3789-3794), a BT-5 locus (Michael et al., 1999, Mech. Dev. 85:35-47), or an Oct4 locus (Wallace et al., 2000, Nucleic Acids Res. 28:1455-1464). Targeting nucleotide sequences to transcriptionally active loci are described, e.g., in US 7,473,557.

Alternatively, the nucleotide sequence that rescues fertility can be coupled with an inducible promoter so as to facilitate optimal expression in the appropriate cells and/or tissues, *e*.*g*., reproductive tissues. Exemplary inducible promoters include promoters activated by physical (*e*.*g*., heat shock promoter) and/or chemical means (*e*.*g*., IPTG or Tetracycline).

Further, expression of the nucleotide sequence can be linked to other genes so as to achieve expression at specific stages of development or within specific tissues. Such expression can be achieved by placing the nucleotide sequence in operable linkage with the promoter of a gene expressed at a specific stage of development. For example, immunoglobulin sequences from one species engineered into the genome of a host species are place in operable linkage with a promoter sequence of a CD19 gene (a B cell specific gene) from the host species. B cell-specific expression at precise developmental stages when immunoglobulins are expressed is achieved.

Yet another method to achieve robust expression of an inserted nucleotide sequence is to employ a constitutive promoter. Exemplary constitutive promoters include SV40, CMV, UBC, EF1A, PGK and CAGG. In a similar fashion, the desired nucleotide sequence is placed in operable linkage with a selected constitutive promoter, which provides high level of expression of the protein(s) encoded by the nucleotide sequence.

The term "ectopic" is intended to include a displacement, or a placement at a position that is not normally encountered in nature (*e*.*g*., placement of a nucleic acid sequence at a position that is not the same position as the nucleic acid sequence is found in a wild-type mouse). The term in various aspects is used in the sense of its object being out of its normal, or proper, position. For example, the phrase "an ectopic nucleotide sequence encoding ..." refers to a nucleotide sequence that appears at a position at which it is not normally encountered in the mouse. For example, in the case of an ectopic nucleotide sequence encoding a mouse ADAM6 protein (or an ortholog or homolog or fragment thereof that provides the same or similar fertility benefit on male mice), the sequence can be placed at a different position in the mouse's genome than is normally found in a wild-type mouse. In such cases, novel sequence junctions of mouse sequence will be created by placing the sequence at a different position in the genome of the mouse than in a willd-type mouse. A functional homolog or ortholog of mouse ADAM6 is a sequence that confers a rescue of fertility loss (*e*.*g*., loss of the ability of a male mouse to generate offspring by mating) that is observed in an ADAM6^{-/-} mouse. Functional homologs or orthologs include proteins that have at least about 89% identity or more, *e*.*g*., up to 99% identity, to the amino acid sequence of ADAM6a and/or to the amino acid sequence of ADAM6b, and that can complement, or rescue ability to successfully mate, of a mouse that has a genotype that includes a deletion or knockout of ADAM6a and/or ADAM6b.

The ectopic position can be anywhere (*e*.*g*., as with random insertion of a transgene containing a mouse ADAM6 sequence), or can be, *e*.*g*., at a position that approximates (but is not precisely the same as) its location in a wild-type mouse (*e*.*g*., in a modified endogenous immunoglobulin locus, but either upstream or downstream of its natural position, *e*.*g*., within a modified immunoglobulin locus but between different gene segments, or at a different position in a mouse V-D intergenic sequence). One example of an ectopic placement is maintaining the position normally found in wild-type mice within the endogenous immunoglobulin heavy chain locus while rendering the surrounding endogenous heavy chain gene segments in capable of rearranging to encode a functional heavy chain containing an endogenous heavy chain constant region. In this example, this may be accomplished by inversion of the chromosomal fragment containing the endogenous immunoglobulin heavy chain variable loci, *e*.*g*. using engineered site-specific recombination sites placed at positions flanking the variable region locus. Thus, upon recombination the endogenous heavy chain variable region loci are placed at a great distance away from the endogenous heavy chain constant region genes thereby preventing rearrangement to encode a functional heavy chain containing an endogenous heavy chain constant region. Other exemplary methods to achieve functional silencing of the endogenous immunoglobulin heavy chain variable gene locus while maintaining a functional ADAM6 locus will be apparent to persons of skill upon reading this disclosure and/or in combination with methods known in the art. With such a placement of the endogenous heavy chain locus, the endogenous ADAM6 genes are maintained and the endogenous immunoglobulin heavy chain locus is functionally silenced.

Another example of an ectopic placement is placement within a modified immunoglobulin heavy chain locus. For example, a mouse comprising a replacement of one or more endogenous V_{H} gene segments with human V_{L} gene segments, wherein the replacement removes an endogenous ADAM6 sequence, can be engineered to have a mouse ADAM6 sequence located within sequence that contains the human V_{L} gene segments. The resulting modification would generate a (ectopic) mouse ADAM6 sequence within a human gene sequence, and the (ectopic) placement of the mouse ADAM6 sequence within the human gene sequence can approximate the position of the human ADAM6 pseudogene (*i*.*e*., between two V segments) or can approximate the position of the mouse ADAM6 sequence (*i*.*e*., within the V-D intergenic region). The resulting sequence junctions created by the joining of a (ectopic) mouse ADAM6 sequence within or adjacent to a human gene sequence (*e*.*g*., an immunoglobulin light chain gene sequence) within the germline of the mouse would be novel as compared to the same or similar position in the genome of a wild-type mouse.

In various aspects, non-human animals are described that lack an ADAM6 or ortholog or homolog thereof, wherein the lack renders the non-human animal infertile, or substantially reduces fertility of the non-human animal. In various aspects, the lack of ADAM6 or ortholog or homolog thereof is due to a modification of an endogenous immunoglobulin heavy chain locus. A substantial reduction in fertility is, *e*.*g*., a reduction in fertility (*e*.*g*., breeding frequency, pups per litter, litters per year, etc.) of about 50%, 60%, 70%, 80%, 90%, or 95% or more. In various aspects, the non-human animals are supplemented with a mouse ADAM6 gene or ortholog or homolog or functional fragment thereof that is functional in a male of the non-human animal, wherein the supplemented ADAM6 gene or ortholog or homolog or functional fragment thereof rescues the reduction in fertility in whole or in substantial part. A rescue of fertility in substantial part is, *e*.*g*., a restoration of fertility such that the non-human animal exhibits a fertility that is at least 70%, 80%, or 90% or more as compared with an unmodified (*i*.*e*., an animal without a modification to the ADAM6 gene or ortholog or homolog thereof) heavy chain locus.

The sequence that confers upon the genetically modified animal (*i*.*e*., the animal that lacks a functional ADAM6 or ortholog or homolog thereof, due to, *e*.*g*., a modification of a immunoglobulin heavy chain locus) is, in various aspects, selected from an ADAM6 gene or ortholog or homolog thereof. For example, in a mouse, the loss of ADAM6 function is rescued by adding, in one aspect, a mouse ADAM6 gene. In one aspect, the loss of ADAM6 function in the mouse is rescued by adding an ortholog or homolog of a closely related specie with respect to the mouse, *e*.*g*., a rodent, *e*.*g*., a mouse of a different strain or species, a rat of any species, a rodent; wherein the addition of the ortholog or homolog to the mouse rescues the loss of fertility due to loss of ADAM6 function or loss of an ADAM6 gene. Orthologs and homologs from other species, in various aspects, are selected from a phylogenetically related species and, in various aspects, exhibit a percent identity with the endogenous ADAM6 (or ortholog) that is about 80% or more, 85% or more, 90% or more, 95% or more, 96% or more, or 97% or more; and that rescue ADAM6-related or (in a non-mouse) ADAM6 ortholog-related loss of fertility. For example, in a genetically modified male rat that lacks ADAM6 function (*e*.*g*., a rat with an endogenous immunoglobulin heavy chain variable region replaced with a human immunoglobulin heavy chain variable region, or a knockout in the rat immunoglobulin heavy chain region), loss of fertility in the rat is rescued by addition of a rat ADAM6 or, in some aspects, an ortholog of a rat ADAM6 (*e*.*g*., an ADAM6 ortholog from another rat strain or species, or, in one aspect, from a mouse).

Thus, in various aspects, genetically modified animals that exhibit no fertility or a reduction in fertility due to modification of a nucleic acid sequence encoding an ADAM6 protein (or ortholog or homolog thereof) or a regulatory region operably linked with the nucleic acid sequence, comprise a nucleic acid sequence that complements, or restores, the loss in fertility where the nucleic acid sequence that complements or restores the loss in fertility is from a different strain of the same species or from a phylogenetically related species. In various aspects, the complementing nucleic acid sequence is an ADAM6 ortholog or homolog or functional fragment thereof. In various aspects, the complementing ADAM6 ortholog or homolog or functional fragment thereof is from a non-human animal that is closely related to the genetically modified animal having the fertility defect. For example, where the genetically modified animal is a mouse of a particular strain, an ADAM6 ortholog or homolog or functional fragment thereof can be obtained from a mouse of another strain, or a mouse of a related species. In one aspect, where the genetically modified animal comprising the fertility defect is of the order Rodentia, the ADAM6 ortholog or homolog or functional fragment thereof is from another animal of the order Rodentia. In one aspect, the genetically modified animal comprising the fertility defect is of a suborder Myomoropha (e.g., jerboas, jumping mice, mouse-like hamsters, hamsters, New World rats and mice, voles, true mice and rats, gerbils, spiny mice, crested rats, climbing mice, rock mice, white-tailed rats, malagasy rats and mice, spiny dormice, mole rats, bamboo rats, zokors), and the ADAM6 ortholog or homolog or functional fragment thereof is selected from an animal of order Rodentia, or of the suborder Myomorpha.

In one aspect, the genetically modified animal is from the superfamily Dipodoidea, and the ADAM6 ortholog or homolog or functional fragment thereof is from the superfamily Muroidea. In one aspect, the genetically modified animal is from the superfamily Muroidea, and the ADAM6 ortholog or homolog or functional fragment thereof is from the superfamily Dipodoidea.

In one aspect, the genetically modified animal is a rodent. In one aspect, the rodent is selected from the superfamily Muroidea, and the ADAM6 ortholog or homolog is from a different species within the superfamily Muroidea. In one aspect, the genetically modified animal is from a family selected from Calomyscidae (*e*.*g*., mouse-like hamsters), Cricetidae (*e*.*g*., hamster, New World rats and mice, voles), Muridae (true mice and rats, gerbils, spiny mice, crested rats), Nesomyidae (climbing mice, rock mice, with-tailed rats, Malagasy rats and mice), Platacanthomyidae (*e*.*g*., spiny dormice), and Spalacidae (*e*.*g*., mole rates, bamboo rats, and zokors); and the ADAM6 ortholog or homolog is selected from a different species of the same family. In a specific aspect, the genetically modified rodent is selected from a true mouse or rat (family Muridae), and the ADAM6 ortholog or homolog is from a species selected from a gerbil, spiny mouse, or crested rat. In one aspect, the genetically modified mouse is from a member of the family Muridae, and the ADAM6 ortholog or homolog is from a different species of the family Muridae. In a specific aspect, the genetically modified rodent is a mouse of the family Muridae, and the ADAM6 ortholog or homolog is from a rat, gerbil, spiny mouse, or crested rat of the family Muridae.

In various aspects, one or more rodent ADAM6 orthologs or homologs or functional fragments thereof of a rodent in a family restores fertility to a genetically modified rodent of the same family that lacks an ADAM6 ortholog or homolog (*e*.*g*., Cricetidae (*e*.*g*., hamsters, New World rats and mice, voles); Muridae (*e*.*g*., true mice and rats, gerbils, spiny mice, crested rats)).

In various aspects, ADAM6 orthologs, homologs, and fragments thereof are assessed for functionality by ascertaining whether the ortholog, homolog, or fragment restores fertility to a genetically modified male non-human animal that lacks ADAM6 activity (*e*.*g*., a rodent, *e*.*g*., a mouse or rat, that comprises a knockout of ADAM6 or its ortholog). In various aspects, functionality is defined as the ability of a sperm of a genetically modified animal lacking an endogenous ADAM6 or ortholog or homolog thereof to migrate an oviduct and fertilize an ovum of the same specie of genetically modified animal.

In various aspects, mice that comprise deletions or replacements of the endogenous heavy chain variable region locus or portions thereof can be made that contain an ectopic nucleotide sequence that encodes a protein that confers similar fertility benefits to mouse ADAM6 (*e*.*g*., an ortholog or a homolog or a fragment thereof that is functional in a male mouse). The ectopic nucleotide sequence can include a nucleotide sequence that encodes a protein that is an ADAM6 homolog or ortholog (or fragment thereof) of a different mouse strain or a different species, *e*.*g*., a different rodent species, and that confers a benefit in fertility, *e*.*g*., increased number of litters over a specified time period, and/or increased number of pups per litter, and/or the ability of a sperm cell of a male mouse to traverse through a mouse oviduct to fertilize a mouse egg.

In one aspect, the ADAM6 is a homolog or ortholog that is at least 89% to 99% identical to a mouse ADAM6 protein (e.g., at least 89% to 99% identical to mouse ADAM6a or mouse ADAM6b). In one aspect, the ectopic nucleotide sequence encodes one or more proteins independently selected from a protein at least 89% identical to mouse ADAM6a, a protein at least 89% identical to mouse ADAM6b, and a combination thereof. In one aspect, the homolog or ortholog is a rat, hamster, mouse, or guinea pig protein that is or is modified to be about 89% or more identical to mouse ADAM6a and/or mouse ADAM6b. In one aspect, the homolog or ortholog is or is at least 90%, 91%, 92%, 93%, 94%, 95%, 96%, 97%, 98%, or 99% identical to a mouse ADAM6a and/or mouse ADAM6b. In a specific aspect, the mouse ADAM6a comprises SEQ ID NO: 1 or a functional fragment thereof, and the mouse ADAM6b comprises SEQ ID NO: 2 or a functional fragment thereof.

In one aspect, non-human animals are described, wherein the non-human animals comprise (a) an insertion of one or more human V_{L} and J_{L} gene segments upstream of a non-human immunoglobulin heavy chain constant region, (b) an insertion of one or more human V_{L}, and J_{L} gene segments upstream of a non-human immunoglobulin light chain constant region, and (c) a nucleotide sequence that encodes an ADAM6 protein or a functional fragment thereof. In one aspect, the non-human heavy and/or light chain constant regions are rodent constant regions (*e*.*g*., selected from mouse, rat or hamster constant regions). In one aspect, the non-human light chain constant region is a rodent constant region. In a specific aspect, the light chain constant region is a mouse Cκ or a rat Cκ region. In a specific aspect, the light chain constant region is a mouse Cλ or a rat Cκ region. In one aspect, the human V_{L} and J_{L} gene segments are Vκ and Jκ gene segments. In one aspect, the human V_{L} and J_{L} gene segments are Vλ and Jλ gene segments. In one aspect, the non-human animal further comprises one or more human D_{H} gene segments present between the human V_{L} and J_{L} gene segments. Suitable non-human animals include rodents, *e*.*g*, mice, rats and hamsters. In one aspect, the rodent is a mouse or a rat.

In one aspect, the non-human animal comprises at least six to at least 40 human Vκ gene segments and at least one to at least five human Jκ gene segments. In a specific aspect, the non-human animal comprises six human Vκ gene segments and five human Jκ gene segments. In a specific aspect, the non-human animal comprises 16 human Vκ gene segments and five human Jκ gene segments. In a specific aspect, the non-human animal comprises 30 human Vκ gene segments and five human Jκ gene segments. In a specific aspect, the non-human animal comprises 40 human Vκ gene segments and five human Jκ gene segments. In various aspects, the human Jκ gene segments are selected from Jκ1, Jκ2, Jκ3, Jκ4, Jκ5, and a combination thereof.

In one aspect, the nucleotide sequence that encodes an ADAM6 protein or functional fragment thereof is ectopic in the non-human animal. In one aspect, the nucleotide sequence that encodes an ADAM6 protein or functional fragment thereof (that is functional in the non-human animal) is present the same location as compared to a wild-type type non-human ADAM6 locus. In one aspect, the non-human animal is a mouse and the nucleotide sequence encodes a mouse ADAM6 protein or functional fragment thereof and is present at an ectopic location in the genome of the non-human animal. In one aspect the non-human animal is a mouse and the nucleotide sequence encodes a mouse ADAM6 protein or functional fragment thereof and is present within immunoglobulin gene segments. In a specific aspect, the immunoglobulin gene segments are heavy chain gene segments of the non-human animal. In a specific aspect, the immunoglobulin gene segments are light chain gene segments of another species. In one aspect, the light chain gene segments are human κ light chain gene segments. In one aspect, the mouse comprises an ectopic contiguous sequence comprising one or more endogenous unrearranged heavy chain gene segments, and the ADAM6 sequence is within the ectopic contiguous sequence.

In one aspect, the non-human animal lacks an endogenous immunoglobulin V_{L} and/or a J_{L} gene segment at an endogenous immunoglobulin light chain locus. In one aspect, the non-human animal comprises endogenous immunoglobulin V_{L} and/or J_{L} gene segments that are incapable of rearranging to form an immunoglobulin V_{L} domain in the non-human animal. In one aspect, all or substantially all endogenous immunoglobulin Vκ and Jκ gene segments are replaced with one or more human Vκ and Jκ gene segments. In one aspect, all or substantially all endogenous immunoglobulin Vλ and Jλ gene segments are deleted in whole or in part. In one aspect, all or substantially all endogenous immunoglobulin V_{L} and J_{L} gene segments are intact in the non-human animal and the non-human animal comprises one or more human Vκ gene segments and one or more human Jκ gene segments inserted between endogenous immunoglobulin V_{L} and/or J_{L} gene segments and an endogenous immunoglobulin light chain constant region. In a specific aspect, the intact endogenous immunoglobulin V_{L} and J_{L} gene segments are rendered incapable of rearranging to form a V_{L} domain of an antibody in the non-human animal. In one aspect, the endogenous immunoglobulin light chain locus of the non-human animal is an immunoglobulin κ light chain locus. In one aspect, the endogenous immunoglobulin V_{L} and J_{L} gene segments are Vκ and Jκ gene segments.

In one aspect, cells and/or tissues derived from non-human animals as described herein are described, wherein the cells and/or tissues comprise (a) an insertion of one or more human Vκ and Jκ gene segments upstream of an non-human immunoglobulin light chain constant region, (b) an insertion of one or more human Vκ and Jκ gene segments upstream of an non-human immunoglobulin heavy chain constant region, and (c) a nucleotide sequence that encodes an ADAM6 protein or a functional fragment thereof. In one aspect, the non-human heavy and/or light chain constant regions are mouse constant regions. In one aspect, the non-human heavy and/or light chain constant regions are rat constant regions. In one aspect, the non-human heavy and/or light chain constant regions are hamster constant regions.

In one aspect, the nucleotide sequence that encodes an ADAM6 protein or functional fragment thereof is ectopic in the cell and/or tissue. In one aspect, the nucleotide sequence that encodes an ADAM6 protein or functional fragment thereof is present the same location as compared to a wild-type type non-human ADAM6 locus. In one aspect the non-human cell and/or tissue is derived from a mouse and the nucleotide sequence encodes a mouse ADAM6 protein or functional fragment thereof and is present at an ectopic location. In one aspect, the non-human cell and/or tissue is derived from a mouse and the nucleotide sequence encodes a mouse ADAM6 protein or functional fragment thereof and is present within immunoglobulin gene segments. In a specific aspect, the immunoglobulin gene segments are heavy chain gene segments. In a specific aspect, the immunoglobulin gene segments are light chain gene segments. In one aspect, a contiguous sequence of endogenous heavy chain gene segments are placed ectopically in the non-human animal, wherein the contiguous sequence of ectopically placed endogenous heavy chain gene segments comprises an ADAM6 gene that is functional in the mouse (e.g., in a male mouse).

In one aspect, use of a non-human animal as described herein to make an antigen-binding protein is described, wherein the non-human animal expresses (a) an antibody that comprises (i) an immunoglobulin light chain that comprises a human Vκ domain and a non-human light chain constant region and (ii) an immunoglobulin heavy chain that comprises a human Vκ domain and a non-human constant region; and (b) an ADAM6 protein or functional fragment thereof. In one aspect, the antigen binding protein is human. In one aspect, the non-human animal is a rodent and the non-human constant regions are rodent constant regions. In a specific aspect, the rodent is a mouse.

In one aspect, a non-human cell or tissue derived from a non-human animal as described herein is described. In one aspect, the non-human cell or tissue comprises one or more human immunoglobulin Vκ gene segments and at least one human immunoglobulin Jκ gene segments contiguous with a non-human immunoglobulin light chain constant region gene and one or more human Vκ and one or more human Jκ gene segments contiguous with a non-human immunoglobulin heavy chain constant region gene, wherein the cell or tissue expresses an ADAM6 protein or functional fragment thereof. In one aspect, the non-human light chain constant region gene is a mouse Cκ.

In one aspect, the nucleotide sequence that encodes the ADAM6 protein or functional fragment thereof is ectopic. In one aspect, the nucleotide sequence that encodes the ADAM6 protein or functional fragment thereof is located at a position that is the same as a wild-type non-human cell. In various aspects, the non-human cell is a mouse B cell. In various aspects, the non-human cell is an embryonic stem cell.

In one aspect, the tissue is derived from spleen, bone marrow or lymph node of the non-human animal.

In one aspect, use of a cell or tissue derived from a non-human animal as described herein to make a hybridoma or quadroma is described.

In one aspect, a non-human cell comprising a modified genome as described herein is described, wherein the non-human cell is an oocyte, a host embryo, or a fusion of a cell from a non-human animal as described herein and a cell from a different non-human animal.

In one aspect, use of a cell or tissue derived from a non-human animal as described herein to make a human antigen-binding protein is described. In one aspect, the human antigen-binding protein comprises a human Vκ domain isolated from a non-human animal as described herein.

In one aspect, a method for making an antigen-binding protein that binds to an antigen of interest is described, wherein the method comprises (a) exposing a non-human animal as described herein to an antigen of interest, (b) isolating one or more B lymphocytes of the non-human animal, wherein the one or more B lymphocytes express a V_{L} binding protein that binds the antigen of interest, and (c) identifying a nucleic acid sequence that encodes a V_{L} domain of the V_{L} binding protein that binds the antigen of interest, wherein the V_{L} binding protein comprises a human Vκ domain and a non-human light chain constant domain and a human Vκ domain and a non-human heavy chain constant domain, and (d) employing the nucleic acid sequence of (c) with a human immunoglobulin constant region nucleic acid sequence to make a human antigen-binding protein that binds the antigen of interest.

In one aspect, the non-human light chain constant domain of the V_{L} binding protein is a mouse Cκ. In one aspect, the non-human heavy chain chain constant domain of the V_{L} binding protein is a mouse Cγ. In one aspect, the non-human animal is a mouse.

In one aspect, a fertile male mouse comprising a modification at an immunoglobulin heavy chain locus is described, wherein the fertile male mouse comprises an ectopic ADAM6 sequence that is functional in the male mouse.

### Ectopic ADAM6 in Modified Immunoglobulin Heavy Chain Loci

Developments in gene targeting, *e*.*g*., the development of bacterial artificial chromosomes (BACs), now enable the recombination of relatively large genomic fragments. BAC engineering has allowed for the ability to make large deletions, and large insertions, into mouse ES cells.

Mice that make human antibodies (*i*.*e*., human variable regions) have been available for some time now. Although they represent an important advance in the development of human therapeutic antibodies, these mice display a number of significant abnormalities that limit their usefulness. For example, they display compromised B cell development. The compromised development may be due to a variety of differences between the transgenic mice and wild-type mice.

Human antibodies might not optimally interact with mouse pre B cell or B cell receptors on the surface of mouse cells that signal for maturation, proliferation, or survival during clonal selection. Fully human antibodies might not optimally interact with a mouse Fc receptor system; mice express Fc receptors that do not display a one-to-one correspondence with human Fc receptors. Finally, various mice that make fully human antibodies do not include all genuine mouse sequences, *e*.*g*., downstream enhancer elements and other locus control elements, which may be required for wild-type B cell development.

Mice that make fully human antibodies generally comprise endogenous immunoglobulin loci that are disabled in some way, and human transgenes that comprise variable and constant immunoglobulin gene segments are introduced into a random location in the mouse genome. As long as the endogenous locus is sufficiently disabled so as not to rearrange gene segments to form a functional immunoglobulin gene, the goal of making fully human antibodies in such a mouse can be achieved-albeit with compromised B cell development.

Although compelled to make fully human antibodies from the human transgene locus, generating human antibodies in a mouse is apparently an unfavored process. In some mice, the process is so unfavored as to result in formation of chimeric human variable/mouse constant heavy chains (but not light chains) through the mechanism of *trans-*switching. By this mechanism, transcripts that encode fully human antibodies undergo isotype switching in *trans* from the human isotype to a mouse isotype. The process is in *trans*, because the fully human transgene is located apart from the endogenous locus that retains an undamaged copy of a mouse heavy chain constant region gene. Although in such mice *trans*-switching is readily apparent the phenomenon is still insufficient to rescue B cell development, which remains frankly impaired. In any event, *trans*-switched antibodies made in such mice retain fully human light chains, since the phenomenon of *trans*-switching apparently does not occur with respect to light chains; *trans*-switching presumably relies on switch sequences in endogenous loci used (albeit differently) in normal isotype switching in *cis.* Thus, even when mice engineered to make fully human antibodies select a *trans-*switching mechanism to make antibodies with mouse constant regions, the strategy is still insufficient to rescue normal B cell development.

A primary concern in making antibody-based human therapeutics is making a sufficiently large diversity of human immunoglobulin variable region sequences to identify useful variable domains that specifically recognize particular epitopes and bind them with a desirable affinity, usually-but not always-with high affinity. Prior to the development of VELOCIMMUNE® mice (described herein), there was no indication that mice expressing human variable regions with mouse constant regions would exhibit any significant differences from mice that made human antibodies from a transgene. That supposition, however, was incorrect.

VELOCIMMUNE® mice, which contain a precise replacement of mouse immunoglobulin variable regions with human immunoglobulin variable regions at the endogenous loci, display a surprising and remarkable similarity to wild-type mice with respect to B cell development. In a surprising and stunning development, VELOCIMMUNE® mice displayed an essentially normal, wild-type response to immunization that differed only in one significant respect from wild-type mice-the variable regions generated in response to immunization are fully human.

VELOCIMMUNE® mice contain a precise, large-scale replacement of germline variable regions of mouse immunoglobulin heavy chain (IgH) and immunoglobulin light chain (e.g., κ light chain, Igκ) with corresponding human immunoglobulin variable regions, at the endogenous loci. In total, about six megabases of mouse loci are replaced with about 1.5 megabases of human genomic sequence. This precise replacement results in a mouse with hybrid immunoglobulin loci that make heavy and light chains that have a human variable regions and a mouse constant region. The precise replacement of mouse V_{H}-D_{H}-J_{H} and Vκ-Jκ segments leave flanking mouse sequences intact and functional at the hybrid immunoglobulin loci. The humoral immune system of the mouse functions like that of a wild-type mouse. B cell development is unhindered in any significant respect and a rich diversity of human variable regions is generated in the mouse upon antigen challenge.

VELOCIMMUNE® mice are possible because immunoglobulin gene segments for heavy and κ light chains rearrange similarly in humans and mice, which is not to say that their loci are the same or even nearly so-clearly they are not. However, the loci are similar enough that humanization of the heavy chain variable gene locus can be accomplished by replacing about three million base pairs of contiguous mouse sequence that contains all the V_{H}, D_{H}, and J_{H} gene segments with about one million bases of contiguous human genomic sequence covering basically the equivalent sequence from a human immunoglobulin locus.

In some aspects, further replacement of certain mouse constant region gene sequences with human gene sequences (*e*.*g*., replacement of mouse C_{H}1 sequence with human C_{H}1 sequence, and replacement of mouse C_{L} sequence with human C_{L} sequence) results in mice with hybrid immunoglobulin loci that make antibodies that have human variable regions and partly human constant regions, suitable for, *e*.*g*., making fully human antibody fragments, *e*.*g*., fully human Fab's. Mice with hybrid immunoglobulin loci exhibit normal variable gene segment rearrangement, normal somatic hypermutation, and normal class switching. These mice exhibit a humoral immune system that is indistinguishable from wild type mice, and display normal cell populations at all stages of B cell development and normal lymphoid organ structures-even where the mice lack a full repertoire of human variable region gene segments. Immunizing these mice results in robust humoral responses that display a wide diversity of variable gene segment usage.

The precise replacement of mouse germline variable region gene segments allows for making mice that have partly human immunoglobulin loci. Because the partly human immunoglobulin loci rearrange, hypermutate, and class switch normally, the partly human immunoglobulin loci generate antibodies in a mouse that comprise human variable regions. Nucleotide sequences that encode the variable regions can be identified and cloned, then fused (*e*.*g*., in an *in vitro* system) with any sequences of choice, *e*.*g*., any immunoglobulin isotype suitable for a particular use, resulting in an antibody or antigen-binding protein derived wholly from human sequences.

Large-scale humanization by recombineering methods were used to modify mouse embryonic stem (ES) cells to create a unique immunoglobulin heavy chain locus by precisely replacing up to three megabases of the mouse heavy chain immunoglobulin locus including essentially all of the mouse V_{H}, D_{H}, and J_{H} gene segments with up to a one-half megabase segment of the human genome comprising one of two repeats encoding essentially all human Vκ and Jκ gene segments. Additionally, up to a one-half megabase segment of the human genome comprising one of two repeats encoding essentially all human Vκ and Jκ gene segments was used to replace a three megabase segment of the mouse immunoglobulin κ light chain locus containing essentially all of the mouse Vκ and Jκ gene segments. Mice with such replaced immunoglobulin loci can comprise a disruption or deletion of the mouse ADAM6 locus, which is normally found between the 3'-most V_{H} gene segment and the 5'-most D_{H} gene segment at the mouse immunoglobulin heavy chain locus. Disruption in this region can lead to reduction or elimination of functionality of the mouse ADAM6 locus.

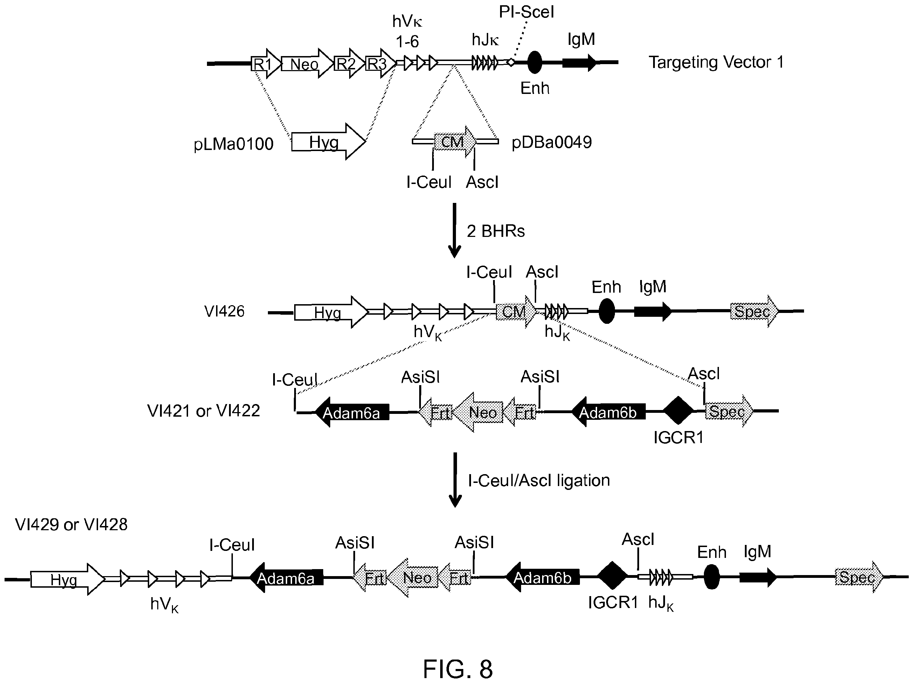

Mice are described that comprise the replaced loci as described above, and that also comprise an ectopic nucleic acid sequence encoding a mouse ADAM6, where the mice exhibit essentially normal fertility. In one aspect, the ectopic nucleic acid sequence is placed between a human V_{L} gene segment and a human J_{L} gene segment or upstream of a 5'-most human V_{L} gene segment at the modified endogenous heavy chain locus. The direction of transcription of the ADAM6 genes may be opposite (FIG. 7) or the same (FIG. 8) with respect to the direction of transcription of the surrounding human V_{L} gene segments. Although examples herein show rescue of fertility by placing the ectopic sequence between the indicated human V_{L} and J_{L} gene segments or upstream of a 5'-most human V_{L} gene segment, skilled persons will recognize that placement of the ectopic sequence at any suitable transcriptionally-permissive locus in the mouse genome (or even extra-chromosomally) will be expected to similarly rescue fertility in a male mouse. In various aspects, the ectopic nucleic acid sequence is selected from SEQ ID NO: 3, SEQ ID NO: 4 and SEQ ID NO: 5, wherein the ectopic sequence encodes one or more ADAM6 proteins, wherein the one or more ADAM6 proteins comprise SEQ ID NO: 1, SEQ ID NO: 2 or a combination thereof.

The phenomenon of complementing a mouse that lacks a functional ADAM6 locus with an ectopic sequence that comprises a mouse ADAM6 gene or ortholog or homolog or functional fragment thereof is a general method that is applicable to rescuing any mice with nonfunctional or minimally functional endogenous ADAM6 loci. Thus, a great many mice that comprise an ADAM6-disrupting modification of the immunoglobulin heavy chain locus can be rescued with the compositions and methods described herein. Accordingly, the present disclosure comprises mice with a wide variety of modifications of immunoglobulin heavy chain loci that compromise endogenous ADAM6 function. Some (non-limiting) examples are provided in this description. In addition to the mice described, the compositions and methods related to ADAM6 can be used in a great many applications, e.g., when modifying a heavy chain locus in a wide variety of ways.

In one aspect, a mouse is described that comprises an ectopic ADAM6 sequence that encodes a functional ADAM6 protein (or ortholog or homolog or functional fragment thereof), a replacement of all or substantially all mouse V_{H} gene segments with one or more human V_{L} gene segments, a replacement of all or substantially all mouse D_{H} gene segments and J_{H} gene segments with human J_{L} gene segments; wherein the mouse lacks a C_{H}1 and/or hinge region. In one aspect, the mouse makes a single variable domain binding protein that is a dimer of immunoglobulin chains selected from: (a) human V_{L} - mouse C_{H}1 - mouse C_{H}2 - mouse C_{H}3; (b) human V_{L} -mouse hinge - mouse C_{H}2 - mouse C_{H}3; and, (c) human V_{L} - mouse C_{H}2 - mouse C_{H}3.

In one aspect, the nucleotide sequence that rescues fertility is placed within a human immunoglobulin light chain variable region sequence (*e*.*g*., between human Vκ4-1 and Jκ1 gene segments) in a mouse that has a replacement of all or substantially all mouse immunoglobulin heavy chain variable gene segments (mV_{H}'s, mD_{H}'s, and mJ_{H}'s) with one or more human immunoglobulin κ light chain variable gene segments (hVκ's and hJκ's), and the mouse further comprises a replacement of all or substantially all mouse immunoglobulin κ light chain variable gene segments (mVκ's, mJκ's) with one or more human immunoglobulin κ light chain variable gene segments (hVκ's and hJκ's).

In one aspect, a functional mouse ADAM6 locus (or ortholog or homolog or functional fragment thereof) can be placed in the midst of human V_{L} gene segments or upstream of a 5'-most human V_{L} gene segment, wherein the human V_{L} gene segments replace endogenous V_{H} gene segments. In one aspect, all or substantially all mouse V_{H} gene segments are removed and replaced with one or more human V_{L} gene segments, and the mouse ADAM6 locus is placed immediately adjacent to the 5' end of the 5'-most human V_{L} gene segments, or between two human V_{L} gene segments. In a specific aspect, the mouse ADAM6 locus is placed between two V_{L} gene segments near the 3' terminus of the inserted human V_{L} gene segments. In a specific aspect, the arrangement of human V_{L} gene segments is then the following (from upstream to downstream with respect to direction of transcription of the human V_{L} gene segments): human Vκ5-2-mouse ADAM6 locus-human Vκ4-1. In a specific aspect, the arrangement of human V_{L} gene segments is then the following (from upstream to downstream with respect to direction of transcription of the human V_{L} gene segments): mouse ADAM6 locus-human Vκ2-40, wherein human Vκ2-40 is the 5'-most human V_{L} gene segment at the modified immunoglobulin heavy chain locus. In one aspect, the orientation of one or more of mouse ADAM6a and mouse ADAM6b of the mouse ADAM6 locus is opposite with respect to direction of transcription as compared with the orientation of the human V_{L} gene segments. In one aspect, the orientation of one or more of mouse ADAM6a and mouse ADAM6b of the mouse ADAM6 locus is the same with respect to direction of transcription as compared with the orientation of the human V_{L} gene segments.

In one aspect, a functional mouse ADAM6 locus (or ortholog or homolog or functional fragment thereof) can be placed between a human V_{L} gene segment and a human J_{L} gene segment (*i*.*e*., in the intergenic region between the 3'-most human V_{L} gene segment and the 5'-most J_{L} gene segment), wherein the human V_{L} and J_{L} gene segments replace endogenous V_{H} gene segments. In one aspect, all or substantially all mouse V_{H} gene segments are removed and replaced with one or more human V_{L} gene segments and one or more human J_{L} gene segments, and the mouse ADAM6 locus is placed immediately adjacent to the 3' end of the 3'-most human V_{L} gene segment and immediately adjacent to the 5' end of the 5'-most human J_{L} gene segment. In a specific aspect, the one or more human V_{L} gene segments and one or more human J_{L} gene segments are VK and JK gene segments. In a specific aspect, the arrangement of human V_{L} gene segments is then the following (from upstream to downstream with respect to direction of transcription of the human V_{L} gene segments): human Vκ4-1-mouse ADAM6 locus-human Jκ1. In one aspect, the orientation of one or more of mouse ADAM6a and mouse ADAM6b of the mouse ADAM6 locus is opposite with respect to direction of transcription as compared with the orientation of the human V_{L} gene segments. In one aspect, the orientation of one or more of mouse ADAM6a and mouse ADAM6b of the mouse ADAM6 locus is the same with respect to direction of transcription as compared with the orientation of the human V_{L} gene segments.

A mouse modified with one or more human V_{L} gene segments (*e*.*g*., Vκ or Vλ segments) replacing all or substantially all endogenous V_{H} gene segments can be modified so as to either maintain the endogenous ADAM6 locus, as described above, e.g., by employing a targeting vector having a downstream homology arm that includes a mouse ADAM6 locus or functional fragment thereof, or to replace a damaged mouse ADAM6 locus with an ectopic sequence positioned between two human V_{L} gene segments or between the human V_{L} gene segments and a D_{H} gene segment (whether human or mouse, *e*.*g*., Vλ + m/hD_{H}), or a J gene segment (whether human or mouse, *e*.*g*., Vκ + J_{H}). In one aspect, the replacement includes two or more human V_{L} gene segments, and the mouse ADAM6 locus or functional fragment thereof is placed between the two 3'-most V_{L} gene segments. In a specific aspect, the arrangement of human V_{L} gene segments is then the following (from upstream to downstream with respect to direction of transcription of the human gene segments): human V_{L}3'-1 - mouse ADAM6 locus - human V_{L}3'. In one aspect, the orientation of one or more of mouse ADAM6a and mouse ADAM6b of the mouse ADAM6 locus is opposite with respect to direction of transcription as compared with the orientation of the human V_{L} gene segments. Alternatively, the mouse ADAM6 locus can be placed in the intergenic region between the 3'-most human V_{L} gene segment and the 5'-most J_{L} gene segment.

In one aspect, a mouse is described with a replacement of one or more endogenous V_{H} gene segments, and that comprises at least one endogenous D_{H} gene segment. In such a mouse, the modification of the endogenous V_{H} gene segments can comprise a modification of one or more of the 3'-most V_{H} gene segments, but not the 5'-most D_{H} gene segment, where care is taken so that the modification of the one or more 3'-most V_{H} gene segments does not disrupt or render the endogenous ADAM6 locus nonfunctional. For example, in one aspect the mouse comprises a replacement of all or substantially all endogenous V_{H} gene segments with one or more human V_{L} gene segments, and the mouse comprises one or more endogenous D_{H} gene segments and a functional endogenous ADAM6 locus.

In another aspect, the mouse comprises the modification of endogenous 3'-most V_{H} gene segments, and a modification of one or more endogenous D_{H} gene segments, and the modification is carried out so as to maintain the integrity of the endogenous ADAM6 locus to the extent that the endogenous ADAM6 locus remains functional. In one example, such a modification is done in two steps: (1) replacing the 3'-most endogenous V_{H} gene segments with one or more human V_{L} gene segments employing a targeting vector with an upstream homology arm and a downstream homology arm wherein the downstream homology arm includes all or a portion of a functional mouse ADAM6 locus; (2) then replacing and endogenous D_{H} gene segment with a targeting vector having an upstream homology arm that includes a all or a functional portion of a mouse ADAM6 locus.

In various aspects, employing mice that contain an ectopic sequence that encodes a mouse ADAM6 protein or an ortholog or homolog or functional homolog thereof are useful where modifications disrupt the function of endogenous mouse ADAM6. The probability of disrupting endogenous mouse ADAM6 function is high when making modifications to mouse immunoglobulin loci, in particular when modifying mouse immunoglobulin heavy chain variable regions and surrounding sequences. Therefore, such mice provide particular benefit when making mice with immunoglobulin heavy chain loci that are deleted in whole or in part, are humanized in whole or in part, or are replaced (*e*.*g*., with Vκ or Vλ sequences) in whole or in part. Methods for making the genetic modifications described for the mice described below are known to those skilled in the art.

Mice containing an ectopic sequence encoding a mouse ADAM6 protein, or a substantially identical or similar protein that confers the fertility benefits of a mouse ADAM6 protein, are particularly useful in conjunction with modifications to a mouse immunoglobulin heavy chain variable gene locus that disrupt or delete the endogenous ADAM6 sequence. Although primarily described in connection with mice that express antibodies with human variable regions and mouse constant regions, such mice are useful in connection with any genetic modifications that disrupt endogenous ADAM6 genes. Persons of skill will recognize that this encompasses a wide variety of genetically modified mice that contain modifications of mouse immunoglobulin heavy chain variable gene loci. These include, for example, mice with a deletion or a replacement of all or a portion of mouse immunoglobulin heavy chain gene segments, regardless of other modifications. Non-limiting examples are described below.

In some aspects, genetically modified mice are described that comprise an ectopic mouse, rodent, or other ADAM6 gene (or ortholog or homolog or fragment) functional in a mouse, and one or more human immunoglobulin variable and/or constant region gene segments. In various aspects, other ADAM6 gene orthologs or homologs or fragments functional in a mouse may include sequences from bovine, canine, primate, rabbit or other non-human sequences.

In one aspect, a mouse is described that comprises an ectopic ADAM6 sequence that encodes a functional ADAM6 protein, a replacement of all or substantially all mouse V_{H} gene segments with one or more human V_{L} gene segments; a replacement of all or substantially all mouse D_{H} and J_{H} gene segments with one or more human J_{L} gene segments.

In one aspect, the mouse further comprises a replacement of a mouse C_{H}1 nucleotide sequence with a human C_{H}1 nucleotide sequence. In one aspect, the mouse further comprises a replacement of a mouse hinge nucleotide sequence with a human hinge nucleotide sequence. In one aspect, the mouse further comprises a replacement of an immunoglobulin light chain variable locus (V_{L} and J_{L}) with a human immunoglobulin light chain variable locus. In one aspect, the mouse further comprises a replacement of a mouse immunoglobulin light chain constant region nucleotide sequence with a human immunoglobulin light chain constant region nucleotide sequence. In a specific aspect, the V_{L}, J_{L}, and C_{L} are immunoglobulin κ light chain sequences. In a specific aspect, the mouse comprises a mouse C_{H}2 and a mouse C_{H}3 immunoglobulin constant region sequence fused with a human hinge and a human C_{H}1 sequence, such that the mouse immunoglobulin loci rearrange to form a gene that encodes a binding protein comprising (a) a heavy chain that has a human variable region, a human C_{H}1 region, a human hinge region, and a mouse C_{H}2 and a mouse C_{H}3 region; and (b) a gene that encodes an immunoglobulin light chain that comprises a human variable domain and a human constant region.

In one aspect, a mouse is described that comprises an ectopic ADAM6 sequence that encodes a functional ADAM6 protein, a replacement of all or substantially all mouse V_{H} gene segments with one or more human V_{L} gene segments, and optionally a replacement of all or substantially all D_{H} gene segments and/or J_{H} gene segments with one or more human D_{H} gene segments and/or human J_{H} gene segments, or optionally a replacement of all or substantially all D_{H} gene segments and J_{H} gene segments with one or more human J_{L} gene segments.

In one aspect, the mouse comprises a replacement of all or substantially all mouse V_{H}, D_{H}, and J_{H} gene segments with one or more V_{L}, one or more D_{H}, and one or more J gene segments (*e*.*g*., Jκ or Jλ), wherein the gene segments are operably linked to an mouse hinge region, wherein the mouse forms a rearranged immunoglobulin chain gene that contains, from 5' to 3' in the direction of transcription, human V_{L} - human or mouse D_{H} - human or mouse J - mouse hinge - mouse C_{H}2 - mouse C_{H}3. In one aspect, the J region is a human Jκ region. In one aspect, the J region is a human J_{H} region. In one aspect, the J region is a human Jλ region. In one aspect, the human V_{L} region is selected from a human Vλ region and a human Vκ region.

In specific aspects, the mouse expresses a single variable domain antibody having a mouse or human constant region and a variable region derived from a human Vκ, a human D_{H} and a human Jκ; a human Vκ, a human D_{H}, and a human J_{H}; a human Vλ, a human D_{H}, and a human Jλ; a human Vλ, a human D_{H}, and a human J_{H}; a human Vκ, a human D_{H}, and a human Jλ; a human Vλ, a human D_{H}, and a human Jκ. In specific aspect, recombination recognition sequences are modified so as to allow for productive rearrangements to occur between recited V, D, and J gene segments or between recited V and J gene segments.

In one aspect, a mouse is described that comprises an ectopic ADAM6 sequence that encodes a functional ADAM6 protein (or ortholog or homolog or functional fragment thereof), a replacement of all or substantially all mouse V_{H} gene segments with one or more human V_{L} gene segments, a replacement of all or substantially all mouse D_{H} gene segment and J_{H} gene segments with human J_{L} gene segments; wherein the mouse lacks a C_{H}1 and/or hinge region.

In one aspect, the mouse lacks a sequence encoding a C_{H}1 domain. In one aspect, the mouse lacks a sequence encoding a hinge region. In one aspect, the mouse lacks a sequence encoding a C_{H}1 domain and a hinge region.

In a specific aspect, the mouse expresses a binding protein that comprises a human immunoglobulin light chain variable domain (λ or κ) fused to a mouse C_{H}2 domain that is attached to a mouse C_{H}3 domain.

In one aspect, a mouse is described that comprises an ectopic ADAM6 sequence that encodes a functional ADAM6 protein (or ortholog or homolog or functional fragment thereof), a replacement of all or substantially all mouse V_{H} gene segments with one or more human V_{L} gene segments, a replacement of all or substantially all mouse D_{H} and J_{H} gene segments with human J_{L} gene segments.

In one aspect, the mouse comprises a deletion of an immunoglobulin heavy chain constant region gene sequence encoding a C_{H}1 region, a hinge region, a C_{H}1 and a hinge region, or a C_{H}1 region and a hinge region and a C_{H}2 region.

In one aspect, the mouse makes a single variable domain binding protein comprising a homodimer selected from the following: (a) human V_{L} - mouse C_{H}1 - mouse C_{H}2 - mouse C_{H}3; (b) human V_{L} - mouse hinge - mouse C_{H}2 - mouse C_{H}3; (c) human V_{L} - mouse C_{H}2 - mouse C_{H}3.

In one aspect, a non-human animal is described, comprising a modified immunoglobulin heavy chain locus, wherein the modified immunoglobulin heavy chain locus comprises a non-human ADAM6 sequence or ortholog or homolog thereof.

In one aspect, the non-human animal is a rodent selected from a mouse, a rat, and a hamster.

In one aspect, the non-human ADAM6 ortholog or homolog is a sequence that is orthologous and/or homologous to a mouse ADAM6 sequence, wherein the ortholog or homolog is functional in the non-human animal.

In one aspect, the non-human animal is selected from a mouse, a rat, and a hamster and the ADAM6 ortholog or homolog is from a non-human animal selected from a mouse, a rat, and a hamster. In a specific aspect, the non-human animal is a mouse and the ADAM6 ortholog or homolog is from an animal that is selected from a different mouse species, a rat, and a hamster. In specific aspect, the non-human animal is a rat, and the ADAM6 ortholog or homolog is from a rodent that is selected from a different rat species, a mouse, and a hamster. In a specific aspect, the non-human animal is a hamster, and the ADAM6 ortholog or homolog is form a rodent that is selected from a different hamster species, a mouse, and a rat.

In a specific aspect, the non-human animal is from the suborder Myomorpha, and the ADAM6 sequence is from an animal selected from a rodent of superfamily Dipodoidea and a rodent of the superfamily Muroidea. In a specific aspect, the rodent is a mouse of superfamily Muroidea, and the ADAM6 ortholog or homolog is from a mouse or a rat or a hamster of superfamily Muroidea.

In one aspect, the modified immunoglobulin heavy chain locus comprises one or more human V_{L} gene segments and one or more human J_{L} gene segments. In a specific aspect, the one or more human V_{L} gene segments and one or more human J_{λ} gene segments are operably linked to one or more human, chimeric and/or rodent (e.g., mouse or rat) constant region genes. In one aspect, the constant region genes are mouse. In one aspect, the constant region genes are rat. In one aspect, the constant region genes are hamster. In one aspect, the constant region genes comprise a sequence selected from a hinge, a C_{H}2, a C_{H}3, and a combination thereof. In specific aspect, the constant region genes comprise a hinge, a C_{H}2, and a C_{H}3 sequence. In one aspect, the human VL and JL gene segments are human Vκ and Jκ gene segments.

In one aspect, the non-human ADAM6 sequence is contiguous with a human immunoglobulin light chain sequence. In one aspect, the non-human ADAM6 sequence is positioned within a human immunoglobulin light chain sequence. In a specific aspect, the human immunoglobulin light chain sequence comprises a V and/or J gene segment.

In one aspect, the non-human ADAM6 sequence is juxtaposed with a V gene segment. In one aspect, the non-human ADAM6 sequence is positioned between two V gene segments. In one aspect, the non-human ADAM6 sequence is juxtaposed between a V and a J gene segment. In one aspect, the mouse ADAM6 sequence is juxtaposed between two J gene segments.

In one aspect, a genetically modified non-human animal is described, comprising a B cell that expresses a human V_{L} domain cognate with a human V_{L} domain from an immunoglobulin locus, wherein the non-human animal expresses a non-immunoglobulin non-human protein from the immunoglobulin locus. In one aspect, the non-immunoglobulin non-human protein is an ADAM protein. In a specific aspect, the ADAM protein is an ADAM6 protein or homolog or ortholog or functional fragment thereof.

In one aspect the non-human animal is a rodent (*e*.*g*., mouse or rat). In one aspect, the rodent is of family Muridae. In one aspect, the rodent is of subfamily Murinae. In a specific aspect, the rodent of subfamily Murinae is selected from a mouse and a rat.

In one aspect, the non-immunoglobulin non-human protein is a rodent protein. In one aspect, the rodent is of family Muridae. In one aspect, the rodent is of subfamily Murinae. In a specific aspect, the rodent is selected from a mouse, a rat, and a hamster.

In one aspect, the human V_{L} domains are attached directly or through a linker to an immunoglobulin constant domain sequence. In a specific aspect, the constant domain sequence comprises a sequence selected from a hinge, a C_{H}2 a C_{H}3, and a combination thereof. In a specific aspect, the human V_{L} domain is selected from a Vκ or a Vλ domain.

In various aspects, the human V_{L} domains are human Vκ domains.

In one aspect, a genetically modified non-human animal is described, comprising in its germline a human immunoglobulin sequence, wherein the sperm of a male non-human animal is characterized by an *in vivo* migration defect. In one aspect, the *in vivo* migration defect comprises an inability of the sperm of the male non-human animal to migrate from a uterus through an oviduct of a female non-human animal of the same species. In one aspect, the non-human animal lacks a nucleotide sequence that encodes and ADAM6 protein or functional fragment thereof. In a specific aspect, the ADAM6 protein or functional fragment thereof includes an ADAM6a and/or an ADAM6b protein or functional fragments thereof. In one aspect, the non-human animal is a rodent. In a specific aspect, the rodent is selected from a mouse, a rat, and a hamster.

In one aspect, a non-human animal is described, comprising a human immunoglobulin sequence contiguous with a non-human sequence that encodes an ADAM6 protein or ortholog or homolog or functional fragment thereof. In one aspect, the non-human animal is a rodent. In a specific aspect, the rodent is selected from a mouse, a rat, and a hamster.

In one aspect, the human immunoglobulin sequence is an immunoglobulin light chain sequence. In one aspect, the immunoglobulin sequence comprises one or more V_{L} gene segments. In one aspect, the human immunoglobulin sequence comprises one or more J_{L} gene segments. In one aspect, the human immunoglobulin sequence comprises one or more V_{L} gene segments and one or more J_{L} gene segments. In various aspects, the human V_{L} and J_{L} gene segments are human Vκ and Jκ gene segments.

In one aspect, a mouse is described with a disabled endogenous immunoglobulin heavy chain locus, comprising a disabled or deleted endogenous ADAM6 locus, wherein the mouse comprises a nucleic acid sequence that expresses a human or mouse or human/mouse or other chimeric antibody. In one aspect, the nucleic acid sequence is present on a transgene integrated that is randomly integrated into the mouse genome. In one aspect, the nucleic acid sequence is on an episome (e.g., a chromosome) not found in a wild-type mouse.

In one aspect, a mouse is described with a disabled endogenous immunoglobulin heavy chain locus, comprising a functional endogenous ADAM6 locus, wherein the mouse comprises a nucleic acid sequence that expresses a human or mouse or human/mouse or other chimeric antibody. In one aspect, the nucleic acid sequence is present at the endogenous immunoglobulin heavy chain locus at a position upstream from one or more endogenous heavy chain constant region genes. In one aspect, the nucleic acid sequence is present on a transgene integrated that is randomly integrated into the mouse genome. In one aspect, the nucleic acid sequence is on an episome (e.g., a chromosome) not found in a wild-type mouse.

### Bispecific-Binding Proteins

The binding proteins described herein, and nucleotide sequences encoding them, can be used to make multispecific binding proteins, e.g., bispecific binding proteins. In this aspect, a first polypeptide consisting essentially of a first V_{L} domain fused with a C_{H} region can associate with a second polypeptide consisting essentially of a second V_{L} domain fused with a C_{H} region. Where the first V_{L} domain and the second V_{L} domain specifically bind a different epitope, a bispecific-binding molecule can be made using the two V_{L} domains. The C_{H} region can be the same or different. In one aspect, *e*.*g*., one of the C_{H} regions can be modified so as to eliminate a protein A binding determinant, whereas the other heavy chain constant region is not so modified. This particular arrangement simplifies isolation of the bispecific binding protein from, *e*.*g*., a mixture of homodimers (*e*.*g*., homodimers of the first or the second polypeptides).

In one aspect, the methods and compositions described herein are used to make bispecific-binding proteins. In this aspect, a first V_{L} that is fused to a C_{H} region and a second V_{L} that is fused to a C_{H} region are each independently cloned in frame with a human IgG sequence of the same isotype (*e*.*g*., a human IgG1, IgG2, IgG3, or IgG4). The first V_{L} specifically binds a first epitope, and the second V_{L} specifically binds a second epitope. The first and second epitopes may be on different antigens, or on the same antigen.

In one aspect, the IgG isotype of the C_{H} region fused to the first V_{L} and the IgG isotype of the C_{H} region fused to the second V_{L} are the same isotype, but differ in that one IgG isotype comprises at least one amino acid substitution. In one aspect, the at least one amino acid substitution renders the heavy chain bearing the substitution unable or substantially unable to bind protein A as compared with the heavy chain that lacks the substitution.

In one aspect, the first C_{H} region comprises a first C_{H}3 domain of a human IgG selected from IgG1, IgG2, and IgG4; and the second C_{H} region comprises a second C_{H}3 domain of a human IgG selected from IgG1, IgG2, and IgG4, wherein the second C_{H}3 domain comprises a modification that reduces or eliminates binding of the second C_{H}3 domain to protein A.

In one aspect, the second C_{H}3 domain comprises a 435R modification, numbered according to the EU index of Kabat. In another aspect, the second C_{H}3 domain further comprises a 436F modification, numbered according to the EU index of Kabat.

In one aspect, the second C_{H}3 domain is that of a human IgG1 that comprises a modification selected from the group consisting of D356E, L358M, N384S, K392N, V397M, and V422I, numbered according to the EU index of Kabat.

In one aspect, the second C_{H}3 domain is that of a human IgG2 that comprises a modification selected from the group consisting of N384S, K392N, and V422I, numbered according to the EU index of Kabat.

In one aspect, the second C_{H}3 domain is that of a human IgG4 comprising a modification selected from the group consisting of Q355R, N384S, K392N, V397M, R409K, E419Q, and V422I, numbered according to the EU index of Kabat.

In one aspect, the binding protein comprises C_{H} regions having one or more modifications as recited herein, wherein the constant region of the binding protein is nonimmunogenic or substantially nonimmunogenic in a human. In a specific aspect, the C_{H} regions comprise amino acid sequences that do not present an immunogenic epitope in a human. In another specific aspect, the binding protein comprises a C_{H} region that is not found in a wild-type human heavy chain, and the C_{H} region does not comprise a sequence that generates a T-cell epitope.

### EXAMPLES

The following examples are provided so as to describe how to make and use methods and compositions described herein. Unless indicated otherwise, temperature is indicated in Celsius, and pressure is at or near atmospheric.

### Example 1. Introduction of Human Light Chain Gene Segments Into A Heavy Chain Locus

Various targeting constructs were made using VELOCIGENE® genetic engineering technology (see, *e*.*g*., US Pat. No. 6,586,251 and Valenzuela et al. (2003), High-throughput engineering of the mouse genome coupled with high-resolution expression analysis, Nat Biotechnol 21:652-659) to modify mouse genomic Bacterial Artificial Chromosome (BAC) libraries. Mouse BAC DNA was modified by homologous recombination to inactivate the endogenous heavy chain locus through targeted deletion of V_{H}, D_{H} and J_{H} gene segments for the ensuing insertion of unrearranged human germline κ light chain gene sequences (*e*.*g*., see top of FIG. 2).

Briefly, the mouse heavy chain locus was deleted in two successive targeting events using recombinase-mediated recombination. The first targeting event included a targeting at the 5' end of the mouse heavy chain locus using a targeting vector comprising from 5' to 3' a 5' mouse homology arm, a recombinase recognition site, a neomycin cassette and a 3' homology arm. The 5' and 3' homology arms contained sequence 5' of the mouse heavy chain locus. The second targeting event included a targeting at the 3' end of the mouse heavy chain locus in the region of the J_{H} gene segments using a second targeting vector that contained from 5' to 3' a 5' mouse homology arm, a 5' recombinase recognition site, a second recombinase recognition site, a hygromycin cassette, a third recombinase recognition site, and a 3' mouse homology arm. The 5' and 3' homology arms contained sequence flanking the mouse J_{H} gene segments and 5' of the intronic enhancer and constant regions. Positive ES cells containing a modified heavy chain locus targeted with both targeting vectors (as described above) were confirmed by karyotyping. DNA was then isolated from the double-targeted ES cells and subjected to treatment with a recombinase thereby mediating the deletion of genomic DNA of the mouse heavy chain locus between the 5' recombinase recognition site in the first targeting vector and the 5' recombinase recognition site in the second targeting vector, leaving a single recombinase recognition site and the hygromycin cassette flanked by two recombinase recognition sites (top of FIG. 2). Thus a modified mouse heavy chain locus containing intact C_{H} genes was created for progressively inserting human κ germline gene segments in a precise manner using targeting vectors described below.

Four separate targeting vectors were engineered to progressively insert 40 human Vκ gene segments and five human Jκ gene segments into the inactivated mouse heavy chain locus (described above) using standard molecular techniques recognized in the art (FIG. 2). The human κ gene segments used for engineering the four targeting constructs are naturally found in proximal contig of the germline human κ light chain locus (bottom of FIG. 1 and Table 1).

A ∼110,499 bp human genomic fragment containing the first six human Vκ gene segments and five human Jκ gene segments was engineered to contain a PI-Scel site 431 bp downstream (3') of the human Jκ5 gene segment. Another PI-Scel site was engineered at the 5' end of a ∼7,852 bp genomic fragment containing the mouse heavy chain intronic enhancer, the IgM switch region (Sµ) and the IgM gene of the mouse heavy chain locus. This mouse fragment was used as a 3' homology arm by ligation to the ∼110.5 kb human fragment, which created a 3' junction containing, from 5' to 3', ∼110.5 kb of genomic sequence of the human κ light chain locus containing the first six consecutive Vκ gene segments and five Jκ gene segments, a PI-Scel site, ∼7,852 bp of mouse heavy chain sequence containing the mouse intronic enhancer, 3µ and the mouse IgM constant gene. Upstream (5') from the human Vκ1-6 gene segment was an additional 3,710 bp of human κ sequence before the start of the 5' mouse homology arm, which contained 19,752 bp of mouse genomic DNA corresponding to sequence 5' of the mouse heavy chain locus. Between the 5' homology arm and the beginning of the human κ sequence was a neomycin cassette flanked by three recombinase recognition sites (see Targeting Vector 1, FIG. 2). The final targeting vector for the first insertion of human κ sequence from 5' to 3' included a 5' homology arm containing ∼20 kb of mouse genomic sequence 5' of the heavy chain locus, a first recombinase recognition site (R1), a neomycin cassette, a second recombinase recognition site (R2), a third recombinase recognition site (R3), ∼110.5 kb of human genomic κ sequence containing the first six consecutive human Vκ gene segments and five human Jκ gene segments, a PI-Scel site, and a 3' homology arm containing ∼8 kb of mouse genomic sequence including the intronic enhancer, Sµ and the mouse IgM constant gene (FIG. 2, Targeting Vector 1). Homologous recombination with this targeting vector created a modified mouse heavy chain locus containing six human Vκ gene segments and five human Jκ gene segments operably linked to the endogenous heavy chain constant genes which, upon recombination, leads to the formation of a hybrid heavy chain (*i*.*e*., a human Vκ domain and a mouse C_{H} region).

**TABLE 1**

| Targeting Vector | Size of Human κ Sequence | Human κ Gene Segments Added | |
|---|---|---|---|
| | | Vκ | Jκ |
| 1 | kb | 4-1, 5-2, 7-3, 2-4, 1-5, 1-6 | 1 - 5 |
| 2 | ∼140 kb | 3-7, 1-8, 1-9, 2-10, 3-11, 1-12, 1-13, 2-14, 3-15, 1-16 | - |
| 3 | ∼161 kb | 1-17, 2-18, 2-19, 3-20, 6-21, 1-22, 1-23, 2-24, 3-25, 2-26, 1-27, 2-28, 2-29, 2-30 | - |
| 4 | ∼90 kb | 3-31, 1-32, 1-33, 3-34, 1-35, 2-36, 1-37, 2-38, 1-39, 2-40 | - |

**Introduction of ten additional human Vx gene segments into a hybrid heavy chain locus.** A second targeting vector was engineered for introduction of 10 additional human Vκ gene segments to the modified mouse heavy chain locus described above (see FIG. 2, Targeting Vector 2). A 140,058 bp human genomic fragment containing 12 consecutive human Vκ gene segments from the human κ light chain locus was engineered with a 5' homology arm containing mouse genomic sequence 5' of the mouse heavy chain locus and a 3' homology arm containing human genomic κ sequence. Upstream (5') from the human Vκ1-16 gene segment was an additional 10,170 bp of human κ sequence before the start of the 5' mouse homology arm, which was the same 5' homology arm used for construction of Targeting Vector 1 (FIG. 2). Between the 5' homology arm and the beginning of the human κ sequence was a hygromycin cassette flanked by recombinase recognition sites. The 3' homology arm included a 31,165 bp overlap of human genomic κ sequence corresponding to the equivalent 5' end of the ∼110.5 kb fragment of human genomic κ sequence of Targeting Vector 1 (FIG. 2). The final targeting vector for the insertion of 10 additional human Vκ gene segments, from 5' to 3', included a 5' homology arm containing ∼20 kb of mouse genomic sequence 5' of the heavy chain locus, a first recombinase recognition site (R1), a hygromycin cassette, a second recombinase recognition site (R2) and ∼140 kb of human genomic κ sequence containing 12 consecutive human Vλ, gene segments, ∼31 kb of which overlaps with the 5' end of the human κ sequence of Targeting Vector 1 and serves as the 3' homology arm for this targeting construct. Homologous recombination with this targeting vector created a modified mouse heavy chain locus containing 16 human Vκ gene segments and five human Jκ gene segments operably linked to the mouse heavy chain constant genes which, upon recombination, leads to the formation of a hybrid heavy chain.

**Introduction of fourteen additional human Vx gene segments into a hybrid heavy chain locus.** A third targeting vector was engineered for introduction of 14 additional human Vκ gene segments to the modified mouse heavy chain locus described above (FIG. 2, Targeting Vector 3). A 160,579 bp human genomic fragment containing 15 consecutive human Vκ gene segments was engineered with a 5' homology arm containing mouse genomic sequence 5' of the mouse heavy chain locus and a 3' homology arm containing human genomic κ sequence. Upstream (5') from the human Vκ2-30 gene segment was an additional 14,687 bp of human κ sequence before the start of the 5' mouse homology arm, which was the same 5' homology used for the previous two targeting vectors (described above, see also FIG. 2). Between the 5' homology arm and the beginning of the human κ sequence was a neomycin cassette flanked by recombinase recognition sites. The 3' homology arm included a 21,275 bp overlap of human genomic κ sequence corresponding to the equivalent 5' end of the ∼140 kb fragment of human genomic κ sequence of Targeting Vector 2 (FIG. 20). The final targeting vector for the insertion of 14 additional human Vκ gene segments, from 5' to 3' included a 5' homology arm containing -20 kb of mouse genomic sequence 5' of the mouse heavy chain locus, a first recombinase recognition site (R1), a neomycin cassette, a second recombinase recognition site (R2) and ∼161 kb of human genomic κ sequence containing 15 human Vκ gene segments, ∼21 kb of which overlaps with the 5' end of the human κ sequence of Targeting Vector 2 and serves as the 3' homology arm for this targeting construct. Homologous recombination with this targeting vector created a modified mouse heavy chain locus containing 30 human Vκ gene segments and five human Jκ gene segments operably linked to the mouse heavy chain constant genes which, upon recombination, leads to the formation of a chimeric κ heavy chain.

**Introduction of ten additional human Vκ gene segments into a hybrid heavy chain locus.** A fourth targeting vector was engineered for introduction of 10 additional human Vκ gene segments to the modified mouse heavy chain locus described above (FIG. 2, Targeting Vector 4). A 90,398 bp human genomic fragment containing 16 consecutive human Vκ gene segments was engineered with a 5' homology arm containing mouse genomic sequence 5' of the mouse heavy chain locus and a 3' homology arm containing human genomic κ sequence. Upstream (5') from the human Vκ2-40 gene segment was an additional 8,484 bp of human κ sequence before the start of the 5' mouse homology arm, which was the same 5' homology as the previous targeting vectors (described above, FIG. 2). Between the 5' homology arm and the beginning of the human κ sequence was a hygromycin cassette flanked by recombinase recognition sites. The 3' homology arm included a 61,615 bp overlap of human genomic κ sequence corresponding to the equivalent 5' end of the ∼160 kb fragment of human genomic κ sequence of Targeting Vector 3 (FIG. 2). The final targeting vector for the insertion of 10 additional human Vκ gene segments, from 5' to 3', included a 5' homology arm containing ∼**2**0 kb of mouse genomic sequence 5' of the mouse heavy chain locus, a first recombinase recognition site (R1), a hygromycin cassette, a second recombinase recognition site (R2) and ∼90 kb of human genomic κ sequence containing 16 human Vκ gene segments, ∼62 kb of which overlaps with the 5' end of the human κ sequence of Targeting Vector 3 and serves as the 3' homology arm for this targeting construct. Homologous recombination with this targeting vector created a modified mouse heavy chain locus containing 40 human Vκ gene segments and five human Jκ gene segments operably linked to the mouse heavy chain constant genes which, upon recombination, leads to the formation of a chimeric κ heavy chain (bottom of FIG. 2).

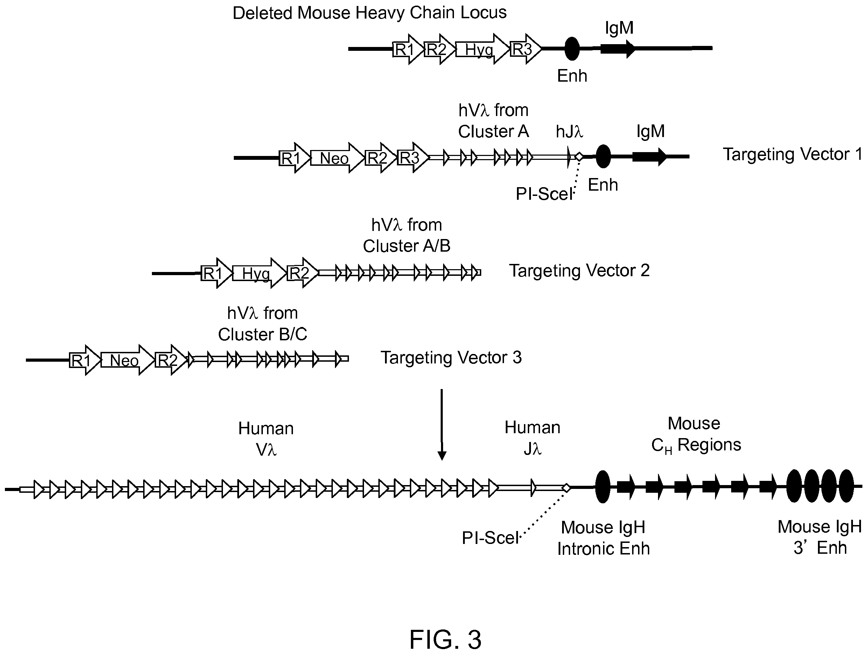

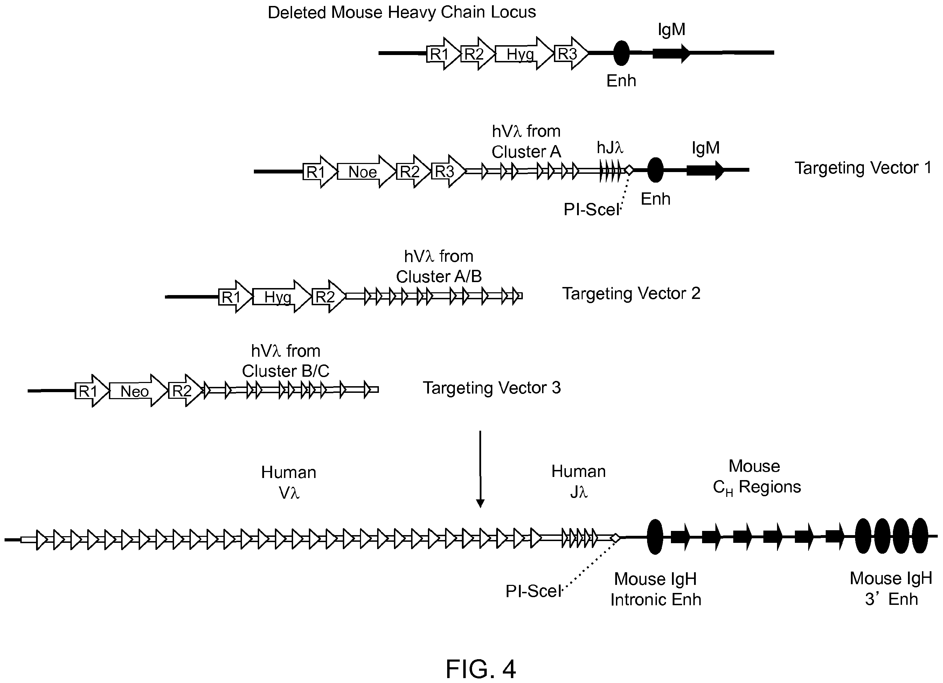

Using a similar approach as described above, other combinations of human light chain variable domains in the context of mouse heavy chain constant regions are constructed. Additional light chain variable domains may be derived from human Vλ, and Jλ, gene segments (FIG. 3 and FIG. 4).

The human λ light chain locus extends over 1,000 kb and contains over 80 genes that encode variable (V) or joining (J) segments. Among the 70 Vλ, gene segments of the human λ light chain locus, anywhere from 30-38 appear to be functional gene segments according to published reports. The 70 Vλ, sequences are arranged in three clusters, all of which contain different members of distinct V gene family groups (clusters A, B and C). Within the human λ light chain locus, over half of all observed Vλ, domains are encoded by the gene segments 1-40, 1-44, 2-8, 2-14, and 3-21. There are seven Jλ, gene segments, only four of which are regarded as generally functional Jλ, gene segments↓Jλ1, Jλ2, Jλ3, and Jλ7. In some alleles, a fifth Jλ,-Cλ, gene segment pair is reportedly a pseudo gene (CPλ6) Incorporation of multiple human Jλ, gene segments into a hybrid heavy chain locus, as described herein, is constructed by de novo synthesis. In this way, a genomic fragment containing multiple human Jλ, gene segments in germline configuration is engineered with multiple human Vλ, gene segments and allow for normal V-J recombination in the context of a heavy chain constant region.

Coupling light chain variable domains with heavy chain constant regions represents a potentially rich source of diversity for generating unique V_{L} binding proteins with human V_{L} regions in non-human animals. Exploiting this diversity of the human λ light chain locus (or human κ locus as described above) in mice results in the engineering of unique hybrid heavy chains and gives rise to another dimension of binding proteins to the immune repertoire of genetically modified animals and their subsequent use as a next generation platform for the generation of therapeutics.

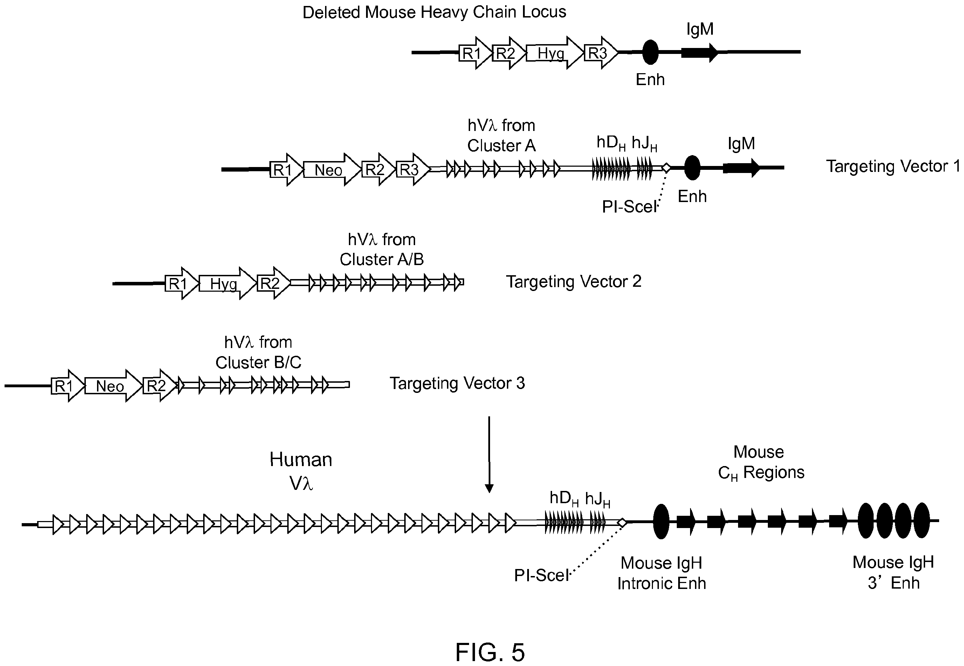

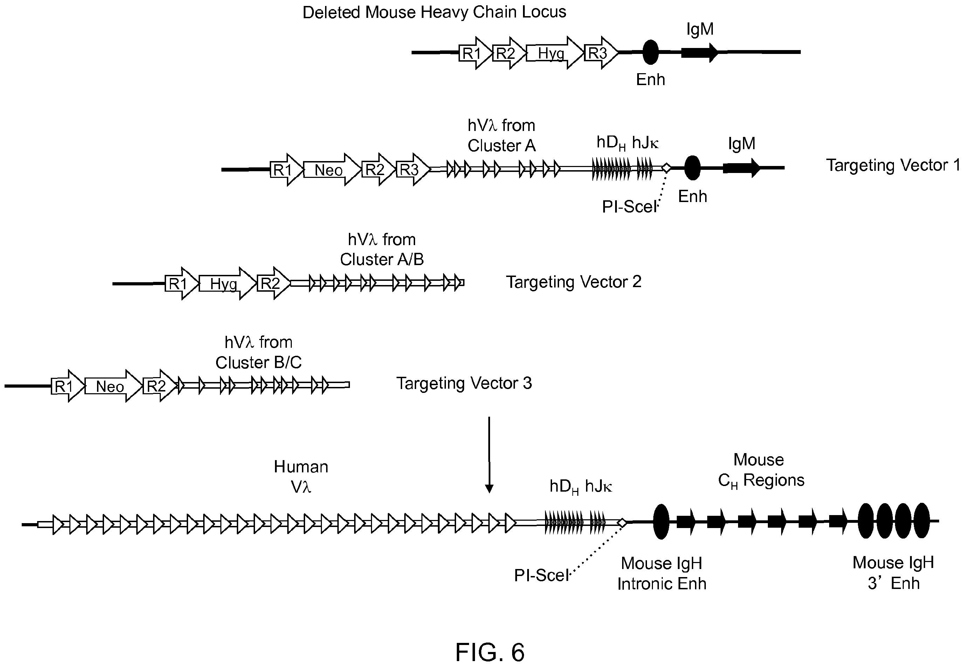

Additionally, human D_{H} and J_{H} (or Jκ) gene segments can be incorporated with either human Vκ or Vλ gene segments to construct novel hybrid loci that will give rise, upon recombination, to novel engineered variable domains (FIG. 5 and 6). In this latter case, engineering combinations of gene segments that are not normally contained in a single locus would require specific attention to the recombination signal sequences (RSS) that are associated with respective gene segments such that normal recombination can be achieved when they are combined into a single locus. For example, V(D)J recombination is known to be guided by conserved noncoding DNA sequences, known as heptamer and nonamer sequences that are found adjacent to each gene segment at the precise location at which recombination takes place. Between these noncoding DNA sequences are nonconserved spacer regions that either 12 or 23 base pairs (bp) in length. Generally, recombination only occurs at gene segments located on the same chromosome and those gene segments flanked by a 12-bp spacer can be joined to a gene segment flanked by a 23-bp spacer, *i.e.* the 12/23 rule, although joining two of D_{H} gene segments (each flanked by 12-bp spacers) has been observed in a small proportion of antibodies. To allow for recombination between gene segments that do not normally have compatible spacers (*e*.*g*., Vκ and a D_{H} or D_{H} and Jλ), unique, compatible spacers are synthesized in adjacent locations with the desired gene segments for construction of unique hybrid heavy chains that allow for successful recombination to form unique heavy chains containing light chain variable regions.

Thus, using the strategy outlined above for incorporation of human κ light chain gene segments into an endogenous heavy chain locus allows for the use of other combinations of human λ light chain gene segments as well as specific human heavy chain gene segments (*e*.*g*., D_{H} and J_{H}) and combinations thereof.

### Example 2. Identification of Targeted ES Cells and Generation of Genetically Modified Mice Bearing Human Light Chain Gene Segments at an Endogenous Heavy Chain Locus

The targeted BAC DNA made in the foregoing Examples is used to electroporate mouse ES cells to created modified ES cells for generating chimeric mice that express V_{L} binding proteins *(i.e.,* human κ light chain gene segments operably linked to mouse heavy chain constant regions). Targeted ES cells containing an insertion of unrearranged human κ light chain gene segments are identified by a quantitative PCR assay, TAQMAN® (Lie, Y.S., and Petropoulos, C.J. (1998) Advances in quantitative PCR technology: 5' nuclease assays. Curr Opin Biotechnol 9(1): 43-48). Specific primers sets and probes are designed to detect insertion of human κ sequences and associated selection cassettes, loss of mouse heavy chain sequences and retention of mouse sequences flanking the endogenous heavy chain locus.

ES cells bearing the human κ light chain gene segments can be transfected with a construct that expresses a recombinase in order to remove any undesired selection cassette introduced by the insertion of the targeting construct containing human κ gene segments. Optionally, mice bearing an engineered heavy chain locus containing the human κ light chain gene segments can be bred to a FLPe deletor mouse strain (see, *e*.*g*., Rodriguez, C.I. et al. (2000) High-efficiency deletor mice show that FLPe is an alternative to Cre-IoxP. Nature Genetics 25: 139-140; US 6,774,279) in order to remove any Frt'ed cassette introduced by the targeting vector that is not removed, *e*.*g*., at the ES cell stage or in the embryo. Optionally, the selection cassette is retained in the mice.

Targeted ES cells described above are used as donor ES cells and introduced into an 8-cell stage mouse embryo by the VELOCIMOUSE® method (*supra*). Mice bearing a modified heavy chain locus bearing human Vκ and Jκ gene segments operably linked to the mouse immunoglobulin heavy chain constant region genes are identified by genotyping using a modification of allele assay (Valenzuela et al., *supra*) that detected the presence and/or absence of cassette sequences, the human Vκ and Jκ gene segments and endogenous heavy chain sequences.

Pups are genotyped and a pup heterozygous for a modified heavy chain locus containing human κ light chain gene segments operably linked to the endogenous mouse immunoglobulin heavy chain constant genes is selected for characterizing the immunoglobulin heavy chain repertoire.

### Example 3. Propagation of Mice Expressing V_{L} Binding Proteins

To create a new generation of V_{L} binding proteins, mice bearing the unrearranged human κ gene segments can be bred to another mouse containing a deletion of the opposite or untargeted endogenous heavy chain allele (*i*.*e*., a mouse heterozygous for the modification). In this manner, the progeny obtained would express only hybrid heavy chains as described in Example 1. Breeding is performed by standard techniques recognized in the art and, alternatively, by commercial companies, *e*.*g*., The Jackson Laboratory. Mouse strains bearing a modified heavy chain locus are screened for presence of the unique heavy chains containing human light chain variable domains.

Alternatively, mice bearing the unrearranged human κ gene segments at the mouse heavy chain locus can be optimized by breeding to other mice containing one or more deletions in the mouse light chain loci (κ and λ). In this manner, the progeny obtained would express unique human κ heavy chain only antibodies as described in Example 1. Breeding is similarly performed by standard techniques recognized in the art and, alternatively, by commercial companies, *e*.*g*., The Jackson Laboratory. Mouse strains bearing a modified heavy chain locus and one or more deletions of the mouse light chain loci are screened for presence of the unique heavy chains containing human Vκ domains and mouse heavy chain constant domains and absence of endogenous light chains.

Mice bearing a modified heavy chain locus (described above) are also bred with mice that contain a replacement of the endogenous κ light chain variable gene locus with the human κ light chain variable gene locus (see US 6,596,541, Regeneron Pharmaceuticals, The VELOCIMMUNE® Humanized Mouse Technology). The VELOCIMMUNE® Humanized Mouse includes, in part, having a genome comprising human κ light chain variable regions operably linked to endogenous κ light chain variable constant region loci such that the mouse produces antibodies comprising a human κ light chain variable domain and a mouse heavy chain constant domain in response to antigenic stimulation. The DNA encoding the variable regions of the light chains of the antibodies can be isolated and operably linked to DNA encoding the human light chain constant regions. The DNA can then be expressed in a cell capable of expressing the fully human light chain of the antibody. Upon a suitable breeding schedule, mice bearing a replacement of the endogenous κ light chain with the human κ light chain locus and a modified heavy chain locus according to Example 1 are obtained. Unique V_{L} binding proteins containing somatically mutated human Vκ domains can be isolated upon immunization with an antigen of interest.

### Example 4. Reengineering of ADAM Genes into a Modified Heavy Chain Locus

Mice with modified immunoglobulin heavy chain loci in which the endogenous variable region gene segments *(i.e.,* VDJ) have been replaced and/or deleted lack expression of endogenous ADAM6 genes. In particular, male mice comprising such modifications of the immunoglobulin heavy chain loci demonstrate a reduction in fertility. This Example demonstrates two methods to reengineer the capability to express ADAM6 into the mice with the modified heavy chain loci according to Example 1, thus allowing for the maintenance of the modified mouse strains using normal breeding methods.

**Reengineering of ADAM6 Genes Within Human Light Chain Gene Segments.** A modified immunoglobulin heavy chain locus containing human Vκ and Jκ gene segments was reengineered to contain a genomic fragment encoding mouse ADAM6a and ADAM6b by homologous recombination using BAC DNA. This was accomplished by VELOCIGENE® genetic engineering technology (*supra*) in a series of six steps that included modification of BAC DNA containing mouse and human sequences that yielded a final targeting vector containing human Vκ and Jκ gene segments contiguous with mouse ADAM6 genes and mouse heavy chain constant regions.

A mouse BAC clone (VI149) containing, from 5' to 3', a unique restriction site (I-Ceul), mouse Adam6a and Adam6b genes, an IGCR1 regulatory element (Guo *et al*., 2011), immunoglobulin D_{H} and J_{H} gene segments, an Eµ enhancer, and an IgM constant region gene was used as starting material for reengineering ADAM6 genes in to a modified heavy chain locus containing V_{L} and J_{L} gene segments (FIG. 7). VI149 was modified by bacterial homologous recombination (BHR) to delete all of the D_{H} and J_{H} gene segments and the IgM gene from about 53 bp 5' of the most distal D segment (DFL16.1) to the 3' end of the BAC. This region was replaced by a spectinomycin-resistance cassette (pSVi0029) that contained a unique Ascl site at its 5' end to yield the BAC clone VI413.

Additional BHR modifications were made to create BAC clones containing the mouse Adam6a and Adam6b genes, as well as the IGCR1 element. The first BAC clone was created by replacing a 47199 bp region between Adam6a and Adam6b with a Frt'ed neomycin-resistance cassette with unique I-Ceul (5') and Ascl (3') restriction sites (pLMa0294). This deletion spanned the region from 4779 bp 3' of the Adam6b CDS to 290 bp 5' of the Adam6b CDS. The resulting BAC clone was named VI421. The second BAC clone was created by inserting the same Frt'ed cassette between Adam6a and Adam6b at a position 4782 bp 3' of the Adam6a CDS in VI413 to yield VI422.

The VI421 BAC clone contained, from 5' to 3', a unique I-Ceul site, Adam6a including 751 bp 5' and 4779 bp 3' of the CDS, the Frt'ed neomycin-resistance cassette, Adam6b including 290 bp 5' and 7320 bp 3' of the CDS, IGCR1, and a unique Ascl site (SEQ ID NO: 3).

The VI422 BAC clone contained, from 5' to 3', a unique I-Ceul site, Adam6a including 751 bp 5' and 4779 bp 3' of the CDS, the Frt'ed hygromycin-resistance cassette, Adam6b including 47490 bp 5' and 7320 bp 3' of the CDS, IGCR1, and a unique Ascl site (SEQ ID NO: 4).

Reengineering of ADAM6 genes was accomplished by insertion of VI421 and VI422 into the intergenic region of modified version of Targeting Vector 1 (FIG. 2) as described in Example 1. Targeting Vector 1 was modified by two BHR steps to insert the mouse ADAM6 fragments from VI421 and VI422. The first BHR step, the neomycin cassette from Targeting Vector 1 was deleted with a hygromycin cassette (pLMa0100). The resulting BAC clone was named VI425, which contained, from 5' to 3', a hygromycin-resistance cassette, the four most proximal human Vκ segments, a 23,552 bp Vκ-Jκ intergenic region, and all five human Jκ segments, which are functionally linked to an 8 kb 3' mouse homology arm containing the mouse Eµ enhancer and IgM constant region gene. For the second BHR, VI425 was modified to replace 740 bp within the Vκ-Jκ intergenic region with a chloramphenicol-resistance cassette flanked by unique I-Ceul and Ascl restriction sites (pDBa0049; FIG. 8). The location of the 740 bp deletion was from 16,858 to17,597 bp 3' of the most proximal human Vκ gene segment (Vκ4-1). The resulting BAC clone from both BHRs was named VI426 (FIG. 8).

The DNA fragment containing mouse ADAM6 genes from VI421 and VI422 were independently used to replace the chloramphenicol cassette of VI426 by I-Ceul/Ascl digestion and relegation of compatible ends. FIG. 8 shows the final targeting vectors, named VI429 and VI428, respectively. Each were used to electroporate into ES cells previously modified with Targeting Vector 4 (as described in Example 1, see FIG. 2) to allow for recombination with the unique heavy chain locus modified in accordance with Example 1 and insertion of the DNA fragment encoding mouse ADAM6 genes. Positive colonies were selected with neomycin.

**Reengineering of ADAM6 Genes Flanking Human Light Chain Gene Segments.** A modified immunoglobulin heavy chain locus containing human Vκ and Jκ gene segments located upstream of all the endogenous heavy chain constant regions was reengineered to contain a genomic fragment encoding mouse ADAM6a and ADAM6b by homologous recombination using BAC DNA. This was accomplished by VELOCIGENE® genetic engineering technology (*supra*) in a series of steps that included modification of BAC DNA containing mouse and human sequences that yielded a final targeting vector containing human Vκ and Jκ gene segments contiguous with mouse ADAM6 genes and mouse heavy chain constant regions.

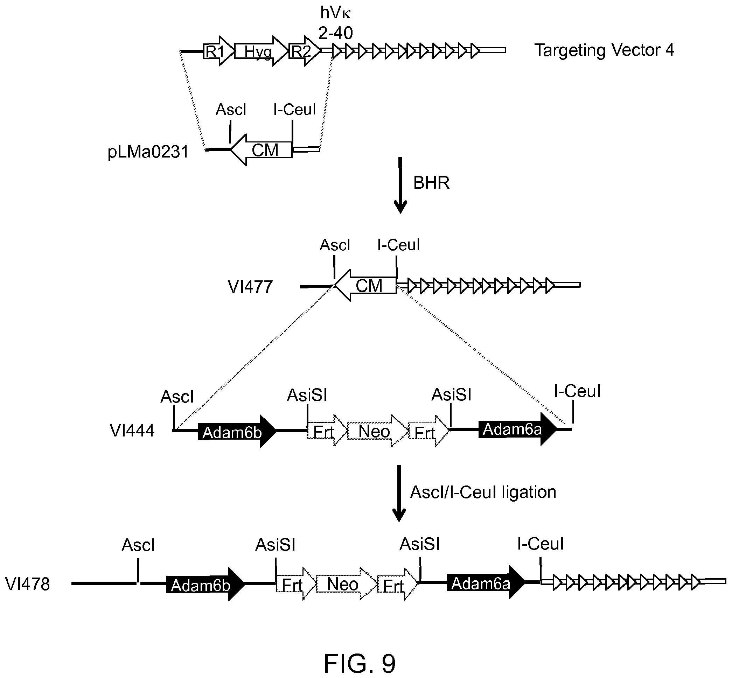

Targeting Vector 4 made in accordance with Example 1 (see FIG. 2 and top of FIG. 9) was modified by BHR to replace the Frt'ed hygromycin cassette with a chloramphenicol cassette containing unique Ascl (5') and I-Ceul (3') restriction sites (pLMa0231; FIG. 9). Targeting Vector 4 contained, from 5' to 3', an ∼20 kb mouse distal IgH homology arm, a Frt'ed hygromycin-resistance cassette, and human Vκ2-40 to human Vκ3-25 gene segments.

Next, a BAC clone named VI444 was used to insert a DNA fragment encoding mouse ADAM6 genes at a position 5' of the human Vκ gene segments of the VI477 BAC clone by Ascl/I-Ceul digestion and relegation of compatible ends. The VI444 clone contained, from 5' to 3', a unique I-Ceul site, the Adam6a gene including 751 bp 5' and 4779 bp 3' of the CDS, a Frt'ed neomycin-resistance cassette, the Adam6b gene including 290 bp 5' and 1633 bp 3' of the CDS, and a unique Ascl site (SEQ ID NO: 5). The resulting BAC clone used as the targeting vector for insertion of mouse ADAM6 genes upstream of human Vκ gene segments was named VI478, which, in contrast to VI421 and VI422, positioned the mouse ADAM6 genes in VI478 are in reverse orientation (*i*.*e*., the same transcriptional direction relative to the human Vκ gene segments; FIG. 9). The final targeting vector for insertion of mouse ADAM6 genes at the distal end of the human Vκ gene segments contained, from 5' to 3', the ∼20 kb mouse distal IgH homology arm, a unique Ascl site, mouse Adam6b, a Frt'ed neomycin-resistance cassette, mouse Adam6a, a unique I-Ceul site, and human Vκ2-40 to human Vκ3-25 gene segments. This targeting vector was used to electroporate into ES cells previously modified with Targeting Vector 4 (FIG. 2) to allow for recombination with the unique heavy chain locus modified in accordance with Example 1 and insertion of the DNA fragment encoding mouse ADAM6 genes. Positive colonies were selected with neomycin.

**Selection and Confirmation of targeted ES cells.** Each of the final targeting vectors (described above) were used to electroporate mouse ES cells to create modified ES cells comprising a mouse genomic sequence ectopically placed that comprises mouse ADAM6a and ADAM6b sequences within modified heavy chain locus containing human Vκ and Jκ gene segments. Positive ES cells containing the ectopic mouse genomic fragment within the modified heavy chain locus were identified by a quantitative PCR assay using TAQMAN™ probes (Lie and Petropoulos (1998), *supra*).

Targeted ES cells described above were used as donor ES cells and introduced into an 8-cell stage mouse embryo by the VELOCIMOUSE® mouse engineering method (see, *e*.*g*., US Pat. Nos. 7,6598,442; 7,576,259; and 7,294,754). Mice bearing a modified heavy chain locus containing human κ light chain gene segments and an ectopic mouse genomic sequence comprising mouse ADAM6a and ADAM6b sequences were identified by genotyping using a modification of allele assay (Valenzuela *et al*., 2003) that detected the presence of the mouse ADAM6a and ADAM6b genes within the modified heavy chain locus as well as human κ light chain sequences.

Pups are genotyped and a pup heterozygous for a modified heavy chain locus containing an ectopic mouse genomic fragment that comprises mouse ADAM6a and ADAM6b sequences is selected for characterizing mouse ADAM6 gene expression and fertility.

## Claims

1. A mouse whose genome comprises:
(a) an insertion of one or more human VL gene segments and one or more human J_{L} gene segments upstream of a mouse immunoglobulin light chain constant region gene, wherein the one or more human V_{L} gene segments and one or more human J_{L} gene segments are operably linked to the mouse immunoglobulin light chain constant region gene;
(b) an insertion of one or more human V_{L} gene segments and one or more human J_{L} gene segments upstream of a mouse immunoglobulin heavy chain constant region gene, wherein the one or more human V_{L} gene segments and one or more human J_{L} gene segments are operably linked to the mouse immunoglobulin heavy chain constant region gene; and
(c) an ectopic nucleic acid sequence that encodes a mouse ADAM6a protein or a functional fragment thereof and an ectopic nucleic acid sequence that encodes a mouse ADAM6b protein or a functional fragment thereof, wherein the mouse ADAM6a and ADAM6b proteins or functional fragments thereof are expressed from the ectopic nucleic acid sequences,
wherein the mouse expresses an antibody comprising (i) a polypeptide that comprises a human light chain variable domain linked to a mouse heavy chain constant domain; and (ii) a polypeptide that comprises a human light chain variable domain linked to a mouse light chain constant domain.

2. The mouse of claim 1, wherein the mouse immunoglobulin light chain constant region gene is a mouse CK.

3. The mouse of claim 1 or 2, wherein the one or more human V_{L} gene segments and one or more human J_{L} gene segments upstream of the mouse immunoglobulin light chain constant region gene are one or more human Vκ gene segments and one or more human Jκ gene segments.

4. The mouse of any one of the preceding claims, wherein the one or more human V_{L} gene segments and one or more human J_{L} gene segments upstream of the mouse immunoglobulin heavy chain constant region gene are one or more human Vκ gene segments and one or more human Jκ gene segments.

5. The mouse of any one of claims 1-4, wherein the ectopic nucleic acid sequences are present between two of the inserted one or more human V_{L} gene segments and one or more human J_{L} gene segments of (b).

6. The mouse of claim 5, wherein the inserted one or more human V_{L} gene segments and one or more human J_{L} gene segments are human κ light chain gene segments or human λ light chain gene segments.

7. The mouse of any one of the preceding claims, wherein the inserted one or more human V_{L} gene segments includes Vκ5-2 and Vκ4-1 and the ectopic nucleic acid sequences that encode the mouse ADAM6a and ADAM6b proteins or functional fragments thereof are present between Vκ5-2 and Vκ4-1.

8. The mouse of any one of claims 1-6, wherein the inserted one or more human V_{L} gene segments includes Vκ4-1 and the inserted one or more J_{L} gene segments include Jκ1 and the ectopic nucleic acid sequences that encode the mouse ADAM6a and ADAM6b proteins or functional fragments thereof are present between Vκ4-1 and Jκ1.

9. The mouse of any one of the preceding claims, wherein the mouse comprises one or more endogenous V_{L} and/or one or more endogenous J_{L} gene segments that are incapable of rearranging to form an immunoglobulin light chain variable sequence in the mouse.

10. The mouse of any one of the preceding claims, wherein the mouse comprises deletion of, or replacement of, one or more endogenous immunoglobulin heavy chain gene sequences.

11. A cell derived from the mouse of claim 1.

12. The cell of claim 11, wherein the cell is a B cell.

13. A hybridoma made from the B cell of claim 12.

14. A method for making an antibody that binds to an antigen of interest, wherein the method comprises
(a) exposing a mouse of claim 1 to an antigen of interest;
(b) isolating one or more B cells of the mouse, wherein the one or more B cells express an antibody that binds the antigen of interest;
(c) identifying a nucleic acid sequence that encodes one of the light variable chain domains of the antibody that binds that antigen of interest, wherein the antibody comprises a human immunoglobulin κ light chain variable domain linked to a mouse light chain constant domain and a human immunoglobulin κ light chain variable domain linked to a mouse heavy chain constant domain; and
(d) employing the nucleic acid sequence of (c) with a human immunoglobulin constant region nucleic acid sequence to make a human antibody that binds the antigen of interest.

## Patentansprüche

1. Maus, deren Genom Folgendes umfasst:
(a) eine Insertion von einem oder mehreren menschlichen V_{L}-Gensegmenten und einem oder mehreren menschlichen J_{L}-Gensegmenten stromaufwärts von einem Gen einer konstanten Maus-Immunglobulin-Leichtkettenregion, wobei das eine oder die mehreren menschlichen V_{L}-Gensegmente und ein oder mehrere menschlichen J_{L}-Gensegmente funktionsfähig mit dem Gen einer konstanten Maus-Immunglobulin-Leichtkettenregion verbunden sind;
(b) eine Insertion von einem oder mehreren menschlichen V_{L}-Gensegmenten und einem oder mehreren menschlichen J_{L}-Gensegmenten stromaufwärts von einem Gen einer konstanten Maus-Immunglobulin-Schwerkettenregion, wobei das eine oder die mehreren menschlichen V_{L}-Gensegmente und das eine oder die mehreren menschlichen J_{L}-Gensegmente funktionsfähig mit dem Gen einer konstanten Maus-Immunglobulin-Schwerkettenregion verbunden sind; und
(c) eine ektopische Nukleinsäuresequenz, die ein Maus-ADAM6a-Protein oder ein funktionelles Fragment davon kodiert, und eine ektopische Nukleinsäuresequenz, die ein Maus-ADAM6b-Protein oder ein funktionelles Fragment davon kodiert, wobei das Maus-ADAM6a- und ADAM6b-Protein oder funktionelle Fragmente davon aus den ektopischen Nukleinsäuresequenzen exprimiert werden,
wobei die Maus einen Antikörper exprimiert, der (i) ein Polypeptid umfasst, das eine menschliche variable Leichtkettendomäne umfasst, die mit einer konstanten Maus-Schwerkettendomäne verbunden ist; und (ii) ein Polypeptid, das eine menschliche variable Leichtkettendomäne umfasst, die mit einer konstanten Maus-Leichtkettendomäne verbunden ist.

2. Maus nach Anspruch 1, wobei das Gen einer konstanten Maus-Immunglobulin-Leichtkettenregion ein Maus-Cκ ist.

3. Maus nach Anspruch 1 oder 2, wobei das eine oder die mehreren menschlichen V_{L}-Gensegmente und das eine oder die mehreren menschlichen J_{L}-Gensegmente stromaufwärts von dem Gen einer konstanten Maus-Immunglobulin-Leichtkettenregion ein oder mehrere menschliche Vκ-Gensegmente und ein oder mehrere menschliche Jκ-Gensegmente sind.

4. Maus nach einem der vorherigen Ansprüche, wobei das eine oder die mehreren menschlichen V_{L}-Gensegmente und das eine oder die mehreren menschlichen J_{L}-Gensegmente stromaufwärts von dem Gen einer konstanten Maus-Immunglobulin-Schwerkettenregion ein oder mehrere menschliche Vκ-Gensegmente und ein oder mehrere menschliche Jκ-Gensegmente sind.

5. Maus nach einem der Ansprüche 1-4, wobei die ektopischen Nukleinsäuresequenzen zwischen zwei von dem eingefügten einen oder den eingefügten mehreren menschlichen V_{L}-Gensegmenten und einem oder mehreren menschlichen J_{L}-Gensegmenten von (b) vorhanden sind.

6. Maus nach Anspruch 5, wobei das eingefügte eine oder die eingefügten mehreren menschlichen V_{L}-Gensegmente und das eine oder die mehreren menschlichen J_{L}-Gensegmente menschliche κ-Leichtkettengensegmente oder menschliche λ-Leichtkettengensegmente sind.

7. Maus nach einem der vorherigen Ansprüche, wobei das eingefügte eine oder die mehreren menschlichen V_{L}-Gensegmente Vκ5-2 und Vκ4-1 beinhalten und die ektopischen Nukleinsäuresequenzen, die das Maus-ADAM6a- und -ADAM6b-Protein oder funktionelle Fragmente davon kodieren, zwischen Vκ5-2 und Vκ4-1 vorhanden sind.

8. Maus nach einem der Ansprüche 1-6, wobei das eingefügte eine oder die eingefügten mehreren menschlichen V_{L}-Gensegmente Vκ4-1 und das eingefügte eine oder die eingefügten mehreren J_{L}-Gensegmente Jκ1 beinhalten, und die ektopischen Nukleinsäuresequenzen, die das Maus-ADAM6a- und -ADAM6b-Protein oder funktionelle Fragmente davon kodieren, zwischen Vκ4-1 und Jκ1 vorhanden sind.

9. Maus nach einem der vorherigen Ansprüche, wobei die Maus ein oder mehrere endogene V_{L}- und/oder ein oder mehrere endogene J_{L}-Gensegmente umfasst, die nicht in der Lage sind, sich umzulagern, um eine variable Immunglobulin-Leichtkettensequenz der in der Maus zu bilden.

10. Maus nach einem der vorherigen Ansprüche, wobei die Maus eine Deletion oder einen Ersatz von einer oder mehreren endogenen Immunglobulin-Schwerketten-Gensequenzen umfasst.

11. Zelle, die von der Maus nach Anspruch 1 abgeleitet ist.

12. Zelle nach Anspruch 11, wobei die Zelle eine B-Zelle ist.

13. Hybridom, die aus der B-Zelle nach Anspruch 12 ist.

14. Verfahren zum Herstellen eines Antikörpers, der an ein Antigen von Interesse bindet, wobei das Verfahren Folgendes umfasst
(a) Aussetzen einer Maus nach Anspruch 1 an ein Antigen von Interesse;
(b) Isolieren einer oder mehrerer B-Zellen der Maus, wobei die eine oder mehreren B-Zellen einen Antikörper exprimieren, der das Antigen von Interesse bindet;
(c) Identifizieren einer Nukleinsäuresequenz, die eine der variablen Leichtkettendomänen des Antikörpers kodiert, der dieses Antigen von Interesse bindet, wobei der Antikörper eine menschliche variable Immunglobulin-κ-Leichtkettendomäne, die mit einer konstanten Maus-Leichtkettendomäne verbunden ist, und eine menschliche variable Immunglobulin-κ-Leichtkettendomäne, die mit einer konstanten Maus-Schwerkettendomäne verbunden ist, umfasst; und
(d) Verwenden der Nukleinsäuresequenz von (c) mit einer Nukleinsäuresequenz einer menschlichen konstanten Immunglobulinregion, um einen menschlichen Antikörper herzustellen, der das Antigen von Interesse bindet.

## Revendications

1. Souris dont le génome comprend :
(a) une insertion d'au moins un segment du gène V_{L} humain et au moins un segment du gène J_{L} humain en amont d'un gène de région constante à chaîne légère de l'immunoglobuline de souris, l'au moins un segment du gène V_{L} humain et l'au moins un segment du gène J_{L} humain étant liés de manière fonctionnelle au gène de région constante à chaîne légère de l'immunoglobuline de souris ;
(b) une insertion d'au moins un segment du gène V_{L} humain et d'au moins un segment du gène J_{L} humain en amont d'un gène de région constante à chaîne lourde de l'immunoglobuline de souris, l'au moins un segment du gène V_{L} humain et l'au moins un segment du gène J_{L} humain étant liés de manière fonctionnelle au gène de région constante à chaîne lourde de l'immunoglobuline de souris ; et
(c) une séquence ectopique d'acides nucléiques qui code pour une protéine ADAM6a de souris ou un fragment fonctionnel de celle-ci et une séquence ectopique d'acides nucléiques qui code pour une protéine ADAM6b de souris ou un fragment fonctionnel de celle-ci, les protéines de ADAM6a et ADAM6b de souris ou des fragments fonctionnels de celles-ci étant exprimées à partir des séquences ectopiques d'acides nucléiques,
la souris exprimant un anticorps comprenant (i) un polypeptide qui comprend un domaine variable humain à chaîne légère associé à un domaine constant à chaîne lourde de souris ; et (ii) un polypeptide qui comprend un domaine variable humain à chaîne légère associé à un domaine constant à chaîne légère de souris.

2. Souris selon la revendication 1, dans lequel le gène de région constante à chaîne légère de l'immunoglobuline de souris est un Cκ de souris.

3. Souris selon la revendication 1 ou 2, dans laquelle l'au moins un segment du gène V_{L} humain et l'au moins un segment du gène J_{L} humain en amont du gène de région constante à chaîne légère de l'immunoglobuline de souris constitue au moins un segment du gène Vκ humain et au moins un segment du gène Jκ humain.

4. Souris selon l'une quelconque des revendications précédentes, dans laquelle l'au moins un segment du gène V_{L} humain et l'au moins un segment du gène J_{L} humain en amont du gène de région constante à chaîne lourde de l'immunoglobuline de souris constitue au moins un segment du gène Vκ humain et au moins un segment du gène Jκ humain.

5. Souris selon l'une quelconque des revendications 1 à 4, dans laquelle les séquences ectopiques d'acides nucléiques sont présentes entre deux de l'au moins un segment du gène V_{L} humain inséré et de l'au moins un segment du gène J_{L} humain inséré selon (b).

6. Souris selon la revendication 5, dans laquelle l'au moins un segment du gène V_{L} humain inséré et l'au moins un segment du gène J_{L} humain inséré sont des segments géniques humains à chaîne légère κ ou des segments géniques humains à chaîne légère λ.

7. Souris selon l'une quelconque des revendications précédentes, dans laquelle l'au moins un segment du gène V_{L} humain comprend Vκ5-2 et Vκ4-1 et les séquences ectopiques d'acides nucléiques qui codent pour les protéines ADAM6a et ADAM6b de souris ou des fragments fonctionnels de celles-ci sont présentes entre Vκ5-2 et Vκ4-1.

8. Souris selon l'une quelconque des revendications 1 à 6, dans laquelle l'au moins un segment du gène humain V_{L} inséré comprend Vκ4-1 et l'au moins un segment du gène J_{L} inséré comprend Jκ1 et les séquences ectopiques d'acides nucléiques qui codent pour les protéines ADAM6a et ADAM6b de souris ou des fragments fonctionnels de celles-ci sont présentes entre Vκ4-1 et Jκ1.

9. Souris selon l'une quelconque des revendications précédentes, la souris comprenant au moins un segment du gène endogène V_{L} et au moins un segment du gène endogène J_{L} qui ne sont pas capables de se réarranger pour former une séquence variable à chaîne légère d'immunoglobuline chez la souris.

10. Souris selon l'une quelconque des revendications précédentes, la souris comprenant la suppression ou le remplacement de séquences géniques à chaîne lourde de l'immunoglobuline endogène.

11. Cellule provenant de la souris selon la revendication 1.

12. Cellule selon la revendication 11, la cellule étant une cellule B.

13. Hybridome constitué de la cellule B selon la revendication 12.

14. Procédé de fabrication d'un anticorps qui se lie à un antigène d'intérêt, le procédé comprenant
(a) l'exposition d'une souris selon la revendication 1 à un antigène d'intérêt ;
(b) l'isolation d'au moins une cellule B de la souris, l'au moins une cellule B exprimant un anticorps qui se lie à l'antigène d'intérêt;
(c) l'identification d'une séquence d'acides nucléiques qui code pour un des domaines à chaîne variable légère de l'anticorps qui se lie à un antigène d'intérêt, l'anticorps comprenant un domaine variable à chaîne légère κ de l'immunoglobuline humaine associé à un domaine constant à chaîne légère de souris et un domaine variable à chaîne légère κ de l'immunoglobuline humaine associé à un domaine constant à chaîne lourde de souris ; et
(d) l'emploi de la séquence d'acides nucléiques selon (c) avec une séquence d'acides nucléiques de région constante de l'immunoglobuline humaine pour fabriquer un anticorps humain qui se lie à l'antigène d'intérêt.
